# EUROPEAN PATENT APPLICATION

(11) **EP 3 395 365 A1**
(43) Date of publication of application: **31.10.2018**
(21) Application number: 18169941.4
(22) Date of filing: 27.04.2018
(51) Int. Cl.: A61K 45/00, C07C 323/00

(54) **IMMUNOSTIMULATING AGENT**

(30) Priority: 28.04.2017 JP 2017090851
(71) Applicant: AJINOMOTO CO., INC., Chuo-ku Tokyo 104-8315 (JP)
(72) Inventor: MATSUMOTO, Kayo, Kawasaki-shi, Kanagawa 210-8681 (JP); KUROSAWA, Wataru, Kawasaki-shi, Kanagawa 210-8681 (JP); TANAKA, Kenji, Kawasaki-shi, Kanagawa 210-8681 (JP); SEKI, Takuya, Kawasaki-shi, Kanagawa 210-8681 (JP); UMISHIO, Hiroshi, Kawasaki-shi, Kanagawa 210-8681 (JP)
(74) Representative: Dunne, Paul David

(57) **Abstract**

The present invention aims to provide an immunostimulating agent superior in an immunostimulatory effect, particularly a compound useful as a vaccine adjuvant, a pharmaceutical composition containing the compound, a vaccine containing the compound and an antigen.

An immunostimulating agent containing at least one kind of a compound represented by the formula (I): 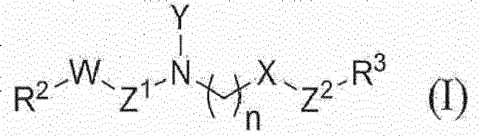 wherein each symbol is as defined in the present specification, or a salt thereof.

## Description

### Technical Field

The present invention relates to an immunostimulating agent superior in an immunostimulatory effect, particularly a compound useful as a vaccine adjuvant, a pharmaceutical composition containing the compound, a vaccine containing the compound and an antigen and the like.

### Background Art

Vaccine includes live vaccine wherein pathogen is attenuated, whole particle vaccine wherein pathogen is inactivated, and split vaccine wherein pathogen is decomposed and only a particular component is extracted and purified. Of these, split vaccine requires addition of a compound or composition called adjuvant to enhance immunostimulatory ability thereof. Also, it is said that mucosal vaccine, cancer vaccine and vaccine for a certain kind of allergy that are being increasingly researched and developed in recent years also require addition of an adjuvant for the expression of the effects thereof. Examples of the adjuvants approved inside the country at present include aluminum salt (aluminum hydroxide gel etc.) as precipitated adjuvant, squalane as oil adjuvant, MPL that is a variant of lipopolysaccharide LPS which is a constituent component of gram negative bacteria cell wall outer membrane intrinsically having immunogenicity. The research and development of adjuvant at the global level are also advancing taking note of nucleic acid derived from CpG and Poly (I:C) and the like, variants of bacteria constituent components that activate Toll-like receptor (TLR), variants of cytokines that stimulate immune system and the like. However, these existing adjuvants including those already approved inside the country and those under research and development have the following problems.

As for aluminum salt which is a precipitated adjuvant, the adjuvant effect is questioned in some vaccines such as influenza HA vaccine, foot-and-mouth disease vaccine and the like. Also from the safety aspect, aluminum salt is known to often show granulation in the inoculation site, cause hyperimmunoglobulinemia E and the like. As for oil adjuvant such as squalene and the like, inoculation may be sometimes painful since viscosity increases by emulsifying, and inoculation site sometimes indurates since it has property to resist dispersion in the body and stay at the inoculation site. On the other hand, since MPL is a variant of LPS having immunogenicity, simultaneous inoculation with vaccine sometimes initiates strong inflammatory reaction, and sometimes accompanies pain and fever. Furthermore, adjuvants under development are also held to have safety problems such as allergy induction, strong inflammation reaction, fever initiation and the like. As for nucleic acid adjuvant, new problems are surfacing such as problems in synthesizing as a pharmaceutical product, for example, difficulty in chemical synthesis up to a chain length considered to afford an effective adjuvant effect and the like. Although adjuvants are requested to simultaneously show effectiveness and safety, conventional adjuvants already approved inside the country and those under research and development fail to completely satisfy such request as the situation stands.

In the meantime, National Institute of Allergy and Infectious Diseases (NIAID) indicates the following 12 points regarding the safety of vaccine adjuvant. 1) free of induction of autoimmune response, 2) free of antigen having crossreactivity with human antigen, 3) free of induction of allergic hypersensitive reaction, 4) should be synthesized chemically pure, 5) free of carcinogenicity, 6) free of induction of response other than the object immune response, 7) should be a substance to be quickly metabolized in the body, 8) should be safe irrespective of inoculation method, 9) should be free of teratogenicity and reproductive toxicity, 10) should have preservation stability for at least one year, 11) should be selected for the object, 12) should tolerate side reaction developed at low frequency. Also, in the guideline of EMA (European Medicines Agency) which is an organization responsible for examination of vaccine in Europe, 1) histological damage and granuloma formation of inoculation spot, 2) hypersensitivity and anaphylaxis, 3) pyrogenicity, 4) systemic toxicity, 5) reproduction toxicity, 6) genotoxicity (synthetic adjuvant alone) are recited as non-clinical toxicity test of adjuvant alone. While some parts are common or not common between US and Europe, at least the vaccine adjuvants to be developed from now should satisfy these requests.

As amino acid analog compounds having an immunoregulating effect, for example, the compounds described in patent documents 1 - 4 (diacylcystine etc.) have been reported as acyl amino acids, and the compound described in patent document 5 (citrulline) has been reported as amino acid. However, these compounds are different from the compound represented by the formula (I) of the present invention.

### [Document List]

### [Patent documents]

patent document 1: WO 2015/163488
patent document 2: JP-A-4-230359
patent document 3: JP-A-59-219262
patent document 4: WO 01/059067
patent document 5: WO 2012/124631

### [SUMMARY OF THE INVENTION]

### [Problems to be Solved by the Invention]

An object of the present invention is to provide an immunostimulating agent superior in an immunostimulatory effect, particularly a compound useful as a vaccine adjuvant, a pharmaceutical composition containing the compound, and a vaccine containing the compound and an antigen.

### [Means of Solving the Problems]

The present inventors have conducted intensive studies in an attempt to solve the aforementioned problems and found that a compound represented by the following formula (I) (hereinafter sometimes to be referred to as compound (I)) and a salt thereof have a superior immunostimulatory effect that enables enhancement of antigen-specific IgG1 subclass antibody production. They have conducted further studies based on the finding and completed the present invention. Also, the present inventors have found that compound (I) and a salt thereof can enhance production of an antigen-specific IgG2a subclass antibody and can sometimes suppress induction of IgE antibody production.

Therefore, the present invention provides the following.
[1] An immunostimulating agent comprising at least one kind of a compound represented by the formula (I): 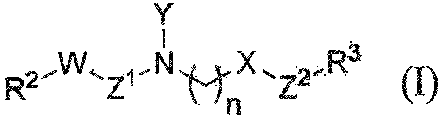 wherein
   R² is an optionally substituted C₁₋₂₄ hydrocarbon group;
   R³ is (a) a mono- or di-C₁₋₁₆ alkylamino group optionally substituted by a hydroxyl group or a C₁₋₄ alkoxy group, (b) a C₇₋₁₆ arylalkoxy group, (c) a hydrogen atom, (d) -C(=NH)R⁴ wherein R⁴ is an amino group, a C₁₋₁₆ alkyl-carbonylamino group or a C₁₋₄ alkyl group, or (e) a basic heterocyclic group;
   W is a bond, -O-, -NH- or -CO-;
   X is
      (i) a group represented by the following formula 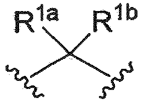 wherein
         R^{1a} is
         (1) a guanidyl-C₁₋₆ alkyl group,
         (2) a C₁₋₁₂ alkyl group,
         (3) an amino-C₁₋₆ alkyl group optionally substituted by an acetyl group or an aminocarbonyl group,
         (4) a group represented by the following formula 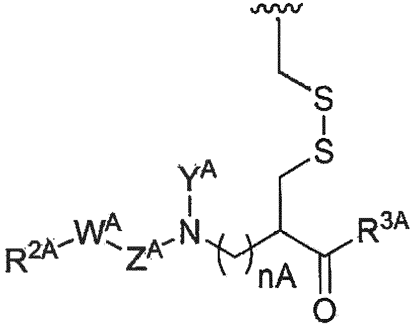 wherein
            R^{2a} is an optionally substituted C₁₋₂₄ hydrocarbon group;
            R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
            W^{A} is a bond, -O-, -NH- or -CO-;
            Y^{A} is a hydrogen atom, a C₁₋₁₀ alkyl group or a C₇₋₁₆ arylalkyl group;
            Z^{A} is -CO- or a bond;
            nA is an integer of 0 - 3 or
         (5) a hydrogen atom;
            R^{1b} is a C₁₋₄ alkyl group or a hydrogen atom;
            when R^{1a} is a methyl group, Y is a methyl group and n is 2, then R^{1a} and Y are optionally bonded to form a ring,
      (ii) -NR⁵- wherein R⁵ is a hydrogen atom or a methyl group, or
      (iii) a bond;
   Y is a hydrogen atom, a C₁₋₁₀ alkyl group or a C₇₋₁₆ arylalkyl group;
   Z¹ and Z² are the same or different and each is -CO- or a bond;
   n is an integer of 0 - 6;
   when X is -N(CH₃)-, Y is a methyl group and n is 2, then the methyl group for X and Y are optionally bonded to form a ring,
   provided that
   a case in which R² is a C₁₋₂₄ alkyl group, R³ is a mono- or di-C₁₋₆ alkylamino group, W is a bond, X is the aforementioned (i), R^{1a} is a 3-guanidylpropyl group, a methyl group, an isopropyl group, an isobutyl group, a sec-butyl group, a 4-aminobutyl group or a hydrogen atom, R^{1b} is a hydrogen atom, Y is a hydrogen atom, Z¹ and Z² are both -CO-, and n is 0, and
      a case in which R² is a C₁₋₂₄ alkyl group, R³ is -C(=NH)NH₂, W is a bond, X is -NH-, Y is a hydrogen atom, Z¹ is -CO-, Z² is a bond, and n is 4 are excluded,
   or a salt thereof.
[2] The immunostimulating agent of [1], wherein R² is an optionally substituted C₆₋₂₁ hydrocarbon group.
[3] The immunostimulating agent of [1] or [2], wherein R³ is (a) a mono- or di-C₁₋₁₆ alkylamino group optionally substituted by a hydroxyl group or a C₁₋₄ alkoxy group, (c) a hydrogen atom, or (d) -C(=NH)R⁴ wherein R⁴ is an amino group, a C₁₋₁₆ alkyl-carbonylamino group or a C₁₋₄ alkyl group.
[4] The immunostimulating agent of any of [1] to [3], wherein X is
   (i) a group represented by the following formula 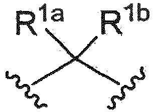 wherein
      R^{1a} is
      (1) a guanidyl-C₁₋₆ alkyl group,
      (2) a C₁₋₁₂ alkyl group,
      (3) an amino-C₁₋₆ alkyl group optionally substituted by an acetyl group or an aminocarbonyl group,
      (4) a group represented by the following formula 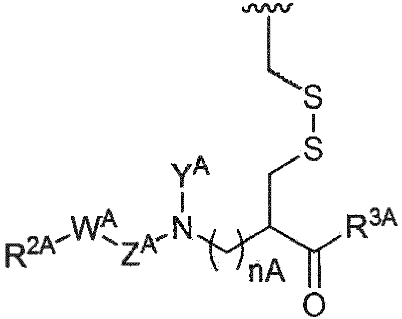 wherein
         R^{2A} is an optionally substituted C₁₋₂₄ hydrocarbon group;
         R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
         W^{A} is a bond, -O-, -NH- or -CO-;
         Y^{A} is a hydrogen atom, a C₁₋₁₀ alkyl group or a C₇₋₁₆ arylalkyl group;
         Z^{A} is -CO- or a bond;
         nA is an integer of 0 - 3, or
      (5) a hydrogen atom;
         R^{1b} is a C₁₋₄ alkyl group or a hydrogen atom;
         when R^{1a} is a methyl group, Y is a methyl group and n is 2, then R^{1a} and Y are optionally bonded to form a ring, or
   (ii) -NR⁵- wherein R⁵ is a hydrogen atom or a methyl group.
[5] The immunostimulating agent of any of [1] to [4], wherein the compound represented by the formula (I) or a salt thereof is selected from the group consisting of
   N-[(1S)-1-(dodecylcarbamoyl)-4-guanidino-butyl]hexadecanamide,
   N-[(1S)-1-(dodecylcarbamoyl)-4-guanidino-butyl]octadecanamide,
   N-[(1S)-1-(dodecylcarbamoyl)-4-guanidino-butyl]docosanamide,
   N-[(1S)-3-methyl-1-(undecylcarbamoyl)butyl]docosanamide,
   N-[(1S)-1-(dodecylcarbamoyl)-3-methyl-butyl]hexadecanamide,
   N-[(1S)-2-(dodecylamino)-1-methyl-2-oxo-ethyl]docosanamide,
   N-[2-(dodecylamino)-1,1-dimethyl-2-oxo-ethyl]docosanamide,
   N-[3-(dodecylamino)-3-oxo-propyl]docosanamide,
   N-[3-(dodecylamino)-2,2-dimethyl-3-oxo-propyl]docosanamide,
   (2S)-N-dodecyl-5-guanidino-2-(hexadecylcarbamoylamino)pentanamide,
   Hexadecyl N-[(1S)-1-(dodecylcarbamoyl)-4-guanidino-butyl]carbamate,
   N-[(1S)-5-amino-1-(dodecylcarbamoyl)pentyl]hexadecanamide,
   N-[(1S)-1-(dodecylcarbamoyl)pentyl]docosanamide,
   1-docosanoyl-N-dodecyl-piperidine-4-carboxamide,
   N-[(1S)-1-(diheptylcarbamoyl)-4-guanidino-butyl]docosanamide,
   N-[(1S)-5-amino-1-(dodecylcarbamoyl)pentyl]docosanamide,
   (E)-N-[(1S)-1-(dodecylcarbamoyl)-4-guanidino-butyl]octadeca-9-enamide,
   N-[(1S)-4-guanidino-1-(heptylcarbamoyl)butyl]docosanamide,
   N-[3-(dodecylamino)-3-oxo-pentyl]docosanamide,
   N-[(1S)-4-amino-1-(dodecylcarbamoyl)butyl]docosanamide,
   N-[(1S)-1-(dodecylcarbamoyl)-5-guanidino-pentyl]docosanamide,
   N-[(1S)-5-acetamide-1-(dodecylcarbamoyl)pentyl]docosanamide,
   (2S)-5-guanidino-2-(hexadecylcarbamoylamino)-N-hexyl-pentanamide,
   Hexadecyl N-[(1S)-4-guanidino-1-(hexylcarbamoyl)butyl]carbamate,
   N-[(1S)-5-guanidino-1-(hexylcarbamoyl)pentyl]docosanamide,
   N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide,
   (2S)-2-(dibenzylamino)-N-dodecyl-5-guanidino-pentanamide,
   N-(5-guanidinopentyl)hexadecanamide,
   N-(5-guanidinopentyl)docosanamide,
   N-[(1S)-1-(dodecylcarbamoyl)-4-guanidino-butyl]-2-oxo-octanamide,
   Hexadecyl N-(4-guanidinobutyl)carbamate,
   N-[4-(ethanimidoylamino)butyl]hexadecanamide,
   N-[(1S)-1-(dodecylcarbamoyl)-4-guanidino-butyl]icosanamide,
   N-[(1S)-4-guanidino-1-(octylcarbamoyl)butyl]hexadecanamide,
   N-[(1S)-4-guanidino-1-(octylcarbamoyl)butyl]octadecanamide,
   N-[(1S)-4-guanidino-1-(octylcarbamoyl)butyl]icosanamide,
   N-[(1S)-5-amino-1-(octylcarbamoyl)pentyl]octadecanamide,
   N-[(1S)-5-amino-1-(dodecylcarbamoyl)pentyl]octadecanamide,
   Octadecyl N-[(1S)-1-(dodecylcarbamoyl)-4-guanidino-butyl]carbamate,
   Dodecyl N-[(1S)-1-(dodecylcarbamoyl)-4-guanidino-butyl]carbamate,
   Hexadecyl N-[(1S)-5-amino-1-(octylcarbamoyl)pentyl]carbamate,
   Hexadecyl N-[(1S)-5-amino-1-(dodecylcarbamoyl)pentyl]carbamate,
   1-(4-guanidinobutyl)-3-hexadecyl-urea,
   N-[(1R)-1-(dodecylcarbamoyl)-4-guanidino-butyl]octadecanamide,
   (2R)-N-dodecyl-5-guanidino-2-(hexadecylcarbamoylamino)pentanamide,
   N-[(1R)-5-amino-1-(dodecylcarbamoyl)pentyl]hexadecanamide,
   N-[(1S)-1-(dodecylcarbamoyl)-3-methyl-butyl]octadecanamide,
   N-[(1S)-3-methyl-1-(octylcarbamoyl)butyl]octadecanamide,
   N-[(1S)-1-(dodecylcarbamoyl)-3-methyl-butyl]docosanamide,
   N-[(1S)-3-methyl-1-(octylcarbamoyl)butyl]docosanamide,
   N-[(1S)-3-methyl-1-(octylcarbamoyl)butyl]hexadecanamide,
   (2S)-N-dodecyl-2-(hexadecylcarbamoylamino)-4-methylpentanamide,
   (2S)-2-(hexadecylcarbamoylamino)-4-methyl-N-octyl-pentanamide,
   Hexadecyl N-[(1S)-1-(dodecylcarbamoyl)-3-methyl-butyl]carbamate,
   Hexadecyl N-[(1S)-3-methyl-1-(octylcarbamoyl)butyl]carbamate,
   N-[3-(dodecylamino)-2,2-dimethyl-3-oxo-propyl]octadecanamide,
   N-[(1S)-1-(dodecylcarbamoyl)-5-guanidino-pentyl]octadecanamide,
   N-[(1S)-1-(hexylcarbamoyl)-5-guanidino-pentyl]octadecanamide,
   (2S)-2-(diheptylamino)-N-dodecyl-5-guanidino-pentanamide,
   N-[2-(4-pyridyl)ethyl]docosanamide,
   (2S)-5-guanidino-2-(hexadecylcarbamoylamino)-N-octyl-pentanamide,
   (2S)-N-dodecyl-5-guanidino-2-(octadecylcarbamoylamino)pentanamide,
   (2S)-N-dodecyl-2-(dodecylcarbamoylamino)-5-guanidino-pentanamide,
   (2S)-6-amino-2-(hexadecylcarbamoylamino)-N-octyl-hexanamide,
   (2S)-6-amino-N-dodecyl-2-(hexadecylcarbamoylamino)hexanamide,
   Hexadecyl N-[(1S)-4-guanidino-1-(octylcarbamoyl)butyl]carbamate,
   and salts thereof.
[5-1] The immunostimulating agent of any of [1] to [4], wherein the compound represented by the formula (I) or a salt thereof is selected from the group consisting of
   N-[(1S)-1-(dodecylcarbamoyl)-4-guanidino-butyl]hexadecanamide,
   N-[(1S)-1-(dodecylcarbamoyl)-4-guanidino-butyl]octadecanamide,
   N-[(1S)-1-(dodecylcarbamoyl)-4-guanidino-butyl]docosanamide,
   N-[(1S)-3-methyl-1-(undecylcarbamoyl)butyl]docosanamide,
   N-[(1S)-1-(dodecylcarbamoyl)-3-methyl-butyl]hexadecanamide,
   N-[(1S)-2-(dodecylamino)-1-methyl-2-oxo-ethyl]docosanamide,
   N-[2-(dodecylamino)-1,1-dimethyl-2-oxo-ethyl]docosanamide,
   N-[3-(dodecylamino)-3-oxo-propyl]docosanamide,
   N-[3-(dodecylamino)-2,2-dimethyl-3-oxo-propyl]docosanamide,
   (2S)-N-dodecyl-5-guanidino-2-(hexadecylcarbamoylamino)pentanamide,
   Hexadecyl N-[(1S)-1-(dodecylcarbamoyl)-4-guanidino-butyl]carbamate,
   N-[(1S)-5-amino-1-(dodecylcarbamoyl)pentyl]hexadecanamide,
   N-[(1S)-1-(dodecylcarbamoyl)pentyl]docosanamide,
   1-docosanoyl-N-dodecyl-piperidine-4-carboxamide,
   N-[(1S)-1-(diheptylcarbamoyl)-4-guanidino-butyl]docosanamide,
   N-[(1S)-5-amino-1-(dodecylcarbamoyl)pentyl]docosanamide,
   (E)-N-[(1S)-1-(dodecylcarbamoyl)-4-guanidino-butyl]octadeca-9-enamide,
   N-[(1S)-4-guanidino-1-(heptylcarbamoyl)butyl]docosanamide,
   N-[3-(dodecylamino)-3-oxo-pentyl]docosanamide,
   N-[(1S)-4-amino-1-(dodecylcarbamoyl)butyl]docosanamide,
   N-[(1S)-1-(dodecylcarbamoyl)-5-guanidino-pentyl]docosanamide,
   N-[(1S)-5-acetamide-1-(dodecylcarbamoyl)pentyl]docosanamide,
   (2S)-5-guanidino-2-(hexadecylcarbamoylamino)-N-hexyl-pentanamide,
   Hexadecyl N-[(1S)-4-guanidino-1-(hexylcarbamoyl)butyl]carbamate,
   N-[(1S)-5-guanidino-1-(hexylcarbamoyl)pentyl]docosanamide,
   N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide,
   (2S)-2-(dibenzylamino)-N-dodecyl-5-guanidino-pentanamide,
   N-(5-guanidinopentyl)hexadecanamide,
   N-(5-guanidinopentyl)docosanamide,
   N-[(1S)-1-(dodecylcarbamoyl)-4-guanidino-butyl]-2-oxo-octanamide,
   Hexadecyl N-(4-guanidinobutyl)carbamate,
   N-[4-(ethanimidoylamino)butyl]hexadecanamide,
   and salts thereof.
[6] The immunostimulating agent of any of [1] to [5], wherein the aforementioned immunostimulating agent is a vaccine adjuvant.
[7] A pharmaceutical composition comprising at least one kind of a compound represented by the formula (I): 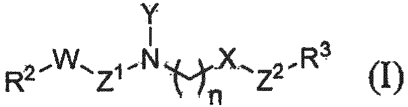 wherein
   R² is an optionally substituted C₁₋₂₄ hydrocarbon group;
   R³ is (a) a mono- or di-C₁₋₁₆ alkylamino group optionally substituted by a hydroxyl group or a C₁₋₄ alkoxy group, (b) a C₇₋₁₆ arylalkoxy group, (c) a hydrogen atom, (d) -C(=NH)R⁴ wherein R⁴ is an amino group, a C₁₋₁₆ alkyl-carbonylamino group or a C₁₋₄ alkyl group, or (e) a basic heterocyclic group;
   W is a bond, -O-, -NH- or -CO-;
   X is
      (i) a group represented by the following formula 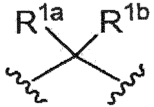 wherein
         R^{1a} is
         (1) a guanidyl-C₁₋₆ alkyl group,
         (2) a C₁₋₁₂ alkyl group,
         (3) an amino-C₁₋₆ alkyl group optionally substituted by an acetyl group or an aminocarbonyl group,
         (4) a group represented by the following formula 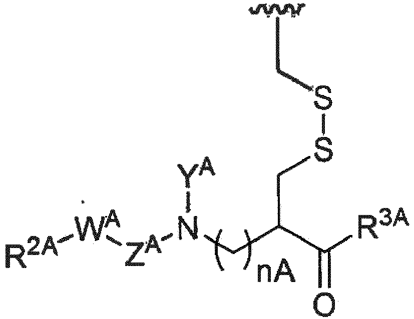 wherein
            R^{2A} is an optionally substituted C₁₋₂₄ hydrocarbon group;
            R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
            W^{A} is a bond, -O-, -NH- or -CO-;
            Y^{A} is a hydrogen atom, a C₁₋₁₀ alkyl group or a C₇₋₁₆ arylalkyl group;
            Z^{A} is -CO- or a bond;
            nA is an integer of 0 - 3, or
         (5) a hydrogen atom;
            R^{1b} is a C₁₋₄ alkyl group or a hydrogen atom;
            when R^{1a} is a methyl group, Y is a methyl group and n is 2, then R^{1a} and Y are optionally bonded to form a ring,
      (ii) -NR⁵- wherein R⁵ is a hydrogen atom or a methyl group, or
      (iii) a bond;
   Y is a hydrogen atom, a C₁₋₁₀ alkyl group or a C₇₋₁₆ arylalkyl group;
   Z¹ and Z² are the same or different and each is -CO- or a bond;
   n is an integer of 0 - 6;
   when X is -N(CH₃)-, Y is a methyl group and n is 2, then the methyl group for X and Y are optionally bonded to form a ring,
   provided that
   a case in which R² is a C₁₋₂₄ alkyl group, R³ is a mono- or di-C₁₋₆ alkylamino group, W is a bond, X is the aforementioned (i), R^{1a} is a 3-guanidylpropyl group, a methyl group, an isopropyl group, an isobutyl group, a sec-butyl group, a 4-aminobutyl group or a hydrogen atom, R^{1b} is a hydrogen atom, Y is a hydrogen atom, Z¹ and Z² are both -CO-, and n is 0, and
   a case in which R² is a C₁₋₂₄ alkyl group, R³ is -C(=NH)NH₂, W is a bond, X is -NH-, Y is a hydrogen atom, Z¹ is -CO-, Z² is a bond and n is 4 are excluded,
   or a salt thereof, and α-cyclodextrin.
[8] The pharmaceutical composition of [7], wherein R² is an optionally substituted C₆₋₂₁ hydrocarbon group.
[9] The pharmaceutical composition of [7] or [8], wherein
   R³ is (a) a mono- or di-C₁₋₁₆ alkylamino group optionally substituted by a hydroxyl group or a C₁₋₄ alkoxy group, (c) a hydrogen atom, or (d) -C(=NH)R⁴ wherein R⁴ is an amino group, a C₁₋₁₆ alkyl-carbonylamino group or a C₁₋₄ alkyl group.
[10] The pharmaceutical composition of any of [7] to [9], wherein
   X is
   (i) a group represented by the following formula 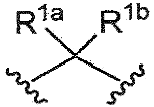 wherein
      R^{1a} is
      (1) a guanidyl-C₁₋₆ alkyl group,
      (2) a C₁₋₁₂ alkyl group,
      (3) an amino-C₁₋₆ alkyl group optionally substituted by an acetyl group or an aminocarbonyl group,
      (4) a group represented by the following formula 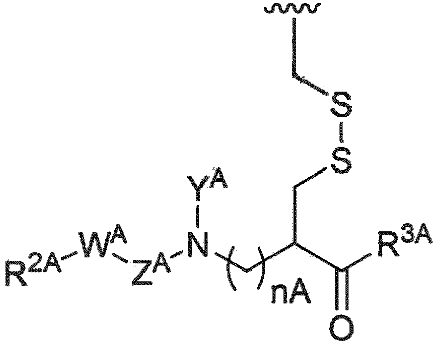 wherein
         R^{2A} is an optionally substituted C₁₋₂₄ hydrocarbon group;
         R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
         W^{A} is a bond, -O-, -NH- or -CO-;
         Y^{A} is a hydrogen atom, a C₁₋₁₀ alkyl group or a C₇₋₁₆ arylalkyl group;
         Z^{A} is -CO- or a bond;
         nA is an integer of 0 - 3, or
      (5) a hydrogen atom;
         R^{1b} is a C₁₋₄ alkyl group or a hydrogen atom;
         when R^{1a} is a methyl group, Y is a methyl group and n is 2, then R^{1a} and Y are optionally bonded to form a ring, or
   (ii) -NR⁵- wherein R⁵ is a hydrogen atom or a methyl group.
[11] The pharmaceutical composition of any of [7] to [10], wherein the compound represented by the formula (I) or a salt thereof is selected from the group consisting of
   N-[(1S)-1-(dodecylcarbamoyl)-4-guanidino-butyl]hexadecanamide,
   N-[(1S)-1-(dodecylcarbamoyl)-4-guanidino-butyl]octadecanamide,
   N-[(1S)-1-(dodecylcarbamoyl)-4-guanidino-butyl]docosanamide,
   N-[(1S)-3-methyl-1-(undecylcarbamoyl)butyl]docosanamide,
   N-[(1S)-1-(dodecylcarbamoyl)-3-methyl-butyl]hexadecanamide,
   N-[(1S)-2-(dodecylamino)-1-methyl-2-oxo-ethyl]docosanamide,
   N-[2-(dodecylamino)-1,1-dimethyl-2-oxo-ethyl]docosanamide,
   N-[3-(dodecylamino)-3-oxo-propyl]docosanamide,
   N-[3-(dodecylamino)-2,2-dimethyl-3-oxo-propyl]docosanamide,
   (2S)-N-dodecyl-5-guanidino-2-(hexadecylcarbamoylamino)pentanamide,
   Hexadecyl N-[(1S)-1-(dodecylcarbamoyl)-4-guanidino-butyl]carbamate,
   N-[(1S)-5-amino-1-(dodecylcarbamoyl)pentyl]hexadecanamide,
   N-[(1S)-1-(dodecylcarbamoyl)pentyl]docosanamide,
   1-docosanoyl-N-dodecyl-piperidine-4-carboxamide,
   N-[(1S)-1-(diheptylcarbamoyl)-4-guanidino-butyl]docosanamide,
   N-[(1S)-5-amino-1-(dodecylcarbamoyl)pentyl]docosanamide,
   (E)-N-[(1S)-1-(dodecylcarbamoyl)-4-guanidino-butyl]octadeca-9-enamide,
   N-[(1S)-4-guanidino-1-(heptylcarbamoyl)butyl]docosanamide,
   N-[3-(dodecylamino)-3-oxo-pentyl]docosanamide,
   N-[(1S)-4-amino-1-(dodecylcarbamoyl)butyl]docosanamide,
   N-[(1S)-1-(dodecylcarbamoyl)-5-guanidino-pentyl]docosanamide,
   N-[(1S)-5-acetamide-1-(dodecylcarbamoyl)pentyl]docosanamide,
   (2S)-5-guanidino-2-(hexadecylcarbamoylamino)-N-hexyl-pentanamide,
   Hexadecyl N-[(1S)-4-guanidino-1-(hexylcarbamoyl)butyl]carbamate,
   N-[(1S)-5-guanidino-1-(hexylcarbamoyl)pentyl]docosanamide,
   N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide,
   (2S)-2-(dibenzylamino)-N-dodecyl-5-guanidino-pentanamide,
   N-(5-guanidinopentyl)hexadecanamide,
   N-(5-guanidinopentyl)docosanamide,
   N-[(1S)-1-(dodecylcarbamoyl)-4-guanidino-butyl]-2-oxo-octanamide,
   Hexadecyl N-(4-guanidinobutyl)carbamate,
   N-[4-(ethanimidoylamino)butyl]hexadecanamide,
   N-[(1S)-1-(dodecylcarbamoyl)-4-guanidino-butyl]icosanamide,
   N-[(1S)-4-guanidino-1-(octylcarbamoyl)butyl]hexadecanamide,
   N-1(1S)-4-guanidino-1-(octylcarbamoyl)butyl]octadecanamide,
   N-[(1S)-4-guanidino-1-(octylcarbamoyl)butyl]icosanamide,
   N-[(1S)-5-amino-1-(octylcarbamoyl)pentyl]octadecanamide,
   N-[(1S)-5-amino-1-(dodecylcarbamoyl)pentyl]octadecanamide,
   Octadecyl N-[(1S)-1-(dodecylcarbamoyl)-4-guanidino-butyl]carbamate,
   Dodecyl N-[(1S)-1-(dodecylcarbamoyl)-4-guanidino-butyl]carbamate,
   Hexadecyl N-[(1S)-5-amino-1-(octylcarbamoyl)pentyl]carbamate,
   Hexadecyl N-[(1S)-5-amino-1-(dodecylcarbamoyl)pentyl]carbamate,
   1-(4-guanidinobutyl)-3-hexadecyl-urea,
   N-[(1R)-1-(dodecylcarbamoyl)-4-guanidino-butyl]octadecanamide,
   (2R)-N-dodecyl-5-guanidino-2-(hexadecylcarbamoylamino)pentanamide,
   N-[(1R)-5-amino-1-(dodecylcarbamoyl)pentyl]hexadecanamide,
   N-[(1S)-1-(dodecylcarbamoyl)-3-methyl-butyl]octadecanamide,
   N-[(1S)-3-methyl-1-(octylcarbamoyl)butyl]octadecanamide,
   N-[(1S)-1-(dodecylcarbamoyl)-3-methyl-butyl]docosanamide,
   N-[(1S)-3-methyl-1-(octylcarbamoyl)butyl]docosanamide,
   N-[(1S)-3-methyl-1-(octylcarbamoyl)butyl]hexadecanamide,
   (2S)-N-dodecyl-2-(hexadecylcarbamoylamino)-4-methyl-pentanamide,
   (2S)-2-(hexadecylcarbamoylamino)-4-methyl-N-octyl-pentanamide,
   Hexadecyl N-[(1S)-1-(dodecylcarbamoyl)-3-methyl-butyl]carbamate,
   Hexadecyl N-[(1S)-3-methyl-1-(octylcarbamoyl)butyl]carbamate,
   N-[3-(dodecylamino)-2,2-dimethyl-3-oxo-propyl]octadecanamide,
   N-[(1S)-1-(dodecylcarbamoyl)-5-guanidino-pentyl]octadecanamide,
   N-[(1S)-1-(hexylcarbamoyl)-5-guanidino-pentyl]octadecanamide,
   (2S)-2-(diheptylamino)-N-dodecyl-5-guanidino-pentanamide,
   N-[2-(4-pyridyl)ethyl]docosanamide,
   (2S)-5-guanidino-2-(hexadecylcarbamoylamino)-N-octyl-pentanamide,
   (2S)-N-dodecyl-5-guanidino-2-(octadecylcarbamoylamino)pentanamide,
   (2S)-N-dodecyl-2-(dodecylcarbamoylamino)-5-guanidino-pentanamide,
   (2S)-6-amino-2-(hexadecylcarbamoylamino)-N-octyl-hexanamide,
   (2S)-6-amino-N-dodecyl-2-(hexadecylcarbamoylamino)hexanamide,
   Hexadecyl N-[(1S)-4-guanidino-1-(octylcarbamoyl)butyl]carbamate,
   and salts thereof.
[11-1] The pharmaceutical composition of any of [7] to [10], wherein the compound represented by the formula (I) or a salt thereof is selected from the group consisting of
   N-[(1S)-1-(dodecylcarbamoyl)-4-guanidino-butyl]hexadecanamide,
   N-[(1S)-1-(dodecylcarbamoyl)-4-guanidino-butyl]octadecanamide,
   N-[(1S)-1-(dodecylcarbamoyl)-4-guanidino-butyl]docosanamide,
   N-[(1S)-3-methyl-1-(undecylcarbamoyl)butyl]docosanamide,
   N-[(1S)-1-(dodecylcarbamoyl)-3-methyl-butyl]hexadecanamide,
   N-[(1S)-2-(dodecylamino)-1-methyl-2-oxo-ethyl]docosanamide,
   N-[2-(dodecylamino)-1,1-dimethyl-2-oxo-ethyl]docosanamide,
   N-[3-(dodecylamino)-3-oxo-propyl]docosanamide,
   N-[3-(dodecylamino)-2,2-dimethyl-3-oxo-propyl]docosanamide,
   (2S)-N-dodecyl-5-guanidino-2-(hexadecylcarbamoylamino)pentanamide,
   Hexadecyl N-[(1S)-1-(dodecylcarbamoyl)-4-guanidino-butyl]carbamate,
   N-[(1S)-5-amino-1-(dodecylcarbamoyl)pentyl]hexadecanamide,
   N-[(1S)-1-(dodecylcarbamoyl)pentyl]docosanamide,
   1-docosanoyl-N-dodecyl-piperidine-4-carboxamide,
   N-[(1S)-1-(diheptylcarbamoyl)-4-guanidino-butyl]docosanamide,
   N-[(1S)-5-amino-1-(dodecylcarbamoyl)pentyl]docosanamide,
   (E)-N-[(1S)-1-(dodecylcarbamoyl)-4-guanidino-butyl]octadeca-9-enamide,
   N-[(1S)-4-guanidino-1-(heptylcarbamoyl)butyl]docosanamide,
   N-[3-(dodecylamino)-3-oxo-pentyl]docosanamide,
   N-[(1S)-4-amino-1-(dodecylcarbamoyl)butyl]docosanamide,
   N-[(1S)-1-(dodecylcarbamoyl)-5-guanidino-pentyl]docosanamide,
   N-[(1S)-5-acetamide-1-(dodecylcarbamoyl)pentyl]docosanamide,
   (2S)-5-guanidino-2-(hexadecylcarbamoylamino)-N-hexyl-pentanamide,
   Hexadecyl N-[(1S)-4-guanidino-1-(hexylcarbamoyl)butyl]carbamate,
   N-[(1S)-5-guanidino-1-(hexylcarbamoyl)pentyl]docosanamide,
   N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide,
   (2S)-2-(dibenzylamino)-N-dodecyl-5-guanidino-pentanamide,
   N-(5-guanidinopentyl)hexadecanamide,
   N-(5-guanidinopentyl)docosanamide,
   N-[(1S)-1-(dodecylcarbamoyl)-4-guanidino-butyl]-2-oxo-octanamide,
   Hexadecyl N-(4-guanidinobutyl)carbamate,
   N-[4-(ethanimidoylamino)butyl]hexadecanamide,
   and salts thereof.
[12] A vaccine comprising at least one kind of a compound represented by the formula (I): 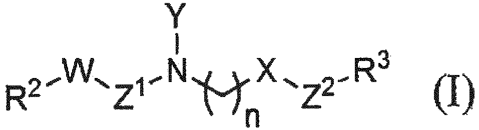 wherein
   R² is an optionally substituted C₁₋₂₄ hydrocarbon group;
   R³ is (a) a mono- or di-C₁₋₁₆ alkylamino group optionally substituted by a hydroxyl group or a C₁₋₄ alkoxy group, (b) a C₇₋₁₆ arylalkoxy group, (c) a hydrogen atom, (d) -C(=NH)R⁴ wherein R⁴ is an amino group, a C₁₋₁₆ alkyl-carbonylamino group or a C₁₋₄ alkyl group, or (e) a basic heterocyclic group;
   W is a bond, -O-, -NH- or -CO-;
   X is
      (i) a group represented by the following formula 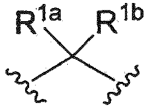 wherein
         R^{1a} is
         (1) a guanidyl-C₁₋₆ alkyl group,
         (2) a C₁₋₁₂ alkyl group,
         (3) an amino-C₁₋₆ alkyl group optionally substituted by an acetyl group or an aminocarbonyl group,
         (4) a group represented by the following formula 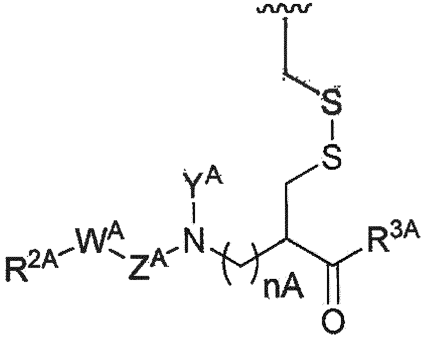 wherein
            R^{2A} is an optionally substituted C₁₋₂₄ hydrocarbon group;
            R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
            W^{A} is a bond, -O-, -NH- or -CO-;
            Y^{A} is a hydrogen atom, a C₁₋₁₀ alkyl group or a C₇₋₁₆ arylalkyl group;
            Z^{A} is -CO- or a bond;
            nA is an integer of 0 - 3, or
         (5) a hydrogen atom;
            R^{1b} is a C₁₋₄ alkyl group or a hydrogen atom;
            when R^{1a} is a methyl group, Y is a methyl group and n is 2, R^{1a} and Y are optionally bonded to form a ring,
      (ii) -NR⁵- wherein R⁵ is a hydrogen atom or a methyl group, or
      (iii) a bond;
   Y is a hydrogen atom, a C₁₋₁₀ alkyl group or a C₇₋₁₆ arylalkyl group;
   Z¹ and Z² are the same or different and each is -CO- or a bond;
   n is an integer of 0 - 6;
   when X is -N(CH₃)-, Y is a methyl group and n is 2, then the methyl group for X and Y are optionally bonded to form a ring,
   provided that
   a case in which R² is a C₁₋₂₄ alkyl group, R³ is a mono- or di-C₁₋₆ alkylamino group, W is a bond, X is the aforementioned (i), R^{1a} is a 3-guanidylpropyl group, a methyl group, an isopropyl group, an isobutyl group, a sec-butyl group, a 4-aminobutyl group or a hydrogen atom, R^{1b} is a hydrogen atom, Y is a hydrogen atom, Z¹ and Z² are both -CO-, and n is 0, and
   a case in which R² is a C₁₋₂₄ alkyl group, R³ is -C (=NH) NH₂, W is a bond, X is -NH-, Y is a hydrogen atom, Z¹ is -CO-, Z² is a bond, and n is 4 are excluded,
   or a salt thereof, and an antigen.
[13] The vaccine of [12], wherein R² is an optionally substituted C₆₋₂₁ hydrocarbon group.
[14] The vaccine of [12] or [13], wherein R³ is (a) a mono- or di-C₁₋₁₆ alkylamino group optionally substituted by a hydroxyl group or a C₁₋₄ alkoxy group, (c) a hydrogen atom, or (d)-C(=NH)R⁴ wherein R⁴ is an amino group, a C₁₋₁₆ alkyl-carbonylamino group or a C₁₋₄ alkyl group.
[15] The vaccine of any of [12] to [14], wherein
   X is
   (i) a group represented by the following formula 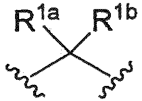 wherein
      R^{1a} is
      (1) a guanidyl-C₁₋₆ alkyl group,
      (2) a C₁₋₁₂ alkyl group,
      (3) an amino-C₁₋₆ alkyl group optionally substituted by an acetyl group or an aminocarbonyl group,
      (4) a group represented by the following formula 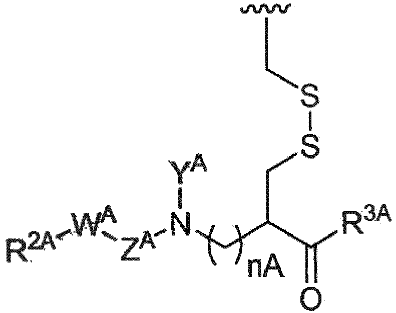 wherein
         R^{2A} is an optionally substituted C₁₋₂₄ hydrocarbon group;
         R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
         W^{A} is a bond, -O-, -NH- or -CO-;
         Y^{A} is a hydrogen atom, a C₁₋₁₀ alkyl group or a C₇₋₁₆ arylalkyl group;
         Z^{A} is -CO- or a bond;
         nA is an integer of 0 - 3, or
      (5) a hydrogen atom;
         R^{1b} is a C₁₋₄ alkyl group or a hydrogen atom;
         when R^{1a} is a methyl group, Y is a methyl group and n is 2, then R^{1a} and Y are optionally bonded to form a ring, or
   (ii) -NR⁵- wherein R⁵ is a hydrogen atom or a methyl group.
[16] The vaccine of any of [12] to [15], wherein the compound represented by the formula (I) or a salt thereof is selected from the group consisting of
   N-[(1S)-1-(dodecylcarbamoyl)-4-guanidino-butyl]hexadecanamide,
   N-[(1S)-1-(dodecylcarbamoyl)-4-guanidino-butyl]octadecanamide,
   N-[(1S)-1-(dodecylcarbamoyl)-4-guanidino-butyl]docosanamide,
   N-[(1S)-3-methyl-1-(undecylcarbamoyl)butyl]docosanamide,
   N-[(1S)-1-(dodecylcarbamoyl)-3-methyl-butyl]hexadecanamide,
   N-[(1S)-2-(dodecylamino)-1-methyl-2-oxo-ethyl]docosanamide,
   N-[2-(dodecylamino)-1,1-dimethyl-2-oxo-ethyl]docosanamide,
   N-[3-(dodecylamino)-3-oxo-propyl]docosanamide,
   N-[3-(dodecylamino)-2,2-dimethyl-3-oxo-propyl]docosanamide,
   (2S)-N-dodecyl-5-guanidino-2-(hexadecylcarbamoylamino)pentanamide,
   Hexadecyl N-[(1S)-1-(dodecylcarbamoyl)-4-guanidino-butyl]carbamate,
   N-[(1S)-5-amino-1-(dodecylcarbamoyl)pentyl]hexadecanamide,
   N-[(1S)-1-(dodecylcarbamoyl)pentyl]docosanamide,
   1-docosanoyl-N-dodecyl-piperidine-4-carboxamide,
   N-[(1S)-1-(diheptylcarbamoyl)-4-guanidino-butyl]docosanamide,
   N-[(1S)-5-amino-1-(dodecylcarbamoyl)pentyl]docosanamide,
   (E)-N-[(1S)-1-(dodecylcarbamoyl)-4-guanidino-butyl]octadeca-9-enamide,
   N-[(1S)-4-guanidino-1-(heptylcarbamoyl)butyl]docosanamide,
   N-[3-(dodecylamino)-3-oxo-pentyl]docosanamide,
   N-[(1S)-4-amino-1-(dodecylcarbamoyl)butyl]docosanamide,
   N-[(1S)-1-(dodecylcarbamoyl)-5-guanidino-pentyl]docosanamide,
   N-[(1S)-5-acetamide-1-(dodecylcarbamoyl)pentyl]docosanamide,
   (2S)-5-guanidino-2-(hexadecylcarbamoylamino)-N-hexyl-pentanamide,
   Hexadecyl N-[(1S)-4-guanidino-1-(hexylcarbamoyl)butyl]carbamate,
   N-[(1S)-5-guanidino-1-(hexylcarbamoyl)pentyl]docosanamide,
   N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide,
   (2S)-2-(dibenzylamino)-N-dodecyl-5-guanidino-pentanamide,
   N-(5-guanidinopentyl)hexadecanamide,
   N-(5-guanidinopentyl)docosanamide,
   N-[(1S)-1-(dodecylcarbamoyl)-4-guanidino-butyl]-2-oxo-octanamide,
   Hexadecyl N-(4-guanidinobutyl)carbamate,
   N-[4-(ethanimidoylamino)butyl]hexadecanamide,
   N-[(1S)-1-(dodecylcarbamoyl)-4-guanidino-butyl]icosanamide,
   N-[(1S)-4-guanidino-1-(octylcarbamoyl)butyl]hexadecanamide,
   N-[(1S)-4-guanidino-1-(octylcarbamoyl)butyl]octadecanamide,
   N-[(1S)-4-guanidino-1-(octylcarbamoyl)butyl]icosanamide,
   N-[(1S)-5-amino-1-(octylcarbamoyl)pentyl]octadecanamide,
   N-[(1S)-5-amino-1-(dodecylcarbamoyl)pentyl]octadecanamide,
   Octadecyl N-[(1S)-1-(dodecylcarbamoyl)-4-guanidino-butyl]carbamate,
   Dodecyl N-[(1S)-1-(dodecylcarbamoyl)-4-guanidino-butyl]carbamate,
   Hexadecyl N-[(1S)-5-amino-1-(octylcarbamoyl)pentyl]carbamate,
   Hexadecyl N-[(1S)-5-amino-1-(dodecylcarbamoyl)pentyl]carbamate,
   1-(4-guanidinobutyl)-3-hexadecyl-urea,
   N-[(1R)-1-(dodecylcarbamoyl)-4-guanidino-butyl]octadecanamide,
   (2R)-N-dodecyl-5-guanidino-2-(hexadecylcarbamoylamino)pentanamide,
   N-[(1R)-5-amino-1-(dodecylcarbamoyl)pentyl]hexadecanamide,
   N-[(1S)-1-(dodecylcarbamoyl)-3-methyl-butyl]octadecanamide,
   N-[(1S)-3-methyl-1-(octylcarbamoyl)butyl]octadecanamide,
   N-[(1S)-1-(dodecylcarbamoyl)-3-methyl-butyl]docosanamide,
   N-[(1S)-3-methyl-1-(octylcarbamoyl)butyl]docosanamide,
   N-[(1S)-3-methyl-1-(octylcarbamoyl)butyl]hexadecanamide,
   (2S)-N-dodecyl-2-(hexadecylcarbamoylamino)-4-methyl-pentanamide,
   (2S)-2-(hexadecylcarbamoylamino)-4-methyl-N-octyl-pentanamide,
   Hexadecyl N-[(1S)-1-(dodecylcarbamoyl)-3-methyl-butyl]carbamate,
   Hexadecyl N-[(1S)-3-methyl-1-(octylcarbamoyl)butyl]carbamate,
   N-[3-(dodecylamino)-2,2-dimethyl-3-oxo-propyl]octadecanamide,
   N-[(1S)-1-(dodecylcarbamoyl)-5-guanidino-pentyl]octadecanamide,
   N-[(1S)-1-(hexylcarbamoyl)-5-guanidino-pentyl]octadecanamide,
   (2S)-2-(diheptylamino)-N-dodecyl-5-guanidino-pentanamide,
   N-[2-(4-pyridyl)ethyl]docosanamide,
   (2S)-5-guanidino-2-(hexadecylcarbamoylamino)-N-octyl-pentanamide,
   (2S)-N-dodecyl-5-guanidino-2-(octadecylcarbamoylamino)pentanamide,
   (2S)-N-dodecyl-2-(dodecylcarbamoylamino)-5-guanidino-pentanamide,
   (2S)-6-amino-2-(hexadecylcarbamoylamino)-N-octyl-hexanamide,
   (2S)-6-amino-N-dodecyl-2-(hexadecylcarbamoylamino)hexanamide,
   Hexadecyl N-[(1S)-4-guanidino-1-(octylcarbamoyl)butyl]carbamate,
   and salts thereof.
[16-1] The vaccine of any of [12] to [15], wherein the compound represented by the formula (I) or a salt thereof is selected from the group consisting of
   N-[(1S)-1-(dodecylcarbamoyl)-4-guanidino-butyl]hexadecanamide,
   N-[(1S)-1-(dodecylcarbamoyl)-4-guanidino-butyl]octadecanamide,
   N-[(1S)-1-(dodecylcarbamoyl)-4-guanidino-butyl]docosanamide,
   N-[(1S)-3-methyl-1-(undecylcarbamoyl)butyl]docosanamide,
   N-[(1S)-1-(dodecylcarbamoyl)-3-methyl-butyl]hexadecanamide,
   N-[(1S)-2-(dodecylamino)-1-methyl-2-oxo-ethyl]docosanamide,
   N-[2-(dodecylamino)-1,1-dimethyl-2-oxo-ethyl]docosanamide,
   N-[3-(dodecylamino)-3-oxo-propyl]docosanamide,
   N-[3-(dodecylamino)-2,2-dimethyl-3-oxo-propyl]docosanamide,
   (2S)-N-dodecyl-5-guanidino-2-(hexadecylcarbamoylamino)pentanamide,
   Hexadecyl N-[(1S)-1-(dodecylcarbamoyl)-4-guanidino-butyl]carbamate,
   N-[(1S)-5-amino-1-(dodecylcarbamoyl)pentyl]hexadecanamide,
   N-[(1S)-1-(dodecylcarbamoyl)pentyl]docosanamide,
   1-docosanoyl-N-dodecyl-piperidine-4-carboxamide,
   N-[(1S)-1-(diheptylcarbamoyl)-4-guanidino-butyl]docosanamide,
   N-[(1S)-5-amino-1-(dodecylcarbamoyl)pentyl]docosanamide,
   (E)-N-[(1S)-1-(dodecylcarbamoyl)-4-guanidino-butyl]octadeca-9-enamide,
   N-[(1S)-4-guanidino-1-(heptylcarbamoyl)butyl]docosanamide,
   N-[3-(dodecylamino)-3-oxo-pentyl]docosanamide,
   N-[(1S)-4-amino-1-(dodecylcarbamoyl)butyl]docosanamide,
   N-[(1S)-1-(dodecylcarbamoyl)-5-guanidino-pentyl]docosanamide,
   N-[(1S)-5-acetamide-1-(dodecylcarbamoyl)pentyl]docosanamide,
   (2S)-5-guanidino-2-(hexadecylcarbamoylamino)-N-hexyl-pentanamide,
   Hexadecyl N-[(1S)-4-guanidino-1-(hexylcarbamoyl)butyl]carbamate,
   N-[(1S)-5-guanidino-1-(hexylcarbamoyl)pentyl]docosanamide,
   N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide,
   (2S)-2-(dibenzylamino)-N-dodecyl-5-guanidino-pentanamide,
   N-(5-guanidinopentyl)hexadecanamide,
   N-(5-guanidinopentyl)docosanamide,
   N-[(1S)-1-(dodecylcarbamoyl)-4-guanidino-butyl]-2-oxo-octanamide,
   Hexadecyl N-(4-guanidinobutyl)carbamate,
   N-[4-(ethanimidoylamino)butyl]hexadecanamide,
   and salts thereof.
[17] A compound represented by the formula (I'): 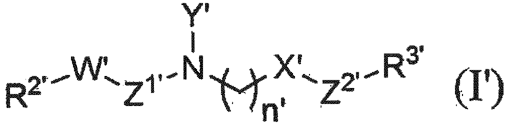 wherein
   X' is a group represented by the following formula 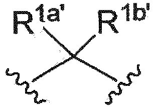
   wherein
   R^{1a}' is
   (1) a guanidyl-C₃₋₄ alkyl group,
   (2) a C₄ alkyl group,
   (3) an amino-C₃₋₄ alkyl group optionally substituted by an acetyl group or an aminocarbonyl group, or
   (4) a group represented by the following formula 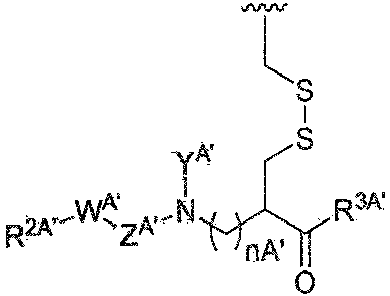
   wherein
   R^{2A}' is a C₁₈₋₂₄ hydrocarbon group;
   R^{3A}' is a mono- or di-C₆₋₁₆ alkylamino group;
   W^{A}' is a bond;
   Y^{A}' is a hydrogen atom;
   Z^{A}' is -CO-;
   nA' is 0;
   R^{1b}' is a hydrogen atom;
   Y' is a hydrogen atom;
   in R²', R³' and W',
      (I') when R^{1a}' is the aforementioned (1),
         (I'-1) R²' is a C₁₈₋₂₄ hydrocarbon group, R³' is a mono- or di-C₇₋₁₆ alkylamino group and W' is a bond,
         (I'-2) R²' is a C₁₂₋₂₄ hydrocarbon group, R³' is a mono- or di-C₆₋₁₆ alkylamino group and W' is -O- or -NH-, or
         (I'-3) R²' is a C₅₋₂₄ hydrocarbon group, R³' is a mono- or di-C₆₋₁₆ alkylamino group and W' is -CO-,
      (II') when R^{1a}' is the aforementioned (2),
         (II'-1) R²' is a C₁₂₋₂₄ hydrocarbon group, R³' is a mono- or di-C₇₋₁₆ alkylamino group and W' is a bond (provided that a case in which R²' is a C₂₁ hydrocarbon group and R³' is a mono-C₈ alkylamino group is excluded),
         (II'-2) R²' is a C₁₂₋₂₄ hydrocarbon group, R³' is a mono- or di-C₆₋₁₆ alkylamino group and W' is -O- or -NH-, or
         (II'-3) R²' is a C₅₋₂₄ hydrocarbon group, R³' is a mono- or di-C₆₋₁₆ alkylamino group and W' is -CO-,
      (III') when R^{1a}' is the aforementioned (3),
         (III'-1) R²' is a C₁₂₋₂₄ alkyl group, R³' is a mono- or di-C₇₋₁₆ alkylamino group and W' is a bond,
         (III'-2) R²' is a C₁₂₋₂₄ hydrocarbon group, R³' is a mono- or di-C₆₋₁₆ alkylamino group and W' is -O- or -NH-, or
         (III'-3) R²' is a C₅₋₂₄ hydrocarbon group, R³' is a mono- or di-C₆₋₁₆ alkylamino group and W' is -CO-,
      (IV') when R^{1a}' is the aforementioned (4),
         (IV'-1) R²' is a C₁₈₋₂₄ hydrocarbon group, R³' is a mono- or di-C₇₋₁₆ alkylamino group and W' is a bond,
         (IV'-2) R²' is a C₁₂₋₂₄ hydrocarbon group, R³' is a mono- or di-C₆₋₁₆ alkylamino group and W' is -O- or -NH-, or
         (IV'-3) R²' is a C₅₋₂₄ hydrocarbon group, R³' is a mono- or di-C₆₋₁₆ alkylamino group and W' is -CO-;
         Z¹' and Z²' are each -CO-;
         n' is 0,
   or a salt thereof.
[18] A compound selected from the group consisting of
   N-[(1S)-1-(dodecylcarbamoyl)-4-guanidino-butyl]hexadecanamide,
   N-[(1S)-1-(dodecylcarbamoyl)-4-guanidino-butyl]octadecanamide,
   N-[(1S)-1-(dodecylcarbamoyl)-4-guanidino-butyl]docosanamide,
   N-[(1S)-3-methyl-1-(undecylcarbamoyl)butyl]docosanamide,
   N-[(1S)-1-(dodecylcarbamoyl)-3-methyl-butyl]hexadecanamide,
   (2S)-N-dodecyl-5-guanidino-2-(hexadecylcarbamoylamino)pentanamide,
   Hexadecyl N-[(1S)-1-(dodecylcarbamoyl)-4-guanidino-butyl]carbamate,
   N-[(1S)-5-amino-1-(dodecylcarbamoyl)pentyl]hexadecanamide,
   N-[(1S)-1-(dodecylcarbamoyl)pentyl]docosanamide,
   N-[(1S)-1-(diheptylcarbamoyl)-4-guanidino-butyl]docosanamide,
   N-[(1S)-5-amino-1-(dodecylcarbamoyl)pentyl]docosanamide,
   (E)-N-[(1S)-1-(dodecylcarbamoyl)-4-guanidino-butyl]octadeca-9-enamide,
   N-[(1S)-4-guanidino-1-(heptylcarbamoyl)butyl]docosanamide,
   N-[(1S)-4-amino-1-(dodecylcarbamoyl)butyl]docosanamide,
   N-[(1S)-1-(dodecylcarbamoyl)-5-guanidino-pentyl]docosanamide,
   N-[(1S)-5-acetamide-1-(dodecylcarbamoyl)pentyl]docosanamide,
   (2S)-5-guanidino-2-(hexadecylcarbamoylamino)-N-hexyl-pentanamide,
   Hexadecyl N-[(1S)-4-guanidino-1-(hexylcarbamoyl)butyl]carbamate,
   N-[(1S)-5-guanidino-1-(hexylcarbamoyl)pentyl]docosanamide,
   N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide,
   N-[(1S)-1-(dodecylcarbamoyl)-4-guanidino-butyl]-2-oxo-octanamide,
   N-[(1S)-1-(dodecylcarbamoyl)-4-guanidino-butyl]icosanamide,
   N-[(1S)-4-guanidino-1-(octylcarbamoyl)butyl]icosanamide,
   N-[(1S)-5-amino-1-(octylcarbamoyl)pentyl]octadecanamide,
   N-[(1S)-5-amino-1-(dodecylcarbamoyl)pentyl]octadecanamide,
   Octadecyl N-[(1S)-1-(dodecylcarbamoyl)-4-guanidino-butyl] carbamate,
   Dodecyl N-[(1S)-1-(dodecylcarbamoyl)-4-guanidino-butyl]carbamate,
   Hexadecyl N-[(1S)-5-amino-1-(octylcarbamoyl)pentyl] carbamate,
   Hexadecyl N-[(1S)-5-amino-1-(dodecylcarbamoyl)pentyl] carbamate,
   (2R)-N-dodecyl-5-guanidino-2-(hexadecylcarbamoylamino)pentanamide,
   N-[(1R)-5-amino-1-(dodecylcarbamoyl)pentyl]hexadecanamide,
   N-[(1S)-1-(dodecylcarbamoyl)-3-methyl-butyl]octadecanamide,
   N-[(1S)-3-methyl-1-(octylcarbamoyl)butyl]octadecanamide,
   N-[(1S)-1-(dodecylcarbamoyl)-3-methyl-butyl]docosanamide,
   N-[(1S)-3-methyl-1-(octylcarbamoyl)butyl]hexadecanamide,
   (2S)-N-dodecyl-2-(hexadecylcarbamoylamino)-4-methyl-pentanamide,
   (2S)-2-(hexadecylcarbamoylamino)-4-methyl-N-octyl-pentanamide,
   Hexadecyl N-[(1S)-1-(dodecylcarbamoyl)-3-methyl-butyl]carbamate,
   Hexadecyl N-[(1S)-3-methyl-1-(octylcarbamoyl)butyl]carbamate,
   (2S)-5-guanidino-2-(hexadecylcarbamoylamino)-N-octyl-pentanamide,
   (2S)-N-dodecyl-5-guanidino-2-(octadecylcarbamoylamino)pentanamide,
   (2S)-N-dodecyl-2-(dodecylcarbamoylamino)-5-guanidino-pentanamide,
   (2S)-6-amino-2-(hexadecylcarbamoylamino)-N-octyl-hexanamide,
   (2S)-6-amino-N-dodecyl-2-(hexadecylcarbamoylamino)hexanamide, and
   Hexadecyl N-[(1S)-4-guanidino-1-(octylcarbamoyl)butyl]carbamate
   or a salt thereof.
[18-1] A compound selected from the group consisting of N-[(1S)-1-(dodecylcarbamoyl)-4-guanidino-butyl]hexadecanamide,
   N-[(1S)-1-(dodecylcarbamoyl)-4-guanidino-butyl]octadecanamide,
   N-[(1S)-1-(dodecylcarbamoyl)-4-guanidino-butyl]docosanamide,
   N-[(1S)-3-methyl-1-(undecylcarbamoyl)butyl]docosanamide,
   N-[(1S)-1-(dodecylcarbamoyl)-3-methyl-butyl]hexadecanamide,
   (2S)-N-dodecyl-5-guanidino-2-(hexadecylcarbamoylamino)pentanamide,
   Hexadecyl N-[(1S)-1-(dodecylcarbamoyl)-4-guanidino-butyl]carbamate,
   N-[(1S)-5-amino-1-(dodecylcarbamoyl)pentyl]hexadecanamide,
   N-[(1S)-1-(dodecylcarbamoyl)pentyl]docosanamide,
   N-[(1S)-1-(diheptylcarbamoyl)-4-guanidino-butyl]docosanamide,
   N-[(1S)-5-amino-1-(dodecylcarbamoyl)pentyl]docosanamide,
   (E)-N-[(1S)-1-(dodecylcarbamoyl)-4-guanidino-butyl]octadeca-9-enamide,
   N-[(1S)-4-guanidino-1-(heptylcarbamoyl)butyl]docosanamide,
   N-[(1S)-4-amino-1-(dodecylcarbamoyl)butyl]docosanamide,
   N-[(1S)-1-(dodecylcarbamoyl)-5-guanidino-pentyl]docosanamide,
   N-[(1S)-5-acetamide-1-(dodecylcarbamoyl)pentyl]docosanamide,
   (2S)-5-guanidino-2-(hexadecylcarbamoylamino)-N-hexyl-pentanamide,
   Hexadecyl N-[(1S)-4-guanidino-1-(hexylcarbamoyl)butyl]carbamate,
   N-[(1S)-5-guanidino-1-(hexylcarbamoyl)pentyl]docosanamide,
   N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide, and
   N-[(1S)-1-(dodecylcarbamoyl)-4-guanidino-butyl]-2-oxo-octanamide
   or a salt thereof.
[19] A compound selected from the group consisting of
   N-[(1S)-1-(dodecylcarbamoyl)-4-guanidino-butyl]hexadecanamide,
   N-[(1S)-1-(dodecylcarbamoyl)-4-guanidino-butyl]octadecanamide,
   (E)-N-[(1S)-1-(dodecylcarbamoyl)-4-guanidino-butyl]octadeca-9-enamide,
   N-[(1S)-4-guanidino-1-(octylcarbamoyl)butyl]hexadecanamide,
   N-[(1S)-4-guanidino-1-(octylcarbamoyl)butyl]octadecanamide,
   N-[(1R)-1-(dodecylcarbamoyl)-4-guanidino-butyl]octadecanamide,
   N-[(1S)-3-methyl-1-(octylcarbamoyl)butyl]docosanamide,
   N-[(1S)-1-(dodecylcarbamoyl)-5-guanidino-pentyl]octadecanamide,
   N-[(1S)-1-(hexylcarbamoyl)-5-guanidino-pentyl]octadecanamide,
   (2S)-2-(diheptylamino)-N-dodecyl-5-guanidino-pentanamide, and
   N-[2-(4-pyridyl)ethyl]docosanamide or a salt thereof.
[19-1] A compound selected from the group consisting of
   N-[(1S)-1-(dodecylcarbamoyl)-4-guanidino-butyl]hexadecanamide,
   N-[(1S)-1-(dodecylcarbamoyl)-4-guanidino-butyl]octadecanamide, and
   (E)-N-[(1S)-1-(dodecylcarbamoyl)-4-guanidino-butyl]octadeca-9-enamide,
   or a salt thereof.
[20] A compound represented by the formula (I"): 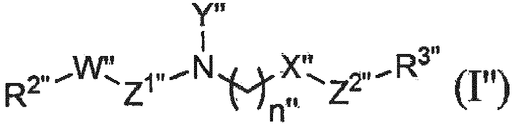 wherein
   in R²", R³" and W",
   (I"-1) R²" is a C₁₂₋₂₄ hydrocarbon group, R³" is a mono- or di-C₇₋₁₆ alkylamino group and W" is a bond,
   (I"-2) R²" is a C₁₂₋₂₄ hydrocarbon group, R³" is a mono- or di-C₆₋₁₆ alkylamino group and W" is -O- or -NH-, or
   (I"-3) R²" is a C₅₋₂₄ hydrocarbon group, R³" is a mono- or di-C₆₋₁₆ alkylamino group and W" is -CO-;
      X" is a group represented by the following formula 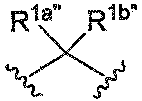
   wherein
   R^{1a}" is a hydrogen atom or a methyl group;
   R^{1b}" is a hydrogen atom or a methyl group;
   Y" is a hydrogen atom or a methyl group;
   Z¹" and Z²" are each -CO-;
   n" is an integer of 0 - 3;
   when R^{1a}" is a methyl group, R^{1b}" is a hydrogen atom, Y" is a hydrogen atom and n" is 2, then R^{1a}" and Y" are optionally bonded to form a ring,
   or a salt thereof.
[21] A compound selected from the group consisting of
   N-[(1S)-2-(dodecylamino)-1-methyl-2-oxo-ethyl]docosanamide,
   N-[2-(dodecylamino)-1,1-dimethyl-2-oxo-ethyl]docosanamide,
   N-[3-(dodecylamino)-3-oxo-propyl]docosanamide,
   N-[3-(dodecylamino)-2,2-dimethyl-3-oxopropyl]docosanamide,
   1-docosanoyl-N-dodecyl-piperidine-4-carboxamide,
   N-[3-(dodecylamino)-3-oxo-pentyl]docosanamide, and
   N-[3-(dodecylamino)-2,2-dimethyl-3-oxopropyl]octadecanamide
   or a salt thereof.
[21-1] A compound selected from the group consisting of
   N-[(1S)-2-(dodecylamino)-1-methyl-2-oxo-ethyl]docosanamide,
   N-[2-(dodecylamino)-1,1-dimethyl-2-oxo-ethyl]docosanamide,
   N-[3-(dodecylamino)-3-oxo-propyl]docosanamide,
   N-[3-(dodecylamino)-2,2-dimethyl-3-oxo-propyl]docosanamide,
   1-docosanoyl-N-dodecyl-piperidine-4-carboxamide, and
   N-[3-(dodecylamino)-3-oxo-pentyl]docosanamide
   or a salt thereof.
[22] A compound represented by the formula (I"'): 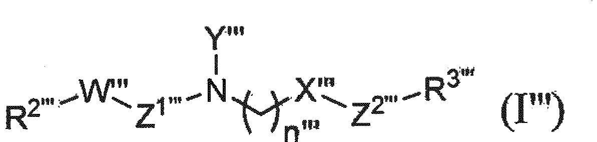 wherein
   R³"' is -C(=NH)R⁴"' wherein R⁴"' is an amino group or a C₁₋₄ alkyl group;
   in R²"' and W"',
      (I"'-1) R²"' is a C₁₂₋₂₄ hydrocarbon group and W"' is a bond, -O-, or -NH-, or
      (I"'-2) R²"' is a C₅₋₂₄ hydrocarbon group and W"' is -CO-; in X"', Y"' and n"',
      (II"'-1) X'" is -NH-, Y'" is a hydrogen atom, n"' is 4 or 5 (provided that a case in which W"' is a bond, n"' is 4 and R⁴"' is an amino group is excluded), or
      (II"'-2) X"' is -NR⁵"' - wherein R⁵"' is a methyl group, Y"' is a methyl group, n"' is 2 and R⁵"' and Y"' are bonded to form a ring;
         Z¹"' is -CO-; and
         Z²"' is a bond,
   or a salt thereof.
[23] The compound or a salt thereof of [22], wherein
   in X"', Y"' and n"',
   (II"'-1') X"' is -NH-, Y"' is a hydrogen atom, n"' is 4 or 5 (provided that a case in which n"' is 4 and R⁴"' is an amino group is excluded), or
   (II"'-2) X"' is -NR⁵"'- wherein R⁵"' is a methyl group, Y"' is a methyl group, n"' is 2 and R⁵"' and Y"' are bonded to form a ring.
[24] A compound selected from the group consisting of
   N-(5-guanidinopentyl)hexadecanamide,
   N-(5-guanidinopentyl)docosanamide,
   Hexadecyl N-(4-guanidinobutyl)carbamate,
   N-[4-(ethanimidoylamino)butyl]hexadecanamide, and
   1-(4-guanidinobutyl)-3-hexadecyl-urea
   or a salt thereof.
[24-1] A compound selected from the group consisting of
   N-(5-guanidinopentyl)hexadecanamide,
   N-(5-guanidinopentyl)docosanamide,
   Hexadecyl N-(4-guanidinobutyl)carbamate, and
   N-[4-(ethanimidoylamino)butyl]hexadecanamide
   or a salt thereof.
[25] (2S)-2-(dibenzylamino)-N-dodecyl-5-guanidino-pentanamide or a salt thereof.
[26] The immunostimulating agent of [1] or [6], the pharmaceutical composition of [7], or the vaccine of [12], wherein the compound represented by the formula (I) is a compound represented by the formula (II): 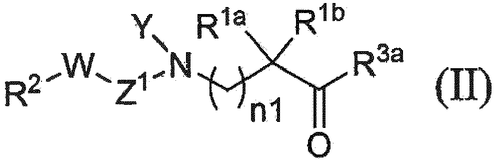 wherein
   R^{1a} is (1) a guanidyl-C₁₋₆ alkyl group, (2) a C₁₋₁₂ alkyl group, (3) an amino-C₁₋₆ alkyl group optionally substituted by an acetyl group or an aminocarbonyl group, (4) 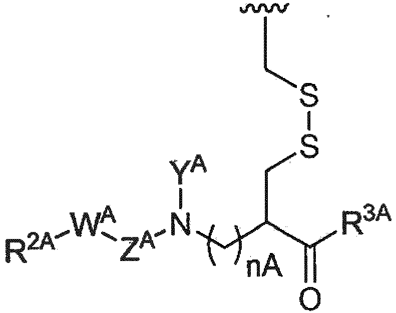 wherein
   R^{2A} is an optionally substituted C₁₋₂₄ hydrocarbon group;
   R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
   W^{A} is a bond, -O-, -NH- or -CO-;
   Y^{A} is a hydrogen atom, a C₁₋₁₆ alkyl group or a C₇₋₁₆ arylalkyl group;
   Z^{A} is -CO- or a bond;
   nA is an integer of 0 - 3, or (5) a hydrogen atom;
   R^{1b} is a C₁₋₄ alkyl group or a hydrogen atom;
   when R^{1a} is a methyl group, Y is a methyl group and n1 is 2, then R^{1a} and Y are optionally bonded to form a ring;
   R² is an optionally substituted C₁₋₂₄ hydrocarbon group;
   R^{3a} is (a) a mono- or di-C₁₋₁₆ alkylamino group optionally substituted by a hydroxyl group or a C₁₋₄ alkoxy group, or (b) a C₇₋₁₆ arylalkoxy group;
   W is a bond, -O-, -NH- or -CO-;
   Y is a hydrogen atom, a C₁₋₁₀ alkyl group or a C₇₋₁₆ arylalkyl group;
   Z¹ is -CO- or a bond;
   n1 is an integer of 0 - 3,
   provided that a case in which R^{1a} is a 3-guanidylpropyl group, a methyl group, an isopropyl group, an isobutyl group, a sec-butyl group, a 4-aminobutyl group or a hydrogen atom, R^{1b} is a hydrogen atom, R² is a C₁₋₂₄ alkyl group, R^{3a} is a mono- or di-C₁₋₆ alkylamino group, W is a bond, Y is a hydrogen atom, Z¹ is -CO-, and n1 is 0 is excluded.
[27] The immunostimulating agent of [1] or [6], the pharmaceutical composition of [7], or the vaccine of [12], wherein the compound represented by the formula (I) is a compound represented by the formula (III): 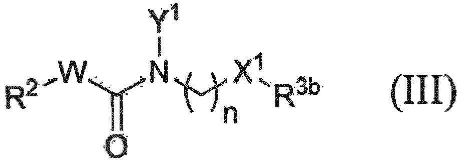 wherein
   R² is an optionally substituted C₁₋₂₄ hydrocarbon group;
   R^{3b} is (c) a hydrogen atom, (d) -C(=NH)R⁴ wherein R⁴ is an amino group, a C₁₋₁₆ alkyl-carbonylamino group or a C₁₋₄ alkyl group, or (e) a basic heterocyclic group;
   W is a bond, -0-, -NH- or -CO-;
   X¹ is (ii) -NR⁵- wherein R⁵ is a hydrogen atom or a methyl group, or (iii) a bond;
   Y¹ is a hydrogen atom or a methyl group;
   n is an integer of 0 - 6;
   when X¹ is -N(CH₃)-, Y¹ is a methyl group and n is 2, then the methyl group for X¹ and Y¹ are optionally bonded to form a ring,
   provided that a case in which R² is a C₁₋₂₄ alkyl group, R^{3b} is -C(=NH)NH₂, W is a bond, X¹ is -NH-, Y¹ is a hydrogen atom, and n is 4 is excluded.

### [Effect of the Invention]

Since compound (I) and a salt thereof have an antigen-specific IgG1 subclass antibody production-enhancing effect (immunostimulatory effect), they are useful as immunostimulating agents. Particularly, compound (I) and a salt thereof have an immunostimulatory effect equivalent to or not less than that of conventional aluminum gel adjuvants. In addition, compound (I) and a salt thereof also show an IgG2a subclass antibody production-enhancing effect (immunostimulatory effect). Furthermore, since compound (I) and a salt thereof suppress induction of IgE antibody production and sometimes suppress problematic allergy inducing activity of conventional aluminum gel adjuvants, they can be effective and safe adjuvants.

According to the present invention, moreover, a pharmaceutical composition containing compound (I) or a salt thereof, and α-cyclodextrin, which can be easily dissolved or dispersed in saline can be provided. The pharmaceutical composition is superior in an antigen-specific IgG1 subclass antibody production-enhancing effect (immunostimulatory effect), and can be used as an immunostimulating agent.

According to the present invention, a vaccine comprising compound (I) or a salt thereof, and an antigen is provided.

### [BRIEF DESCRIPTION OF THE DRAWINGS]

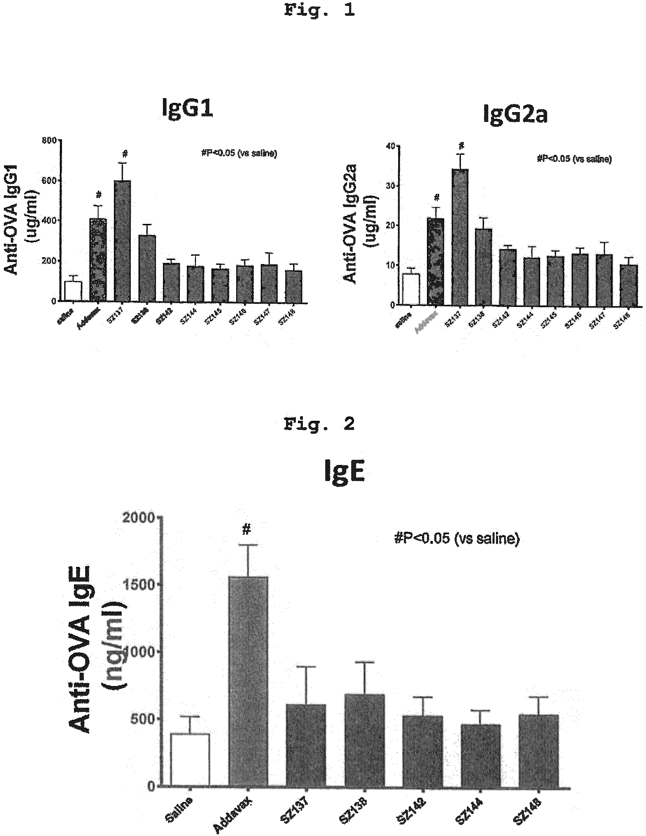

Fig. 1 shows graphs exhibiting the results of an evaluation test of the adjuvant activity (production amounts of IgG1 and IgG2a) of N-[(1S)-1-(dodecylcarbamoyl)-4-guanidino-butyl]octadecanamide hydrochloride (SZ137), N-[(1S)-1-(dodecylcarbamoyl)-4-guanidino-butyl]docosanamide hydrochloride (SZ138), N-[(1S)-3-methyl-1-(undecylcarbamoyl)butyl]docosanamide (SZ142), N-[(1S)-1-(dodecylcarbamoyl)-3-methyl-butyl]hexadecanamide (SZ144), N-[(1S)-2-(dodecylamino)-1-methyl-2-oxo-ethyl]docosanamide (SZ145), N-[2-(dodecylamino)-1,1-dimethyl-2-oxo-ethyl]docosanamide (SZ146), N-[3-(dodecylamino)-3-oxo-propyl]docosanamide (SZ147), N-[3-(dodecylamino)-2,2-dimethyl-3-oxo-propyl]docosanamide (SZ148) after secondary immunization in Experimental Example 1 (left: production amount of IgG1, right: production amount of IgG2a). "saline" shows OVA single administration group, "Addavax" shows Addavax™ administration group, "SZ137" shows SZ137 administration group, "SZ138" shows SZ138 administration group, "SZ142" shows SZ142 administration group, "SZ144" shows SZ144 administration group, "SZ145" shows SZ145 administration group, "SZ146" shows SZ146 administration group, "SZ147" shows SZ147 administration group, and "SZ148" shows SZ148 administration group.
Fig. 2 is a graph showing the results of an evaluation test of the allergy inducing activity (IgE production amount) of N-[(1S)-1-(dodecylcarbamoyl)-4-guanidino-butyl]octadecanamide hydrochloride (SZ137), N-[(1S)-1-(dodecylcarbamoyl)-4-guanidino-butyl]docosanamide hydrochloride (SZ138), N-[(1S)-3-methyl-1-(undecylcarbamoyl)butyl]docosanamide (SZ142), N-[(1S)-1-(dodecylcarbamoyl)-3-methyl-butyl]hexadecanamide (SZ144), N-[3-(dodecylamino)-2,2-dimethyl-3-oxo-propyl]docosanamide (SZ148) after secondary immunization in Experimental Example 1. "Saline" shows OVA single administration group, "Addavax" shows Addavax™ administration group, "SZ137" shows SZ137 administration group, "SZ138" shows SZ138 administration group, "SZ142" shows SZ142 administration group, "SZ144" shows SZ144 administration group and "SZ148" shows SZ148 administration group.

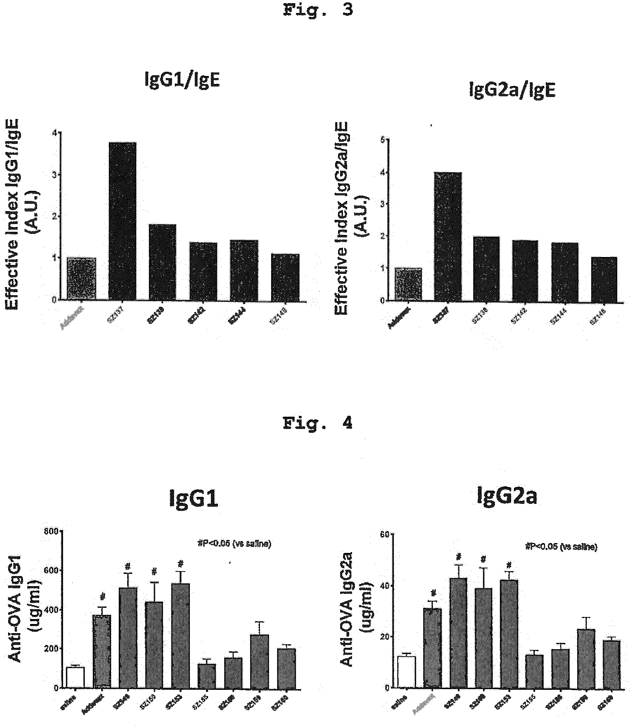

Fig. 3 shows graphs exhibiting relative values (standardized by relative value of Addavax™ administration group) of the adjuvant activity (production amounts of IgG1 and IgG2a) and the allergy inducing activity (IgE production amount) of N-[(1S)-1-(dodecylcarbamoyl)-4-guanidino-butyl]octadecanamide hydrochloride (SZ137), N-[(1S)-1-(dodecylcarbamoyl)-4-guanidino-butyl]docosanamide hydrochloride (SZ138), N-[(1S)-3-methyl-1-(undecylcarbamoyl)butyl]docosanamide (SZ142), N-[(1S)-1-(dodecylcarbamoyl)-3-methyl-butyl]hexadecanamide (SZ144), N-[3-(dodecylamino)-2,2-dimethyl-3-oxo-propyl]docosanamide (SZ148) after secondary immunization in Experimental Example 1 (left: IgG1 production amount/IgE production amount, right: IgG2a production amount/IgE production amount). "Addavax" shows Addavax™ administration group (1.0), "SZ137" shows SZ137 administration group, "SZ138" shows SZ138 administration group, "SZ142" shows SZ142 administration group, "SZ144" shows SZ144 administration group and "SZ148" shows SZ148 administration group.
Fig. 4 shows graphs exhibiting the results of an evaluation test of the adjuvant activity (production amounts of IgG1 and IgG2a) of (2S)-N-dodecyl-5-guanidino-2-(hexadecylcarbamoylamino)pentanamide hydrochloride (SZ149), Hexadecyl N-[(1S)-1-(dodecylcarbamoyl)-4-guanidino-butyl]carbamate hydrochloride (SZ150), N-[(1S)-5-amino-1-(dodecylcarbamoyl)pentyl]hexadecanamide hydrochloride (SZ153), N-[(1S)-1-(dodecylcarbamoyl)pentyl]docosanamide (SZ155), 1-docosanoyl-N-dodecyl-piperidine-4-carboxamide (SZ156), N-[(1S)-1-(diheptylcarbamoyl)-4-guanidino-butyl]docosanamide hydrochloride (SZ159), N-[(1S)-5-amino-1-(dodecylcarbamoyl)pentyl]docosanamide hydrochloride (SZ160) after secondary immunization in Experimental Example 2 (left: IgG1 production amount, right: IgG2a production amount). "saline" shows OVA single administration group, "Addavax" shows Addavax™ administration group, "SZ149" shows SZ149 administration group, "SZ150" shows SZ150 administration group, "SZ153" shows SZ153 administration group, "SZ155" shows SZ155 administration group, "SZ156" shows SZ156 administration group, "SZ159" shows SZ159 administration group, "SZ160" shows SZ160 administration group.

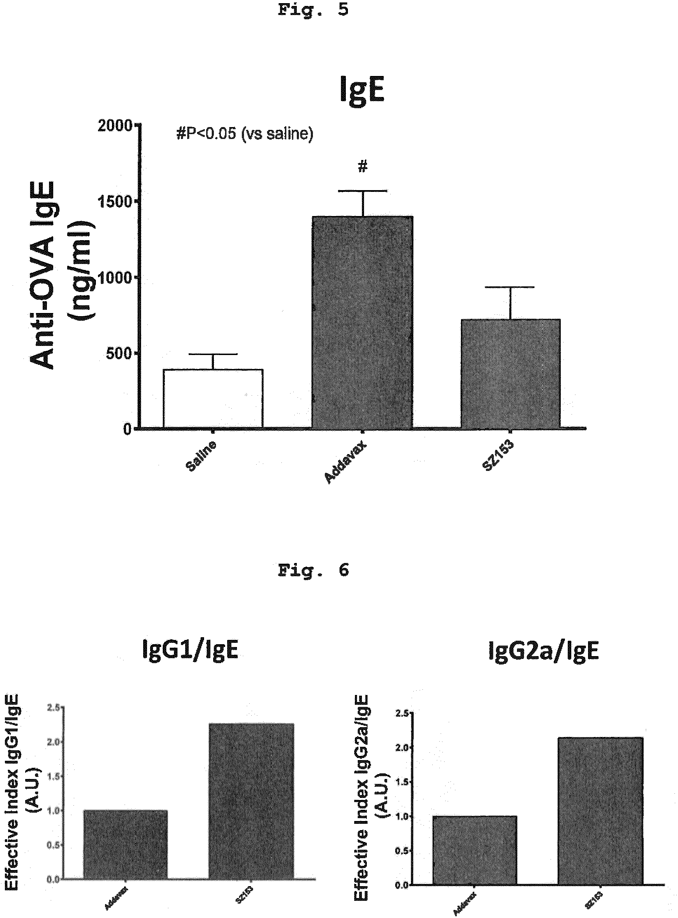

Fig. 5 is a graph showing the results of an evaluation test of the allergy inducing activity (IgE production amount) of N-[(1S)-5-amino-1-(dodecylcarbamoyl)pentyl]hexadecanamide hydrochloride (SZ153) after secondary immunization in Experimental Example 2. "Saline" shows OVA single administration group, "Addavax" shows Addavax™ administration group and "SZ153" shows SZ153 administration group.
Fig. 6 shows graphs exhibiting relative values (standardized by relative value of Addavax™ administration group) of the adjuvant activity (production amounts of IgG1 and IgG2a) and the allergy inducing activity (IgE production amount) of N-[(1S)-5-amino-1-(dodecylcarbamoyl)pentyl]hexadecanamide hydrochloride (SZ153) after secondary immunization in Experimental Example 2 (left: IgG1 production amount/IgE production amount, right: IgG2a production amount/IgE production amount). "Addavax" shows Addavax™ administration group (1.0) and "SZ153" shows SZ153 administration group.

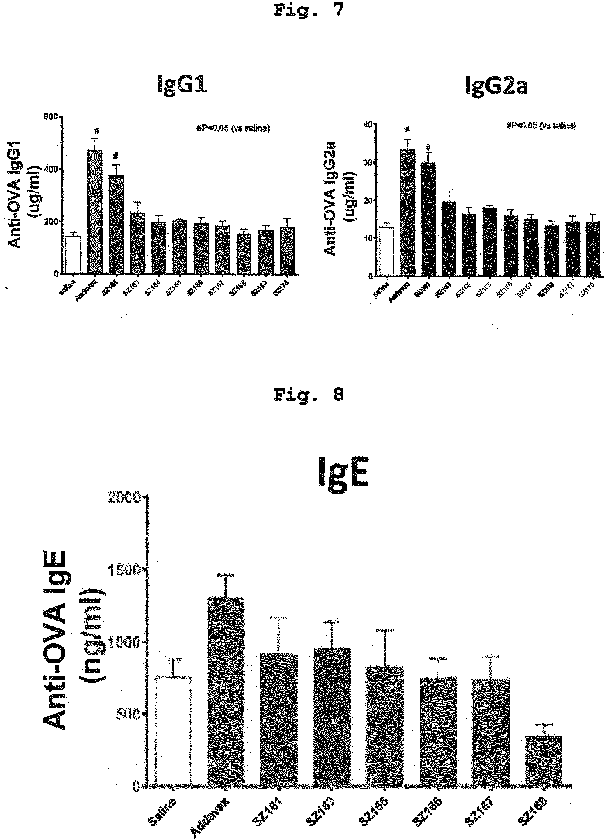

Fig. 7 shows graphs exhibiting the results of an evaluation test of the adjuvant activity (production amounts of IgG1 and IgG2a) of (E)-N-[(1S)-1-(dodecylcarbamoyl)-4-guanidino-butyl]octadeca-9-enamide hydrochloride (SZ161), N-[(1S)-4-guanidino-1-(heptylcarbamoyl)butyl]docosanamide hydrochloride (SZ163), N-[3-(dodecylamino)-3-oxo-pentyl]docosanamide (SZ164), N-[(1S)-4-amino-1-(dodecylcarbamoyl)butyl]docosanamide hydrochloride (SZ165), N-[(1S)-1-(dodecylcarbamoyl)-5-guanidino-pentyl]docosanamide hydrochloride (SZ166), N-[(1S)-5-acetamide-1-(dodecylcarbamoyl)pentyl]docosanamide (SZ167), (2S)-5-guanidino-2-(hexadecylcarbamoylamino)-N-hexyl-pentanamide hydrochloride (SZ168), Hexadecyl N-[(1S)-4-guanidino-1-(hexylcarbamoyl)butyl]carbamate hydrochloride (SZ169), N-[(1S)-5-guanidino-1-(hexylcarbamoyl)pentyl]docosanamide hydrochloride (SZ170) after secondary immunization in Experimental Example 3 (left: IgG1 production amount, right: IgG2a production amount). "saline" shows OVA single administration group, "Addavax" shows Addavax™ administration group, "SZ161" shows SZ161 administration group, "SZ163" shows SZ163 administration group, "SZ164" shows SZ164 administration group, "SZ165" shows SZ165 administration group, "SZ166" shows SZ166 administration group, "SZ167" shows SZ167 administration group, "SZ168" shows SZ168 administration group, "SZ169" shows SZ169 administration group and "SZ170" shows SZ170 administration group.
Fig. 8 is a graph showing the results of an evaluation test of the allergy inducing activity (IgE production amount) of (E)-N-[(1S)-1-(dodecylcarbamoyl)-4-guanidino-butyl]octadeca-9-enamide hydrochloride (SZ161), N-[(1S)-4-guanidino-1-(heptylcarbamoyl)butyl]docosanamide hydrochloride (SZ163), N-[(1S)-4-amino-1-(dodecylcarbamoyl)butyl]docosanamide hydrochloride (SZ165), N-[(1S)-1-(dodecylcarbamoyl)-5-guanidino-pentyl]docosanamide hydrochloride (SZ166), N-[(1S)-5-acetamide-1-(dodecylcarbamoyl)pentyl]docosanamide (SZ167), (2S)-5-guanidino-2-(hexadecylcarbamoylamino)-N-hexyl-pentanamide hydrochloride (SZ168) after secondary immunization in Experimental Example 3. "Saline" shows OVA single administration group, "Addavax" shows Addavax™ administration group, "SZ161" shows SZ161 administration group, "SZ163" shows SZ163 administration group, "SZ165" shows SZ165 administration group, "SZ166" shows SZ166 administration group, "SZ167" shows SZ167 administration group and "SZ168" shows SZ168 administration group.

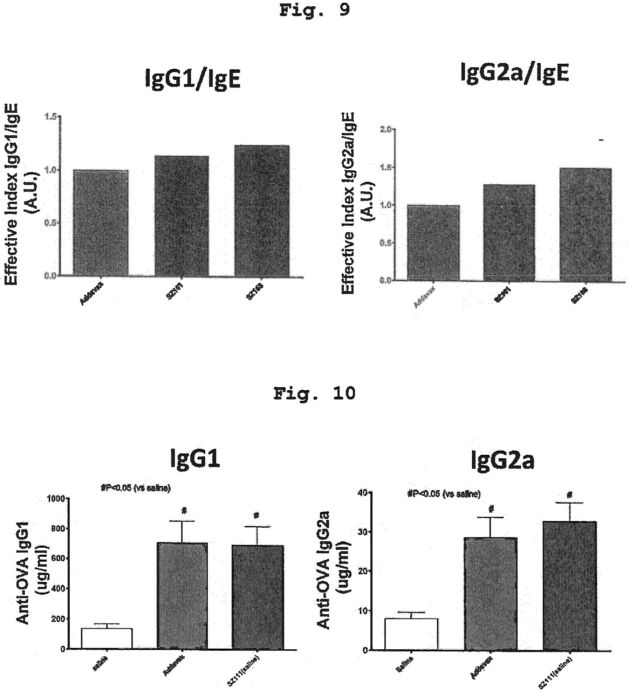

Fig. 9 shows graphs exhibiting relative values (standardized by relative value of Addavax™ administration group) of the adjuvant activity (production amounts of IgG1 and IgG2a) and the allergy inducing activity (IgE production amount) of (E)-N-[(1S)-1-(dodecylcarbamoyl)-4-guanidino-butyl]octadeca-9-enamide hydrochloride (SZ161), (2S)-5-guanidino-2-(hexadecylcarbamoylamino)-N-hexyl-pentanamide hydrochloride (SZ168) after secondary immunization in Experimental Example 3 (left: IgG1 production amount/IgE production amount, right: IgG2a production amount/IgE production amount). "Addavax" shows Addavax™ administration group (1.0), "SZ161" shows SZ161 administration group and "SZ168" shows SZ168 administration group.
Fig. 10 shows graphs exhibiting the results of an evaluation test of the adjuvant activity (production amounts of IgG1 and IgG2a) of N-[(1S)-1-(dodecylcarbamoyl)-4-guanidino-butyl]hexadecanamide hydrochloride (SZ111) after secondary immunization in Experimental Example 4 (left: IgG1 production amount, right: IgG2a production amount). "Saline" shows OVA single administration group, "Addavax" shows Addavax™ administration group and "SZ111 (saline)" shows SZ111 (saline) administration group.

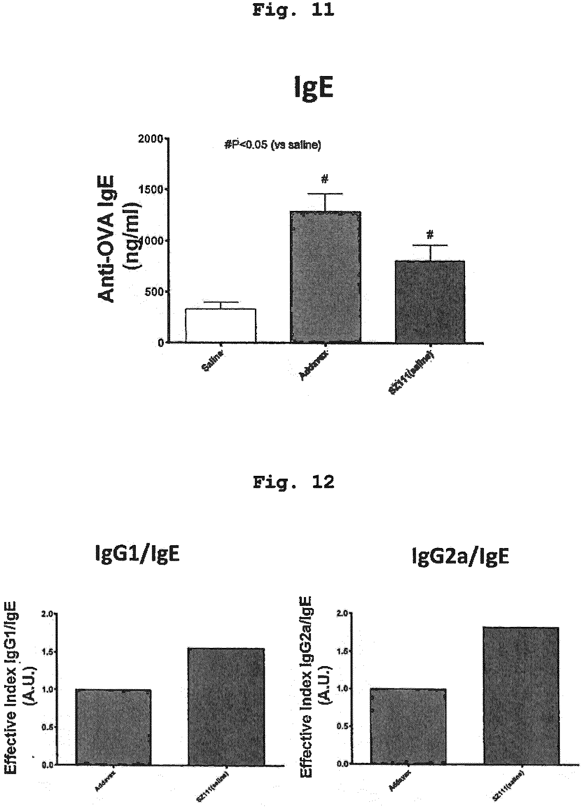

Fig. 11 is a graph showing the results of an evaluation test of the allergy inducing activity (IgE production amount) of N-[(1S)-1-(dodecylcarbamoyl)-4-guanidino-butyl]hexadecanamide hydrochloride (SZ111) in Experimental Example 4. "Saline" shows OVA single administration group, "Addavax" shows Addavax™ administration group and "SZ111 (saline)" shows SZ111 (saline) administration group.
Fig. 12 shows graphs exhibiting relative values (standardized by relative value of Addavax™ administration group) of the adjuvant activity (production amounts of IgG1 and IgG2a) and the allergy inducing activity (IgE production amount) of N-[(1S)-1-(dodecylcarbamoyl)-4-guanidino-butyl]hexadecanamide hydrochloride (SZ111) after secondary immunization in Experimental Example 4 (left: IgG1 production amount/IgE production amount, right: IgG2a production amount/IgE production amount). "Addavax" shows Addavax™ administration group (1.0) and "SZ111 (saline)" shows SZ111 (saline) administration group.

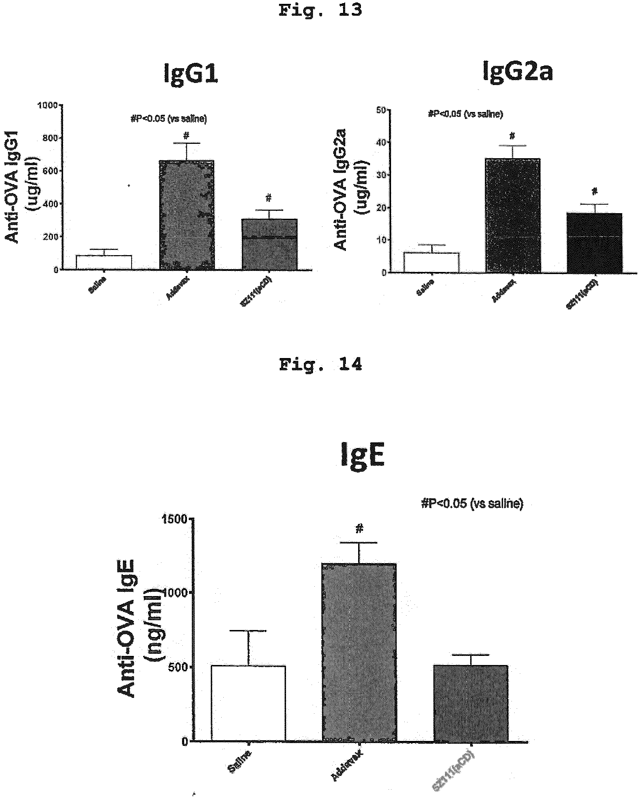

Fig. 13 shows graphs exhibiting the results of an evaluation test of the adjuvant activity (production amounts of IgG1 and IgG2a) of N-[(1S)-1-(dodecylcarbamoyl)-4-guanidino-butyl]hexadecanamide hydrochloride (SZ111) after secondary immunization in Experimental Example 5 (left: IgG1 production amount, right: IgG2a production amount). "Saline" shows OVA single administration group, "Addavax" shows Addavax™ administration group and "SZ111 (αCD)" shows SZ111 (αCD) administration group.
Fig. 14 is a graph showing the results of an evaluation test of the allergy inducing activity (IgE production amount) of N-[(1S)-1-(dodecylcarbamoyl)-4-guanidino-butyl]hexadecanamide hydrochloride (SZ111) after secondary immunization in Experimental Example 5. "Saline" shows OVA single administration group, "Addavax" shows Addavax™ administration group and "SZ111 (αCD)" shows SZ111 (αCD) administration group.

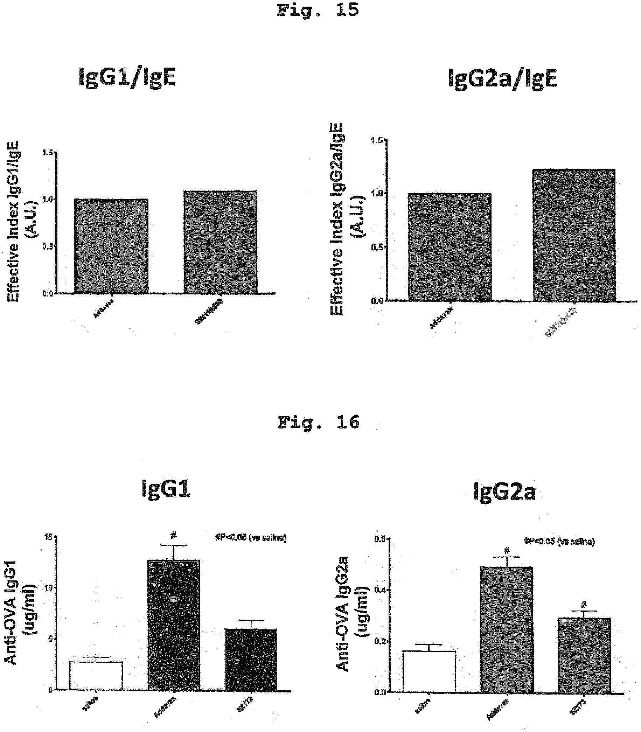

Fig. 15 shows graphs exhibiting relative values (standardized by relative value of Addavax™ administration group) of the adjuvant activity (production amounts of IgG1 and IgG2a) and the allergy inducing activity (IgE production amount) of N-[(1S)-1-(dodecylcarbamoyl)-4-guanidino-butyl]hexadecanamide hydrochloride (SZ111) after secondary immunization in Experimental Example 5 (left: IgG1 production amount/IgE production amount, right: IgG2a production amount/IgE production amount). "Addavax" shows Addavax™ administration group (1.0) and "SZ111 (αCD)" shows SZ111 (αCD) administration group.
Fig. 16 shows graphs exhibiting the results of an evaluation test of the adjuvant activity (production amounts of IgG1 and IgG2a) of N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide (SZ173) after primary immunization in Experimental Example 6 (left: IgG1 production amount, right: IgG2a production amount). "Saline" shows OVA single administration group, "Addavax" shows Addavax™ administration group and "SZ173" shows SZ173 administration group.

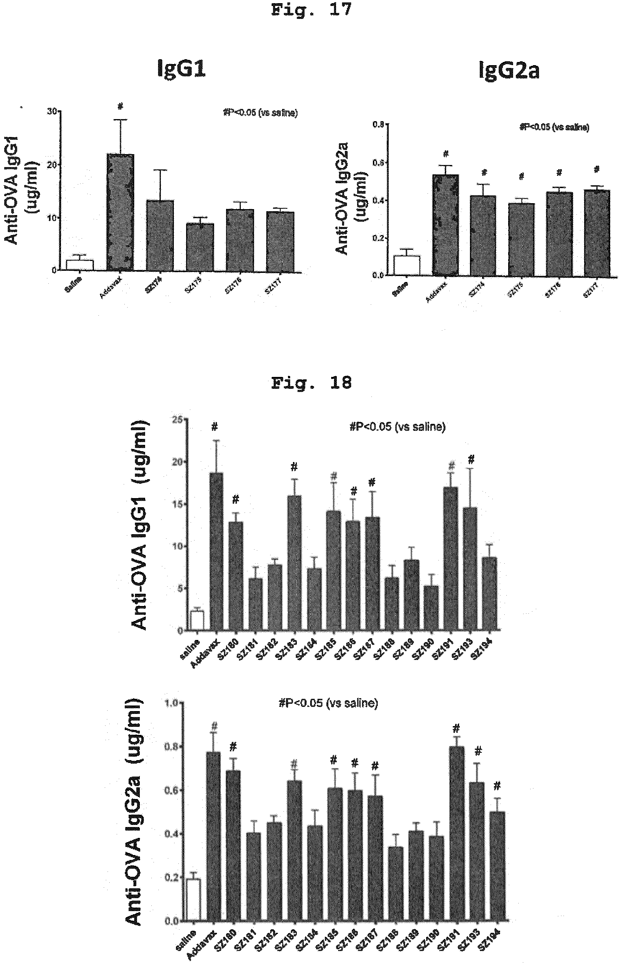

Fig. 17 shows graphs exhibiting the results of an evaluation test of the adjuvant activity (production amounts of IgG1 and IgG2a) of (2S)-2-(dibenzylamino)-N-dodecyl-5-guanidino-pentanamide (SZ174), N-(5-guanidinopentyl)hexadecanamide (SZ175), N-(5-guanidinopentyl)docosanamide (SZ176), N-[(1S)-1-(dodecylcarbamoyl)-4-guanidino-butyl]-2-oxo-octanamide (SZ177) after primary immunization in Experimental Example 7 (left: IgG1 production amount, right: IgG2a production amount). "Saline" shows OVA single administration group, "Addavax" shows Addavax™ administration group, "SZ174" shows SZ174 administration group, "SZ175" shows SZ175 administration group, "SZ176" shows SZ176 administration group and "SZ177" shows SZ177 administration group.
Fig. 18 shows graphs exhibiting the results of an evaluation test of the adjuvant activity (production amounts of IgG1 and IgG2a) of N-[(1S)-1-(dodecylcarbamoyl)-4-guanidino-butyl]icosanamide hydrochloride (SZ180), N-[(1S)-4-guanidino-1-(octylcarbamoyl)butyl]hexadecanamide hydrochloride (SZ181), N-[(1S)-4-guanidino-1-(octylcarbamoyl)butyl]octadecanamide hydrochloride (SZ182), N-[(1S)-4-guanidino-1-(octylcarbamoyl)butyl]icosanamide hydrochloride (SZ183), N-[(1S)-5-amino-1-(octylcarbamoyl)pentyl]octadecanamide hydrochloride (SZ184), N-[(1S)-5-amino-1-(dodecylcarbamoyl)pentyl]octadecanamide hydrochloride (SZ185), octadecyl N-[(1S)-1-(dodecylcarbamoyl)-4-guanidino-butyl]carbamate hydrochloride (SZ186), dodecyl N-[(1S)-1-(dodecylcarbamoyl)-4-guanidino-butyl]carbamate hydrochloride (SZ187), hexadecyl N-[(1S)-5-amino-1-(octylcarbamoyl)pentyl]carbamate hydrochloride (SZ188), hexadecyl N-[(1S)-5-amino-1-(dodecylcarbamoyl)pentyl]carbamate hydrochloride (SZ189), 1-(4-guanidinobutyl)-3-hexadecyl-urea hydrochloride (SZ190), N-[(1R)-1-(dodecylcarbamoyl)-4-guanidino-butyl]octadecanamide hydrochloride (SZ191), (2R)-N-dodecyl-5-guanidino-2-(hexadecylcarbamoylamino)pentanamide hydrochloride (SZ193), N-[(1R)-5-amino-1-(dodecylcarbamoyl)pentyl]hexadecanamide hydrochloride (SZ194) after primary immunization in Experimental Example 8 (upper: IgG1 production amount, lower: IgG2a production amount). "saline" shows OVA single administration group, "Addavax" shows Addavax™ administration group, "SZ180" shows SZ180 administration group, "SZ181" shows SZ181 administration group, "SZ182" shows SZ182 administration group, "SZ183" shows SZ183 administration group, "SZ184" shows SZ184 administration group, "SZ185" shows SZ185 administration group, "SZ186" shows SZ186 administration group, "SZ187" shows SZ187 administration group, "SZ188" shows SZ188 administration group, "SZ189" shows SZ189 administration group, "SZ190" shows SZ190 administration group, "SZ191" shows SZ191 administration group, "SZ193" shows SZ193 administration group and "SZ194" shows SZ194 administration group.

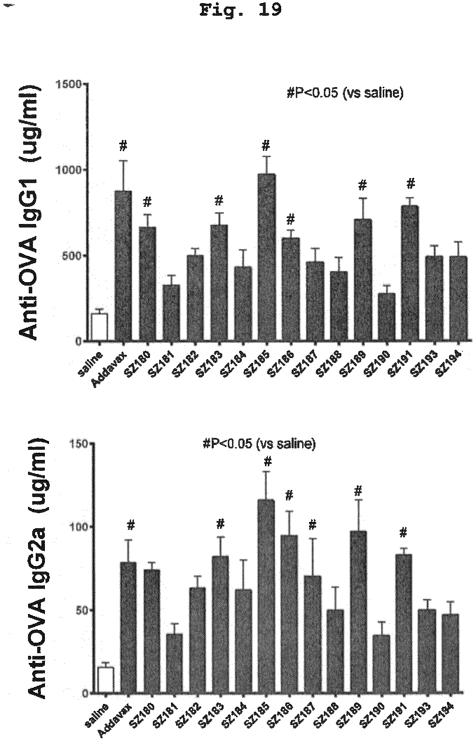

Fig. 19 shows graphs exhibiting the results of an evaluation test of the adjuvant activity (production amounts of IgG1 and IgG2a) of N-[(1S)-1-(dodecylcarbamoyl)-4-guanidino-butyl]icosanamide hydrochloride (SZ180), N-[(1S)-4-guanidino-1-(octylcarbamoyl)butyl]hexadecanamide hydrochloride (SZ181), N-[(1S)-4-guanidino-1-(octylcarbamoyl)butyl]octadecanamide hydrochloride (SZ182), N-[(1S)-4-guanidino-1-(octylcarbamoyl)butyl]icosanamide hydrochloride (SZ183), N-[(1S)-5-amino-1-(octylcarbamoyl)pentyl]octadecanamide hydrochloride (SZ184), N-[(1S)-5-amino-1-(dodecylcarbamoyl)pentyl]octadecanamide hydrochloride (SZ185), octadecyl N-[(1S)-1-(dodecylcarbamoyl)-4-guanidino-butyl]carbamate hydrochloride (SZ186), dodecyl N-[(1S)-1-(dodecylcarbamoyl)-4-guanidino-butyl]carbamate hydrochloride (SZ187), hexadecyl N-[(1S)-5-amino-1-(octylcarbamoyl)pentyl]carbamate hydrochloride (SZ188), hexadecyl N-[(1S)-5-amino-1-(dodecylcarbamoyl)pentyl]carbamate hydrochloride (SZ189), 1-(4-guanidinobutyl)-3-hexadecyl-urea hydrochloride (SZ190), N-[(1R)-1-(dodecylcarbamoyl)-4-guanidino-butyl]octadecanamide hydrochloride (SZ191), (2R)-N-dodecyl-5-guanidino-2-(hexadecylcarbamoylamino)pentanamide hydrochloride (SZ193), N-[(1R)-5-amino-1-(dodecylcarbamoyl)pentyl]hexadecanamide hydrochloride (SZ194) after secondary immunization in Experimental Example 8 (upper: IgG1 production amount, lower: IgG2a production amount). "saline" shows OVA single administration group, "Addavax" shows Addavax™ administration group, "SZ180" shows SZ180 administration group, "SZ181" shows SZ181 administration group, "SZ182" shows SZ182 administration group, "SZ183" shows SZ183 administration group, "SZ184" shows SZ184 administration group, "SZ185" shows SZ185 administration group, "SZ186" shows SZ186 administration group, "SZ187" shows SZ187 administration group, "SZ188" shows SZ188 administration group, "SZ189" shows SZ189 administration group, "SZ190" shows SZ190 administration group, "SZ191" shows SZ191 administration group, "SZ193" shows SZ193 administration group and "SZ194" shows SZ194 administration group.

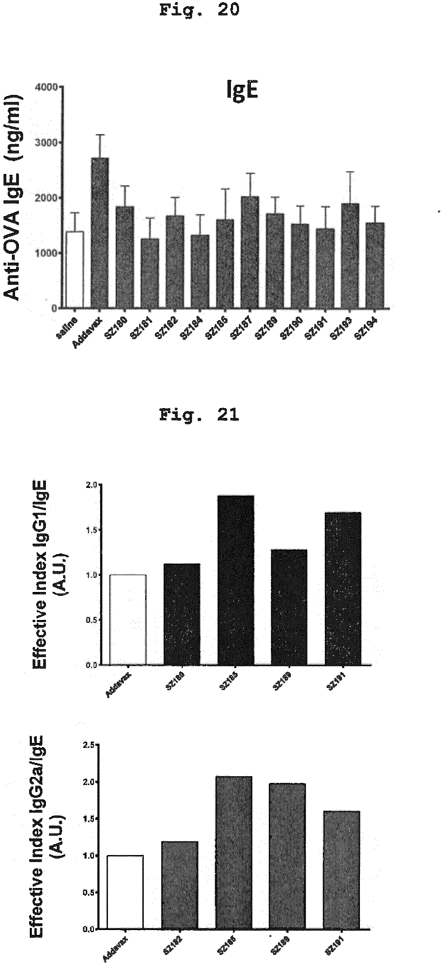

Fig. 20 is a graph showing the results of an evaluation test of the allergy inducing activity (IgE production amount) of N-[(1S)-1-(dodecylcarbamoyl)-4-guanidino-butyl]icosanamide hydrochloride (SZ180), N-[(1S)-4-guanidino-1-(octylcarbamoyl)butyl]hexadecanamide hydrochloride (SZ181), N-[(1S)-4-guanidino-1-(octylcarbamoyl)butyl]octadecanamide hydrochloride (SZ182), N-[(1S)-5-amino-1-(octylcarbamoyl)pentyl]octadecanamide hydrochloride (SZ184), N-[(1S)-5-amino-1-(dodecylcarbamoyl)pentyl]octadecanamide hydrochloride (SZ185), dodecyl N-[(1S)-1-(dodecylcarbamoyl)-4-guanidino-butyl]carbamate hydrochloride (SZ187), hexadecyl N-[(1S)-5-amino-1-(dodecylcarbamoyl)pentyl]carbamate hydrochloride (SZ189), 1-(4-guanidinobutyl)-3-hexadecyl-urea hydrochloride (SZ190), N-[(1R)-1-(dodecylcarbamoyl)-4-guanidino-butyl]octadecanamide hydrochloride (SZ191), (2R)-N-dodecyl-5-guanidino-2-(hexadecylcarbamoylamino)pentanamide hydrochloride (SZ193), N-[(1R)-5-amino-1-(dodecylcarbamoyl)pentyl]hexadecanamide hydrochloride (SZ194) after secondary immunization in Experimental Example 8. "saline" shows OVA single administration group, "Addavax" shows Addavax™ administration group, "SZ180" shows SZ180 administration group, "SZ181" shows SZ181 administration group, "SZ182" shows SZ182 administration group, "SZ184" shows SZ184 administration group, "SZ185" shows SZ185 administration group, "SZ187" shows SZ187 administration group, "SZ189" shows SZ189 administration group, "SZ190" shows SZ190 administration group, "SZ191" shows SZ191 administration group, "SZ193" shows SZ193 administration group and "SZ194" shows SZ194 administration group.
Fig. 21 shows graphs exhibiting relative values (standardized by relative value of Addavax™ administration group) of the adjuvant activity (production amounts of IgG1 and IgG2a) and the allergy inducing activity (IgE production amount) of N-[(1S)-1-(dodecylcarbamoyl)-4-guanidino-butyl]icosanamide hydrochloride (SZ180), N-[(1S)-4-guanidino-1-(octylcarbamoyl)butyl]octadecanamide hydrochloride (SZ182), N-[(1S)-5-amino-1-(dodecylcarbamoyl)pentyl]octadecanamide hydrochloride (SZ185), hexadecyl N-[(1S)-5-amino-1-(dodecylcarbamoyl)pentyl]carbamate hydrochloride (SZ189), N-[(1R)-1-(dodecylcarbamoyl)-4-guanidino-butyl]octadecanamide hydrochloride (SZ191) after secondary immunization in Experimental Example 8 (upper: IgG1 production amount/IgE production amount, lower: IgG2a production amount/IgE production amount). "Addavax" shows Addavax™ administration group (1.0), "SZ180" shows SZ180 administration group, "SZ182" shows SZ182 administration group, "SZ185" shows SZ185 administration group, "SZ189" shows SZ189 administration group, "SZ191" shows SZ191 administration group.

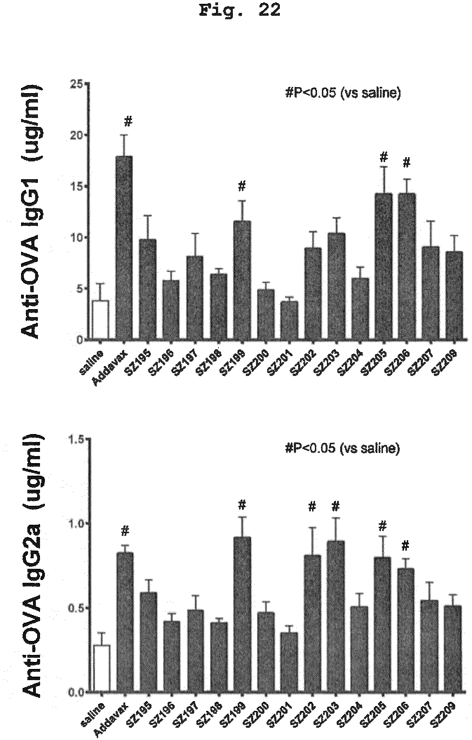

Fig. 22 shows graphs exhibiting the results of an evaluation test of the adjuvant activity (production amounts of IgG1 and IgG2a) of N-[(1S)-1-(dodecylcarbamoyl)-3-methyl-butyl]octadecanamide (SZ195), N-[(1S)-3-methyl-1-(octylcarbamoyl)butyl]octadecanamide (SZ196), N-[(1S)-1-(dodecylcarbamoyl)-3-methyl-butyl]docosanamide (SZ197), N-[(1S)-3-methyl-1-(octylcarbamoyl)butyl]docosanamide (SZ198), N-[(1S)-3-methyl-1-(octylcarbamoyl)butyl]hexadecanamide (SZ199), (2S)-N-dodecyl-2-(hexadecylcarbamoylamino)-4-methyl-pentanamide (SZ200), (2S)-2-(hexadecylcarbamoylamino)-4-methyl-N-octyl-pentanamide (SZ201), hexadecyl N-[(1S)-1-(dodecylcarbamoyl)-3-methyl-butyl]carbamate (SZ202), hexadecyl N-[(1S)-3-methyl-1-(octylcarbamoyl)butyl]carbamate (SZ203), N-[3-(dodecylamino)-2,2-dimethyl-3-oxo-propyl]octadecanamide (SZ204), N-[(1S)-1-(dodecylcarbamoyl)-5-guanidino-pentyl]octadecanamide hydrochloride (SZ205), N-[(1S)-1-(hexylcarbamoyl)-5-guanidino-pentyl]octadecanamide hydrochloride (SZ206), (2S)-2-(diheptylamino)-N-dodecyl-5-guanidino-pentanamide dihydrochloride (SZ207), N-[2-(4-pyridyl)ethyl]docosanamide hydrochloride (SZ209) after primary immunization in Experimental Example 9 (upper: IgG1 production amount, lower: IgG2a production amount). "saline" shows OVA single administration group, "Addavax" shows Addavax™ administration group, "SZ195" shows SZ195 administration group, "SZ196" shows SZ196 administration group, "SZ197" shows SZ197 administration group, "SZ198" shows SZ198 administration group, "SZ199" shows SZ199 administration group, "SZ200" shows SZ200 administration group, "SZ201" shows SZ201 administration group, "SZ202" shows SZ202 administration group, "SZ203" shows SZ203 administration group, "SZ204" shows SZ204 administration group, "SZ205" shows SZ205 administration group, "SZ206" shows SZ206 administration group, "SZ207" shows SZ207 administration group and "SZ209" shows SZ209 administration group.

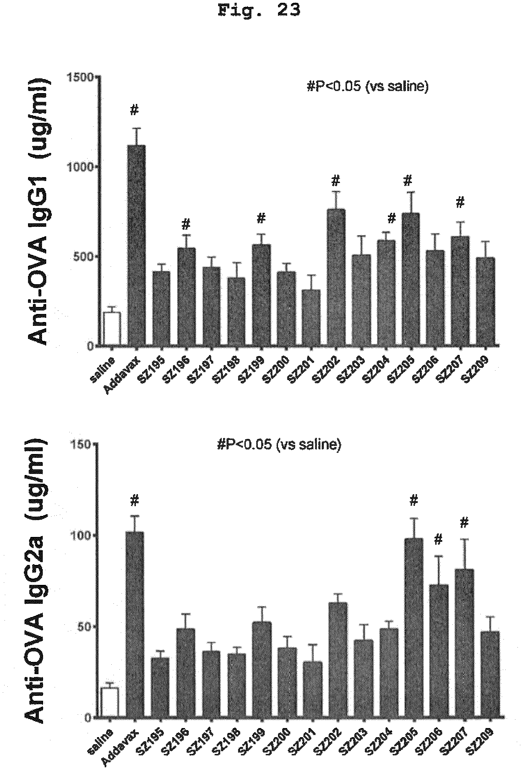

Fig. 23 shows graphs exhibiting the results of an evaluation test of the adjuvant activity (production amounts of IgG1 and IgG2a) of N-[(1S)-1-(dodecylcarbamoyl)-3-methyl-butyl]octadecanamide (SZ195), N-[(1S)-3-methyl-1-(octylcarbamoyl)butyl]octadecanamide (SZ196), N-[(1S)-1-(dodecylcarbamoyl)-3-methyl-butyl]docosanamide (SZ197), N-[(1S)-3-methyl-1-(octylcarbamoyl)butyl]docosanamide (SZ198), N-[(1S)-3-methyl-1-(octylcarbamoyl)butyl]hexadecanamide (SZ199), (2S)-N-dodecyl-2-(hexadecylcarbamoylamino)-4-methyl-pentanamide (SZ200), (2S)-2-(hexadecylcarbamoylamino)-4-methyl-N-octyl-pentanamide (SZ201), hexadecyl N-[(1S)-1-(dodecylcarbamoyl)-3-methyl-butyl]carbamate (SZ202), hexadecyl N-[(1S)-3-methyl-1-(octylcarbamoyl)butyl]carbamate (SZ203), N-[3-(dodecylamino)-2,2-dimethyl-3-oxo-propyl]octadecanamide (SZ204), N-[(1S)-1-(dodecylcarbamoyl)-5-guanidino-pentyl]octadecanamide hydrochloride (SZ205), N-[(1S)-1-(hexylcarbamoyl)-5-guanidino-pentyl]octadecanamide hydrochloride (SZ206), (2S)-2-(diheptylamino)-N-dodecyl-5-guanidino-pentanamide dihydrochloride (SZ207), N-[2-(4-pyridyl)ethyl]docosanamide hydrochloride (SZ209) after secondary immunization in Experimental Example 9 (upper: IgG1 production amount, lower: IgG2a production amount). "saline" shows OVA single administration group, "Addavax" shows Addavax™ administration group, "SZ195" shows SZ195 administration group, "SZ196" shows SZ196 administration group, "SZ197" shows SZ197 administration group, "SZ198" shows SZ198 administration group, "SZ199" shows SZ199 administration group, "SZ200" shows SZ200 administration group, "SZ201" shows SZ201 administration group, "SZ202" shows SZ202 administration group, "SZ203" shows SZ203 administration group, "SZ204" shows SZ204 administration group, "SZ205" shows SZ205 administration group, "SZ206" shows SZ206 administration group, "SZ207" shows SZ207 administration group and "SZ209" shows SZ209 administration group.

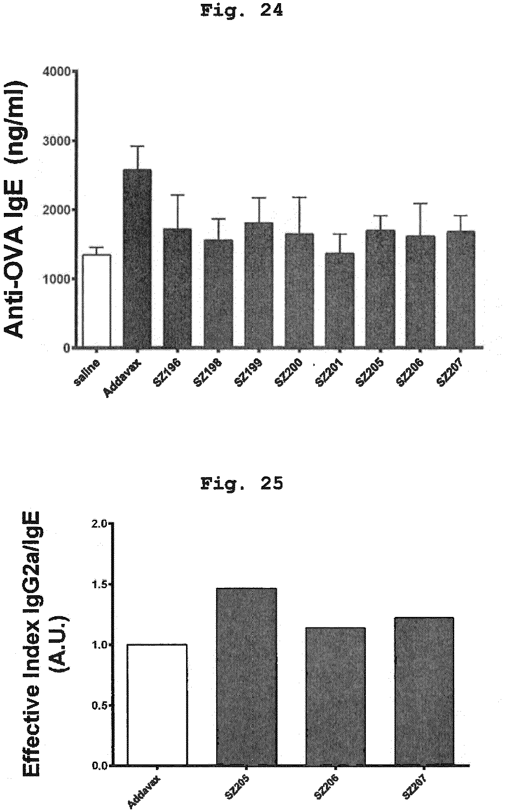

Fig. 24 is a graph showing the results of an evaluation test of the allergy inducing activity (IgE production amount) of N-[(1S)-3-methyl-1-(octylcarbamoyl)butyl]octadecanamide (SZ196), N-[(1S)-3-methyl-1-(octylcarbamoyl)butyl]docosanamide (SZ198), N-[(1S)-3-methyl-1-(octylcarbamoyl)butyl]hexadecanamide (SZ199), (2S)-N-dodecyl-2-(hexadecylcarbamoylamino)-4-methyl-pentanamide (SZ200), (2S)-2-(hexadecylcarbamoylamino)-4-methyl-N-octyl-pentanamide (SZ201), N-[(1S)-1-(dodecylcarbamoyl)-5-guanidino-pentyl]octadecanamide hydrochloride (SZ205), N-[(1S)-1-(hexylcarbamoyl)-5-guanidino-pentyl]octadecanamide hydrochloride (SZ206), (2S)-2-(diheptylamino)-N-dodecyl-5-guanidino-pentanamide dihydrochloride (SZ207) after secondary immunization in Experimental Example 9. "Saline" shows OVA single administration group, "Addavax" shows Addavax™ administration group, "SZ196" shows SZ196 administration group, "SZ198" shows SZ198 administration group, "SZ199" shows SZ199 administration group, "SZ200" shows SZ200 administration group, "SZ201" shows SZ201 administration group, "SZ205" shows SZ205 administration group, "SZ206" shows SZ206 administration group and "SZ207" shows SZ207 administration group.
Fig. 25 is a graph showing relative values (standardized by relative value of Addavax™ administration group) of the adjuvant activity (production amount of IgG2a) and the allergy inducing activity (IgE production amount) of N-[(1S)-1-(dodecylcarbamoyl)-5-guanidino-pentyl]octadecanamide hydrochloride (SZ205), N-[(1S)-1-(hexylcarbamoyl)-5-guanidino-pentyl]octadecanamide hydrochloride (SZ206), (2S)-2-(diheptylamino)-N-dodecyl-5-guanidino-pentanamide dihydrochloride (SZ207) after secondary immunization in Experimental Example 9. "Addavax" shows Addavax™ administration group (1.0), "SZ205" shows SZ205 administration group, "SZ206" shows SZ206 administration group and "SZ207" shows SZ207 administration group.

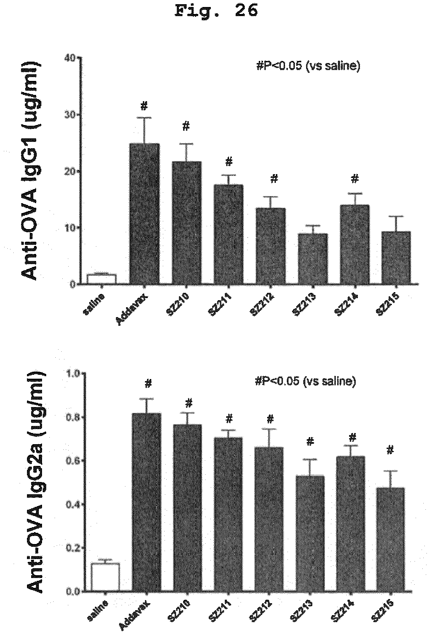

Fig. 26 shows graphs exhibiting the results of an evaluation test of the adjuvant activity (production amounts of IgG1 and IgG2a) of (2S)-5-guanidino-2-(hexadecylcarbamoylamino)-N-octyl-pentanamide hydrochloride (SZ210), (2S)-N-dodecyl-5-guanidino-2-(octadecylcarbamoylamino)pentanamide hydrochloride (SZ211), (2S)-N-dodecyl-2-(dodecylcarbamoylamino)-5-guanidino-pentanamide hydrochloride (SZ212), (2S)-6-amino-2-(hexadecylcarbamoylamino)-N-octyl-hexanamide hydrochloride (SZ213), (2S)-6-amino-N-dodecyl-2-(hexadecylcarbamoylamino)hexanamide hydrochloride (SZ214), hexadecyl N-[(1S)-4-guanidino-1-(octylcarbamoyl)butyl]carbamate hydrochloride (SZ215) after primary immunization in Experimental Example 10 (upper: IgG1 production amount, lower: IgG2a production amount). "saline" shows OVA single administration group, "Addavax" shows Addavax™ administration group, "SZ210" shows SZ210 administration group, "SZ211" shows SZ211 administration group, "SZ212" shows SZ212 administration group, "SZ213" shows SZ213 administration group, "SZ214" shows SZ214 administration group and "SZ215" shows SZ215 administration group.

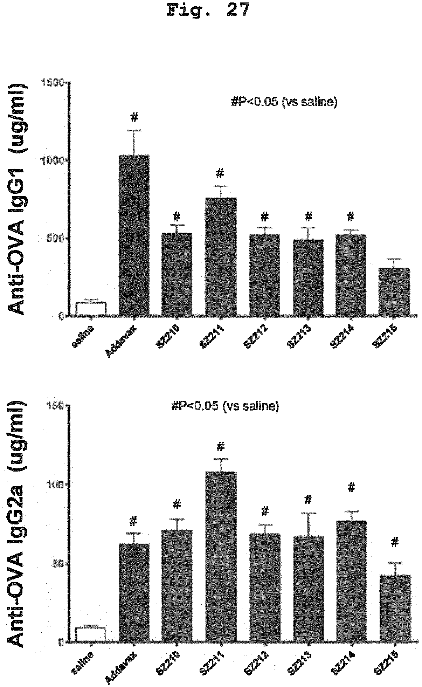

Fig. 27 shows graphs exhibiting the results of an evaluation test of the adjuvant activity (production amounts of IgG1 and IgG2a) of (2S)-5-guanidino-2-(hexadecylcarbamoylamino)-N-octyl-pentanamide hydrochloride (SZ210), (2S)-N-dodecyl-5-guanidino-2-(octadecylcarbamoylamino)pentanamide hydrochloride (SZ211), (2S)-N-dodecyl-2-(dodecylcarbamoylamino)-5-guanidino-pentanamide hydrochloride (SZ212), (2S)-6-amino-2-(hexadecylcarbamoylamino)-N-octyl-hexanamide hydrochloride (SZ213), (2S)-6-amino-N-dodecyl-2-(hexadecylcarbamoylamino)hexanamide hydrochloride (SZ214), hexadecyl N-[(1S)-4-guanidino-1-(octylcarbamoyl)butyl]carbamate hydrochloride (SZ215) after secondary immunization in Experimental Example 10 (upper: IgG1 production amount, lower: IgG2a production amount). "saline" shows OVA single administration group, "Addavax" shows Addavax™ administration group, "SZ210" shows SZ210 administration group, "SZ211" shows SZ211 administration group, "SZ212" shows SZ212 administration group, "SZ213" shows SZ213 administration group, "SZ214" shows SZ214 administration group and "SZ215" shows SZ215 administration group.

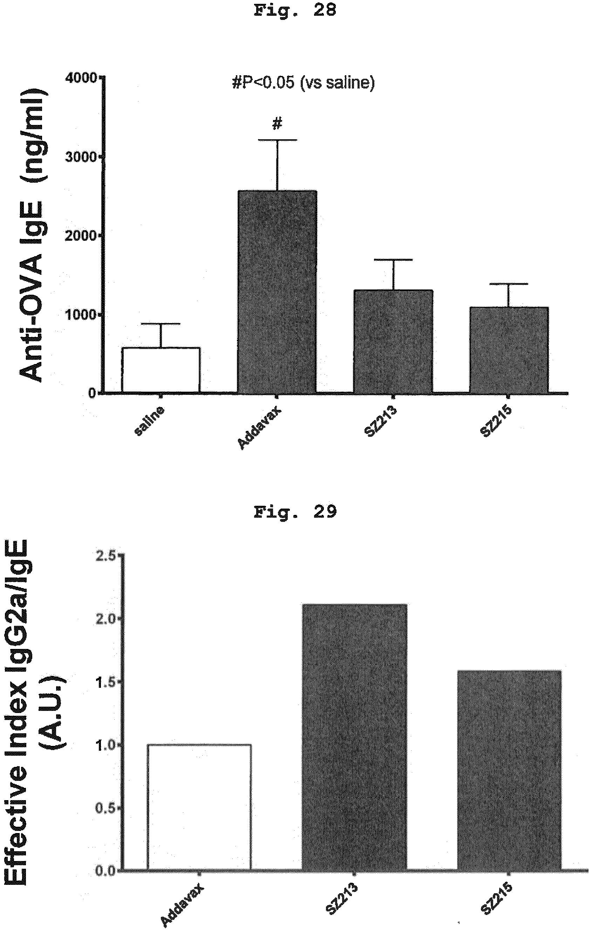

Fig. 28 is a graph showing the results of an evaluation test of the allergy inducing activity (IgE production amount) of (2S)-6-amino-2-(hexadecylcarbamoylamino)-N-octyl-hexanamide hydrochloride (SZ213), hexadecyl N-[(1S)-4-guanidino-1-(octylcarbamoyl)butyl]carbamate hydrochloride (SZ215) after secondary immunization in Experimental Example 10. "saline" shows OVA single administration group, "Addavax" shows Addavax™ administration group, "SZ213" shows SZ213 administration group and "SZ215" shows SZ215 administration group.
Fig. 29 is a graph showing relative values (standardized by relative value of Addavax™ administration group) of the adjuvant activity (production amount of IgG2a) and the allergy inducing activity (IgE production amount) of (2S)-6-amino-2-(hexadecylcarbamoylamino)-N-octyl-hexanamide hydrochloride (SZ213), hexadecyl N-[(1S)-4-guanidino-1-(octylcarbamoyl)butyl]carbamate hydrochloride (SZ215) after secondary immunization in Experimental Example 10. "Addavax" shows Addavax™ administration group (1.0), "SZ213" shows SZ213 administration group and "SZ215" shows SZ215 administration group.

### [Description of Embodiments]

The immunostimulating agent of the present invention comprises at least one kind of a compound represented by the following formula (I) or a salt thereof.

The formula (I): 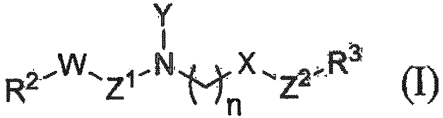 wherein
R² is an optionally substituted C₁₋₂₄ hydrocarbon group;
R³ is (a) a mono- or di-C₁₋₁₆ alkylamino group optionally substituted by a hydroxyl group or a C₁₋₄ alkoxy group, (b) a C₇₋₁₆ arylalkoxy group, (c) a hydrogen atom, (d) -C(=NH)R⁴ wherein R⁴ is an amino group, a C₁₋₁₆ alkyl-carbonylamino group or a C₁₋₄ alkyl group, or (e) a basic heterocyclic group;
W is a bond, -O-, -NH- or -CO-;
X is
   (i) a group represented by the following formula 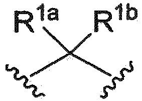 wherein
      R^{1a} is
      (1) a guanidyl-C₁₋₆ alkyl group,
      (2) a C₁₋₁₂ alkyl group,
      (3) an amino-C₁₋₆ alkyl group optionally substituted by an acetyl group or an aminocarbonyl group,
      (4) a group represented by the following formula 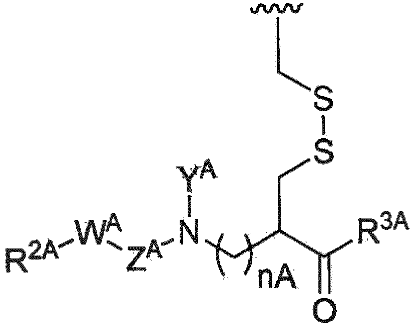 wherein
         R^{2A} is an optionally substituted C₁₋₂₄ hydrocarbon group;
         R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
         W^{A} is a bond, -O-, -NH- or -CO-;
         Y^{A} is a hydrogen atom, a C₁₋₁₀ alkyl group or a C₇₋₁₆ arylalkyl group;
         Z^{A} is -CO- or a bond;
         nA is an integer of 0 - 3 or
      (5) a hydrogen atom;
         R^{1b} is a C₁₋₄ alkyl group or a hydrogen atom;
         when R^{1a} is a methyl group, Y is a methyl group and n is 2, then R^{1a} and Y are optionally bonded to form a ring,
   (ii) -NR⁵- wherein R⁵ is a hydrogen atom or a methyl group, or
   (iii) a bond;
Y is a hydrogen atom, a C₁₋₁₀ alkyl group or a C₇₋₁₆ arylalkyl group;
Z¹ and Z² are the same or different and each is -CO- or a bond;
n is an integer of 0 - 6;
when X is -N(CH₃)-, Y is a methyl group and n is 2, then the methyl group for X and Y are optionally bonded to form a ring.

Each group in the formula (I) is explained below.

R² in the formula (I) is an optionally substituted C₁₋₂₄ hydrocarbon group.

The "C₁₋₂₄ hydrocarbon group" of the "optionally substituted C₁₋₂₄ hydrocarbon group" for R² means a saturated or unsaturated straight chain, branched chain or cyclic hydrocarbon group having 1 - 24 carbon atoms and, for example, a C₁₋₂₄ alkyl group, a C₂₋₂₄ alkenyl group, a C₃₋₈ cycloalkyl group, a C₃₋₈ cycloalkenyl group, a C₆₋₁₄ aryl group, a C₇₋₁₆ arylalkyl group and the like can be mentioned, with preference given to a C₁₋₂₄ alkyl group, a C₂₋₂₄ alkenyl group and a C₇₋₁₆ arylalkyl group. Of these, a C₁₋₂₁ hydrocarbon group (e.g., a C₁₋₂₁ alkyl group, a C₂₋₂₁ alkenyl group and a C₇₋₁₆ arylalkyl group etc.) is preferable, a C₆₋₂₁ hydrocarbon group (e.g., a C₆₋₂₁ alkyl group, a C₆₋₂₁ alkenyl group and a C₇₋₁₆ arylalkyl group etc.) is preferable, a C₁₅₋₂₁ hydrocarbon group (e.g., a C₁₅₋₂₁ alkyl group and a C₁₅₋₂₁ alkenyl group etc.) is preferable.

The "C₁₋₂₄ alkyl group" for R² means a straight chain or a branched chain alkyl having 1 - 24 carbon atoms and, for example, methyl group, ethyl group, propyl group, isopropyl group, butyl group, isobutyl group, sec-butyl group, tert-butyl group, pentyl group, isopentyl group, neopentyl group, 1-ethylpropyl group, hexyl group, isohexyl group, 1,1-dimethylbutyl group, 2,2-dimethylbutyl group, 3,3-dimethylbutyl group, 2-ethylbutyl group, heptyl group, octyl group, nonyl group, decyl group, undecyl group, dodecyl group (lauryl group), tridecyl group, tetradecyl group (myristyl group), pentadecyl group, hexadecyl group (palmityl group, cetyl group), heptadecyl group, octadecyl group (stearyl group), nonadecyl group, icosyl group (arachidinyl group), henicosyl group, docosyl group (behenyl group), tricosyl group, tetracosyl group and the like can be mentioned. Of these, a C₁₋₂₁ alkyl group is preferable, a C₆₋₂₁ alkyl group is more preferable, a C₁₅₋₂₁ alkyl group is further preferable, pentadecyl group, hexadecyl group, heptadecyl group or henicosyl group is particularly preferable.

The "C₂₋₂₄ alkenyl group" for R² means straight chain or branched chain alkenyl having 2 - 24 carbon atoms and, for example, ethenyl group, 1-propenyl group, 2-propenyl group, 2-methyl-1-propenyl group, 1-butenyl group, 2-butenyl group, 3-butenyl group, 3-methyl-2-butenyl group, 1-pentenyl group, 2-pentenyl group, 3-pentenyl group, 4-pentenyl group, 4-methyl-3-pentenyl group, 1-hexenyl group, 3-hexenyl group, 5-hexenyl group, 1-heptenyl group, 1-octenyl group, 1-nonenyl group, 1-decenyl group, 1-undecenyl group, 1-dodecenyl group, 1-tridecenyl group, 1-tetradecenyl group, 1-pentadecenyl group, 1-hexadecenyl group, 1-heptadecenyl group, 1-octadecenyl group, oleyl group, 1-nonadecenyl group, 1-icosenyl group, 1-henicosenyl group, 1-docosenyl group, 1-tricosenyl group, 1-tetracosenyl group and the like can be mentioned. Of these, a C₁₋₂₁ alkenyl group is preferable, a C₆₋₂₁ alkenyl group is more preferable, a C₁₅₋₂₁ alkenyl group is further preferable, and oleyl group is particularly preferable.

Examples of the "C₃₋₈ cycloalkyl group" for R² include cyclopropyl, cyclobutyl, cyclopentyl, cyclohexyl, cycloheptyl and cyclooctyl.

Examples of the "C₃₋₈ cycloalkenyl group" for R² include cyclopropenyl, cyclobutenyl, cyclopentenyl and cyclohexenyl.

The "C₆₋₁₄ aryl group" for R² means monocyclic or polycyclic (including fused ring) aryl having 6 - 14 carbon atoms and, for example, phenyl, 1-naphthyl, 2-naphthyl, 2-anthryl, phenanthryl, acenaphthyl, biphenylyl and the like can be mentioned.

The "C₇₋₁₆ arylalkyl group" for R² means a group in which aryl group is bonded to alkyl group and having 7 - 16 carbon atom in total. For example, benzyl, phenethyl, phenylpropyl, phenylbutyl, phenylpentyl, naphthylmethyl, biphenylylmethyl and the like can be mentioned.

The "optionally substituted" of the "optionally substituted C₁₋₂₄ hydrocarbon group" for R² means optionally having one or more substituents. Examples of the "substituent" include (1) halogen atom, (2) hydroxyl group, (3) nitro group, (4) cyano group, (5) C₁₋₆ alkyl group, (6) C₃₋₈ cycloalkyl group, (7) C₃₋₈ cycloalkenyl group, (8) C₂₋₆ alkenyl group, (9) C₂₋₆ alkynyl group, (10) C₁₋₆ alkoxy group, (11) C₁₋₆ alkylenedioxy group, (12) C₆₋₁₄ aryloxy group, (13) C₆₋₁₄ aryl group, (14) C₇₋₁₄ aralkyl group, (15) C₁₋₆ alkoxy-carbonyl group, (16) C₇₋₁₄ aralkyloxy-carbonyl group, (17) C₆₋₁₄ aryloxy-carbonyl group, (18) acyl group, (19) acyloxy group, (20) C₁₋₆ alkylsulfonyl group, (21) C₆₋₁₄ arylsulfonyl group, (22) C₁₋₆ alkylsulfonyloxy group, (23) C₆₋₁₄ arylsulfonyloxy group, (24) amino group, (25) mono- or di-C₁₋₆ alkylamino group, (26) mono- or di-C₂₋₆ alkenylamino group, (27) mono- or di-C₃₋₈ cycloalkylamino group, (28) mono- or di-C₆₋₁₄ arylamino group, (29) mono- or di-C₇₋₁₃ aralkylamino group, (30) mono- or di-heterocycleamino group, (31) azido group, (32) mercapto group, (33) C₁₋₆ alkylthio group, (34) C₆₋₁₄ arylthio group, (35) oxo group, (36) thioxo group, (37) aminocarbonyl group (carbamoyl group), (38) mono- or di-C₁₋₆ alkyl-aminocarbonyl group, (39) mono- or di-C₂₋₆ alkenyl-aminocarbonyl group, (40) mono- or di-C₃₋₈ cycloalkyl-aminocarbonyl group, (41) mono- or di-C₃₋₈ cycloalkenyl-aminocarbonyl group, (42) mono- or di-C₆₋₁₄ aryl-aminocarbonyl group, (43) mono- or di-C₇₋₁₃ aralkyl-aminocarbonyl group, (44) mono- or di-heterocyclic aminocarbonyl group, (45) thioaminocarbonyl group, (46) mono- or di-C₁₋₆ alkyl-thioaminocarbonyl group, (47) mono- or di-C₂₋₆ alkenyl-thioaminocarbonyl group, (48) mono- or di-C₃₋₈ cycloalkyl-thioaminocarbonyl group, (49) mono- or di-C₃₋₈ cycloalkenyl-thioaminocarbonyl group, (50) mono- or di-C₆₋₁₄ aryl-thioaminocarbonyl group, (51) mono- or di-C₇₋₁₃ aralkyl-thioaminocarbonyl group, (52) mono- or di-heterocyclic thioaminocarbonyl group, (53) aromatic heterocyclic group, (54) non-aromatic heterocyclic group, (55) carboxy group, (56) tri C₁₋₆ alkylsilyl group and the like. When plural substituents are present, the respective substituents may be the same or different.

The above-mentioned substituents are optionally further substituted by the above-mentioned substituents. While the number of the substituents is not particularly limited as long as it is a substitutable number, it is preferably 1 to 5, more preferably 1 to 3. When plural substituents are present, the respective substituents may be the same or different.

R³ in the formula (I) is (a) a mono- or di-C₁₋₁₆ alkylamino group optionally substituted by a hydroxyl group or a C₁₋₄ alkoxy group, (b) a C₇₋₁₆ arylalkoxy group, (c) a hydrogen atom, (d) -C (=NH) R⁴ wherein R⁴ is an amino group, a C₁₋₁₆ alkyl-carbonylamino group or a C₁₋₄ alkyl group, or (e) a basic heterocyclic group. Of these, (a), (c) or (d) is preferable.

The "mono- or di-C₁₋₁₆ alkylamino group" of (a) "a mono- or di-C₁₋₁₆ alkylamino group optionally substituted by a hydroxyl group (i.e., -OH) or a C₁₋₄ alkoxy group" for R³ means an amino group mono- or di-substituted by straight chain or branched chain alkyl having 1 - 16 carbon atoms (C₁₋₁₆ alkyl group). Examples thereof include methylamino group, ethylamino group, propylamino group, isopropylamino group, butylamino group, isobutylamino group, pentylamino group, isopentylamino group, hexylamino group, isohexylamino group, heptylamino group, octylamino group, nonylamino group, decylamino group, undecylamino group, dodecylamino group, tridecylamino group, tetradecylamino group, pentadecylamino group, hexadecylamino group, dimethylamino group, diethylamino group, ethylmethylamino group, dipropylamino group, diisopropylamino group, dibutylamino group, diisobutylamino group, dipentylamino group, diisopentylamino group, dihexylamino group, diisohexylamino group, diheptylamino group, dioctylamino group and the like. Of these, a mono- or di-C₅₋₁₂ alkylamino group which is an amino group mono- or di-substituted by a C₅₋₁₂ alkyl group is preferable, a mono-C₅₋₁₂ alkylamino group which is an amino group mono-substituted by a C₅₋₁₂ alkyl group is more preferable, and pentylamino group, hexylamino group, heptylamino group, octylamino group or dodecylamino group is particularly preferable.

The "C₁₋₄ alkoxy group" of (a) "a mono- or di-C₁₋₁₆ alkylamino group optionally substituted by a hydroxyl group or a C₁₋₄ alkoxy group" for R³ means straight chain or branched chain alkoxy having 1 - 4 carbon atoms. Examples thereof include methoxy group, ethoxy group, propoxy group, isopropoxy group, butoxy group, isobutoxy group, sec-butoxy group, tert-butoxy group and the like, with preference given to methoxy group.

The (b) "C₇₋₁₆ arylalkoxy group" for R³ means a group in which an aryl group is bonded to an alkoxy group, and having a total carbon atom number of 7 - 16. Examples thereof include benzyloxy group, phenethyloxy group, phenylpropyloxy group, phenylbutyloxy group, phenylpentyloxy group, 1-naphthylmethoxy group, 2-naphthylmethoxy group and the like, with preference given to benzyloxy group.

The "C₁₋₁₆ alkyl-carbonylamino group" for R⁴ of (d) "-C (=NH) R⁴ wherein R⁴ is an amino group, a C₁₋₁₆ alkyl-carbonylamino group or a C₁₋₄ alkyl group" for R³ is a carbonylamino group substituted by straight chain or branched chain alkyl having 1 - 16 carbon atoms (C₁₋₁₆ alkyl group) . Examples thereof include acetylamino group, propionylamino group, butyrylamino group, isobutyrylamino group, pentanoylamino group, isopentanoylamino group, hexanoylamino group, isohexanoylamino group, heptanoylamino group, octanoylamino group, nonanoylamino group, decanoylamino group, undecanoylamino group, dodecanoylamino group, tridecanoylamino group, tetradecanoylamino group, pentadecanoylamino group, hexadecanoylamino group and the like. Of these, a C₁₋₁₂ alkyl-carbonylamino group is preferable.

The "C₁₋₄ alkyl group" for R⁴ of (d) "-C(=NH)R⁴ wherein R⁴ is an amino group, a C₁₋₁₆ alkyl-carbonylamino group or a C₁₋₄ alkyl group)" for R³ is straight chain or branched chain alkyl having 1 - 4 carbon atoms. Examples thereof include methyl group, ethyl group, propyl group, isopropyl group, butyl group, isobutyl group, sec-butyl group, tert-butyl group and the like, with preference given to methyl group.

Examples of (e) "basic heterocyclic group" for R³ include basic monocyclic aromatic heterocyclic group, condensed aromatic heterocyclic group, monocyclic non-aromatic heterocyclic group, condensed non-aromatic heterocyclic group and the like. It is preferably a basic monocyclic aromatic heterocyclic group, more preferably 5 or 6-membered basic monocyclic aromatic heterocycle containing 1 or 2 nitrogen atoms, further preferably pyridyl group, imidazolyl group, pyrimidinyl group, particularly preferably pyridyl group.

W in the formula (I) is a bond, -O-, -NH- or -CO-.

X in the formula (I) is
(i) a group represented by the following formula 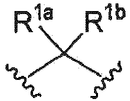 wherein
   R^{1a} is
   (1) a guanidyl-C₁₋₆ alkyl group,
   (2) a C₁₋₁₂ alkyl group,
   (3) an amino-C₁₋₆ alkyl group optionally substituted by an acetyl group or an aminocarbonyl group,
   (4) a group represented by the following formula 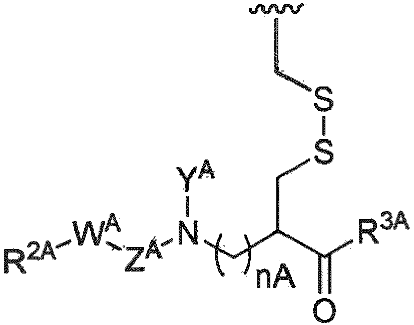 wherein
      R^{2A} is an optionally substituted C₁₋₂₄ hydrocarbon group;
      R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
      W^{A} is a bond, -O-, -NH- or -CQ-;
      Y^{A} is a hydrogen atom, a C₁₋₁₀ alkyl group or a C₇₋₁₆ arylalkyl group;
      Z^{A} is -CO- or a bond;
      nA is an integer of 0 - 3, or
   (5) a hydrogen atom;
      R^{1b} is a C₁₋₄ alkyl group or a hydrogen atom;
      when R^{1a} is a methyl group, Y is a methyl group and n is 2, then R^{1a} and Y are optionally bonded to form a ring,
(ii) -NR⁵- wherein R⁵ is a hydrogen atom or a methyl group, or
(iii) a bond. Of these, (i) or (ii) is preferable.

Each group in (i) a group represented by the following formula 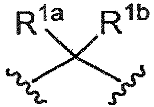 (hereinafter sometimes to be referred to as group (i)) for X is explained below.

R^{1a} in the aforementioned group (i) is
(1) a guanidyl-C₁₋₆ alkyl group,
(2) a C₁₋₁₂ alkyl group,
(3) an amino-C₁₋₆ alkyl group optionally substituted by an acetyl group or an aminocarbonyl group,
(4) a group represented by the following formula 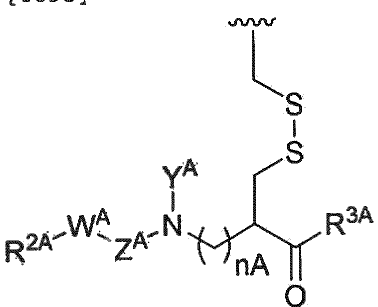 wherein
   R^{2A} is an optionally substituted C₁₋₂₄ hydrocarbon group;
   R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
   W^{A} is a bond, -O-, -NH- or -CO-;
   Y^{A} is a hydrogen atom, a C₁₋₁₀ alkyl group or a C₇₋₁₆ arylalkyl group;
   Z^{A} is -CO- or a bond;
   nA is an integer of 0 - 3, or
(5) a hydrogen atom.

(1) "guanidyl-C₁₋₆ alkyl group" for R^{1a} means a group in which a guanidyl group (i.e., -NH(=NH)NH₂) is bonded to a C₁₋₆ alkyl group and, for example, guanidylmethyl group, guanidylethyl group, 3-guanidylpropyl group, 4-guanidylbutyl group, 5-guanidylpentyl group, 6-guanidylhexyl group and the like can be mentioned. Of these, a guanidyl-C₁₋₄ alkyl group is preferable, 3-guanidylpropyl group or 4-guanidylbutyl group is more preferable.
(2) "C₁₋₁₂ alkyl group" for R^{1a} means straight chain or branched chain alkyl having 1 - 12 carbon atoms and, for example, methyl group, ethyl group, propyl group, isopropyl group, butyl group, isobutyl group, sec-butyl group, tert-butyl group, pentyl group, isopentyl group, neopentyl group, 1-ethylpropyl group, hexyl group, isohexyl group, 1,1-dimethylbutyl group, 2,2-dimethylbutyl group, 3,3-dimethylbutyl group, 2-ethylbutyl group, heptyl group, octyl group, nonyl group, decyl group, undecyl group, dodecyl group (lauryl group) and the like can be mentioned. Of these, a C₁₋₆ alkyl group is preferable, a C₁₋₄ alkyl group is more preferable, methyl group, butyl group or isobutyl group is particularly preferable.

The "amino-C₁₋₆ alkyl group" of (3) "amino-C₁₋₆ alkyl group optionally substituted by acetyl group (i.e., -C(=O)CH₃) or aminocarbonyl group (i.e., -C(=O)NH₂)" for R^{1a} means a group in which amino group (i.e., -NH₂) is bonded to a C₁₋₆ alkyl group and, for example, aminomethyl group, aminoethyl group, 3-aminopropyl group, 4-aminobutyl group, 5-aminopentyl group, 6-aminohexyl group and the like can be mentioned. Of these, an amino-C₁₋₄ alkyl group is preferable, and 3-aminopropyl group or 4-aminobutyl group is more preferable.

Each group in (4) a group represented by the following formula 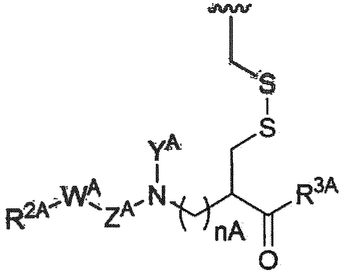 (hereinafter sometimes to be referred to as group (4)) for R^{1a} is explained below.

R^{2A}, W^{A}, Y^{A} and Z^{A} in the aforementioned group (4) are respectively as defined for R², W, Y (mentioned later) and Z¹ (mentioned later) in the formula (I).

The (a) "mono- or di-C₁₋₁₆ alkylamino group optionally substituted by a hydroxyl group or a C₁₋₄ alkoxy group" and (b) "C₇₋₁₆ arylalkoxy group" for R^{3A} in the aforementioned group (4) are respectively as defined for (a) "a mono- or di-C₁₋₁₆ alkylamino group optionally substituted by a hydroxyl group or a C₁₋₄ alkoxy group" and (b) "C₇₋₁₆ arylalkoxy group" for R³ in the formula (I).

nA in the aforementioned group (4) is an integer of 0 - 3. R^{2A}, R^{3A}, W^{A}, Y^{A}, Z^{A} and nA in the aforementioned group (4) each may be the same as or different from, preferably the same as, R², R³, W, Y, Z¹ and n in the formula (I). In the aforementioned group (4) and the formula (I), R² and R^{2A}, R³ and R^{3A}, W and W^{A}, Y and Y^{A}, Z¹ and Z^{A} and n and nA are preferably the same.

R^{1b} in the aforementioned group (i) is a C₁₋₄ alkyl group or a hydrogen atom.

The "C₁₋₄ alkyl group" for R^{1b} means straight chain or branched chain alkyl having 1 - 4 carbon atoms and, for example, methyl group, ethyl group, propyl group, isopropyl group, butyl group, isobutyl group, sec-butyl group, tert-butyl group and the like can be mentioned, with preference given to methyl group.

When R^{1a} in the aforementioned group (i) is a methyl group, Y in the formula (I) (mentioned later) is a methyl group, and n in the formula (I) (mentioned later) is 2, R^{1a} and Y may be bonded to form a 6-membered ring.

In the formula (I), Y is a hydrogen atom, a C₁₋₁₀ alkyl group or a C₇₋₁₆ arylalkyl group.

The "C₁₋₁₀ alkyl group" for Y means straight chain or branched chain alkyl having 1 - 10 carbon atoms. Examples thereof include methyl group, ethyl group, propyl group, isopropyl group, butyl group, isobutyl group, sec-butyl group, tert-butyl group, pentyl group, isopentyl group, neopentyl group, 1-ethylpropyl group, hexyl group, isohexyl group, 1,1-dimethylbutyl group, 2,2-dimethylbutyl group, 3,3-dimethylbutyl group, 2-ethylbutyl group, heptyl group, octyl group, nonyl group, decyl group and the like. Of these, a C₁₋₄ alkyl group is preferable, and methyl group is more preferable. As another embodiment, a C₁₋₈ alkyl group is preferable, and methyl group or heptyl group is more preferable.

The "C₇₋₁₆ arylalkyl group" for Y means a group in which an aryl group is bonded to an alkyl group, and having 7 - 16 carbon atoms in total. For example, benzyl group, phenethyl group, phenylpropyl group, phenylbutyl group, phenylpentyl group, naphthylmethyl group, biphenylylmethyl group and the like can be mentioned. Of these, benzyl group is preferable.

In the formula (I), Z¹ and Z² are the same or different and each is -CO- or a bond.

At least one of Z¹ and Z² is preferably -CO-. Z¹ and Z² may be both -CO-.

In the formula (I), n is an integer of 0 - 6. In one embodiment, n is preferably an integer of 0 - 3, more preferably 0 - 2. In another embodiment, n is preferably an integer of 2 - 6, more preferably 4 - 6.

In the formula (I), when X is -N(CH₃)-, Y is a methyl group and n is 2, then the methyl group for X and Y may be bonded to form a 6-membered ring.

In the formula (I), X is (i) "a group represented by the following formula 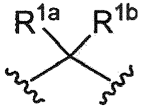 wherein R^{1a} and R^{1b} are as defined above", R³ is preferably (a) "a mono- or di-C₁₋₁₆ alkylamino group optionally substituted by a hydroxyl group or a C₁₋₄ alkoxy group", or (b) "a C₇₋₁₆ arylalkoxy group, particularly preferably (a).

In the formula (I), when X is (ii) "-NR⁵- wherein R⁵ are as defined above", R³ is preferably (c) "a hydrogen atom" or (d) "-C(=NH)R⁴ wherein R⁴ is as defined above".

In the formula (I), when X is (iii) "a bond", R³ is preferably (e) "a basic heterocyclic group" (more preferably, a pyridyl group).

In the formula (I), when X is (i) "a group represented by the following formula 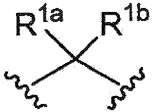 wherein R^{1a} and R^{1b} are as defined above", Z² is preferably -CO-.

In the formula (I), when X is (ii) "-NR⁵- wherein R⁵ is as defined above" or (iii) "a bond", Z¹ is preferably -CO-, and Z² is a bond.

In the formula (I), when X is (i) "a group represented by the following formula 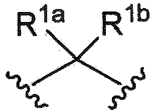 wherein R^{1a} and R^{1b} are as defined above", n is preferably an integer of 0 - 3, more preferably, an integer of 0 - 2.

In the formula (I), when X is (ii) "-NR⁵- wherein R⁵ is as defined above" or (iii) "a bond", n is preferably an integer of 2 - 6, more preferably, an integer of 4 - 6.

Compound (I) may exclude the formula (I) wherein R² is a C₁₋₂₄ alkyl group, R³ is a mono- or di-C₁₋₆ alkylamino group, W is a bond, X is the aforementioned (i) (i.e., -C(R^{1a})(R^{1b})-), R^{1a} is a 3-guanidylpropyl group, a methyl group, an isopropyl group, an isobutyl group, a sec-butyl group, a 4-aminobutyl group or a hydrogen atom, R^{1b} is a hydrogen atom, Y is a hydrogen atom, Z¹ and Z² are both -CO-, and n is 0.

Also, compound (I) may exclude the formula (I) wherein R² is a C₁₋₂₄ alkyl group, R³ is -C(=NH)NH₂, W is a bond, X is -NH-, Y is a hydrogen atom, Z¹ is -CO-, Z² is a bond, and n is 4.

Preferable compound (I) is shown below.

### [Compound (IA)]

Compound (I) wherein
R² is an optionally substituted C₁₋₂₄ hydrocarbon group (preferably, a C₁₋₂₄ alkyl group, a C₂₋₂₄ alkenyl group or a C₇₋₁₆ arylalkyl group);
R³ is (a) a mono- or di-C₁₋₁₆ alkylamino group optionally substituted by a hydroxyl group or a C₁₋₄ alkoxy group, or (b) a C₇₋₁₆ arylalkoxy group;
W is a bond, -O-, -NH- or -CO-;
X is (i) a group represented by the following formula 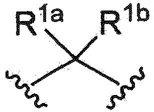
wherein R^{1a} and R^{1b} are as defined above;
Y is a hydrogen atom, a C₁₋₁₀ alkyl group or a C₇₋₁₆ arylalkyl group;
Z¹ is -CO- or a bond;
Z² is -CO-; and
n is an integer of 0 - 3.

### [Compound (IB)]

Compound (I) wherein
R² is an optionally substituted C₁₋₂₄ hydrocarbon group (preferably, a C₁₋₂₄ alkyl group, a C₂₋₂₄ alkenyl group or a C₇₋₁₆ arylalkyl group);
R³ is (a) a mono- or di-C₁₋₁₆ alkylamino group optionally substituted by a hydroxyl group or a C₁₋₄ alkoxy group;
W is a bond, -O-, -NH- or -CO-;
X is (i) a group represented by the following formula 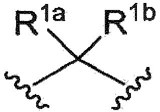
wherein R^{1a} and R^{1b} are as defined above;
Y is a hydrogen atom, a C₁₋₁₀ alkyl group or a C₇₋₁₆ arylalkyl group;
Z¹ is -CO- or a bond;
Z² is -CO-; and
n is an integer of 0 - 3.

### [Compound (IC)]

Compound (I) wherein
R² is an optionally substituted C₆₋₂₁ hydrocarbon group (preferably, a C₆₋₂₁ alkyl group, a C₆₋₂₁ alkenyl group or a C₇₋₁₆ arylalkyl group);
R³ is (a) a mono- or di-C₁₋₁₆ alkylamino group optionally substituted by a hydroxyl group or a C₁₋₄ alkoxy group, or (b) a C₇₋₁₆ arylalkoxy group;
W is a bond, -O-, -NH- or -CO-;
X is (i) a group represented by the following formula 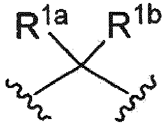
wherein R^{1a} and R^{1b} are as defined above;
Y is a hydrogen atom, a C₁₋₁₀ alkyl group or a C₇₋₁₆ arylalkyl group;
Z¹ is -CO- or a bond;
Z² is -CO-; and
n is an integer of 0 - 3.

### [Compound (ID)]

Compound (I) wherein
R² is an optionally substituted C₆₋₂₁ hydrocarbon group (preferably, a C₆₋₂₁ alkyl group, a C₆₋₂₁ alkenyl group or a C₇₋₁₆ arylalkyl group);
R³ is (a) a mono- or di-C₁₋₁₆ alkylamino group optionally substituted by a hydroxyl group or a C₁₋₄ alkoxy group;
W is a bond, -O-, -NH- or -CO-;
X is (i) a group represented by the following formula 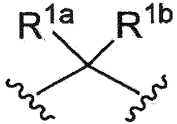
wherein R^{1a} and R^{1b} are as defined above;
Y is a hydrogen atom, a C₁₋₁₀ alkyl group or a C₇₋₁₆ arylalkyl group;
Z¹ is -CO- or a bond;
Z² is -CO-; and
n is an integer of 0 - 3.

### [Compound (IE)]

Compound (I) wherein
R² is an optionally substituted C₁₋₂₄ hydrocarbon group (preferably, a C₁₋₂₄ alkyl group or a C₂₋₂₄ alkenyl group);
R³ is (c) a hydrogen atom, or (d) -C(=NH)R⁴ wherein R⁴ is as defined above;
W is a bond, -O-, -NH- or -CO-;
X is (ii) -NR⁵- wherein R⁵ are as defined above;
Y is a hydrogen atom or a C₁₋₁₀ alkyl group;
Z¹ is -CO-;
Z² is a bond; and
n is an integer of 0 - 6.

### [Compound (IF)]

Compound (I) wherein
R² is an optionally substituted C₆₋₂₁ hydrocarbon group (preferably, a C₆₋₂₁ alkyl group or a C₆₋₂₁ alkenyl group) ;
R³ is (c) a hydrogen atom, or (d) -C(=NH)R⁴ wherein R⁴ is as defined above;
W is a bond, -O-, -NH- or -CO-;
X is (ii) -NR⁵- wherein R⁵ are as defined above;
Y is a hydrogen atom or C₁₋₁₀ alkyl group;
Z¹ is -CO-;
Z² is a bond; and
n is an integer of 2 - 6 (preferably, an integer of 4 - 6).

### [Compound (IG)]

Compound (I) wherein
R² is an optionally substituted C₁₋₂₄ hydrocarbon group (preferably, a C₁₋₂₄ alkyl group or a C₂₋₂₄ alkenyl group);
R³ is (e) a basic heterocyclic group (preferably, a pyridyl group);
W is a bond, -O-, -NH- or -CO-;
X is a bond;
Y is a hydrogen atom or a C₁₋₁₀ alkyl group;
Z¹ is -CO-;
Z² is a bond; and
n is an integer of 0 - 6.

A preferable embodiment of compound (I) is a compound represented by the following formula (II) (hereinafter sometimes to be referred to as compound (II)): 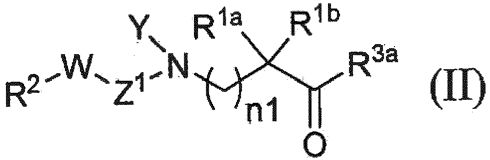 wherein
R^{1a} is
(1) a guanidyl-C₁₋₆ alkyl group,
(2) a C₁₋₁₂ alkyl group,
(3) an amino-C₁₋₆ alkyl group optionally substituted by an acetyl group or an aminocarbonyl group,
(4) a group represented by the following formula 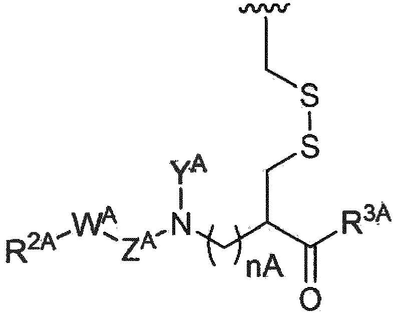 wherein
   R^{2A} is an optionally substituted C₁₋₂₄ hydrocarbon group;
   R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
   W^{A} is a bond, -O-, -NH- or -CO-;
   Y^{A} is a hydrogen atom, a C₁₋₁₀ alkyl group or a C₇₋₁₆ arylalkyl group;
   Z^{A} is -CO- or a bond; and
   nA is an integer of 0 - 3, or
(5) a hydrogen atom;
   R^{1b} is a C₁₋₄ alkyl group or a hydrogen atom;
   when R^{1a} is a methyl group, Y is a methyl group and n1 is 2, then R^{1a} and Y are optionally bonded to form a ring;
   R² is an optionally substituted C₁₋₂₄ hydrocarbon group;
   R^{3a} is (a) a mono- or di-C₁₋₁₆ alkylamino group optionally substituted by a hydroxyl group or a C₁₋₄ alkoxy group, or (b) a C₇₋₁₆ arylalkoxy group;
   W is a bond, -O-, -NH- or -CO-;
   Y is a hydrogen atom, a C₁₋₁₀ alkyl group or a C₇₋₁₆ arylalkyl group;
   Z¹ is -CO- or a bond; and
   n1 is an integer of 0 - 3.

Each group in the formula (II) is explained below.

In the formula (II), R^{1a}, R^{1b}, R², W, Y and Z¹ are each as defined for R^{1a}, R^{1b}, R², W, Y and Z¹ in the formula (I).

In the formula (II), R^{3a} is (a) a mono- or di-C₁₋₁₆ alkylamino group optionally substituted by a hydroxyl group or a C₁₋₄ alkoxy group, or (b) a C₇₋₁₆ arylalkoxy group. Of these, (a) is preferable.

The (a) "mono- or di-C₁₋₁₆ alkylamino group optionally substituted by a hydroxyl group or a C₁₋₄ alkoxy group" and (b) "C₇₋₁₆ arylalkoxy group" for R^{3a} in the formula (II) are respectively defined for (a) "a mono- or di-C₁₋₁₆ alkylamino group optionally substituted by a hydroxyl group or a C₁₋₄ alkoxy group" and (b) "C₇₋₁₆ arylalkoxy group" for R³ in the formula (I).

Compound (II) may exclude the formula (II) wherein R^{1a} is a 3-guanidylpropyl group, a methyl group, an isopropyl group, an isobutyl group, a sec-butyl group, a 4-aminobutyl group or a hydrogen atom, R^{1b} is a hydrogen atom, R² is a C₁₋₂₄ alkyl group, R^{3a} is a mono- or a di-C₁₋₆ alkylamino group, W is a bond, Y is a hydrogen atom, Z¹ is -CO-, and n1 is 0.

Preferable compound (II) is shown below.

### [Compound (IIA)]

Compound (II) wherein
R^{1a} is
(1) a guanidyl-C₁₋₄ alkyl group,
(2) a C₁₋₆ alkyl group,
(3) an amino-C₁₋₄ alkyl group optionally substituted by an acetyl group or an aminocarbonyl group,
(4) a group represented by the following formula 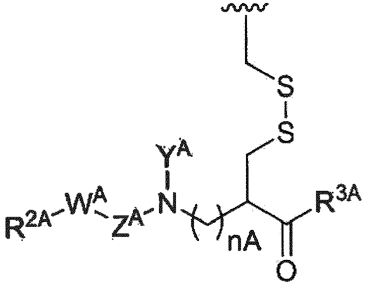 wherein R^{2A}, R^{3A}, W^{A}, Y^{A}, Z^{A} and nA are respectively the same as R², R^{3a}, W, Y, Z¹ and n1, or
(5) a hydrogen atom;
   R^{1b} is a C₁₋₄ alkyl group or a hydrogen atom;
   R² is an optionally substituted C₁₋₂₄ hydrocarbon group (preferably, a C₁₋₂₄ alkyl group, a C₂₋₂₄ alkenyl group or a C₇₋₁₆ arylalkyl group);
   R^{3a} is (a) a mono- or di-C₁₋₁₆ alkylamino group optionally substituted by a hydroxyl group or a C₁₋₄ alkoxy group;
   W is a bond, -O-, -NH- or -CO-;
   Y is a hydrogen atom, a C₁₋₁₀ alkyl group or a C₇₋₁₆ arylalkyl group;
   Z¹ is -CO- or a bond; and
   n1 is an integer of 0 - 3.

### [Compound (IIB)]

Compound (II) wherein
R^{1a} is
(1) a guanidyl-C₁₋₄ alkyl group,
(2) a C₁₋₆ alkyl group,
(3) an amino-C₁₋₄ alkyl group optionally substituted by an acetyl group or an aminocarbonyl group,
(4) a group represented by the following formula 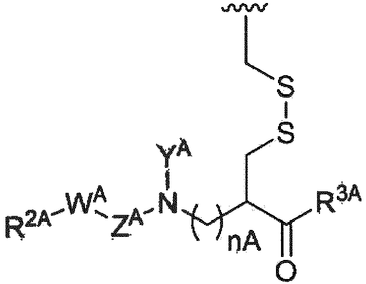 wherein R^{2A}, R^{3A}, W^{A}, Y^{A}, Z^{A} and nA are respectively the same as R², R^{3a}, W, Y, Z¹ and n1, or
(5) a hydrogen atom;
   R^{1b} is a C₁₋₄ alkyl group (preferably, a methyl group) or a hydrogen atom;
   R² is an optionally substituted C₆₋₂₁ hydrocarbon group (preferably, a C₆₋₂₁ alkyl group, a C₆₋₂₁ alkenyl group or a C₇₋₁₆ arylalkyl group);
   R^{3a} is (a) a mono- or di-C₅₋₁₂ alkylamino group optionally substituted by a hydroxyl group or a C₁₋₄ alkoxy group (preferably, a methoxy group);
   W is a bond, -O-, -NH- or -CO-;
   Y is a hydrogen atom, a C₁₋₄ alkyl group (preferably, a methyl group) or a C₇₋₁₆ arylalkyl group (preferably, a benzyl group);
   Z¹ is -CO- or a bond; h
   n1 is an integer of 0 - 3.

### [Compound (IIC)]

Compound (II) wherein
R^{1a} is a optionally substituted by a 3-guanidylpropyl group, a 4-guanidylbutyl group, a methyl group, a butyl group, an isobutyl group, a 3-aminopropyl group optionally substituted by an acetyl group, a 4-aminobutyl group optionally substituted by an acetyl group, a group represented by the following formula 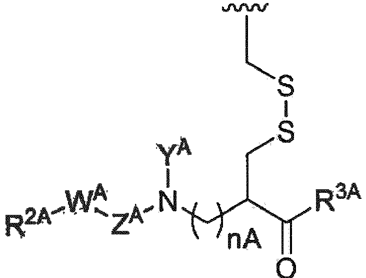
wherein R^{2A}, R^{3A}, W^{A}, Y^{A}, Z^{A} and nA are respectively the same as R², R^{3a}, W, Y, Z¹ and n1 or a hydrogen atom;
R^{1b} is a C₁₋₄ alkyl group (preferably, a methyl group) or a hydrogen atom;
R² is an optionally substituted C₆₋₂₁ hydrocarbon group (preferably, a C₆₋₂₁ alkyl group, a C₆₋₂₁ alkenyl group or a benzyl group);
R^{3a} is (a) a mono- or di-C₅₋₁₂ alkylamino group optionally substituted by a hydroxyl group or a C₁₋₄ alkoxy group (preferably, a methoxy group);
W is a bond, -O-, -NH- or -CO-;
Y is a hydrogen atom, a C₁₋₄ alkyl group (preferably, a methyl group) or a C₇₋₁₆ arylalkyl group (preferably, a benzyl group);
Z¹ is -CO-; and
n1 is an integer of 0 - 3.

Other preferable embodiment of compound (I) is a compound represented by the following formula (III) (hereinafter sometimes to be referred to as compound (III)): 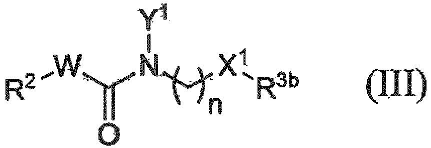 wherein
R² is an optionally substituted C₁₋₂₄ hydrocarbon group;
R^{3b} is (c) a hydrogen atom, (d) -C(=NH)R⁴ wherein R⁴ is an amino group, a C₁₋₁₆ alkyl-carbonylamino group or a C₁₋₄ alkyl group, or (e) a basic heterocyclic group;
W is a bond, -O-, -NH- or -CO-;
X¹ is (ii) -NR⁵- wherein R⁵ is a hydrogen atom or a methyl group) or (iii) a bond;
Y¹ is a hydrogen atom or a methyl group;
n is an integer of 0 - 6;
when X¹ is -N(CH₃)-, Y¹ is a methyl group and n is 2, then the methyl group for X¹ and Y are optionally bonded to form a ring.

Each group in the formula (III) is explained below.

In the formula (III), R², W and n are each as defined for R², W and n in the formula (I).

In the formula (III), R^{3b} is (c) a hydrogen atom, (d) -C (=NH)R⁴ wherein R⁴ is an amino group, a C₁₋₁₆ alkyl-carbonylamino group or a C₁₋₄ alkyl group, or (e) a basic heterocyclic group. Of these, (c) or (d) is preferable.
(d) "-C(=NH)R⁴ wherein R⁴ is an amino group, a C₁₋₁₆ alkyl-carbonylamino group or a C₁₋₄ alkyl group" and (e) "basic heterocyclic group" for R^{3b} in the formula (III) are respectively as defined for (d) "-C(=NH)R⁴ wherein R⁴ is an amino group, a C₁₋₁₆ alkyl-carbonylamino group or a C₁₋₄ alkyl group" and (e) "basic heterocyclic group" for R³ in the formula (I).

In the formula (III), X¹ is (ii) -NR⁵- wherein R⁵ is a hydrogen atom or a methyl group or (iii) a bond.

In the formula (III), Y¹ is a hydrogen atom or a methyl group.

Compound (III) may exclude the formula (III) wherein R² is a C₁₋₂₄ alkyl group, R^{3b} is -C(=NH)NH₂, W is a bond, X¹ is-NH-, Y¹ is a hydrogen atom, and n is 4.

Preferable compound (III) is shown below.

### [Compound (IIIA)]

Compound (III) wherein
R² is an optionally substituted C₁₋₂₄ hydrocarbon group (preferably, a C₁₋₂₄ alkyl group or a C₂₋₂₄ alkenyl group) ;
R^{3b} is (c) a hydrogen atom, (d) -C(=NH)R⁴ wherein R⁴ is an amino group, a C₁₋₁₆ alkyl-carbonylamino group or a C₁₋₄ alkyl group, or (e) a basic heterocyclic group (preferably, a pyridyl group);
W is a bond, -O-, -NH- or -CO-;
X¹ is (ii) -NR⁵- wherein R⁵ is a hydrogen atom or a methyl group or (iii) a bond;
Y¹ is a hydrogen atom or a methyl group; and
n is an integer of 0 - 6.

### [Compound (IIIB)]

Compound (III) wherein
R² is an optionally substituted C₆₋₂₁ hydrocarbon group (preferably, a C₆₋₂₁ alkyl group or a C₂₋₂₄ alkenyl group) ;
R^{3b} is (c) a hydrogen atom, (d) -C(=NH)R⁴ wherein R⁴ is an amino group, a C₁₋₁₂ alkyl-carbonylamino group or a C₁₋₄ alkyl group, or (e) a basic heterocyclic group (preferably, a pyridyl group);
W is a bond, -O-, -NH- or -CO-;
X¹ is (ii) -NR⁵- wherein R⁵ is a hydrogen atom or a methyl group or (iii) a bond;
Y¹ is a hydrogen atom or a methyl group; and
n is an integer of 2 - 6 (preferably, an integer of 4 - 6).

### [Compound (IIIC)]

Compound (III) wherein
R² is an optionally substituted C₆₋₂₁ hydrocarbon group (preferably, a C₆₋₂₁ alkyl group or a C₂₋₂₄ alkenyl group);
R^{3b} is (c) a hydrogen atom, or (d) -C(=NH)R⁴ wherein R⁴ is an amino group, a C₁₋₁₂ alkyl-carbonylamino group or a C₁₋₄ alkyl group (preferably, a methyl group);
W is a bond, -O-, -NH- or -CO-;
X¹ is (ii) -NR⁵- wherein R⁵ is a hydrogen atom or a methyl group;
Y¹ is a hydrogen atom or a methyl group; and
n is an integer of 2 - 6 (preferably, an integer of 4 - 6).

### [Compound (IIID)]

Compound (III) wherein
R² is an optionally substituted C₁₋₂₄ hydrocarbon group (preferably, a C₁₋₂₄ alkyl group or a C₂₋₂₄ alkenyl group) ;
R^{3b} is (e) a basic heterocyclic group (preferably, a pyridyl group);
W is a bond, -O-, -NH- or -CO-;
X¹ is (iii) a bond;
Y¹ is a hydrogen atom or a methyl group; and
n is an integer of 0 - 6.

A salt of compound (I) is not particularly limited as long as it is pharmacologically acceptable. Examples thereof include metal salt, ammonium salt, salts with organic bases, salts with inorganic acids, salts with organic acids and the like. Preferable examples of the metal salt include alkali metal salts such as sodium salt, potassium salt and the like; alkaline earth metal salts such as calcium salt, barium salt and the like; magnesium salt, aluminum salt and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine or the like. Preferable examples of the salt with inorganic acid include salts with hydrochloric acid, hydrobromic acid, nitric acid, sulfuric acid, phosphoric acid or the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid or the like. Of these, salt with hydrochloric acid, salt with acetic acid, and sodium salt are preferable from the aspect of practicability of pharmaceutical product.

Specific examples of preferable compound (I) or a salt thereof include
N-[(1S)-1-(dodecylcarbamoyl)-4-guanidino-butyl]hexadecanamide,
N-[(1S)-1-(dodecylcarbamoyl)-4-guanidino-butyl]octadecanamide,
N-[(1S)-1-(dodecylcarbamoyl)-4-guanidino-butyl]docosanamide,
N-[(1S)-3-methyl-1-(undecylcarbamoyl)butyl]docosanamide,
N-[(1S)-1-(dodecylcarbamoyl)-3-methyl-butyl]hexadecanamide,
N-[(1S)-2-(dodecylamino)-1-methyl-2-oxo-ethyl]docosanamide,
N-[2-(dodecylamino)-1,1-dimethyl-2-oxo-ethyl]docosanamide,
N-[3-(dodecylamino)-3-oxo-propyl]docosanamide,
N-[3-(dodecylamino)-2,2-dimethyl-3-oxo-propyl]docosanamide,
(2S)-N-dodecyl-5-guanidino-2-(hexadecylcarbamoylamino)pentanamide,
Hexadecyl N-[(1S)-1-(dodecylcarbamoyl)-4-guanidino-butyl]carbamate,
N-[(1S)-5-amino-1-(dodecylcarbamoyl)pentyl]hexadecanamide,
N-[(1S)-1-(dodecylcarbamoyl)pentyl]docosanamide,
1-docosanoyl-N-dodecyl-piperidine-4-carboxamide,
N-[(1S)-1-(diheptylcarbamoyl)-4-guanidino-butyl]docosanamide,
N-[(1S)-5-amino-1-(dodecylcarbamoyl)pentyl]docosanamide,
(E)-N-[(1S)-1-(dodecylcarbamoyl)-4-guanidino-butyl]octadeca-9-enamide,
N-[(1S)-4-guanidino-1-(heptylcarbamoyl)butyl]docosanamide,
N-[3-(dodecylamino)-3-oxo-pentyl]docosanamide,
N-[(1S)-4-amino-1-(dodecylcarbamoyl)butyl]docosanamide,
N-[(1S)-1-(dodecylcarbamoyl)-5-guanidino-pentyl]docosanamide,
N-[(1S)-5-acetamide-1-(dodecylcarbamoyl)pentyl]docosanamide,
(2S)-5-guanidino-2-(hexadecylcarbamoylamino)-N-hexyl-pentanamide,
Hexadecyl N-[(1S)-4-guanidino-1-(hexylcarbamoyl)butyl]carbamate,
N-[(1S)-5-guanidino-1-(hexylcarbamoyl)pentyl]docosanamide,
N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide,
(2S)-2-(dibenzylamino)-N-dodecyl-5-guanidino-pentanamide,
N-(5-guanidinopentyl)hexadecanamide,
N-(5-guanidinopentyl)docosanamide,
N-[(1S)-1-(dodecylcarbamoyl)-4-guanidino-butyl]-2-oxo-octanamide,
Hexadecyl N-(4-guanidinobutyl)carbamate,
N-[4-(ethanimidoylamino)butyl]hexadecanamide,
N-[(1S)-1-(dodecylcarbamoyl)-4-guanidino-butyl]icosanamide,
N-[(1S)-4-guanidino-1-(octylcarbamoyl)butyl]hexadecanamide,
N-[(1S)-4-guanidino-1-(octylcarbamoyl)butyl]octadecanamide,
N-[(1S)-4-guanidino-1-(octylcarbamoyl)butyl]icosanamide,
N-[(1S)-5-amino-1-(octylcarbamoyl)pentyl]octadecanamide,
N-[(1S)-5-amino-1-(dodecylcarbamoyl)pentyl]octadecanamide,
Octadecyl N-[(1S)-1-(dodecylcarbamoyl)-4-guanidino-butyl]carbamate,
Dodecyl N-[(1S)-1-(dodecylcarbamoyl)-4-guanidino-butyl] carbamate,
Hexadecyl N-[(1S)-5-amino-1-(octylcarbamoyl)pentyl]carbamate,
Hexadecyl N-[(1S)-5-amino-1-(dodecylcarbamoyl)pentyl]carbamate,
1-(4-guanidinobutyl)-3-hexadecyl-urea,
N-[(1R)-1-(dodecylcarbamoyl)-4-guanidino-butyl]octadecanamide,
(2R)-N-dodecyl-5-guanidino-2-(hexadecylcarbamoylamino)pentanamide,
N-[(1R)-5-amino-1-(dodecylcarbamoyl) pentyl] hexadecanamide,
N-[(1S)-1-(dodecylcarbamoyl)-3-methyl-butyl]octadecanamide,
N-[(1S)-3-methyl-1-(octylcarbamoyl)butyl]octadecanamide,
N-[(1S)-1-(dodecylcarbamoyl)-3-methyl-butyl]docosanamide,
N-[(1S)-3-methyl-1-(octylcarbamoyl)butyl]docosanamide,
N-[(1S)-3-methyl-1-(octylcarbamoyl)butyl]hexadecanamide,
(2S)-N-dodecyl-2-(hexadecylcarbamoylamino)-4-methyl-pentanamide,
(2S)-2-(hexadecylcarbamoylamino)-4-methyl-N-octyl-pentanamide,
Hexadecyl N-[(1S)-1-(dodecylcarbamoyl)-3-methyl-butyl] carbamate,
Hexadecyl N-[(1S)-3-methyl-1-(octylcarbamoyl)butyl]carbamate,
N-[3-(dodecylamino)-2,2-dimethyl-3-oxo-propyl]octadecanamide,
N-[(1S)-1-(dodecylcarbamoyl)-5-guanidino-pentyl]octadecanamide,
N-[(1S)-1-(hexylcarbamoyl)-5-guanidino-pentyl]octadecanamide,
(2S)-2-(diheptylamino)-N-dodecyl-5-guanidino-pentanamide,
N-[2-(4-pyridyl)ethyl]docosanamide,
(2S)-5-guanidino-2-(hexadecylcarbamoylamino)-N-octyl-pentanamide,
(2S)-N-dodecyl-5-guanidino-2-(octadecylcarbamoylamino)pentanamide,
(2S)-N-dodecyl-2-(dodecylcarbamoylamino)-5-guanidino-pentanamide,
(2S)-6-amino-2-(hexadecylcarbamoylamino)-N-octyl-hexanamide,
(2S)-6-amino-N-dodecyl-2-(hexadecylcarbamoylamino)hexanamide,
Hexadecyl N-[(1S)-4-guanidino-1-(octylcarbamoyl)butyl]carbamate,
and salts thereof and the like.

Of these, in view of superior immunostimulatory effect and lower allergy inducing activity,
N-[(1S)-1-(dodecylcarbamoyl)-4-guanidino-butyl]hexadecanamide salt (preferably, hydrochloride),
N-[(1S)-1-(dodecylcarbamoyl)-4-guanidino-butyl]octadecanamide salt (preferably, hydrochloride),
N-[(1S)-1-(dodecylcarbamoyl)-4-guanidino-butyl]docosanamide salt (preferably, hydrochloride),
N-[(1S)-3-methyl-1-(undecylcarbamoyl)butyl]docosanamide,
N-[(1S)-1-(dodecylcarbamoyl)-3-methyl-butyl]hexadecanamide,
N-[3-(dodecylamino)-2,2-dimethyl-3-oxo-propyl]docosanamide,
(2S)-N-dodecyl-5-guanidino-2-(hexadecylcarbamoylamino)pentanamide salt (preferably, hydrochloride),
Hexadecyl N-[(1S)-1-(dodecylcarbamoyl)-4-guanidino-butyl]carbamate salt (preferably, hydrochloride),
N-[(1S)-5-amino-1-(dodecylcarbamoyl)pentyl]hexadecanamide salt (preferably, hydrochloride),
(E)-N-[(1S)-1-(dodecylcarbamoyl)-4-guanidino-butyl]octadeca-9-enamide salt (preferably, hydrochloride),
(2S)-5-guanidino-2-(hexadecylcarbamoylamino)-N-hexyl-pentanamide salt (preferably, hydrochloride)
are preferable, and
N-[(1S)-1-(dodecylcarbamoyl)-4-guanidino-butyl]hexadecanamide salt (preferably, hydrochloride),
N-[(1S)-1-(dodecylcarbamoyl)-4-guanidino-butyl]octadecanamide salt (preferably, hydrochloride),
N-[(1S)-1-(dodecylcarbamoyl)-4-guanidino-butyl]docosanamide salt (preferably, hydrochloride),
(2S)-N-dodecyl-5-guanidino-2-(hexadecylcarbamoylamino)pentanamide salt (preferably, hydrochloride),
N-[(1S)-5-amino-1-(dodecylcarbamoyl)pentyl]hexadecanamide salt (preferably, hydrochloride) are particularly preferable.

In another embodiment, in view of superior immunostimulatory effect and lower allergy inducing activity,
N-[(1S)-1-(dodecylcarbamoyl)-4-guanidino-butyl]hexadecanamide salt (preferably, hydrochloride),
N-[(1S)-1-(dodecylcarbamoyl)-4-guanidino-butyl]octadecanamide salt (preferably, hydrochloride),
N-[(1S)-1-(dodecylcarbamoyl)-4-guanidino-butyl]docosanamide salt (preferably, hydrochloride),
N-[(1S)-3-methyl-1-(undecylcarbamoyl)butyl]docosanamide,
N-[(1S)-1-(dodecylcarbamoyl)-3-methyl-butyl]hexadecanamide,
N-[3-(dodecylamino)-2,2-dimethyl-3-oxo-propyl]docosanamide,
(2S)-N-dodecyl-5-guanidino-2-(hexadecylcarbamoylamino)pentanamide salt (preferably, hydrochloride),
Hexadecyl N-[(1S)-1-(dodecylcarbamoyl)-4-guanidino-butyl]carbamate salt (preferably, hydrochloride),
N-[(1S)-5-amino-1-(dodecylcarbamoyl)pentyl]hexadecanamide salt (preferably, hydrochloride),
(E)-N-[(1S)-1-(dodecylcarbamoyl)-4-guanidino-butyl]octadeca-9-enamide salt (preferably, hydrochloride),
(2S)-5-guanidino-2-(hexadecylcarbamoylamino)-N-hexyl-pentanamide salt (preferably, hydrochloride),
N-[(1S)-1-(dodecylcarbamoyl)-4-guanidino-butyl]icosanamide salt (preferably, hydrochloride),
N-[(1S)-4-guanidino-1-(octylcarbamoyl)butyl]octadecanamide salt (preferably, hydrochloride),
N-[(1S)-5-amino-1-(dodecylcarbamoyl)pentyl]octadecanamide salt (preferably, hydrochloride),
Octadecyl N-[(1S)-1-(dodecylcarbamoyl)-4-guanidino-butyl]carbamate salt (preferably, hydrochloride),
Hexadecyl N-[(1S)-5-amino-1-(octylcarbamoyl)pentyl]carbamate salt (preferably, hydrochloride),
Hexadecyl N-[(1S)-5-amino-1-(dodecylcarbamoyl)pentyl]carbamate salt (preferably, hydrochloride),
N-[(1R)-1-(dodecylcarbamoyl)-4-guanidino-butyl]octadecanamide salt (preferably, hydrochloride),
N-[(1S)-1-(dodecylcarbamoyl)-5-guanidino-pentyl]octadecanamide salt (preferably, hydrochloride),
N-[(1S)-1-(hexylcarbamoyl)-5-guanidino-pentyl]octadecanamide salt (preferably, hydrochloride),
(2S)-2-(diheptylamino)-N-dodecyl-5-guanidino-pentanamide salt (preferably, hydrochloride),
(2S)-5-guanidino-2-(hexadecylcarbamoylamino)-N-octyl-pentanamide salt (preferably, hydrochloride),
(2S)-N-dodecyl-5-guanidino-2-(octadecylcarbamoylamino)pentanamide salt (preferably, hydrochloride),
(2S)-N-dodecyl-2-(dodecylcarbamoylamino)-5-guanidino-pentanamide salt (preferably, hydrochloride),
(2S)-6-amino-2-(hexadecylcarbamoylamino)-N-octyl-hexanamide salt (preferably, hydrochloride),
(2S)-6-amino-N-dodecyl-2-(hexadecylcarbamoylamino)hexanamide salt (preferably, hydrochloride),
Hexadecyl N-[(1S)-4-guanidino-1-(octylcarbamoyl)butyl]carbamate salt (preferably, hydrochloride)
is preferable,
N-[(1S)-1-(dodecylcarbamoyl)-4-guanidino-butyl]hexadecanamide salt (preferably, hydrochloride),
N-[(1S)-1-(dodecylcarbamoyl)-4-guanidino-butyl]octadecanamide salt (preferably, hydrochloride),
N-[(1S)-1-(dodecylcarbamoyl)-4-guanidino-butyl]docosanamide salt (preferably, hydrochloride),
(2S)-N-dodecyl-5-guanidino-2-(hexadecylcarbamoylamino)pentanamide salt (preferably, hydrochloride),
N-[(1S)-5-amino-1-(dodecylcarbamoyl)pentyl]hexadecanamide salt (preferably, hydrochloride),
N-[(1S)-5-amino-1-(dodecylcarbamoyl)pentyl]octadecanamide salt (preferably, hydrochloride),
Hexadecyl N-[(1S)-5-amino-1-(dodecylcarbamoyl)pentyl]carbamate salt (preferably, hydrochloride),
N-[(1R)-1-(dodecylcarbamoyl)-4-guanidino-butyl]octadecanamide salt (preferably, hydrochloride),
(2S)-N-dodecyl-5-guanidino-2-(octadecylcarbamoylamino)pentanamide salt (preferably, hydrochloride),
(2S)-6-amino-2-(hexadecylcarbamoylamino)-N-octyl-hexanamide salt (preferably, hydrochloride)
are particularly preferable.

Of compounds (I) and salts thereof, at least a compound represented by the following formula (I'), the formula (I") or the formula (I"') and a salt thereof are novel compounds not reported as of the filing date of the present application.

The formula (I'): 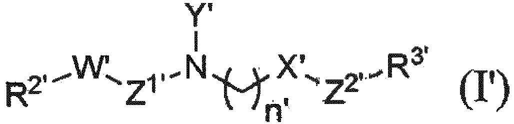 wherein
X' is a group represented by the following formula 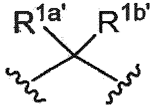 wherein
R^{1a}' is
(1) a guanidyl-C₃₋₄ alkyl group,
(2) a C₄ alkyl group,
(3) an amino-C₃₋₄ alkyl group optionally substituted by an acetyl group or an aminocarbonyl group, or
(4) a group represented by the following formula 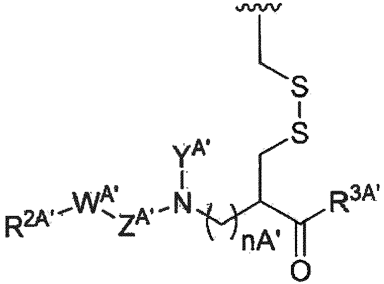 wherein
   R^{2A}' is a C₁₈₋₂₄ hydrocarbon group;
   R^{3A}' is a mono- or di-C₆₋₁₆ alkylamino group;
   W^{A}' is a bond;
   Y^{A}' is a hydrogen atom;
   Z^{A}' is -CO-;
   nA' is 0;
   R^{1b}' is a hydrogen atom;
   Y' is a hydrogen atom;
   in R²', R³' and W',
      (I') when R^{1a}' is the aforementioned (1),
         (I'-1) R²' is a C₁₈₋₂₄ hydrocarbon group, R³' is a mono- or di-C₇₋₁₆ alkylamino group and W' is a bond,
         (I'-2) R²' is a C₁₂₋₂₄ hydrocarbon group, R³' is a mono- or di-C₆₋₁₆ alkylamino group and W' is -O- or -NH-, or (I'-3) R²' is a C₅₋₂₄ hydrocarbon group, R³' is a mono- or di-C₆₋₁₆ alkylamino group and W' is -CO-,
      (II') when R^{1a}' is the aforementioned (2),
         (II'-1) R²' is a C₁₂₋₂₄ hydrocarbon group, R³' is a mono- or di-C₇₋₁₆ alkylamino group and W' is a bond (provided that a case in which R²' is a C₂₁ hydrocarbon group and R³' is a mono-C₈ alkylamino group is excluded),
         (II' -2) R²' is a C₁₂₋₂₄ hydrocarbon group, R³' is a mono- or di-C₆₋₁₆ alkylamino group and W' is -O- or -NH-, or
         (II'-3) R²' is a C₅₋₂₄ hydrocarbon group, R³' is a mono- or di-C₆₋₁₆ alkylamino group and W' is -CO-,
      (III') when R^{1a}' is the aforementioned (3),
         (III' -1) R²' is a C₁₂₋₂₄ alkyl group, R³' is a mono- or di-C₇₋₁₆ alkylamino group and W' is a bond,
         (III'-2) R²' is a C₁₂₋₂₄ hydrocarbon group, R³' is a mono- or di-C₆₋₁₆ alkylamino group and W' is -O- or -NH-, or
         (III'-3) R²' is a C₅₋₂₄ hydrocarbon group, R³' is a mono- or di-C₆₋₁₆ alkylamino group and W' is -CO-,
      (IV') when R^{1a}' is the aforementioned (4),
         (IV'-1) R²' is a C₁₈₋₂₄ hydrocarbon group, R³' is a mono- or di-C₇₋₁₆ alkylamino group and W' is a bond,
         (IV' -2) R²' is a C₁₂₋₂₄ hydrocarbon group, R³' is a mono- or di-C₆₋₁₆ alkylamino group and W' is -O- or -NH-, or
         (IV'-3) R²' is a C₅₋₂₄ hydrocarbon group, R³' is a mono- or di-C₆₋₁₆ alkylamino group and W' is -CO-;
            Z¹' and Z²' are each -CO-; and
            n' is 0.

The "C₁₈₋₂₄ hydrocarbon group", "C₁₂₋₂₄ hydrocarbon group", "C₅₋₂₄ hydrocarbon group" for R²' respectively mean the "C₁₋₂₄ hydrocarbon group" for the aforementioned R² wherein the carbon atom number is 18 - 24, 12 - 24, or 5 - 24.

The "C₁₂₋₂₄ alkyl group" for R²' means the "C₁₋₂₄ alkyl group" for the aforementioned R² having a carbon atom number of 12 - 24.

The "mono- or di-C₇₋₁₆ alkylamino group", "mono- or di-C₆₋₁₆ alkylamino group" for R³' respectively mean the "mono- or di-C₁₋₁₆ alkylamino group" for the aforementioned R³ wherein the amino group is mono- or di-substituted by a C₇₋₁₆ alkyl group or a C₆₋₁₆ alkyl group. The "C₇₋₁₆ alkyl group", "C₆₋₁₆ alkyl group" respectively mean the aforementioned "C₁₋₁₆ alkyl group" having a carbon atom number of 7 - 16 or 6 - 16.

The "guanidyl-C₃₋₄ alkyl group" for R^{1a}' mean the "guanidyl-C₁₋₆ alkyl group" for the aforementioned R^{1a} wherein a guanidyl group is bonded to an alkyl having a carbon atom number of 3 or 4.

The "amino-C₃₋₄ alkyl group" of the "amino-C₃₋₄ alkyl group optionally substituted by an acetyl group or an aminocarbonyl group" for R^{1a}' is the aforementioned "amino-C₁₋₆ alkyl group" for R^{1a} wherein an amino group is bonded to an alkyl having 3 or 4 carbon atoms.

The "C₁₈₋₂₄ hydrocarbon group" for R^{2A}' is the aforementioned "C₁₋₂₄ hydrocarbon group" for R² having 18 - 24 carbon atoms.

The "mono- or di-C₆₋₁₆ alkylamino group" for R^{3A}' means the aforementioned "mono- or di-C₁₋₁₆ alkylamino group" for R³, wherein the amino group is mono- or di-substituted by a C₆₋₁₆ alkyl group.

Specific examples of the compound represented by the formula (I') (hereinafter sometimes to be referred to as compound (I')) or a salt thereof include
N-[(1S)-1-(dodecylcarbamoyl)-4-guanidino-butyl]docosanamide,
N-[(1S)-3-methyl-1-(undecylcarbamoyl)butyl]docosanamide,
N-[(1S)-1-(dodecylcarbamoyl)-3-methyl-butyl]hexadecanamide,
(2S)-N-dodecyl-5-guanidino-2-(hexadecylcarbamoylamino)pentanamide,
Hexadecyl N-[(1S)-1-(dodecylcarbamoyl)-4-guanidino-butyl]carbamate,
N-[(1S)-5-amino-1-(dodecylcarbamoyl)pentyl]hexadecanamide,
N-[(1S)-1-(dodecylcarbamoyl)pentyl]docosanamide,
N-[(1S)-1-(diheptylcarbamoyl)-4-guanidino-butyl]docosanamide,
N-[(1S)-5-amino-1-(dodecylcarbamoyl)pentyl]docosanamide,
N-[(1S)-4-guanidino-1-(heptylcarbamoyl)butyl]docosanamide,
N-[(1S)-4-amino-1-(dodecylcarbamoyl)butyl]docosanamide,
N-[(1S)-1-(dodecylcarbamoyl)-5-guanidino-pentyl]docosanamide,
N-[(1S)-5-acetamide-1-(dodecylcarbamoyl)pentyl]docosanamide,
(2S)-5-guanidino-2-(hexadecylcarbamoylamino)-N-hexyl-pentanamide,
Hexadecyl N-[(1S)-4-guanidino-1-(hexylcarbamoyl)butyl]carbamate,
N-[(1S)-5-guanidino-1-(hexylcarbamoyl)pentyl]docosanamide,
N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide,
N-[(1S)-1-(dodecylcarbamoyl)-4-guanidino-butyl]-2-oxo-octanamide,
N-[(1S)-1-(dodecylcarbamoyl)-4-guanidino-butyl]icosanamide,
N-[(1S)-4-guanidino-1-(octylcarbamoyl)butyl]icosanamide,
N-[(1S)-5-amino-1-(octylcarbamoyl)pentyl]octadecanamide,
N-[(1S)-5-amino-1-(dodecylcarbamoyl)pentyl]octadecanamide,
Octadecyl N-[(1S)-1-(dodecylcarbamoyl)-4-guanidino-butyl]carbamate,
Dodecyl N-[(1S)-1-(dodecylcarbamoyl)-4-guanidino-butyl]carbamate,
Hexadecyl N-[(1S)-5-amino-1-(octylcarbamoyl)pentyl]carbamate,
Hexadecyl N-[(1S)-5-amino-1-(dodecylcarbamoyl)pentyl]carbamate,
(2R)-N-dodecyl-5-guanidino-2-(hexadecylcarbamoylamino)pentanamide,
N-[(1R)-5-amino-1-(dodecylcarbamoyl)pentyl]hexadecanamide,
N-[(1S)-1-(dodecylcarbamoyl)-3-methyl-butyl]octadecanamide,
N-[(1S)-3-methyl-1-(octylcarbamoyl)butyl]octadecanamide,
N-[(1S)-1-(dodecylcarbamoyl)-3-methyl-butyl]docosanamide,
N-[(1S)-3-methyl-1-(octylcarbamoyl)butyl]hexadecanamide,
(2S)-N-dodecyl-2-(hexadecylcarbamoylamino)-4-methyl-pentanamide,
(2S)-2-(hexadecylcarbamoylamino)-4-methyl-N-octyl-pentanamide,
Hexadecyl N-[(1S)-1-(dodecylcarbamoyl)-3-methyl-butyl]carbamate,
Hexadecyl N-[(1S)-3-methyl-1-(octylcarbamoyl)butyl]carbamate,
(2S)-5-guanidino-2-(hexadecylcarbamoylamino)-N-octyl-pentanamide,
(2S)-N-dodecyl-5-guanidino-2-(octadecylcarbamoylamino)pentanamide,
(2S)-N-dodecyl-2-(dodecylcarbamoylamino)-5-guanidino-pentanamide,
(2S)-6-amino-2-(hexadecylcarbamoylamino)-N-octyl-hexanamide,
(2S)-6-amino-N-dodecyl-2-(hexadecylcarbamoylamino)hexanamide,
Hexadecyl N-[(1S)-4-guanidino-1-(octylcarbamoyl)butyl]carbamate,
and salts thereof and the like.

N-[(1S)-1-(dodecylcarbamoyl)-4-guanidino-butyl]hexadecanamide,
N-[(1S)-1-(dodecylcarbamoyl)-4-guanidino-butyl]octadecanamide,
(E)-N-[(1S)-1-(dodecylcarbamoyl)-4-guanidino-butyl]octadeca-9-enamide,
N-[(1S)-4-guanidino-1-(octylcarbamoyl)butyl]hexadecanamide,
N-[(1S)-4-guanidino-1-(octylcarbamoyl)butyl]octadecanamide,
N-[(1R)-1-(dodecylcarbamoyl)-4-guanidino-butyl]octadecanamide,
N-[(1S)-3-methyl-1-(octylcarbamoyl)butyl]docosanamide,
N-[(1S)-1-(dodecylcarbamoyl)-5-guanidino-pentyl]octadecanamide,
N-[(1S)-1-(hexylcarbamoyl)-5-guanidino-pentyl]octadecanamide,
(2S)-2-(diheptylamino)-N-dodecyl-5-guanidino-pentanamide,
N-[2-(4-pyridyl)ethyl]docosanamide,
and salts thereof are also novel compounds.

The formula (I"): 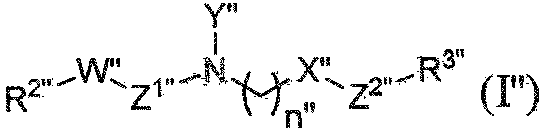 wherein
in R²", R³" and W",
(I"-1) R²" is a C₁₂₋₂₄ hydrocarbon group, R³" is a mono- or di-C₇₋₁₆ alkylamino group and W" is a bond,
(I"-2) R²" is a C₁₂₋₂₄ hydrocarbon group, R³" is a mono- or di-C₆₋₁₆ alkylamino group and W" is -O- or -NH-, or
(I"-3) R²" is a C₅₋₂₄ hydrocarbon group, R³" is a mono- or di-C₆₋₁₆ alkylamino group and W" is -CO-;
   X" is a group represented by the following formula 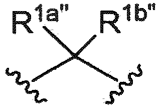
wherein
R^{1a}" is a hydrogen atom or a methyl group;
R^{1b}" is a hydrogen atom or a methyl group;
Y" is a hydrogen atom or a methyl group;
Z¹" and Z²" are each -CO-;
n" is an integer of 0 - 3;
when R^{1a}" is a methyl group, R^{1b}" is a hydrogen atom, Y" is a hydrogen atom and n" is 2, then R^{1a}" and Y" are optionally bonded to form a ring.

The "C₁₂₋₂₄ hydrocarbon group", "C₅₋₂₄ hydrocarbon group" for R²" respectively mean the aforementioned "C₁₋₂₄ hydrocarbon group" for R² having 12 - 24 carbon atoms or 5 - 24 carbon atoms. The "mono- or di-C₇₋₁₆ alkylamino group", "mono- or di-C₆₋₁₆ alkylamino group" for R³" mean the "mono- or di-C₁₋₁₆ alkylamino group" for the aforementioned R³ wherein the amino group is mono- or di-substituted by a C₇₋₁₆ alkyl group or a C₆₋₁₆ alkyl group. The "C₇₋₁₆ alkyl group", "C₆₋₁₆ alkyl group" respectively mean the aforementioned "C₁₋₁₆ alkyl group" having 7 - 16 carbon atoms or 6 - 16 carbon atoms.

Specific examples of compound represented by the formula (I") (hereinafter sometimes to be referred to as compound (I")) or a salt thereof include
N-[(1S)-2-(dodecylamino)-1-methyl-2-oxoethyl]docosanamide,
N-[2-(dodecylamino)-1,1-dimethyl-2-oxoethyl]docosanamide,
N-[3-(dodecylamino)-3-oxo-propyl]docosanamide,
N-[3-(dodecylamino)-2,2-dimethyl-3-oxo-propyl]docosanamide,
1-docosanoyl-N-dodecyl-piperidine-4-carboxamide,
N-[3-(dodecylamino)-3-oxo-pentyl]docosanamide,
N-[3-(dodecylamino)-2,2-dimethyl-3-oxo-propyl]octadecanamide,
and salts thereof and the like.

The formula (I"'): 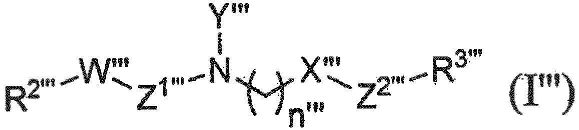 wherein
R³"' is -C(=NH)R⁴"' wherein R⁴"' is an amino group or a C₁₋₄ alkyl group;
in R²"' and W"',
(I"'-1) R²"' is a C₁₂₋₂₄ hydrocarbon group and W"' is a bond, -O-, or -NH- or
(I"'-2) R²"' is a C₅₋₂₄ hydrocarbon group and W"' is -CO-;
in X"', Y"' and n"',
(II"'-1) X"' is -NH-, Y"' is a hydrogen atom, n"', is 4 or 5 (provided that a case in which W"' is a bond, n"', is 4 and R⁴"' is an amino group is excluded), or
(II"'-2) X"' is -NR⁵"'- wherein R⁵"' is a methyl group, Y"' is a methyl group, n"', is 2 and R⁵"' and Y"' are bonded to form a ring;
   Z¹"' is -CO-; and
   Z²"' is a bond.

The "C₁₂₋₂₄ hydrocarbon group", "C₅₋₂₄ hydrocarbon group" for R²"' respectively mean the aforementioned "C₁₋₂₄ hydrocarbon group" for R² having 12 - 24 carbon atoms or 5 - 24 carbon atoms.

The "C₁₋₄ alkyl group" for R⁴"' is as defined for the aforementioned "C₁₋₄ alkyl group" for R⁴.

In X"', Y"' and n"', in the formula (I"'), as one embodiment,
(II"'-1') X"' is -NH-, Y"' is a hydrogen atom, n"', is 4 or 5 (provided that a case in which n"', is 4 and R⁴"' is an amino group is excluded), or
(II"'-2) X"' is -NR⁵"'- wherein R⁵"' is a methyl group, Y"' is a methyl group, n"'is 2 and R⁵"' and Y"' are bonded to form a ring.

Specific examples of the compound represented by the formula (I"') (hereinafter sometimes to be referred to as compound (I"')) or a salt thereof include
N-(5-guanidinopentyl)hexadecanamide,
N-(5-guanidinopentyl)docosanamide,
Hexadecyl N-(4-guanidinobutyl)carbamate,
N-[4-(ethanimidoylamino)butyl]hexadecanamide,
1-(4-guanidinobutyl)-3-hexadecyl-urea,
and salts thereof and the like.

(2S)-2-(dibenzylamino)-N-dodecyl-5-guanidino-pentanamide and salts thereof are also novel compounds.

### (Synthesis of compound of the present invention)

While the production methods of compound (I), compound (I'), compound (I"), compound (I'") and salts thereof (hereinafter sometimes to be generically referred to as the compound of the present invention) is not particularly limited, they can be produced by a known method or an appropriate combination of a method analogous thereto.

As the production method of the compound of the present invention, representative production methods (production methods 1 - 7) are described below by referring to compound (I) as an example. The production method of the compound of the present invention is not limited to these.

Compound (I) can be produced by the methods shown in the following production methods 1 - 7, the methods of the below-mentioned Examples, a method analogous thereto and the like. Compound (I'), compound (I") and compound (I"') can also be produced respectively by a method similar to the production method of compound (I). Each symbol in the formula (I) corresponds to each symbol in the formula (I'), the formula (I") and the formula (I"'). For example, the symbol "R²" in the formula (I) corresponds to the symbol "R²'" in the formula (I'), the symbol "R²"" in the formula (I") and the symbol "R²"'" in the formula (I"').

### <Production method 1>

Of compounds (I), a compound of the formula (II) wherein R^{1a} is "(1) a guanidyl-C₁₋₆ alkyl group" or "(3) an amino-C₁₋₆ alkyl group optionally substituted by an acetyl group or an aminocarbonyl group", R^{3a} is "(a) a mono- or di-C₁₋₁₆ alkylamino group optionally substituted by a hydroxyl group or a C₁₋₄ alkoxy group" and W is a bond can be produced by the following production method or a method analogous thereto. 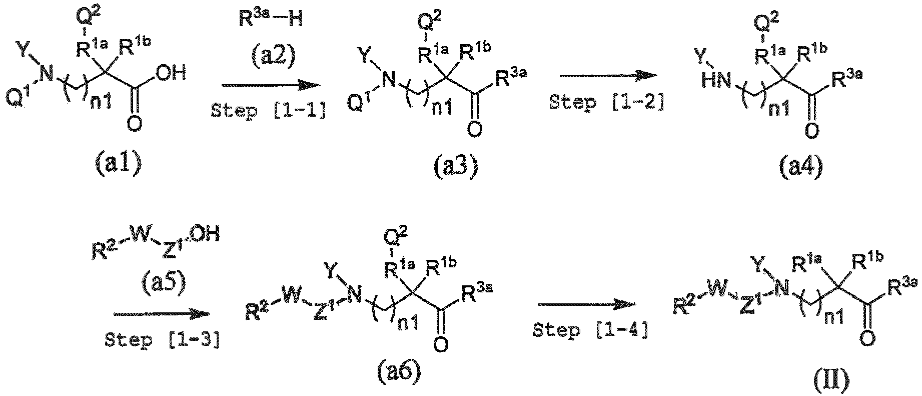 wherein Q¹ and Q² are the same or different and each is a protecting group, and other symbols are as defined above.

### Step [1-1]

In this step, compound (a1) and compound (a2) are reacted in the presence of a condensing agent to produce compound (a3).

The starting compound (a1) may be a commercially available product, or can be produced by a method known per se (e.g., the method described in "Etemad-Moghadam, Guita et al., European Journal of Medicinal Chemistry, 1988, 23(6), 577-585") or a method analogous thereto.

The starting compound (a2) may be a commercially available product, or can be produced by a method known per se (e.g., the method described in "Ayedi, Mohamed Ali et al., Synthetic Communications, 2013, 43(16), 2127-2133") or a method analogous thereto.

The amount of compound (a2) to be used is generally 1 - 10 equivalents, preferably 1 - 4 equivalents, relative to 1 equivalent of compound (a1).

Examples of the condensing agent include carbodiimides such as 1,3-dicyclohexylcarbodiimide, 1-cyclohexyl-3-morpholinoethylcarbodiimide, 1-cyclohexyl-3-(4-diethylaminocyclohexyl)carbodiimide, 1,3-diethylcarbodiimide, 1,3-diisopropylcarbodiimide, 1-ethyl-3-(3-dimethylaminopropyl)carbodiimide and the like or a salt thereof, uranium salts such as O-(7-azabenzotriazol-1-yl)-N,N,N',N'-tetramethyluronium, (1-cyano-2-ethoxy-2-oxoethylidenaminooxy)dimethylamino-morpholino-carbenium and the like, and the like.

The amount of the condensing agent to be used is generally 1 - 10 equivalents, preferably 1 - 4 equivalents, relative to 1 equivalent of compound (a1).

This reaction may be performed, when desired, in the presence of a base. Examples of the base include alkali metal hydroxides (e.g., lithium hydroxide, sodium hydroxide, potassium hydroxide etc.), alkaline earth metal hydroxides (e.g., magnesium hydroxide, calcium hydroxide etc.), alkali metal carbonates (e.g., sodium carbonate, potassium carbonate etc.), alkali metal hydrogen carbonates (e.g., sodium hydrogen carbonate, potassium hydrogen carbonate etc.), organic bases (e.g., trimethylamine, triethylamine, diisopropylethylamine, pyridine, 4-dimethylaminopyridine, picoline, N-methylpyrrolidine, N-methylmorpholine, N,N-dimethylaniline, 1,5-diazabicyclo[4.3.0]-5-nonene, 1,4-diazabicyclo[2.2.2]octane, 1,8-diazabicyclo[5.4.0]-7-undecene, tetramethylguanidine etc.), organic lithiums (e.g., methyllithium, n-butyllithium, sec-butyllithium, tert-butyllithium etc.), lithium amides (e.g., lithium diisopropylamide etc.) and the like.

The amount of the base to be used is generally 1 - 10 equivalents, preferably 1 - 4 equivalents, relative to 1 equivalent of compound (a1).

This reaction may be performed, when desired, in the presence of a condensation promoter. Examples of the condensation promoter include 1-hydroxybenzotriazole (HOBt), a hydrate thereof and the like.

The amount of the condensation promoter to be used is generally 0.01 - 10 equivalents, preferably 1 - 4 equivalents, relative to 1 equivalent of compound (a1).

In this reaction, a mixed acid anhydride of compound (a1) may also be used instead of compound (a1). The mixed acid anhydride can be obtained by, for example, reacting compound (a1) and chloroformic acid alkyl (e.g., methyl chloroformate, ethyl chloroformate, isobutyl chloroformate etc.) and the like in the presence of a base.

This reaction is preferably performed in a solvent inert to the reaction. While such solvent is not particularly limited as long as the reaction proceeds, examples thereof include ethers (e.g., 1,4-dioxane, tetrahydrofuran, diethyl ether, tert-butyl methyl ether, diisopropyl ether, ethylene glycol dimethyl ether etc.), esters (e.g., ethyl formate, ethyl acetate, butyl acetate etc.), halogenated hydrocarbons (e.g., dichloromethane, chloroform, carbon tetrachloride, trichloroethylene etc.), hydrocarbons (e.g., hexane, benzene, toluene etc.), amides (e.g., N,N-dimethylformamide, N,N-dimethylacetamide etc.), sulfoxides (e.g., dimethyl sulfoxide etc.) and the like. Two or more kinds of these solvents may be mixed and used at an appropriate ratio.

The reaction temperature is generally -80 - 150°C, preferably 5 - 80°C.

While the reaction time is not particularly limited, it is generally 0.5 - 48 hr, preferably 1 - 30 hr.

### Step [1-2]

In this step, protecting group Q¹ is removed from compound (a3) to produce compound (a4).

As the protecting group Q¹, those generally used in the peptide chemical and the like may be used. For example, one described in "Protective Groups in Organic Synthesis, 3rd Ed." (Theodora W. Greene, Peter G. M. Wuts) Wiley-Interscience, 1999 can be mentioned. Specifically, for example, an acyl group (e.g., formyl group, acetyl group, propionyl group, benzoyl group etc.), a C₁₋₆ alkyl-oxycarbonyl group (e.g., methoxycarbonyl group, ethoxycarbonyl group, tert-butoxycarbonyl group etc.), a C₆₋₁₄ aryl-oxycarbonyl group (e.g., phenoxycarbonyl group etc.), a C₇₋₁₀ aralkyl-carbonyl group (e.g., benzyloxycarbonyl group etc.), benzyl group, benzhydryl group, trityl group, phthaloyl group and the like can be mentioned. These protecting groups may have a substituent, and examples of the substituent include halogen atom (fluorine atom, chlorine atom, bromine atom, iodine atom and the like), a C₁₋₆ alkyl-carbonyl group (acetyl, propionyl, butylcarbonyl group and the like), nitro group and the like. The number of the substituents is generally 1 - 3.

The protecting group can be removed by a method known per se or, for example, the method described in "Protective Groups in Organic Synthesis, 3rd Ed." (Theodora W. Greene, Peter G. M. Wuts) Wiley-Interscience, 1999 and the like, or a method analogous thereto. For example, a method for treating with acid, base, reduction, ultraviolet rays, hydrazine, phenylhydrazine, sodium N-methyldithiocarbamate, tetrabutylammoniumfluoride, palladium acetate etc. can be utilized.

### Step [1-3]

In this step, compound (a4) and compound (a5) are reacted in the presence of a condensing agent to produce compound (a6).

Compound (a5) may be a commercially available product, or can be produced by a method known per se (e.g., the method described in "Jocelyn G. Millar, Allan C. Oehlschlager, John W. Wong, J. Org. Chem., 1983, 48 (23), 4404-4407") or a method analogous thereto.

The amount of compound (a5) to be used is generally 1 - 10 equivalents, preferably 1 - 4 equivalents, relative to 1 equivalent of compound (a4).

Examples of the condensing agent include those similar to the examples of usable condensing agent recited in Step [1-1].

The amount of the condensing agent to be used is generally 1 - 10 equivalents, preferably 1 - 4 equivalents, relative to 1 equivalent of compound (a4).

This reaction may be performed, when desired, in the presence of a base. Examples of the base include those similar to the examples of usable base recited in Step [1-1].

The amount of the base to be used is generally 1 - 10 equivalents, preferably 2 - 6 equivalents, relative to 1 equivalent of compound (a4).

This reaction may be performed in the presence of a condensation promoter when desired. Examples of the condensation promoter include those similar to the examples of usable condensation promoter recited in Step [1-1].

The amount of the condensation promoter to be used is generally 0.01 - 10 equivalents, preferably 1 - 4 equivalents, relative to 1 equivalent of compound (a4).

In this reaction, acid chloride of compound (a5) may also be used instead of compound (a5). The acid chloride may be a commercially available product, or can be obtained by, for example, reacting compound (a5) and oxalyl dichloride or thionyl chloride and the like by adding, as necessary, a catalytic amount of N,N-dimethylformamide.

In this reaction, a mixed acid anhydride of compound (a5) may also be used instead of compound (a5). The mixed acid anhydride can be obtained by a method similar to that for the mixed acid anhydride in Step [1-1].

This reaction is preferably performed in a solvent inert to the reaction. Examples of such solvent include those similar to the examples of usable solvent recited in Step [1-1]. Two or more kinds of solvents may be used in a mixture at an appropriate ratio.

The reaction temperature is generally -80 - 150°C, preferably 5 - 80°C.

While the reaction time is not particularly limited, it is generally 0.5 - 48 hr, preferably 1 - 30 hr.

### Step [1-4]

In this step, the protecting group Q² of compound (a6) is removed to produce compound (II).

Examples of the protecting group Q² include those similar to the examples recited as protecting group Q¹, and removal of the protecting group can also be performed by a similar method.

### <Production method 2>

Of compounds (I), a compound shown by the formula (II) wherein R^{1a} is "(2) a C₁₋₁₂ alkyl group", R^{3a} is "(a) a mono- or di-C₁₋₁₆ alkylamino group optionally substituted by a hydroxyl group or a C₁₋₄ alkoxy group" and W is a bond can be produced by the following production method or a method analogous thereto. 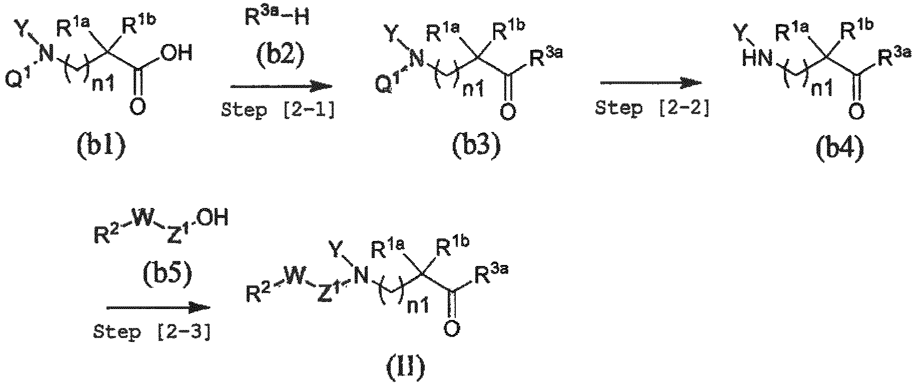 wherein Q¹ is a protecting group and other symbols are as defined above.

### Step [2-1]

In this step, compound (b1) and compound (b2) are reacted in the presence of a condensing agent to produce compound (b3).

The starting compound (b1) may be a commercially available product, or can be produced by a method known per se (e.g., the method described in "Sinha, Manish; Dola, Vasanth R.; Agarwal, Pooja; Srivastava, Kumkum; Haq, Wahajul; Puri, Sunil K.; Katti, Seturam B. Bioorganic & Medicinal Chemistry, 2014, 22(14), 3573-3586") or a method analogous thereto.

The starting compound (b2) may be a commercially available product, or can be produced by a method known per se (e.g., the method described in "Ayedi, Mohamed Ali et al., Synthetic Communications, 2013, 43(16), 2127-2133") or a method analogous thereto.

The amount of compound (b2) to be used is generally 1 - 10 equivalents, preferably 1 - 4 equivalents, relative to 1 equivalent of compound (b1).

Examples of the condensing agent include those similar to the examples of usable condensing agent recited in Production method 1, Step [1-1].

The amount of the condensing agent to be used is generally 1 - 10 equivalents, preferably 1 - 4 equivalents, relative to 1 equivalent of compound (b1).

This reaction may be performed, when desired, in the presence of a base. Examples of the base include those similar to the examples of usable base recited in Production method 1, Step [1-1].

The amount of the base to be used is generally 1 - 10 equivalents, preferably 1 - 4 equivalents, relative to 1 equivalent of compound (b1).

This reaction may be performed, when desired, in the presence of a condensation promoter. Examples of the condensation promoter include those similar to the examples of usable condensation promoter recited in Production method 1, Step [1-1].

The amount of the condensation promoter to be used is generally 0.01 - 10 equivalents, preferably 1 - 4 equivalents, relative to 1 equivalent of compound (b1).

In this reaction, moreover, mixed acid anhydride of compound (b1) may also be used instead of compound (b1). The mixed acid anhydride can be obtained by a method similar to that for the mixed acid anhydride in Production method 1, Step [1-1].

This reaction is preferably performed in a solvent inert to the reaction. Examples of such solvent include those similar to the examples of usable solvent recited in Production method 1, Step [1-1]. Two or more kinds of solvents may be used in a mixture at an appropriate ratio.

The reaction temperature is generally -80 - 150°C, preferably 5 - 80°C.

While the reaction time is not particularly limited, it is generally 0.5 - 48 hr, preferably 1 - 30 hr.

### Step [2-2]

In this step, protecting group Q¹ of compound (b3) is removed to produce compound (b4).

Examples of the protecting group Q¹ include those similar to the examples recited in the explanation of Production method 1, Step [1-2], and the protecting group can also be removed by a similar method.

### Step [2-3]

In this step, compound (b4) and compound (b5) are reacted in the presence of a condensing agent to produce compound (II).

The compound (b5) may be a commercially available product, or can be produced by a method known per se (e.g., the method described in "Jocelyn G. Millar, Allan C. Oehlschlager, John W. Wong, J. Org. Chem., 1983, 48 (23), 4404-4407") or a method analogous thereto.

The amount of compound (b5) to be used is generally 1 - 10 equivalents, preferably 1 - 4 equivalents, relative to 1 equivalent of compound (b4).

Examples of the condensing agent include those similar to the examples of usable condensing agent recited in Production method 1, Step [1-1].

The amount of the condensing agent to be used is generally 1 - 10 equivalents, preferably 1 - 4 equivalents, relative to 1 equivalent of compound (b4).

This reaction may be performed, when desired, in the presence of a base. Examples of the base include those similar to the examples of usable base recited in Production method 1, Step [1-1].

The amount of the base to be used is generally 1 - 10 equivalents, preferably 2 - 6 equivalents, relative to 1 equivalent of compound (b4).

This reaction may be performed, when desired, in the presence of a condensation promoter. Examples of the condensation promoter include those similar to the examples of usable condensation promoter recited in Production method 1, Step [1-1].

The amount of the condensation promoter to be used is generally 0.01 - 10 equivalents, preferably 1 - 4 equivalents, relative to 1 equivalent of compound (b4).

In this reaction, moreover, acid chloride of compound (b5) may also be used instead of compound (b5). The acid chloride can be obtained, for example, by a method similar to that for the acid chloride in Production method 1, Step [1-3].

In this reaction, mixed acid anhydride of compound (b5) may also be used instead of compound (b5). The mixed acid anhydride can be obtained, for example, by a method similar to that for the mixed acid anhydride in Production method 1, Step [1-1].

This reaction is preferably performed in a solvent inert to the reaction. Examples of such solvent include those similar to the examples of usable solvent recited in Production method 1, Step [1-3]. Two or more kinds of solvents may be used in a mixture at an appropriate ratio.

The reaction temperature is generally -80 - 150°C, preferably 5 - 80°C.

While the reaction time is not particularly limited, it is generally 0.5 - 48 hr, preferably 1 - 30 hr.

### <Production method 3>

Of compounds (I), a compound shown by the formula (II) wherein R^{1a} is "(1) a guanidyl-C₁₋₆ alkyl group" or "(3) an amino-C₁₋₆ alkyl group optionally substituted by an acetyl group or an aminocarbonyl group", R^{3a} is "(a) a mono- or di-C₁₋₁₆ alkylamino group optionally substituted by a hydroxyl group or a C₁₋₄ alkoxy group", W is -NH- and Z¹ is -CO- can be produced by the following production method or a method analogous thereto. 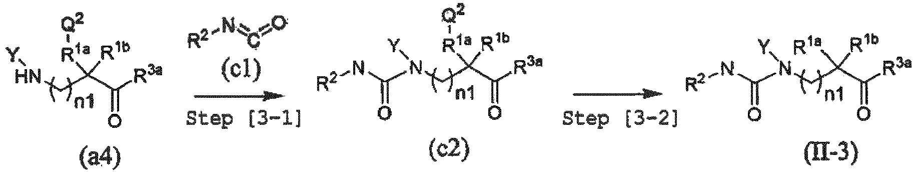 wherein Q² is a protecting group and other symbols are as defined above.

### Step [3-1]

In this step, compound (a4) and compound (c1) are reacted in the presence of a base to produce compound (c2).

The compound (a4) may be a commercially available product, or can be produced by Production method 1 or a method analogous thereto.

The starting compound (c1) may be a commercially available product, or can be produced by a method known per se (e.g., the method described in "Liu, Dazhi et al., Bioorganic & Medicinal Chemistry,2013, 21(11), 2960-2967") or a method analogous thereto.

The amount of compound (c1) to be used is generally 1 - 10 equivalents, preferably 1 - 4 equivalents, relative to 1 equivalent of compound (a4).

Examples of the base include those similar to the examples of usable base recited in Production method 1, Step [1-1].

The amount of the base to be used is generally 1 - 10 equivalents, preferably 2 - 6 equivalents, relative to 1 equivalent of compound (a4).

This reaction is preferably performed in a solvent inert to the reaction. Examples of such solvent include those similar to the examples of usable solvent recited in Production method 1, Step [1-3]. Two or more kinds of solvents may be used in a mixture at an appropriate ratio.

The reaction temperature is generally -80 - 150°C, preferably 0 - 50°C.

While the reaction time is not particularly limited, it is generally 0.5 - 48 hr, preferably 1 - 30 hr.

### Step [3-2]

In this step, protecting group Q² of compound (c2) is removed to produce compound (II-3).

Examples of the protecting group Q² include those similar to the examples recited in the explanation of Production method 1, Step [1-2], and the protecting group can also be removed by a similar method.

### <Production method 4>

Of compounds (I), a compound shown by the formula (II) wherein R^{1a} is "(1) a guanidyl-C₁₋₆ alkyl group" or "(3) an amino-C₁₋₆ alkyl group optionally substituted by an acetyl group or an aminocarbonyl group", R^{3a} is "(a) a mono- or di-C₁₋₁₆ alkylamino group optionally substituted by a hydroxyl group or a C₁₋₄ alkoxy group", W is -CO- and Z¹ is -O- can be produced by the following production method or a method analogous thereto. 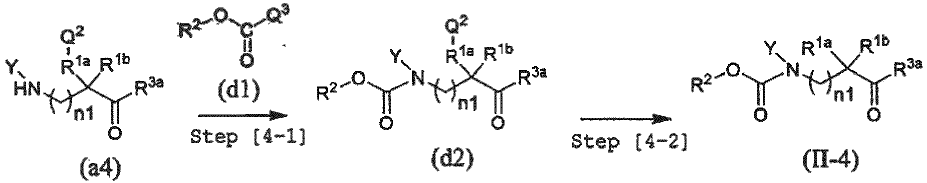 wherein Q² is a protecting group, Q³ is a leaving group, and other symbols are as defined above.

### Step [4-1]

In this step, compound (a4) and compound (d1) are reacted in the presence of a base to produce compound (d2).

Examples of the leaving group Q³ include halogen atom and the like.

The compound (a4) may be a commercially available product, or can be produced by Production method 1 or a method analogous thereto.

The starting compound (d1) may be a commercially available product, or a method known per se (e.g., the method described in "Mailyan, Artur K. et al., Organic Letters, 2016, 18(21), 5532-5535") or a method analogous thereto.

The amount of compound (d1) to be used is generally 1 - 10 equivalents, preferably 1 - 4 equivalents, relative to 1 equivalent of compound (a4).

Examples of the base include those similar to the examples of usable base recited in Production method 1, Step [1-1].

The amount of the base to be used is generally 1 - 10 equivalents, preferably 2 - 6 equivalents, relative to 1 equivalent of compound (a4).

This reaction is preferably performed in a solvent inert to the reaction. Examples of such solvent include those similar to the examples of usable solvent recited in Production method 1, Step [1-3]. Two or more kinds of solvents may be used in a mixture at an appropriate ratio.

The reaction temperature is generally -80 - 150°C, preferably 0 - 50°C.

While the reaction time is not particularly limited, it is generally 0.5 - 48 hr, preferably 1 - 30 hr.

### Step [4-2]

In this step, protecting group Q² of compound (d2) is removed to produce compound (II-4).

Examples of the protecting group Q² include those similar to the examples recited in the explanation of Production method 1, Step [1-2], and the protecting group can also be removed by a similar method.

### <Production method 5>

Of compounds (I), a compound shown by the formula (II) wherein R^{1a} is "(1) a guanidyl-C₁₋₆ alkyl group" or "(3) an amino-C₁₋₆ alkyl group optionally substituted by an acetyl group or an aminocarbonyl group", R^{3a} is "(a) a mono- or di-C₁₋₁₆ alkylamino group optionally substituted by a hydroxyl group or a C₁₋₄ alkoxy group", W is -CO- and Z¹ is -CO- can be produced by the following production method or a method analogous thereto. 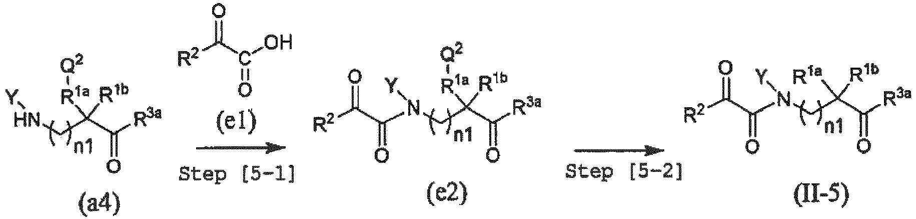 wherein Q² is a protecting group and other symbols are as defined above.

### Step [5-1]

In this step, compound (a4) and compound (e1) are reacted in the presence of a condensing agent to produce compound (e2).

The compound (a4) may be a commercially available product, or can be produced by Production method 1 or a method analogous thereto.

The starting compound (e1) may be a commercially available product, or can be produced by a method known per se (e.g., the method described in "Anatol, J. and Medete, A., Synthesis, 1971, 10, 538-539") or a method analogous thereto.

The amount of compound (e1) to be used is generally 1 - 10 equivalents, preferably 1 - 4 equivalents, relative to 1 equivalent of compound (a4).

Examples of the condensing agent include those similar to the examples of usable condensing agent recited in Production method 1, Step [1-1].

The amount of the condensing agent to be used is generally 1 - 10 equivalents, preferably 1 - 4 equivalents, relative to 1 equivalent of compound (a4).

This reaction may be performed, when desired, in the presence of a base. Examples of the base include those similar to the examples of usable base recited in Production method 1, Step [1-1].

The amount of the base to be used is generally 1 - 10 equivalents, preferably 2 - 6 equivalents, relative to 1 equivalent of compound (a4).

This reaction is preferably performed in a solvent inert to the reaction. Examples of such solvent include those similar to the examples of usable solvent recited in Production method 1, Step [1-3]. Two or more kinds of solvents may be used in a mixture at an appropriate ratio.

The reaction temperature is generally -80 - 150°C, preferably 5 - 80°C.

While the reaction time is not particularly limited, it is generally 0.5 - 48 hr, preferably 1 - 30 hr.

### Step [5-2]

In this step, protecting group Q² of compound (e2) is removed to produce compound (II-5).

Examples of the protecting group Q² include those similar to the examples recited in the explanation of Production method 1, Step [1-2], and the protecting group can also be removed by a similar method.

### <Production method 6>

Of compounds (I), a compound shown by the formula (II) wherein R^{1a} is the aforementioned group (4), R^{3a} is "(a) a mono- or di-C₁₋₁₆ alkylamino group optionally substituted by a hydroxyl group or a C₁₋₄ alkoxy group", W is a bond, and R² and R^{2A}, R^{3a} and R^{3A}, W and W^{A} and Z¹ and Z^{A} are respectively the same can be produced by the following production method or a method analogous thereto. 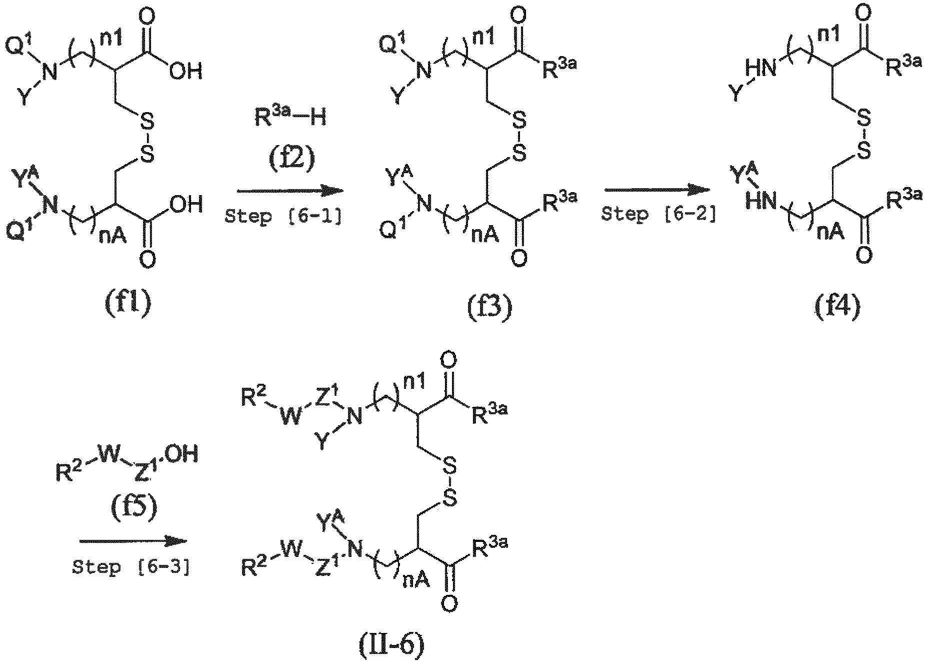 wherein Q¹ is a protecting group and other symbols are as defined above.

### Step [6-1]

In this step, compound (f1) and compound (f2) are reacted in the presence of a condensing agent to produce compound (f3).

The starting compound (f1) may be a commercially available product, or can be produced by a method known per se (e.g., the method described in "Agnihotri, Geetanjali et al., Journal of Medicinal Chemistry, 2011, 54(23), 8148-8160") or a method analogous thereto.

The starting compound (f2) may be a commercially available product, or can be produced by a method known per se (e.g., the method described in "Ayedi, Mohamed Ali et al., Synthetic Communications, 2013, 43(16), 2127-2133") or a method analogous thereto.

The amount of compound (f2) to be used is generally 1 - 10 equivalents, preferably 1 - 4 equivalents, relative to 1 equivalent of compound (f1).

Examples of the condensing agent include those similar to the examples of usable condensing agent recited in Production method 1, Step [1-1].

The amount of the condensing agent to be used is generally 1 - 10 equivalents, preferably 1 - 4 equivalents, relative to 1 equivalent of compound (f1).

This reaction may be performed, when desired, in the presence of a base. Examples of the base include those similar to the examples of usable base recited in Production method 1, Step [1-1].

The amount of the base to be used is generally 1 - 10 equivalents, preferably 1 - 4 equivalents, relative to 1 equivalent of compound (f1).

This reaction may be performed in the presence of a condensation promoter when desired. Examples of the condensation promoter include those similar to the examples of usable condensation promoter recited in Production method 1, Step [1-1].

The amount of the condensation promoter to be used is generally 0.01 - 10 equivalents, preferably 1 - 4 equivalents, relative to 1 equivalent of compound (f1).

This reaction is preferably performed in a solvent inert to the reaction. Examples of such solvent include those similar to the examples of usable solvent recited in Production method 1, Step [1-1]. Two or more kinds of solvents may be used in a mixture at an appropriate ratio.

The reaction temperature is generally -80 - 150°C, preferably 5 - 80°C.

While the reaction time is not particularly limited, it is generally 0.5 - 48 hr, preferably 1 - 30 hr.

### Step [6-2]

In this step, protecting group Q¹ of compound (f3) is removed to produce compound (f4).

Examples of the protecting group Q¹ include those similar to the examples recited in the explanation of Production method 1, Step [1-2], and the protecting group can also be removed by a similar method.

### Step [6-3]

In this step, compound (f4) and compound (f5) are reacted in the presence of a condensing agent to produce compound (II-6).

The compound (f5) may be a commercially available product, or can be produced by a method known per se (e.g., the method described in "Jocelyn G. Millar, Allan C. Oehlschlager, John W. Wong, J. Org. Chem., 1983, 48 (23), 4404-4407") or a method analogous thereto.

The amount of compound (f5) to be used is generally 1 - 10 equivalents, preferably 1 - 4 equivalents, relative to 1 equivalent of compound (f4).

Examples of the condensing agent include those similar to the examples of usable condensing agent recited in Production method 1, Step [1-1].

The amount of the condensing agent to be used is generally 1 - 10 equivalents, preferably 1 - 4 equivalents, relative to 1 equivalent of compound (f4).

This reaction may be performed, when desired, in the presence of a base. Examples of the base include those similar to the examples of usable base recited in Production method 1, Step [1-3].

The amount of the base to be used is generally 1 - 10 equivalents, preferably 2 - 6 equivalents, relative to 1 equivalent of compound (f4).

This reaction may be performed, when desired, in the presence of a condensation promoter. Examples of the condensation promoter include those similar to the examples of usable condensation promoter recited in Production method 1, Step [1-1].

The amount of the condensation promoter to be used is generally 0.01 - 10 equivalents, preferably 1 - 4 equivalents, relative to 1 equivalent of compound (f4).

In this reaction, moreover, acid chloride of compound (f5) may also be used instead of compound (f5). The acid chloride can be obtained, for example, by a method similar to that for the acid chloride in Production method 1, Step [1-3].

This reaction is preferably performed in a solvent inert to the reaction. Examples of such solvent include those similar to the examples of usable solvent recited in Production method 1, Step [1-3]. Two or more kinds of solvents may be used in a mixture at an appropriate ratio.

The reaction temperature is generally -80 - 150°C, preferably 5 - 80°C.

While the reaction time is not particularly limited, it is generally 0.5 - 48 hr, preferably 1 - 30 hr.

### <Production method 7>

Of compounds (I), a compound shown by the formula (III) wherein R^{3b} is "(d) -C(=NH)R⁴ wherein R⁴ is as defined above)" and W is a bond or -O- can be produced by the following production method or a method analogous thereto. 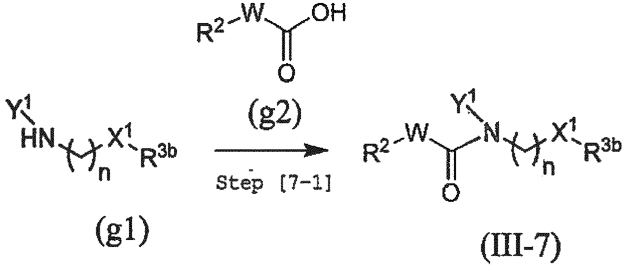 wherein each symbol is as defined above.

### Step [7-1]

In this step, compound (g1) and compound (g2) are reacted in the presence of a base to produce, compound (III-7).

The starting compound (g1) may be a commercially available product, or can be produced by a method known per se (e.g., the method described in "Beria, Italo et al., Journal of Medicinal Chemistry, 2004, 47(10), 2611-2623") or a method analogous thereto.

The starting compound (g2) may be a commercially available product, or can be produced by a method known per se (e.g., the method described in "Jocelyn G. Millar, Allan C. Oehlschlager, John W. Wong, J. Org. Chem., 1983, 48 (23), 4404-4407") or a method analogous thereto.

The amount of compound (g2) to be used is generally 0.5 - 5 equivalents, preferably 0.8 - 3 equivalents, relative to 1 equivalent of compound (g1).

Examples of the base include those similar to the examples of usable base recited in Production method 1, Step [1-1].

The amount of the base to be used is generally 1 - 10 equivalents, preferably 1 - 4 equivalents, relative to 1 equivalent of compound (g1).

This reaction may be performed in the presence of a condensing agent when desired.

Examples of the condensing agent include those similar to the examples of usable condensing agent recited in Production method 1, Step [1-1].

The amount of the condensing agent to be used is generally 1 - 10 equivalents, preferably 1 - 4 equivalents, relative to 1 equivalent of compound (g1).

This reaction may be performed, when desired, in the presence of a condensation promoter.

Examples of the condensation promoter include those similar to the examples of usable condensation promoter recited in Production method 1, Step [1-1].

The amount of the condensation promoter to be used is generally 0.01 - 10 equivalents, preferably 1 - 4 equivalents, relative to 1 equivalent of compound (g1).

In this reaction, moreover, acid chloride of compound (g2) may also be used instead of compound (g2). The acid chloride can be obtained, for example, by a method similar to that for the acid chloride in Production method 1, Step [1-3].

This reaction is preferably performed in a solvent inert to the reaction. Examples of such solvent include those similar to the examples of usable solvent recited in Production method 1, Step [1-3]. Two or more kinds of solvents may be used in a mixture at an appropriate ratio. This reaction can also be performed in the presence of a base such as hydroxylated alkali metals (e.g., lithium hydroxide, sodium hydroxide, potassium hydroxide etc.), hydroxylated alkaline earth metals (e.g., magnesium hydroxide, calcium hydroxide etc.), alkali metal carbonates (e.g., sodium carbonate, potassium carbonate etc.), alkali metal hydrogencarbonates (e.g., sodium hydrogen carbonate, potassium hydrogen carbonate etc.) in water, or a mixed solvent of water and a water-soluble organic solvent (e.g., alcohols such as isopropanol, tert-butanol and the like, acetone, tetrahydrofuran, 1,4-dioxane and the like).

The reaction temperature is generally -80 - 150°C, preferably 5 - 50°C.

While the reaction time is not particularly limited, it is generally 0.5 - 48 hr, preferably 1 - 30 hr.

When the compound of the present invention produced by the above-mentioned method is a free form, it can be converted to a salt with, for example, inorganic acid, organic acid, inorganic base, organic base or the like according to a conventional method; when the compound of the present invention is a salt form, it can also be converted to a free form or other salt according to a conventional method.

The compound of the present invention produced by a method such as the above can be isolated and purified by, for example, general separation means such as column chromatography, recrystallization, solvent washing and the like.

When the compound of the present invention contains an optical isomer, a stereoisomer, a regio isomer or a rotamer, these are also included as the compound of the present invention, and each can be obtained as a single product by a synthesis method and a separation method known per se (concentration, solvent extraction, column chromatography, recrystallization, solvent washing etc.). For example, when an optical isomer is present in the compound of the present invention, an optical isomer resolved from the compound is also encompassed in the compound of the present invention.

An optical isomer of the compound of the present invention can be produced by a method known per se. Specifically, an optical isomer is obtained by using an optically active synthetic intermediate, or optical resolution of the final product racemate according to a conventional method.

The compound of the present invention may be a crystal, and is encompassed in the compound of the present invention whether the crystal form is single or a crystal mixture. A crystal can be produced by crystallization by applying a crystallization method known per se.

The compound of the present invention may be any of a hydrate, a non-hydrate, a solvate and a non-solvate.

Compound (I) labeled with an isotope (e.g., ²H, ³H, ¹³C, ¹⁴C, ¹⁵N, ³⁵S) and the like is also encompassed in the compound of the present invention.

Since the compound of the present invention has an antigen-specific IgG1 subclass antibody production-enhancing effect (immunostimulatory effect), it is useful as an immunostimulating agent. Also, it can enhance production of an antigen-specific IgG2a subclass antibody. The immunostimulating agent of the present invention may be the compound of the present invention per se, or may be obtained by formulating the compound of the present invention by using a pharmacologically acceptable carrier and the like.

As a pharmacologically acceptable carrier that the immunostimulating agent of the present invention may contain, various conventional organic or inorganic carrier substances are used as preparation materials, which are added as excipient, lubricant, binder or disintegrant in solid preparations; solvent, solubilizing agent, suspending agent, isotonicity agent, buffering agent or soothing agent in liquid preparations, and the like. Where necessary, preparation additives such as preservative, antioxidant, colorant, sweetening agent and the like can also be used.

The immunostimulating agent of the present invention may further contain α-cyclodextrin in addition to the compound of the present invention.

In the present invention, α-cyclodextrin refers to cyclic oligosaccharide wherein six D-glucoses form a cyclic structure with α1→4 bond.

The α-cyclodextrin used in the present invention may be in the form of a derivative. While such derivative is not particularly limited as long as it has the skeleton of α-cyclodextrin, examples thereof include derivatives wherein α-cyclodextrin is chemically modified by methylation and the like or enzymatically modified by maltosylation and the like, and the like.

While α-cyclodextrin used in the present invention can be produced by, for example, enzymatically converting starch by cyclodextrin glucanotransferase, and the like, the production method is not limited thereto and it may be produced by a method known per se. In addition, a commercially available product may be used, and it is convenient and preferable.

While the weight ratio of the compound of the present invention and α-cyclodextrin (compound of the present invention : α-cyclodextrin) in the immunostimulating agent of the present invention is not particularly limited, 1:0.0002 - 2.0000 is preferable, 1:0.002 - 0.2 is more preferable.

The immunostimulating agent of the present invention may further contain hydroxypropyl-β-cyclodextrin in addition to the compound of the present invention.

In the present invention, hydroxypropyl-β-cyclodextrin refers to β-cyclodextrin, which is a cyclic oligosaccharide wherein seven D-glucoses form a cyclic structure by α1→4 bond, wherein at least one hydroxyl group is substituted by a hydroxypropyl group, and particularly, 2-hydroxypropyl-β-cyclodextrin wherein the above-mentioned hydroxypropyl group is a 2-hydroxypropyl group is preferable.

Hydroxypropyl-β-cyclodextrin to be used in the present invention is not particularly limited as long as it has the skeleton of β-cyclodextrin, and has at least one hydroxypropyl group in the side chain, and may be subjected to, for example, chemical modification such as methylation and the like, enzyme modification such as maltosylation and the like, and the like.

Hydroxypropyl-β-cyclodextrin to be used in the present invention can also be produced by, for example, reacting β-cyclodextrin with propylene oxide under alkali conditions and the like, though the method is not limited thereto, and can be produced by a method known per se. In addition, a commercially available product may be used, since it is convenient and preferable.

While the weight ratio of the compound of the present invention and hydroxypropyl-β-cyclodextrin (compound of the present invention : hydroxypropyl-β-cyclodextrin) in the immunostimulating agent of the present invention is not particularly limited as long as the compound of the present invention can be in a dissolution state, 1:0.0002 - 2.0000 is preferable, and 1:0.004 - 0.4 is more preferable.

The immunostimulating agent of the present invention may further contain at least one kind selected from the group consisting of carboxymethylcellulose, polysorbate (e.g., Tween80 (registered trade mark) etc.), polyethylene glycol (e.g., macrogol etc.), PBS (Phosphate buffered saline), PBS-EDTA (Phosphate buffered saline with ethylendiaminetetraacetic acid), TG (Tris-Glycine buffer), SDS (sodium dodecyl sulfate), TBS (Tris buffered saline), TBS-T (Tris buffered saline with Tween20), TAE (Tris-Acetate-EDTA buffer), TBE (Tris-Borate-EDTA buffer) and SSC (Saline sodium citrate buffer) in addition to the compound of the present invention.

While the weight ratio of the compound of the present invention and carboxymethyl cellulose (compound of the present invention : carboxymethyl cellulose) in the immunostimulating agent of the present invention is not particularly limited, 1:0.0002 - 2.0000 is preferable, and 1:0.002 - 0.2 is more preferable.

In another embodiment, the weight ratio of the compound of the present invention and carboxymethyl cellulose (compound of the present invention : carboxymethyl cellulose) in the immunostimulating agent of the present invention is preferably 1:0.0005 - 5.0000, more preferably 1:0.005 - 0.5.

While the weight ratio of the compound of the present invention and polysorbate (compound of the present invention : polysorbate) in the immunostimulating agent of the present invention is not particularly limited, 1:0.0002 - 2.0000 is preferable, and 1:0.002 - 0.2 is more preferable.

In another embodiment, the weight ratio of the compound of the present invention and polysorbate (compound of the present invention : polysorbate) in the immunostimulating agent of the present invention is preferably 1:0.0005 - 5.0000, more preferably 1:0.005 - 0.5.

While the weight ratio of the compound of the present invention and polyethylene glycol (compound of the present invention : polyethylene glycol) in the immunostimulating agent of the present invention is not particularly limited, 1:0.0002 - 2.0000 is preferable, and 1:0.002 - 0.2 is more preferable.

In another embodiment, the weight ratio of the compound of the present invention and polyethylene glycol (compound of the present invention : polyethylene glycol) in the immunostimulating agent of the present invention is preferably 1:0.0005 - 5.0000, more preferably 1:0.005 - 0.5.

Examples of the dosage form of the immunostimulating agent of the present invention include oral preparations such as tablet (including sugar-coated tablet, film-coated tablet, sublingual tablet, orally disintegrating tablet), capsule (including soft capsule, microcapsule), granule, powder, troche, syrup, emulsion, suspension and the like; and parenteral agents such as injection (e.g., subcutaneous injection, intravenous injection, intramuscular injection, intraperitoneal injection, drip infusion), external preparation (e.g., dermal preparation, ointment), suppository (e.g., rectal suppository, vaginal suppository), pellet, drip infusion, eye drop, pulmonary preparation (inhalant) and the like. These preparations may be controlled-release preparations (e.g., sustained-release microcapsule) such as immediate-release preparation, sustained-release preparation and the like.

When the immunostimulating agent of the present invention is an oral preparation, coating may be performed where necessary, aiming at masking taste, enteric property or sustainability. Examples of the coating base to be used for coating include various known coating bases.

The immunostimulating agent of the present invention can be produced by a method used conventionally in the technical field of preparation formulation, for example, the method described in the Japanese Pharmacopoeia, 16th Edition and the like.

The immunostimulating agent of the present invention can be processed into a preparation for children, in addition to that for adults.

The subject of administration of the immunostimulating agent of the present invention is not particularly limited as long as it is an animal having an immune system. Examples thereof include mammals (e.g., human, mouse, rat, rabbit, dog, cat, bovine, horse, swine, monkey etc.), birds (e.g., chicken, duck, goose etc.), fishes (e.g., Japanese trout, yellowtail, flounder etc.) and the like. The immunostimulating agent of the present invention can be safely administered orally or parenterally (e.g., topical, rectal, intravenous administration, immersion etc.) to them.

Since the compound of the present invention has a superior adjuvant activity as shown in the below-mentioned Examples, the immunostimulating agent of the present invention is useful as an adjuvant (particularly vaccine adjuvant).

The "adjuvant" in the present invention is a generic term for substances that increase antibody production and enhance immune response when combined with an antigen.

When the immunostimulating agent of the present invention is used as an adjuvant, the dosage form thereof may be, for example, an aqueous or a non-aqueous (e.g., oily etc.) solution, suspension, emulsion and the like. These can be prepared by mixing the compound of the present invention with a pharmacologically acceptable carrier (e.g., solvent, suspending agent etc.) and performing a method such as manual shaking, mechanical shaking, ultrasonic dispersing, dispersing by a homomixer, self emulsification, membrane emulsification, D-phase emulsification method, vacuum emulsification method, ultra-high pressure emulsification method and the like.

The immunostimulating agent of the present invention may be used in combination with other adjuvant. Examples of other adjuvant include Freund's Incomplete Adjuvant, Freund's Complete Adjuvant, Montanide ISA, particulates (e.g., urate crystals, silica, aluminum hydroxide gel (e.g., Alum etc.), polystyrene, asbestos, titanium oxide, black nickel oxide, hydroxyapatite etc.), TLR (Toll-like receptor) agonist (e.g., TLR 1/TLR 2 agonist (e.g., Pam3CSK4 etc.), TLR 2/TLR 6 agonist (e.g., MALP-2 etc.), TLR 3 agonist (e.g., polyinosinic polycytidylic acid (Poly I:C) etc.), TLR 4 agonist (e.g., lipopolysaccharide (LPS), monophosphoryl lipid (MPL) etc.), TLR 5 agonist (e.g., flagellin etc.), TLR 7/TLR 8 agonist (e.g., Imiquimod (R-837), SMIP-7,8, Resiquimod (R-848) etc.), TLR 9 agonist (e.g., sizofiran-CpG complex, CpG-ODNs etc.), TLR 11 agonist (e.g., profilin etc.), TLR agonist other than the above (e.g., BCG-CWS, OK-432, IC31, 1018ISS etc.)), inulin (e.g. Advax™), cholera toxin B subunit (CTB), ricin, chitosan, saponin (e.g., QS-21 etc.), squalene (e.g., MF59 (AddaVax™) etc.), rapamycin, α-GalCer, lipopeptide (e.g., Pam2CSK4, Macrophage-activating lipopeptide 2 etc.), long-chain peptide (e.g., NY-ESO-1 etc.), deoxycholic acid, liposome (e.g., deoxycholic acid-contained liposome, phospholipid-contained liposome etc.), nanoparticle (e.g., γ-PGA nanoparticle, polylactic acid nanoparticle, polystyrene nanoparticle etc.), carbomer homopolymer, ISCOM, biopolymer, β-cyclodextrin, γ-cyclodextrin, surfactant (e.g., benzalkonium-type cationic surfactant, sulfonic acid-type anionic surfactant, saccharide-type non-ionic surfactant, sulfobetaine-type amphoteric surfactant, lung surfactant, surfactin, CAF01 etc.), lipid (e.g., saturated fatty acid, unsaturated fatty acid, cationic lipid, anionic lipid, phospholipid, sphingolipid, lecithin etc.), citrulline, nucleic acid (e.g., ssDNA, dsDNA, ssRNA, dsRNA etc.), c-GMP-AMP, VLP (virus-like particle) (e.g., alphavirus etc.), Mycobacterium tuberculosis adjuvant, acid-fast bacteria-secreted antigen (e.g., Ag85B etc.), probiotic lactobacillus (e.g., lactobacillus plantarum, lactobacillus casei, lactobacillus lactis etc.), cytokine (e.g., interleukin-1, interleukin-2, interleukin-7, interleukin-12, interleukin-15, interleukin-18, TNF-α, GM-CSF, INF-α etc.), CLR (C-type lectin receptor) agonist (e.g., β-glucan, Man9GlcNAc2, TDM, Filamentous actin etc.), cGAS/STING agonist (e.g., cGAMP, cdiGMP, cdiAMP, DMXAA etc.), RIG-1 receptor agonist (e.g., 5-PPP ssRNA etc.), NLR (Nucleotide binding oligomerization domain)-like receptor agonist (e.g., peptidoglycan, KF156, FK565, Murabutide etc.), IRF3 agonist, CD22 agonist and the like.

The immunostimulating agent of the present invention is preferably used in combination with at least one kind selected from the group consisting of aluminum hydroxide gel (e.g., Alum etc.), TLR (Toll-like receptor) agonist (e.g., TLR 1/TLR 2 agonist (e.g., Pam3CSK4 etc.), TLR 2/TLR 6 agonist (e.g., MALP-2 etc.), TLR 3 agonist (e.g., polyinosinic polycytidylic acid (Poly I:C) etc.), TLR 4 agonist (e.g., lipopolysaccharide (LPS), monophosphoryl lipid (MPL) etc.), TLR 5 agonist (e.g., flagellin etc.), TLR 7/TLR 8 agonist (e.g., Imiquimod(R-837), Resiquimod(R-848) etc.), TLR 9 agonist (e.g., sizofiran-CpG complex, CpG-ODNs etc.), TLR 11 agonist (e.g., profilin etc.), TLR agonist other than the above (e.g., BCG-CWS, OK-432, IC31, 1018ISS etc.)), saponin (e.g., QS-21 etc.), squalene (e.g., MF59(AddaVax™) etc.), α-GalCer, lipopeptide (e.g., Pam2CSK4, Macrophage-activating lipopeptide 2 etc.), liposome, nucleic acid (e.g., ssDNA, dsDNA, ssRNA, dsRNA etc.), cGAS/STING agonist (e.g., cGAMP, cdiGMP, cdiAMP, DMXAA etc.), RIG-1 receptor agonist (e.g., 5-PPP ssRNA etc.) and NLR (Nucleotide binding oligomerization domain)-like receptor agonist (e.g., peptidoglycan, KF156, FK565, Murabutide etc.). Of these, a combined use with at least one kind selected from the group consisting of polyinosinic polycytidylic acid (PolyI:C), TLR (Toll-like receptor) agonist, monophosphoryl lipid (MPL), CpG-ODNs, nucleic acid (e.g., ssDNA, dsDNA, ssRNA, dsRNA etc.), cGAS/STING agonist, RIG-1 receptor agonist and NLR-like receptor agonist is more preferable.

The present invention also provides a vaccine containing the compound of the present invention and an antigen. A vaccine containing the compound of the present invention and an antigen is one embodiment of a pharmaceutical composition containing the compound of the present invention and an antigen.

The compound of the present invention to be contained in the vaccine of the present invention may be one similar to the compound of the present invention contained in the immunostimulating agent of the present invention.

The antigen to be used in the present invention is not particularly limited as long as it is a substance capable of inducing an immune reaction, and examples thereof include allergen, pathogen antigen, self antigen in the living body, tumor antigen and the like.

The allergen to be used in the present invention can be pollen allergen, food allergen or house dust allergen. The pollen allergen is not particularly limited, and examples thereof include cedar pollen allergen, Japanese cypress pollen allergen, ragweed allergen, Dactylis glomerata allergen and the like. The food allergen is not particularly limited, and examples thereof include casein, lactalbumin, lactoglobulin, ovomucoid, ovalbumin, conalbumin and the like. The house dust allergen is not particularly limited, and examples thereof include mites allergen, cat allergen and the like.

The pathogen antigen to be used in the present invention can be pathogenic virus antigen, pathogenic microorganism antigen or pathogenic protozoan antigen. The pathogenic virus antigen is not particularly limited, and examples thereof include antigen of virus such as human immunodeficiency virus (HIV), hepatitis virus (e.g., type A, type B, type C, type D and type E hepatitis virus etc.), influenza virus (e.g., type A, type B and type C, influenza virus, for example, antigen described in "Surveillance Report Influenza virus characterization, Summary Europe, September 2015" (http://ecdc.europa.eu/en/publications/surveillance_reports/inf luenza/pages/influenza_virus_characterisation.aspx) etc.), simple herpes virus, West Nile fever virus, human papilloma virus, horse encephalitis virus, human T cell leukemia virus (e.g., HTLV-I etc.), polio virus, varicella-zoster virus, mumps virus, rotavirus, norovirus, RS virus, measles virus, ebola virus and the like, and the antigen of influenza virus is particularly preferably used. The pathogenic microorganism antigen is not particularly limited, and examples thereof include antigens expressed in pathogenic bacterium (e.g., Haemophilus influenzae type B (Hib), pneumococcus, clostridium tetani, corynebacterium diphtheriae, bordetella pertussis, cholera, salmonella, bacillus typhosus, chlamydiae, mycobacteria, legionella etc.), pathogenic yeast (e.g., Aspergillus, Candida etc.) or the like. The pathogenic protozoan antigen is not particularly limited, and examples thereof include antigens expressed in malaria, schistosome or the like.

The self antigen in the living body, which is to be used in the present invention, is not particularly limited, and examples thereof include amyloid β, prion in neurological diseases such as Alzheimer's disease, Creutzfeldt-Jakob disease and the like; ApoB100, angiotensin I, angiotensin II in circulatory diseases such as arteriosclerosis, hypertension and the like; insulin, IL-5 in autoimmune/allergic diseases such as Type I diabetes mellitus, bronchial asthma and the like; IL-6, TNF-α in rheumatoid arthritis, and the like.

The tumor antigen to be used in the present invention can be an antigen of a solid tumor such as epithelial and non-epithelial tumors or an antigen of a tumor in hematopoietic tissue. The solid tumor antigen is not particularly limited, and examples thereof include MART-1/Melan-A, Mage-1, Mage-3, gp100, tyrosinase, tyrosinase-related protein 2 (trp2), CEA, PSA, CA-125, erb-2, Muc-1, Muc-2, TAG-72, AES, FBP, C-lectin, NY-ESO-1, galectin-4/NY-CO-27, Pec60, HER-2/erbB-2/neu, telomerase, G250, Hsp105, point mutated ras oncogene, point mutated p53 oncogene, carcinoembryonic antigen and the like. The antigen of a tumor (e.g., leukemia etc.) in hematopoietic tissue is not particularly limited, and examples thereof include proteinase 3, WT-1, hTERT, PRAME, PML/RAR-a, DEK/CAN, cyclophilin B, TEL-MAL1, BCR-ABL, OFA-iLRP, Survivin, idiotype, Sperm protein 17, SPAN-Xb, CT-27, MUC1 and the like.

The content of the antigen in the vaccine of the present invention may be an effective amount that functions as a vaccine, and the amount can be determined by those of ordinary skill in the art based on, for example, tests using an experiment animal and the like, without requiring undue experiments. Specifically, the content of the antigen in the vaccine of the present invention is generally 1 - 100 µg, based on the total weight of the vaccine.

While the content of the compound of the present invention in the vaccine of the present invention is not particularly limited and may be appropriately adjusted according to, for example, the kind of antigen, subject of administration, administration form, administration route and the like, it is generally 2 µg - 20 mg, preferably 20 µg - 200 µg, based on the total weight of the vaccine, for oral, intramuscular, transdermal, intradermal, subcutaneous or intraperitoneal administration and generally 0.01 µg - 1 mg, preferably 0.1 µg - 100 µg, based on the total weight of the vaccine, for intratracheal, intranasal(transnasal), intraocular, vaginal, rectal, intravenous, intraintestinal or inhalation administration.

The vaccine of the present invention may contain a pharmacologically acceptable carrier in addition to the compound of the present invention and antigen. Examples of the pharmacologically acceptable carrier that the vaccine of the present invention may contain include those recited as examples of the pharmacologically acceptable carrier that the immunostimulating agent of the present invention may contain.

The vaccine of the present invention may further contain other adjuvant. Examples of other adjuvant include those recited as examples of the adjuvant that can be used in combination with the immunostimulating agent of the present invention.

The vaccine of the present invention may contain α-cyclodextrin in addition to the compound of the present invention and an antigen. The α-cyclodextrin that may be contained in the vaccine of the present invention may be similar to α-cyclodextrin that may be contained in the immunostimulating agent of the present invention.

The content of α-cyclodextrin in the vaccine of the present invention is not particularly limited, and 0.005 - 20 wt% is preferable, and 0.05 - 5 wt% is more preferable.

While the weight ratio of the content of the compound of the present invention and the content of α-cyclodextrin (compound of the present invention: α-cyclodextrin) in the vaccine of the present invention is not particularly limited, 1:0.0002 - 2.0000 is preferable, and 1:0.002 - 0.2 is more preferable.

The vaccine of the present invention may contain hydroxypropyl-β-cyclodextrin in addition to the compound of the present invention and an antigen. The hydroxypropyl-β-cyclodextrin that may be contained in the vaccine of the present invention may be similar to hydroxypropyl-β-cyclodextrin that may be contained in the immunostimulating agent of the present invention.

The content of hydroxypropyl-β-cyclodextrin in the vaccine of the present invention is not particularly limited, and 0.005 - 20 wt% is preferable, and 0.05 - 5 wt% is more preferable.

While the weight ratio of the content of the compound of the present invention and the content of hydroxypropyl-β-cyclodextrin (compound of the present invention: hydroxypropyl-β-cyclodextrin) in the vaccine of the present invention is not particularly limited as long as the compound of the present invention can be in a dissolution state, 1:0.002 -2.0000 is preferable, and 1:0.004 - 0.4 is more preferable.

The vaccine of the present invention may contain at least one kind selected from the group consisting of carboxymethylcellulose, polysorbate (e.g., Tween80 (registered trade mark) etc.), polyethylene glycol (e.g., macrogol etc.), PBS, PBS-EDTA, TG, SDS, TBS, TBS-T, TAE, TBE and SSC in addition to the compound of the present invention and an antigen. carboxymethylcellulose, polysorbate, polyethylene glycol, PBS, PBS-EDTA, TG, SDS, TBS, TBS-T, TAE, TBE, SSC that may be contained in the vaccine of the present invention may be similar to carboxymethylcellulose, polysorbate, polyethylene glycol, PBS, PBS-EDTA, TG, SDS, TBS, TBS-T, TAE, TBE, SSC that may be contained in the immunostimulating agent of the present invention.

The each content of carboxymethylcellulose, polysorbate (e.g., Tween80 (registered trade mark) etc.), polyethylene glycol (e.g., macrogol etc.), PBS, PBS-EDTA, TG, SDS, TBS, TBS-T, TAE, TBE, SSC in the vaccine of the present invention is not particularly limited, and 0.005 - 20 wt% is preferable, and 0.05 - 5 wt% is more preferable.

While the weight ratio of the content of the compound of the present invention and the content of carboxymethyl cellulose (compound of the present invention: carboxymethyl cellulose) in the vaccine of the present invention is not particularly limited, 1:0.0002 - 2.0000 is preferable, and 1:0.002 - 0.2 is more preferable.

In another embodiment, the weight ratio of the content of the compound of the present invention and the content of carboxymethyl cellulose (compound of the present invention: carboxymethyl cellulose) in the vaccine of the present invention is not particularly limited, 1:0.0005 - 5.0000 is preferable, and 1:0.005 - 0.5 is more preferable.

While the weight ratio of the content of the compound of the present invention and the content of polysorbate (compound of the present invention: polysorbate) in the vaccine of the present invention is not particularly limited, 1:0.0002 - 2.0000 is preferable, and 1:0.002 - 0.2 is more preferable.

In another embodiment, the weight ratio of the content of the compound of the present invention and the content of polysorbate (compound of the present invention: polysorbate) in the vaccine of the present invention is not particularly limited, 1:0.0005 - 5.0000 is preferable, and 1:0.005 - 0.5 is more preferable.

While the weight ratio of the content of the compound of the present invention and the content of polyethylene glycol (compound of the present invention: polyethylene glycol) in the vaccine of the present invention is not particularly limited, 1:0.0002 - 2.0000 is preferable, and 1:0.002 - 0.2 is more preferable.

In another embodiment, the weight ratio of the content of the compound of the present invention and the content of polyethylene glycol (compound of the present invention: polyethylene glycol) in the vaccine of the present invention is not particularly limited, 1:0.0005 - 5.0000 is preferable, and 1:0.005 - 0.5 is more preferable.

The vaccine of the present invention may contain the immunostimulating agent of the present invention and an antigen.

Examples of the dosage form of the vaccine of the present invention include those recited as examples of the dosage form of the immunostimulating agent of the present invention.

The vaccine of the present invention can be produced by a method used conventionally in the technical field of preparation formulation, for example, the method described in the Japanese Pharmacopoeia, 16th Edition and the like. For example, it can be prepared by mixing the compound of the present invention and a desired antigen and, where necessary, emulsifying or dispersing the mixture, or adding the compound of the present invention to a vaccine containing a desired antigen and, where necessary, emulsifying or dispersing the mixture and the like.

The subject of administration of the vaccine of the present invention is not particularly limited as long as it is an animal having an immune system, and examples thereof include those recited as examples of the administration subject of the immunostimulating agent of the present invention.

The vaccine of the present invention may be administered by single administration or multiple successive administrations. When the vaccine of the present invention is successively administered, the dosing period is not particularly limited and can be appropriately set according to, for example, the kind of antigen, subject of administration, administration form, administration route and the like. It is generally within the range of 1 - 90 days, preferably 1 - 30 days.

While the administration route of the vaccine of the present invention is not particularly limited, the vaccine of the present invention is preferably administered by a route selected from oral administration, intramuscular administration, transdermal administration, intradermal administration, subcutaneous administration, intraperitoneal administration, intratracheal administration, intranasal administration (transnasal administration), intraocular administration, vaginal administration, rectal administration, intravenous administration, intraintestinal administration, and inhalation administration, and particularly preferably administered by subcutaneous administration or intranasal administration (transnasal administration). The vaccine of the present invention is particularly preferably administered by intradermal administration.

By administering the vaccine of the present invention to a target, allergy, infection, tumor and the like can be prevented or treated.

The present invention also provides a pharmaceutical composition containing the compound of the present invention and α-cyclodextrin (hereinafter sometimes to be also conveniently indicated as the pharmaceutical composition A of the present invention).

The compound of the present invention to be contained in the pharmaceutical composition A of the present invention may be one similar to the compound of the present invention which is contained in the immunostimulating agent of the present invention.

As α-cyclodextrin to be contained in the pharmaceutical composition A of the present invention, those similar to α-cyclodextrin that may be contained in the immunostimulating agent of the present invention can be used.

While the content of the compound of the present invention in the pharmaceutical composition A of the present invention is not particularly limited, it is preferably 0.1 - 99.9 wt%, more preferably 1 - 99 wt%.

While the content of α-cyclodextrin in the pharmaceutical composition A of the present invention is not particularly limited, it is preferably 0.005 - 20 wt%, more preferably 0.05 - 5 wt%.

While the weight ratio of the content of the compound of the present invention and that of α-cyclodextrin (compound of the present invention : α-cyclodextrin) in the pharmaceutical composition A of the present invention is not particularly limited, it is preferably 1:0.0002 - 2.0000, more preferably 1:0.002 - 0.2.

The pharmaceutical composition A of the present invention may contain a pharmacologically acceptable carrier in addition to the compound of the present invention and α-cyclodextrin. Examples of the pharmacologically acceptable carrier that the pharmaceutical composition A of the present invention may contain include those similar to those exemplified as the pharmacologically acceptable carrier that the immunostimulating agent of the present invention may contain.

The present invention also provides a pharmaceutical composition containing the compound of the present invention and hydroxypropyl-β-cyclodextrin (hereinafter sometimes conveniently referred to as the pharmaceutical composition B of the present invention).

The compound of the present invention to be contained in the pharmaceutical composition B of the present invention may be one similar to the compound of the present invention which is contained in the immunostimulating agent of the present invention.

The hydroxypropyl-β-cyclodextrin to be contained in the pharmaceutical composition B of the present invention may be one similar to the hydroxypropyl-β-cyclodextrin which is contained in the immunostimulating agent of the present invention.

While the content of the compound of the present invention in the pharmaceutical composition B of the present invention is not particularly limited, it is preferably 0.1 - 99.9 wt%, more preferably 1 - 99 wt%.

While the content of hydroxypropyl-β-cyclodextrin in the pharmaceutical composition B of the present invention is not particularly limited, it is preferably 0.005 - 20 wt%, more preferably 0.05 - 5 wt%.

While the weight ratio of the content of the compound of the present invention and the content of hydroxypropyl-β-cyclodextrin (compound of the present invention : hydroxypropyl-β-cyclodextrin) in the pharmaceutical composition B of the present invention is not particularly limited as long as the compound of the present invention can be in a dissolution state, 1:0.0002 - 2.0000 is preferable, and 1:0.004 - 0.4 is more preferable.

The pharmaceutical composition B of the present invention may contain a pharmacologically acceptable carrier in addition to the compound of the present invention and hydroxypropyl-β-cyclodextrin. Examples of the pharmacologically acceptable carrier that the pharmaceutical composition B of the present invention may contain include those similar to those exemplified as the pharmacologically acceptable carrier that the immunostimulating agent of the present invention may contain.

The present invention also provides a pharmaceutical composition containing the compound of the present invention and at least one kind selected from the group consisting of carboxymethylcellulose, polysorbate (e.g., Tween80 (registered trade mark) etc.), polyethylene glycol (e.g., macrogol etc.), PBS, PBS-EDTA, TG, SDS, TBS, TBS-T, TAE, TBE and SSC (hereinafter sometimes to be also conveniently indicated as the pharmaceutical composition C of the present invention).

The compound of the present invention to be contained in the pharmaceutical composition C of the present invention may be one similar to the compound of the present invention which is contained in the immunostimulating agent of the present invention.

Carboxymethylcellulose, polysorbate, polyethylene glycol, PBS, PBS-EDTA, TG, SDS, TBS, TBS-T, TAE, TBE, SSC that may be contained in the pharmaceutical composition C of the present invention may be similar to carboxymethylcellulose, polysorbate, polyethylene glycol, PBS, PBS-EDTA, TG, SDS, TBS, TBS-T, TAE, TBE, SSC that may be contained in the immunostimulating agent of the present invention.

While the content of the compound of the present invention in the pharmaceutical composition C of the present invention is not particularly limited, it is preferably 0.1 - 99.9 wt%, more preferably 1 - 99 wt%.

The each content of carboxymethylcellulose, polysorbate (e.g., Tween80 (registered trade mark) etc.), polyethylene glycol (e.g., macrogol etc.), PBS, PBS-EDTA, TG, SDS, TBS, TBS-T, TAE, TBE, SSC in the pharmaceutical composition C of the present invention is not particularly limited, and 0.005 - 20 wt% is preferable, and 0.05 - 5 wt% is more preferable.

While the weight ratio of the content of the compound of the present invention and the content of carboxymethyl cellulose (compound of the present invention: carboxymethyl cellulose) in the pharmaceutical composition C of the present invention is not particularly limited, 1:0.0002 - 2.0000 is preferable, and 1:0.002 - 0.2 is more preferable.

In another embodiment, the weight ratio of the content of the compound of the present invention and the content of carboxymethyl cellulose (compound of the present invention: carboxymethyl cellulose) in the pharmaceutical composition C of the present invention is not particularly limited, 1:0.0005 - 5.0000 is preferable, and 1:0.005 - 0.5 is more preferable.

While the weight ratio of the content of the compound of the present invention and the content of polysorbate (compound of the present invention: polysorbate) in the pharmaceutical composition C of the present invention is not particularly limited, 1:0.0002 - 2.0000 is preferable, and 1:0.002 - 0.2 is more preferable.

In another embodiment, the weight ratio of the content of the compound of the present invention and the content of polysorbate (compound of the present invention: polysorbate) in the pharmaceutical composition C of the present invention is not particularly limited, 1:0.0005 - 5.0000 is preferable, and 1:0.005 - 0.5 is more preferable.

While the weight ratio of the content of the compound of the present invention and the content of polyethylene glycol (compound of the present invention: polyethylene glycol) in the pharmaceutical composition C of the present invention is not particularly limited, 1:0.0002 - 2.0000 is preferable, and 1:0.002 - 0.2 is more preferable.

In another embodiment, the weight ratio of the content of the compound of the present invention and the content of polyethylene glycol (compound of the present invention: polyethylene glycol) in the pharmaceutical composition C of the present invention is not particularly limited, 1:0.0005 - 5.0000 is preferable, and 1:0.005 - 0.5 is more preferable.

The pharmaceutical composition C of the present invention may contain a pharmacologically acceptable carrier in addition to the compound of the present invention and at least one kind selected from the group consisting of carboxymethyl cellulose, polysorbate, polyethylene glycol, PBS, PBS-EDTA, TG, SDS, TBS, TBS-T, TAE, TBE and SSC. Examples of the pharmacologically acceptable carrier that the pharmaceutical composition C of the present invention may contain include those similar to those exemplified as the pharmacologically acceptable carrier that the immunostimulating agent of the present invention may contain.

Examples of the dosage form of the pharmaceutical compositions A, B and C of the present invention include those similar to those exemplified as the dosage form of the immunostimulating agent of the present invention.

The pharmaceutical compositions A, B and C of the present invention can be produced by a method used conventionally in the technical field of preparation formulation, for example, the method described in the Japanese Pharmacopoeia, 16th Edition and the like.

Examples of the administration subject of the pharmaceutical compositions A, B and C of the present invention include those recited as examples of the administration subject of the immunostimulating agent of the present invention.

The pharmaceutical composition A of the present invention may also be provided in the form of a kit wherein the compound of the present invention and α-cyclodextrin are separately packaged.

The pharmaceutical composition B of the present invention may also be provided in the form of a kit wherein the compound of the present invention and hydroxypropyl-β-cyclodextrin are separately packaged.

The pharmaceutical composition C of the present invention may also be provided in the form of a kit wherein the compound of the present invention and at least one kind selected from the group consisting of carboxymethyl cellulose, polysorbate, polyethylene glycol, PBS, PBS-EDTA, TG, SDS, TBS, TBS-T, TAE, TBE and SSC are separately packaged.

Since the pharmaceutical compositions A, B and C of the present invention have an antigen-specific IgG1 or IgG2a subclass antibody production-enhancing effect (immunostimulatory effect), respectively, they may be used, for example, as immunostimulating agents, adjuvants (e.g., vaccine adjuvant etc.) and the like.

### [Example]

The present invention is explained in more detail in the following by referring to Synthesis Examples, Examples and Experimental Examples, which do not limit the present invention. The present invention may be modified without departing from the scope of the invention. The reagents, apparatuses and materials used in the present invention are commercially available unless particularly indicated.

### (1) Synthesis of the compound of the present invention Synthetic Example 1 (Synthesis of (2S)-2-amino-N-dodecyl-5-[(N-nitrocarbamimidoyl)amino]pentanamide hydrochloride)

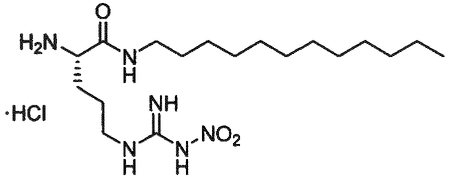

### (step 1)

### Synthesis of tert-butyl N-[(1S)-1-(dodecylcarbamoyl)-4-[(N-nitrocarbamimidoyl) amino]butyl]carbamate

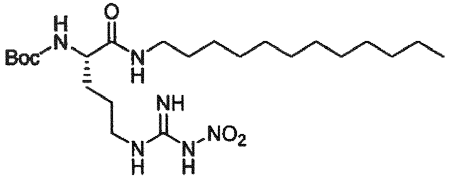

To a tetrahydrofuran (hereinafter to be also referred to as "THF") solution (250 mL) of (2S)-2-(tert-butoxycarbonylamino)-5-[(N-nitrocarbamimidoyl)amino]pentanoic acid (15.0 g, 47 mmol) and triethylamine (5.2 g, 52 mmol) was added ethyl chloroformate (5.7 g, 52 mmol) at -25°C and the mixture was stirred 20 min. To the reaction mixture was added a THF solution (50 mL) of dodecylamine (10.4 g, 56 mmol) and the mixture was stirred at -25°C for 30 min, heated to room temperature and stirred overnight. The reaction mixture was diluted with saturated aqueous sodium hydrogen carbonate solution and extracted with ethyl acetate. The organic layer was washed with saturated aqueous ammonium chloride solution and saturated brine and dried over anhydrous sodium sulfate. The solvent was evaporated under reduced pressure. The obtained residue was purified by silica gel chromatography (dichloromethane/methanol=40/1) to give the title compound (18.5 g, yield 76%).
¹H-NMR (400 MHz, CDCl₃) δ8.69(brs, 1H), 7.70-7.55(m, 2H), 6.51(brs, 1H), 5.45(d, J=6.8Hz, 1H), 4.26-4.16(m, 1H), 3.49-3.11(m, 4H), 1.79-1.67(m, 4H), 1.55-1.51(m, 2H), 1.47(s, 9H), 1.40-1.20(m, 18H), 0.88(t, J=6.8Hz, 3H).

### (step 2)

### Synthesis of (2S)-2-amino-N-dodecyl-5-[(N-nitrocarbamimidoyl)amino]pentanamide hydrochloride

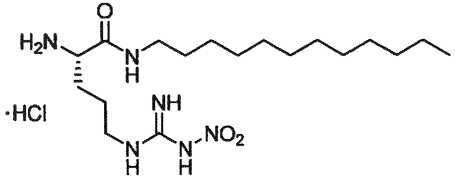

To tert-butyl N-[(1S)-1-(dodecylcarbamoyl)-4-[(N-nitrocarbamimidoyl)amino]butyl]carbamate (10.0 g, 21 mmol) obtained in step 1 was added hydrochloric acid/methanol solution (100 mL) and the mixture was stirred at room temperature for 1 hr. The reaction mixture was concentrated under reduced pressure to give the title compound (8.7 g, yield quantitative).
¹H-NMR (400 MHz, DMSO-d₆) δ8.61(brs, 1H), 8.49(brs, 1H), 8.30-8.10(m, 4H), 7.99-7.90(m, 1H), 5.45(d, J=6.8Hz, 1H), 3.77-3.68(m, 1H), 3.21-3.04(m, 4H), 1.73-1.67(m, 2H), 1.52-1.46(m, 2H), 1.43-1.40(m, 2H), 1.29-1.20(m, 18H), 0.85(t, J=6.8Hz, 3H).

### Example 1

### N-[(1S)-1-(dodecylcarbamoyl)-4-guanidino-butyl]hexadecanamide hydrochloride (SZ111)

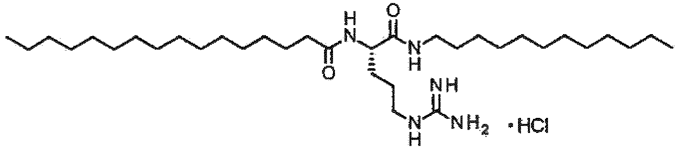

### (step 1)

### Synthesis of N-[(1S)-1-(dodecylcarbamoyl)-4-[(N-nitrocarbamimidoyl)amino]butyl]hexadecanamide

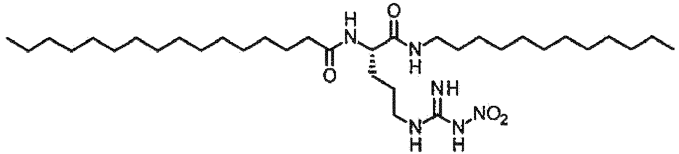

To a solution (100 mL) of hexadecanoic acid (2.60 g, 10.14 mmol), 1-ethyl-3-(3-dimethylaminopropyl)carbodiimide hydrochloride (hereinafter to be also referred to as "EDCI") (3.25 g, 16.91 mmol) and 1-hydroxybenzotriazole (hereinafter to be also referred to as "HOBt") (2.28 g, 16.91 mmol) in N,N-dimethylformamide (hereinafter to be also referred to as "DMF") was added triethylamine (3.41 g, 33.81 mmol), and the mixture was stirred at room temperature for 15 min. To the reaction mixture was added (2S)-2-amino-N-dodecyl-5-[(N-nitrocarbamimidoyl)amino]pentanamide hydrochloride (4.76 g, 11.27 mmol) obtained in Synthetic Example 1 and the mixture was stirred at room temperature for 6 hr. The reaction mixture was diluted with water, and the mixture was extracted with ethyl acetate. The organic layer was washed with saturated brine, dried over anhydrous sodium sulfate, and concentrated to give the title compound (4.55 g, yield 64%).
¹H-NMR (400 MHz, CF₃COOD) δ4.87(s, 1H), 3.72-3.68 (m, 2H), 3.59-3.46(m, 2H), 2.68-2.64(m, 2H), 2.23-1.99(m, 4H), 1.86-1.75(m, 4H), 1.58-1.36(m, 42H), 1.01(t, J=5.2Hz, 6H).

### (step 2)

### Synthesis of N-[(1S)-1-(dodecylcarbamoyl)-4-guanidino-butyl]hexadecanamide hydrochloride

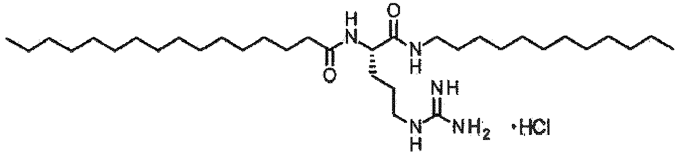

To a methanol solution (50 mL) of N-[(1S)-1-(dodecylcarbamoyl)-4-[(N-nitrocarbamimidoyl)amino]butyl]hexadecanamide (2.50 g, 4.01 mmol) obtained in step 1 were added concentrated hydrochloric acid (0.3 mL) and 10% palladium-carbon (500 mg), and the mixture was stirred under 50 psi hydrogen atmosphere at 50°C overnight. Insoluble material was filtered off, the filtrate was concentrated, to the obtained residue was added ethanol, and the mixture was collected by filtration and dried to give the title compound (2.44 g, yield 99%).
¹H-NMR (400 MHz, CD₃OD) δ4.32-4.29(m, 1H), 3.30-3.13(m, 4H), 2.26(d, J=7.2Hz, 2H), 1.84-1.57(m, 6H), 1.50(t, J=6.8Hz, 2H), 1.35-1.24(m, 42H), 0.90(t, J=6.8Hz, 6H).
MS(ESI)m/z:580[M+H]⁺

### Example 2

### N-[(1S)-1-(dodecylcarbamoyl)-4-guanidino-butyl]octadecanamide hydrochloride (SZ137)

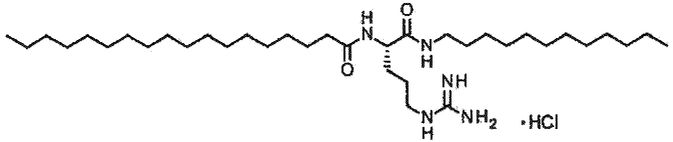

### (step 1)

### Synthesis of N-[(1S)-1-(dodecylcarbamoyl)-4-[(N-nitrocarbamimidoyl)amino]butyl]octadecanamide

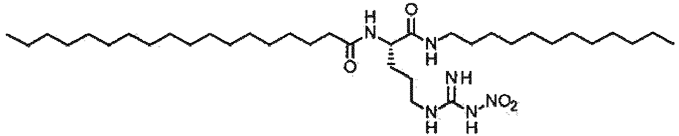

To a DMF solution (20 mL) of stearic acid (604 mg, 2.13 mmol), EDCI (681 mg, 3.55 mmol) and HOBt (479 mg, 3.55 mmol) was added triethylamine (956 mg, 9.47 mmol), and the mixture was stirred at room temperature for 20 min. To the reaction mixture was added (2S)-2-amino-N-dodecyl-5-[(N-nitrocarbamimidoyl)amino]pentanamide hydrochloride (1.0 g, 2.37 mmol) obtained in Synthetic Example 1 and the mixture was stirred at room temperature overnight. Water was added to the reaction mixture, and the mixture was stirred at room temperature for 10 min and the obtained solid was washed with water and dried to give the title compound (1.25 g, yield 90%).
¹H-NMR (400 MHz, CDCl₃) δ7.46-7.33(m, 2H), 6.72(d, J=8.0Hz, 1H), 6.67-6.64(m, 1H), 4.76-4.69(m, 1H), 3.68-3.57(m, 1H), 3.33-3.17(m, 3H), 2.24(t, J=7.6Hz, 2H), 1.81-1.68(m, 4H), 1.55-1.42(m, 4H), 1.34-1.18(m, 46H), 0.88(t, J=6.8Hz, 6H).

### (step 2)

### Synthesis of N-[(1S)-1-(dodecylcarbamoyl)-4-guanidino-butyl]octadecanamide hydrochloride

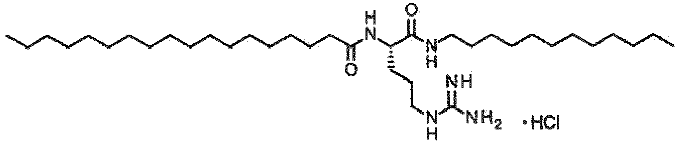

To N-[(1S)-1-(dodecylcarbamoyl)-4-[(N-nitrocarbamimidoyl)amino]butyl]octadecanamide (1.25 g, 1.92 mmol) obtained in step 1 in methanol solution (30 mL) were added concentrated hydrochloric acid (0.5 mL) and 10% palladium-carbon (250 mg), and the mixture was stirred under 50 psi hydrogen atmosphere at 50°C overnight. Insoluble material was filtered off, the filtrate was concentrated and the obtained residue was purified by silica gel chromatography (dichloromethane/methanol=10/1) to give the title compound (560 mg, yield 48%).
¹H-NMR (400 MHz, CD₃OD) δ4.33-4.29(m, 1H), 3.23-3.13(m, 4H), 2.25(t, J=7.6Hz, 2H), 1.83-1.48(m, 8H), 1.36-1.23(m, 46H), 0.90(t, J=6.8Hz, 6H).
MS(ESI)m/z:608[M+H]⁺

### Example 3

N-[(1S)-1-(dodecylcarbamoyl)-4-guanidino-butyl]docosanamide hydrochloride (SZ138)

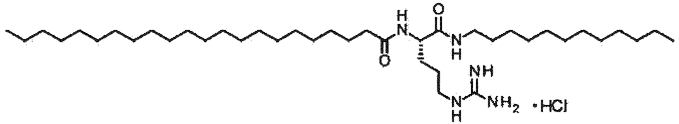

### (step 1)

### Synthesis of N-[(1S)-1-(dodecylcarbamoyl)-4-[(N-nitrocarbamimidoyl)amino]butyl]docosanamide

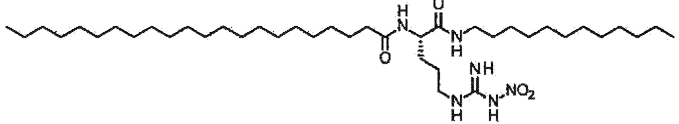

To a dichloromethane solution (20 mL) of behenic acid (1.0 g, 2.94 mmol) were added DMF (0.3 mL) and oxalyl dichloride (0.30 mL, 3.53 mmol) and the mixture was stirred at room temperature for 2 hr. The reaction mixture was concentrated and the obtained residue was dissolved in THF (5 mL). This was added to a THF solution (20 mL) of (2S)-2-amino-N-dodecyl-5-[(N-nitrocarbamimidoyl)amino]pentanamide hydrochloride (1.37 g, 3.23 mmol) obtained in Synthetic Example 1 and triethylamine (1.19 g, 11.76 mmol) and the mixture was stirred at room temperature overnight. Water was added to the reaction mixture, the mixture was stirred at room temperature for 10 min and the obtained solid was washed with water and dried to give the title compound (2.0 g, yield 96%).
¹H-NMR (400 MHz, CDCl₃) δ8.88-8.76(m, 1H), 7.53-7.41(m, 2H), 6.92-6.81(m, 2H), 4.80-4.70(m, 1H), 3.68-3.55(m, 1H), 3.29-3.12(m, 3H), 2.24(t, J=7.6Hz, 2H), 1.77-1.49(m, 8H), 1.37-1.19(m, 54H), 0.88(t, J=6.8Hz, 6H).

### (step 2)

### Synthesis of N-[(1S)-1-(dodecylcarbamoyl)-4-guanidino-butyl]docosanamide hydrochloride

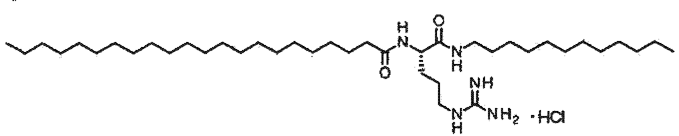

An operation similar to that in Example 2, step 2 was performed using N-[(1S)-1-(dodecylcarbamoyl)-4-[(N-nitrocarbamimidoyl)amino]butyl]docosanamide obtained in step 1 instead of N-[(1S)-1-(dodecylcarbamoyl)-4-[(N-nitrocarbamimidoyl)amino]butyl]octadecanamide to give the title compound (1.10 g, yield 56%).
¹H-NMR (400 MHz, CD₃OD) δ4.35-4.32(m, 1H), 3.26-3.14(m, 4H), 2.29-2.25(m, 2H), 1.85-1.50(m, 8H), 1.40-1.25(m, 54H), 0.92(t, J=6.8Hz, 6H).
MS(ESI)m/z:664[M+H]⁺

### Example 4

### N-[(1S)-3-methyl-1-(undecylcarbamoyl)butyl]docosanamide (SZ142)

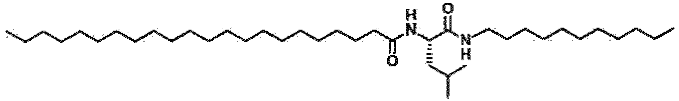

### (step 1)

### Synthesis of benzyl N-[(1S)-1-methyl-2-oxo-2-(undecylamino)ethyl]carbamate

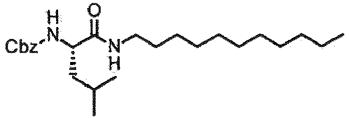

To a DMF solution (100 mL) of (2S)-2-(benzyloxycarbonylamino)-4-methyl-pentanoic acid (3.0 g, 11.3 mmol), HOBt (3.2 g, 23.7 mmol) and EDCI (4.5 g, 23.7 mmol) was added triethylamine (4.8 g, 47.5 mmol) and the mixture was stirred for 15 min. To the reaction mixture was added undecylamine (2.7 g, 15.8 mmol), and the mixture was stirred at room temperature overnight. The reaction mixture was diluted with water and extracted with ethyl acetate. The organic layer was washed with saturated brine, dried over anhydrous sodium sulfate and concentrated under reduced pressure. The obtained residue was purified by silica gel chromatography (petroleum ether/ethyl acetate=10/1) to give the title compound (2.5 g, yield 53%).
¹H-NMR (400 MHz, CDCl₃) δ7.35-7.31(m, 5H), 6.02-6.01(m, 1H), 5.21-5.19(m, 1H), 5.09(s, 2H), 4.15-4.09(m, 1H), 3.23-3.18(m, 2H), 1.65-1.61(m, 2H), 1.52-1.47(m, 3H), 1.25(m, 16H), 0.93-0.92(m, 6H), 0.89-0.86(m, 3H).

### (step 2)

### Synthesis of (2S)-2-amino-4-methyl-N-undecyl-pentanamide

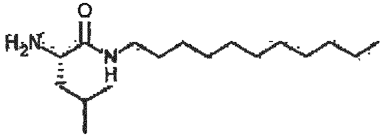

To a methanol solution (50 mL) of benzyl N-[(1S)-1-methyl-2-oxo-2-(undecylamino)ethyl]carbamate (2.5 g, 6.0 mmol) obtained in step 1 was added 10% palladium-carbon (250 mg), and the mixture was stirred under a hydrogen atmosphere at room temperature for 3 hr. Insoluble material was filtered off and the filtrate was concentrated under reduced pressure to give the title compound (1.7 g, yield quantitative) without purification.
MS(ESI)m/z:285[M+H]⁺

### (step 3)

### Synthesis of N-[(1S)-3-methyl-1-(undecylcarbamoyl)butyl]docosanamide

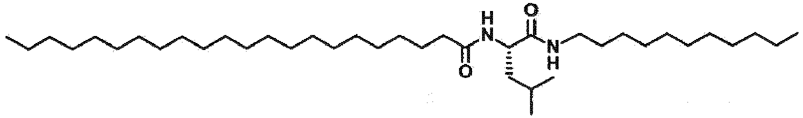

To a DMF solution (10 mL) of behenic acid (998 mg, 2.9 mmol), HOBt (594 mg, 4.4 mmol) and EDCI (840 mg, 4.4 mmol) was added triethylamine (889 mg, 8.8 mmol) and the mixture was stirred at room temperature for 15 min. To the reaction mixture was added (2S)-2-amino-4-methyl-N-undecyl-pentanamide (1.0 g, 3.5 mmol) obtained in step 2 and the mixture was stirred under a nitrogen atmosphere at room temperature overnight. The reaction mixture was poured into water under ice-cooling, and the obtained crude crystals were collected by filtration, washed with water and was purified by silica gel chromatography (dichloromethane/methanol=50/1 - 20/1) to give the title compound (580 mg, yield 32%).
¹H-NMR (400 MHz, CDCl₃) δ6.16-6.13(m, 1H), 5.91-5.89(d, J=8.8Hz, 1H), 4.43-4.37(m, 1H), 3.24-3.18(m, 2H), 2.19-2.16(m, 2H), 1.62-1.59(m, 4H), 1.53-1.46(m, 3H), 1.25(m, 52H), 0.94-0.86(m, 12H) .

### Example 5

### N-[(1S)-1-(dodecylcarbamoyl)-3-methylbutyl]hexadecanamide (SZ144)

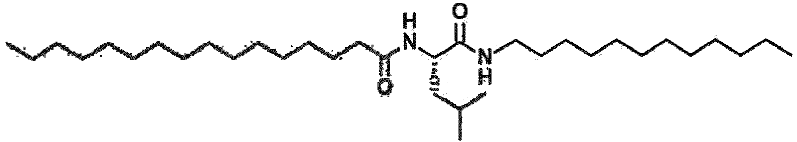

### (step 1)

### Synthesis of benzyl N-[(1S)-1-(dodecylcarbamoyl)-3-methylbutyl]carbamate

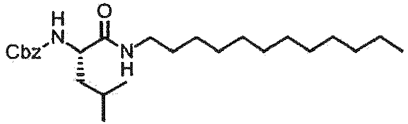

An operation similar to that in Example 4, step 1 was performed using dodecylamine (1.9 g, 10.6 mmol) instead of undecylamine to give the title compound (1.2 g, yield 37%).
¹H-NMR (300 MHz, CDCl₃) δ7.35-7.27(m, 5H), 6.07-6.02 (m, 1H), 5.25-5.21(m, 1H), 5.10(s, 2H), 4.20-4.10(m, 1H), 3.26-3.18(m, 2H), 1.63-1.56(m, 2H), 1.48-1.42(m, 3H), 1.26(m, 18H), 0.94-0.86(m, 9H).

### (step 2)

### Synthesis of (2S)-2-amino-N-dodecyl-4-methyl-pentanamide

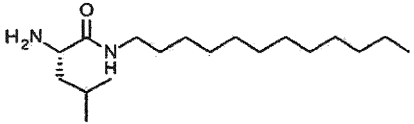

An operation similar to that in Example 4, step 2 was performed using benzyl N-[(1S)-1-(dodecylcarbamoyl)-3-methyl-butyl]carbamate (1.1 g, 2.5 mmol) obtained in step 1 instead of benzyl N-[(1S)-1-methyl-2-oxo-2-(undecylamino)ethyl]carbamate to give the title compound (758 mg, yield quantitative).
MS(ESI)m/z:299[M+H]⁺

### (step 3)

### Synthesis of N-[(1S)-1-(dodecylcarbamoyl)-3-methyl-butyl]hexadecanamide

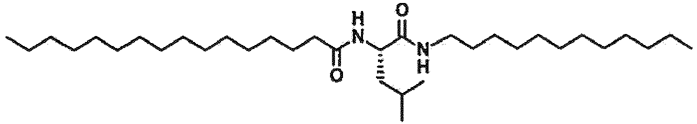

An operation similar to that in Example 4, step 3 was performed using palmitic acid (651 mg, 2.5 mmol) instead of behenic acid to give the title compound (680 mg, yield 50%).
¹H-NMR (400 MHz, CDCl₃) δ6.23-6.20(m, 1H), 5.96-5.94(m, 1H), 4.43-4.38(m, 1H), 3.24-3.17(m, 2H), 2.19-2.16(m, 2H), 1.64-1.60(m, 4H), 1.55-1.44(m, 3H), 1.25(m, 42H), 0.94-0.86(m, 12H).

### Example 6

### N-[(1S)-2-(dodecylamino)-1-methyl-2-oxoethyl]docosanamide (SZ145)

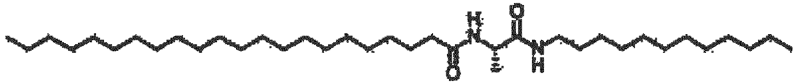

### (step 1)

### Synthesis of tert-butyl N-[(1S)-2-(dodecylamino)-1-methyl-2-oxo-ethyl carbamate

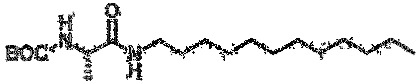

To a DMF solution (100 mL) of (2S)-2-(tert-butoxycarbonylamino)propanoic acid (2.0 g, 10.6 mmol), HOBt (3.0 g, 22.2 mmol) and EDCI (4.2 g, 22.2 mmol) was added triethylamine (4.5 g, 44.4 mmol) and the mixture was stirred for 15 min. To the reaction mixture was added dodecylamine (2.7 g, 14.8 mmol), and the mixture was stirred at room temperature overnight, diluted with water and extracted with ethyl acetate. The organic layer was washed with saturated brine, dried over anhydrous sodium sulfate and concentrated under reduced pressure. The obtained residue was purified by silica gel chromatography (petroleum ether/ethyl acetate=10/1) to give the title compound (2.9 g, yield 78%).
MS(ESI)m/z:257[M+H-Boc]⁺

### (step 2)

### Synthesis of (2S)-2-amino-N-dodecyl-propanamide hydrochloride

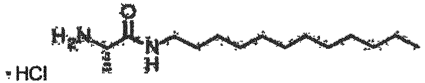

An operation similar to that in Synthetic Example 1, step 2 was performed using tert-butyl N-[(1S)-2-(dodecylamino)-1-methyl-2-oxo-ethyl carbamate (2.9 g, 8.13 mmol) obtained in step 1 instead of tert-butyl N-[(1S)-1-(dodecylcarbamoyl)-4-[(N-nitrocarbamimidoyl)amino]butyl]carbamate to give the title compound (2.3 g, yield quantitative).
MS(ESI m/z:257[M+H]⁺

### (step 3)

### Synthesis of N-[(1S)-2-(dodecylamino)-1-methyl-2-oxoethyl]docosanamide

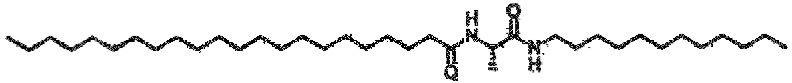

An operation similar to that in Example 4, step 3 was performed using (2S)-2-amino-N-dodecyl-propanamide hydrochloride (2.3 g, 7.9 mmol) obtained in step 2 instead of (2S)-2-amino-4-methyl-N-undecyl-pentanamide to give the title compound (750 mg, yield 17%).
¹H-NMR (300 MHz, CDCl₃) δ6.31-6.30(m, 1H), 6.16-6.14(m, 1H), 4.51-4.42(m, 1H), 3.26-3.20(m, 2H), 2.21-2.16(m, 2H), 1.62-1.57(m, 2H), 1.52-1.45(m, 2H), 1.37-1.35(m, 3H), 1.26-1.22(m, 54H), 0.90-0.86(m, 6H).

### Example 7

### N-[2-(dodecylamino)-1,1-dimethyl-2-oxo-ethyl]docosanamide (SZ146)

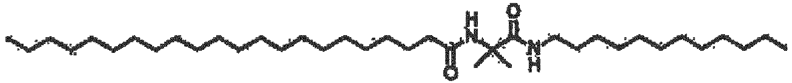

### (step 1)

### Synthesis of N-[2-(dodecylamino)-1,1-dimethyl-2-oxoethyl]carbamate tert-butyl

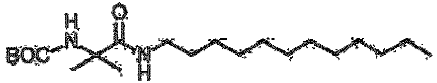

An operation similar to that in Synthetic Example 1, step 1 was performed using 2-(tert-butoxycarbonylamino)-2-methyl-propanoic acid (3.0 g, 14.8 mmol) instead of (2S)-2-(tert-butoxycarbonylamino)-5-[(N-nitrocarbamimidoyl)amino]pentanoic acid to give the title compound (5.1 g, yield 93%).
¹H-NMR (400 MHz, DMSO-d₆) δ7.43(brs, 1H), 6.75(brs, 1H), 4.06(q, J=7.2Hz, 2H), 3.00(q, J=6.4Hz, 4H), 1.36(brs, 15H), 1.26(brs, 13H), 0.85(t, J=6.4Hz, 6H).

### (step 2)

### Synthesis of 2-amino-N-dodecyl-2-methyl-propanamide hydrochloride

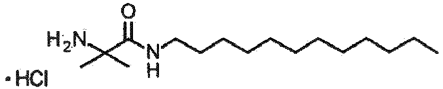

To tert-butyl N-[2-(dodecylamino)-1,1-dimethyl-2-oxoethyl]carbamate (5.1 g, 13.8 mmol) obtained in step 1 was added 2 M hydrochloric acid/1,4-dioxane (50 mL) and the mixture was stirred at room temperature for 2 hr. The reaction mixture was concentrated under reduced pressure to give the title compound (3.1 g, yield 77%) without purification.
¹H-NMR (400 MHz, DMSO-d₆) δ8.07(t, J=5.6Hz, 2H), 3.04(q, J=6.4Hz, 4H), 1.41-1.38(m, 4H), 1.25(s, 15H), 0.85(t, J=6.8Hz, 6H) .

### (step 3)

### Synthesis of N-[2-(dodecylamino)-1,1-dimethyl-2-oxoethyl]docosanamide

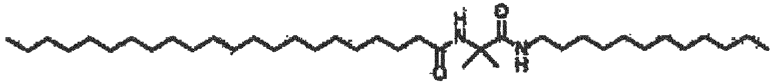

To a dichloromethane solution (20 mL) of behenic acid (1.0 g, 2.9 mmol) were added a catalytic amount of DMF and oxalyl dichloride (442 mg, 3.5 mmol) and the mixture was stirred at room temperature for 1 hr. The reaction mixture was concentrated and the obtained residue was dissolved in THF (2 mL). This was added to a THF solution (20 mL) of 2-amino-N-dodecyl-2-methyl-propanamide hydrochloride (952 mg, 3.5 mmol) obtained in step 2 while adjusting to pH8 or above with 5M aqueous sodium hydroxide solution and the mixture was stirred at room temperature overnight. The precipitate was collected by filtration and recrystallized from methanol to give the title compound (900 mg, yield 39%).
¹H-NMR (400 MHz,CF₃COOD) δ3.51(t, J=7.2Hz, 2H), 2.54(t, J=7.2Hz, 2H), 1.76(s, 6H), 1.76-1.67(m, 2H), 1.36(brs, 56H), 0.86(t, J=6.4Hz, 6H).

### Example 8

### N-[3-(dodecylamino)-3-oxo-propyl]docosanamide (SZ147)

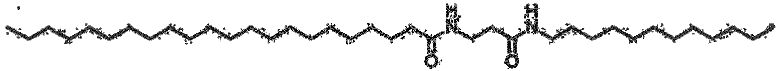

### (step 1)

### Synthesis of tert-butyl N-[3-(dodecylamino)-3-oxopropyl]carbamate

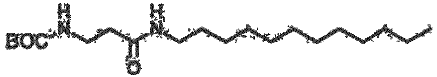

An operation similar to that in Synthetic Example 1, step 1 was performed using 3-(tert-butoxycarbonylamino)propanoic acid (3.0 g, 15.9 mmol) instead of (2S)-2-(tert-butoxycarbonylamino)-5-[(N-nitrocarbamimidoyl)amino]pentanoic acid to give the title compound (4.4 g, yield 77%).
¹H-NMR (400 MHz, CD₃OD) δ3.30-3.28(m, 4H), 3.14(t, J=6.8Hz, 2H), 2.33(t, J=6.8Hz, 2H), 1.48-1.46(m, 2H), 1.42(s, 9H), 1.28(brs, 16H), 0.89(t, J=6.8Hz, 3H).

### (step 2)

### Synthesis of 3-amino-N-dodecyl-propanamide hydrochloride

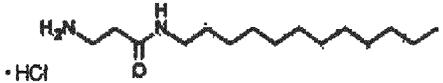

An operation similar to that in Example 7, step 2 was performed using tert-butyl N-[3-(dodecylamino)-3-oxopropyl]carbamate (4.4 g, 13.8 mmol) obtained in step 1 instead of tert-butyl N-[2-(dodecylamino)-1,1-dimethyl-2-oxo-ethyl]carbamate to give the title compound (3.1 g, yield 77%).
¹H-NMR (300 MHz, DMSO-d₆) δ8.10(brs, 1H), 7.88 (brs, 2H), 3.06-2.91(m, 4H), 2.47-2.42(m, 2H), 1.40-1.33(m, 2H), 1.23(brs, 18H), 0.85(t, J=6.0Hz, 3H).

### (step 3)

### Synthesis of N-[3-(dodecylamino)-3-oxo-propyl]docosanamide

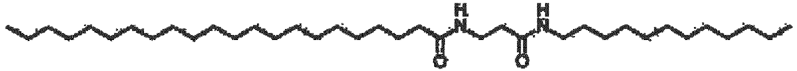

An operation similar to that in Example 7, step 3 was performed using 3-amino-N-dodecyl-propanamide hydrochloride (900 mg, 3.5 mmol) obtained in step 2 instead of 2-amino-N-dodecyl-2-methyl-propanamide hydrochloride to give the title compound (850 mg, yield 42%).
¹H-NMR (400 MHz, CDCl₃) δ3.79(brs, 2H), 3.47(t, J=7.2Hz, 2H), 3.00(brs, 2H), 2.58(t, J=7.2Hz, 2H), 1.74-1.85(m, 4H), 1.35(brs, 56H), 0.85(t, J=6.4Hz, 6H).

### Example 9

N-[3-(dodecylamino)-2,2-dimethyl-3-oxo-propyl]docosanamide (SZ148)

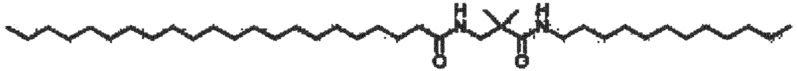

### (step 1)

### Synthesis of tert-butyl N-[3-(dodecylamino)-2,2-dimethyl-3-oxopropyl]carbamate

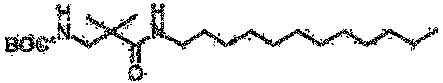

An operation similar to that in Example 6, step 1 was performed using 3-(tert-butoxycarbonylamino)-2,2-dimethyl-propanoic acid (1.0 g, 4.6 mmol) instead of (2S)-2-(tert-butoxycarbonylamino)propanoic acid to give the title compound (1.7 g, yield 96%).
¹H-NMR (300 MHz, CDCl₃) δ5.91(brs, 1H), 5.17(brs, 1H), 3.24-3.18(m, 4H), 1.51-1.43(m, 11H), 1.26(m, 18H), 1.18(s, 6H), 0.88(t, J=6Hz, 3H).

### (step 2)

### Synthesis of 3-amino-N-dodecyl-2,2-dimethyl-propanamide hydrochloride

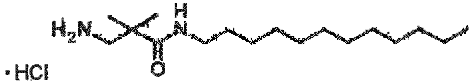

To tert-butyl N-[3-(dodecylamino)-2,2-dimethyl-3-oxopropyl]carbamate (1.3 g, 3.4 mmol) obtained in step 1 was added 7 M hydrochloric acid/methanol (10 mL) and the mixture was stirred at room temperature for 1 min. The reaction mixture was concentrated, methanol (20 mL) was added and the mixture was concentrated to give the title compound (1.5 g, yield 97%).
¹H-NMR (400 MHz, CD₃OD) δ3.18(t, J=7.2Hz, 2H), 2.99(s, 2H), 1.50(s, 2H), 1.28(m, 24H), 0.90(t, J=6Hz, 3H).

### (step 3)

### Synthesis of N-[3-(dodecylamino)-2,2-dimethyl-3-oxopropyl]docosanamide

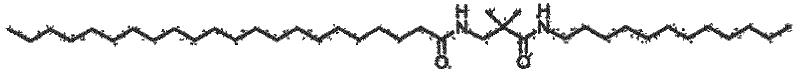

To a dichloromethane solution (10 mL) of behenic acid (1.0 g, 2.96 mmol) were added DMF (0.2 mL) and oxalyl dichloride (470 mg, 3.7 mmol) and the mixture was stirred at room temperature for 2 hr. The reaction mixture was concentrated and the obtained residue was dissolved in THF (10 mL). This was added to a THF solution (20 mL) of 3-amino-N-dodecyl-2,2-dimethyl-propanamide hydrochloride (1.05 g, 3.7 mmol) obtained in step 2 and triethylamine (1.5 g, 14.8 mmol) and the mixture was stirred at room temperature overnight. The precipitated solid was collected by filtration, methanol (50 mL) was added and the mixture was stirred at 70°C for 30 min. The mixture was cooled to room temperature and the solid was collected by filtration to give the title compound (946 mg, yield 43%).
¹H-NMR (400 MHz, CDCl₃) δ6.37(brs, 1H), 5.86(brs, 1H), 3.56-3.42(m, 2H), 3.24-3.19(m, 2H), 3.18-3.14(m, 2H), 1.62-1.61(m, 2H), 1.50-1.49(m, 2H), 1.25(m, 54H), 1.18(s, 6H), 0.88(t, J=6.8Hz, 6H).

### Example 10

### (2S)-N-dodecyl-5-guanidino-2-(hexadecylcarbamoylamino)pentanamide hydrochloride (SZ149)

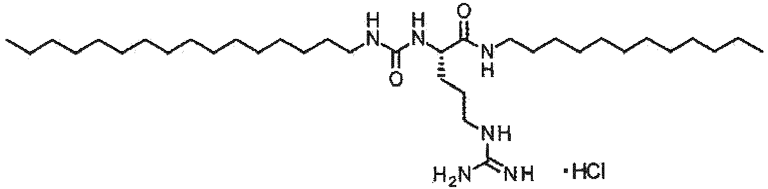

To a THF solution (30 mL) of (2S)-2-amino-N-dodecyl-5-[(N-nitrocarbamimidoyl)amino]pentanamide hydrochloride (1.0 g, 2.37 mmol) obtained in Synthetic Example 1 were added triethylamine (717 mg, 7.11 mmol) and hexadecyl isocyanate (697 mg, 2.61 mmol) and the mixture was stirred at 30°C for 3 hr. The reaction mixture was diluted with water, stirred at room temperature for 10 min and the solid was collected by filtration. The solid was washed with water and dried. The obtained crude crystals (1.2 g, 1.9 mmol, yield 80%) were dissolved in methanol (30 mL), concentrated hydrochloric acid (0.5 mL) and 10% palladium-carbon (240 mg) were added and the mixture was stirred under 50 psi hydrogen atmosphere at 50°C overnight. The mixture was cooled to room temperature and insoluble material was filtered off. The filtrate was concentrated and the obtained residue was purified by silica gel chromatography (dichloromethane/methanol=10/1) to give the title compound (700 mg, yield 59%).
¹H-NMR (400 MHz, CD₃OD) δ4.18(t, J=6.8Hz, 1H), 3.25-3.06(m, 6H), 1.80-1.75(m, 1H), 1.68-1.59(m, 3H), 1.52-1.43(m, 4H), 1.35-1.24(m, 44H), 0.90(t, J=6.8Hz, 6H).
MS(ESI)m/z:609[M+H]⁺

### Example 11

### Hexadecyl N-[(1S)-1-(dodecylcarbamoyl)-4-guanidino-butyl]carbamate hydrochloride (SZ150)

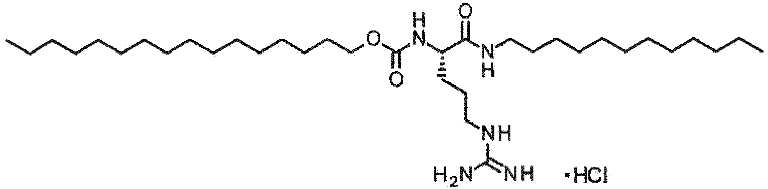

### (step 1)

### Synthesis of hexadecyl N-[(1S)-1-(dodecylcarbamoyl)-4-[(N-nitrocarbamimidoyl)amino]butyl] carbamate

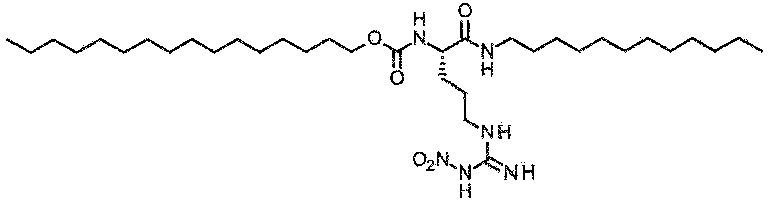

To a THF solution (10 mL) of (2S)-2-amino-N-dodecyl-5-[(N-nitrocarbamimidoyl)amino]pentanamide hydrochloride (692 mg, 1.64 mmol) obtained in Synthetic Example 1 were added triethylamine (497 mg, 4.92 mmol) and hexadecyl chloroformate (500 mg, 1.64 mmol) and the mixture was stirred at 30°C overnight. The reaction mixture was diluted with water, stirred at room temperature for 10 min, and the solid was collected by filtration to give the title compound (900 mg, yield 84%).
¹H-NMR (400 MHz, CDCl₃) δ7.55-7.40(m, 2H), 6.44-6.37(m, 1H), 5.59(d, J=6.0Hz, 1H), 4.27-4.21(m, 1H), 4.06-4.01(m, 2H), 3.46-3.32(m, 2H), 3.27-3.21(m, 2H), 1.80-1.68(m, 4H), 1.59-1.39(m, 4H), 1.34-1.21(m, 44H), 0.88(t, J=7.6Hz, 6H).

### (step 2)

### Synthesis of hexadecyl N-[(1S)-1-(dodecylcarbamoyl)-4-guanidino-butyl]carbamate hydrochloride

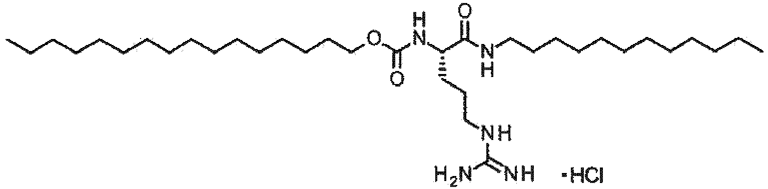

Hexadecyl N-[(1S)-1-(dodecylcarbamoyl)-4-[(N-nitrocarbamimidoyl)amino]butyl] carbamate (900 mg, 1.37 mmol) obtained in step 1 was dissolved in methanol (20 mL), concentrated hydrochloric acid (0.3 mL) and 10% palladium-carbon (180 mg) were added and the mixture was stirred at under 50 psi hydrogen atmosphere at 50°C overnight. Insoluble material was filtered off, the filtrate was concentrated and the obtained residue was purified by silica gel chromatography (dichloromethane/methanol=10/1) to give the title compound (308 mg, yield 34%).
¹H-NMR (400 MHz, CD₃OD) δ4.09-4.00(m, 3H), 3.24-3.16(m, 4H), 1.81-1.48(m, 8H), 1.35-1.24(m, 44H), 0.90(t, J=6.8Hz, 6H). MS(ESI)m/z:610 [M+H]⁺

### Example 12

### N-[(1S)-5-amino-1-(dodecylcarbamoyl)pentyl]hexadecanamide hydrochloride (SZ153)

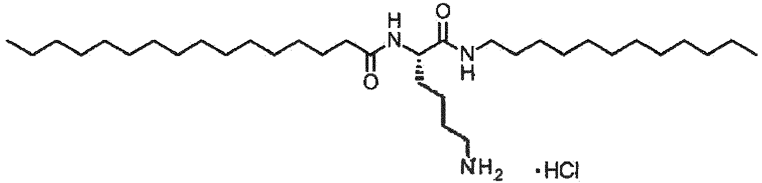

### (step 1)

### Synthesis of (2S)-6-(benzyloxycarbonylamino)-2-(tert-butoxycarbonylamino)hexanoic acid

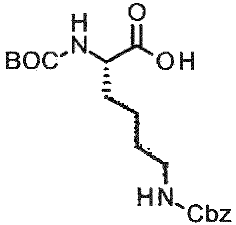

To (2S)-2-amino-6-(benzyloxycarbonylamino)hexanoic acid (10.0 g, 35.7 mmol) was added 1,4-dioxane/water (80 mL/80 mL), 1M aqueous sodium hydroxide solution (35.6 mL) was added under ice-cooling, a 1,4-dioxane solution (50 mL) of di-tert-butyl dicarbonate (8.5 g, 39.2 mmol) was added with stirring and the mixture was stirred at room temperature for 3 hr. The reaction mixture was concentrated under reduced pressure, diluted with water, and acidified with 2N potassium hydrogensulfate aqueous solution. The aqueous layer was extracted with ethyl acetate, and the organic layer was dried over anhydrous sodium sulfate and concentrated to give the title compound (13.5 g, yield quantitative) without purification.
¹H-NMR (300 MHz, DMSO-d₆) 512.40 (s, 1H), 7.35-7.29 (m, 5H), 7.24-7.23(m, 1H), 7.04(d, J=7.8Hz, 1H), 4.99(s, 2H), 3.83-3.76(m, 1H), 2.98-2.92(m, 2H), 1.62-1.50(m, 2H), 1.42-1.37(m, 13H).

### (step 2)

### Synthesis of tert-butyl N-[(1S)-5-(benzyloxycarbonylamino)-1-(dodecylcarbamoyl)pentyl] carbamate

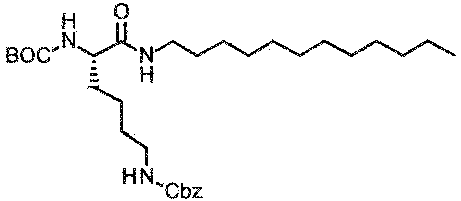

An operation similar to that in Example 6, step 1 was performed using (2S)-6-(benzyloxycarbonylamino)-2-(tert-butoxycarbonylamino)hexanoic acid (13.5 g, 35.5 mmol) obtained in step 1 instead of (2S)-2-(tert-butoxycarbonylamino)propanoic acid to give the title compound (18.0 g, yield 92%).
¹H-NMR (400 MHz, CDCl₃) δ7.36-7.30(m, 5H), 6.15-6.12(m, 1H), 5.14-5.09(m, 3H), 4.88-4.84(m, 1H), 4.00-3.97(m, 1H), 3.23-3.18(m, 4H), 1.85-1.82(m, 1H), 1.66-1.58(m, 5H), 1.52(s, 9H), 1.48-1.43(m, 2H), 1.41-1.25(m, 18H), 0.89-0.86(m, 3H).

### (step 3)

### Synthesis of benzyl N-[(5S)-5-amino-6-(dodecylamino)-6-oxohexyl]carbamate hydrochloride

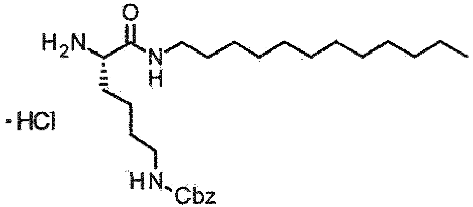

An operation similar to that in Synthetic Example 1, step 2 was performed using tert-butyl N-[(1S)-5-(benzyloxycarbonylamino)-1-(dodecylcarbamoyl)-pentyl]carbamate obtained in step 2 instead of tert-butyl N-[(1S)-1-(dodecylcarbamoyl)-4-[(N-nitrocarbamimidoyl)amino]butyl]carbamate to give the title compound (15.9 g, yield quantitative).
¹H-NMR (300 MHz, CDCl₃) δ8.25-8.19(m, 3H), 7.36-7.31(m, 5H), 5.06(s, 2H), 4.33-4.23(m, 1H), 3.67-3.52(m, 1H), 3.33-3.28(m, 1H), 3.25-3.14(m, 3H), 2.00-1.87(m, 2H), 1.55-1.44(m, 6H), 1.31-1.22(m, 18 H), 0.90-0.85(m, 3H).

### (step 4)

### Synthesis of benzyl N-[(5S)-6-(dodecylamino)-5-(hexadecanoylamino)-6-oxo-hexyl]carbamate

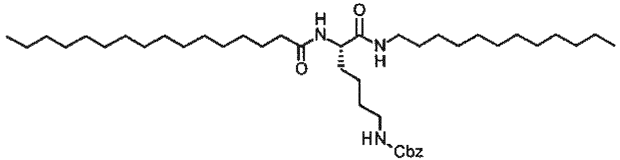

To a dichloromethane solution (40 mL) of palmitic acid (847 mg, 3.30 mmol) were added DMF (0.4 mL) and oxalyl dichloride (524 mg, 4.13 mmol) under ice-cooling and the mixture was stirred at room temperature for 2 hr. The reaction mixture was concentrated and the obtained residue was dissolved in THF (5 mL). This was added to a THF solution (50 mL) of benzyl N-[(5S)-5-amino-6-(dodecylamino)-6-oxo-hexyl]carbamate hydrochloride (2.05 g, 4.13 mmol) obtained in step 3 and triethylamine (1.7 g, 16.5 mmol) and the mixture was stirred at room temperature overnight. The reaction mixture was diluted with water and extracted with ethyl acetate. The organic layer was washed with saturated brine, dried over anhydrous sodium sulfate and concentrated. The obtained residue was purified by silica gel chromatography (dichloromethane/methanol=50/1) to give the title compound (1.1 g, yield 50%).
¹H-NMR (300 MHz, CDCl₃) δ7.37-7.32(m, 5H), 6.47-6.43(m, 1H), 6.33(d, J=8.1Hz, 1H), 5.08(s, 2H), 4.99-4.94(m, 1H), 4.41-4.34(m, 1H), 3.24-3.15(m, 4H), 2.21-2.16(m, 2H), 1.87-1.80(m, 1H), 1.69-1.49(m, 7H), 1.45-1.37(m, 2H), 1.32-1.25(m, 42H), 0.90-0.86(m, 6H).

### (step 5)

### Synthesis of N-[(1S)-5-amino-1-(dodecylcarbamoyl)pentyl]hexadecanamide hydrochloride

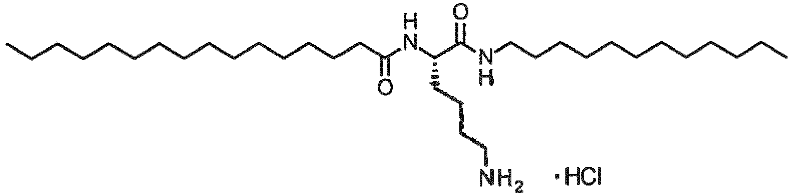

To a methanol solution (22 mL) of benzyl N-[(5S)-6-(dodecylamino)-5-(hexadecanoylamino)-6-oxo-hexyl]carbamate (1.1 g, 1.60 mmol) obtained in step 4 was added 10% palladium-carbon (110 mg) and the mixture was stirred under a hydrogen atmosphere at room temperature for 2 hr. Insoluble material was filtered off and the filtrate was concentrated. To the obtained residue (884 mg, 1.60 mmol) was added a hydrochloric acid/methanol solution (10 mL) and the mixture was stirred at room temperature for 1 hr. The reaction mixture was concentrated, to the residue was added methanol and the mixture was stirred at 70°C for 1 hr. The mixture was slowly cooled to room temperature and the solid was collected by filtration to give the title compound (380 mg, yield 40%).
¹H-NMR (400 MHz, CD₃OD) δ4.30-4.26(m, 1H), 3.21-3.12(m, 2H), 2.93-2.88(m, 2H), 2.26-2.22(m, 2H), 1.83-1.77(m, 1H), 1.69-1.60(m, 5H), 1.50-1.40(m, 4H), 1.38-1.28(m, 42H), 0.91-0.87(m, 6H).

### Example 13

### N-[(1S)-1-(dodecylcarbamoyl)pentyl]docosanamide (SZ155)

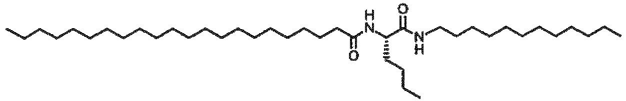

### (step 1)

### Synthesis of tert-butyl N-[(1S)-1-(dodecylcarbamoyl)pentyl]carbamate

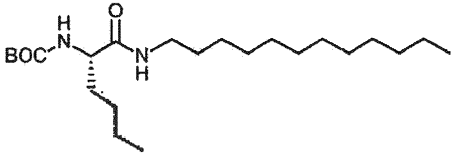

An operation similar to that in Example 6, step 1 was performed using (2S)-2-(tert-butoxycarbonylamino)hexanoic acid (1.0 g, 4.33 mmol) instead of (2S)-2-(tert-butoxycarbonylamino)propanoic acid to give the title compound (1.60 g, yield 93%).

### (step 2)

### Synthesis of (2S)-2-amino-N-dodecyl-hexanamide hydrochloride

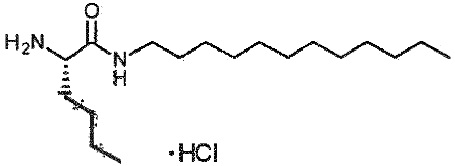

To a methanol solution (5 mL) of tert-butyl N-[(1S)-1-(dodecylcarbamoyl)pentyl]carbamate (1.6 g, 4.02 mmol) obtained in step 1 was added a hydrochloric acid/1,4-dioxane solution (15 mL) and the mixture was stirred at room temperature for 3 hr. The reaction mixture was concentrated under reduced pressure, to obtained residue was added saturated aqueous sodium hydrogen carbonate solution and the mixture was extracted with ethyl acetate. The organic layer was dried over anhydrous sodium sulfate and concentrated under reduced pressure to give the title compound (1.1 g, yield 92%) without purification.

### (step 3)

### Synthesis of N-[(1S)-1-(dodecylcarbamoyl)pentyl]docosanamide

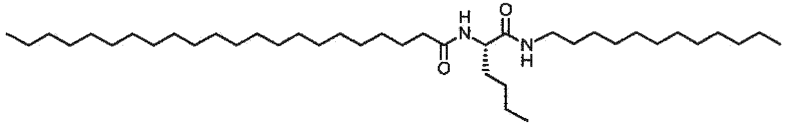

To a dichloromethane solution (20 mL) of behenic acid (1.14 g, 3.36 mmol) were added DMF (0.3 mL) and oxalyl dichloride (0.511 mg, 4.03 mmol) and the mixture was stirred at room temperature for 2 hr. The reaction mixture was concentrated and the obtained residue was dissolved in THF (5 mL). This was added a THF solution (20 mL) of (2S)-2-amino-N-dodecyl-hexanamide hydrochloride (1.10 g, 3.69 mmol) obtained in step 2 and triethylamine (1.36 g, 13.4 mmol) and the mixture was stirred at room temperature overnight. Water was added to the reaction mixture, and the mixture was stirred at room temperature for 10 min. The obtained solid was collected by filtration, and the obtained crude crystals were triturated in methanol and collected by filtration to give the title compound (1.20 g, yield 57%).
¹H-NMR (400 MHz, CDCl₃) δ6.26-6.23(m, 1H), 6.14(d, J=7.6Hz, 1H), 4.42-4.36(m, 1H), 3.32-3.21(m, 2H), 2.23(t, J=7.6Hz, 2H), 1.90-1.81(m, 1H), 1.68-1.60(m, 3H), 1.55-1.48(m, 2H), 1.40-1.19(m, 58H), 0.93-0.88(m, 9H).

### Example 14

### 1-docosanoyl-N-dodecyl-piperidine-4-carboxamide (SZ156)

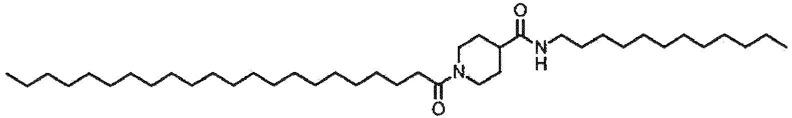

### (step 1)

### Synthesis of tert-butyl 4-(dodecylcarbamoyl)piperidine-1-carboxylate

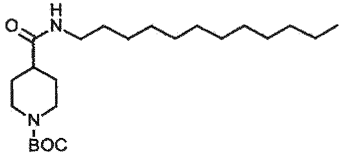

An operation similar to that in Example 6, step 1 was performed using 1-tert-butoxycarbonylpiperidine-4-carboxylic acid (2.25 g, 5.00 mmol) instead of (2S)-2-(tert-butoxycarbonylamino)propanoic acid to give the title compound (1.5 g, yield 76%).
¹H-NMR (300 MHz, CDCl₃) δ5.47(t, J=5.1Hz, 1H), 4.13(brs, 2H), 3.23-3.17(m, 2H), 2.76(t, J=15.3Hz, 2H), 2.02(t, J=15.0Hz, 1H), 1.82-1.76(m, 2H), 1.67-1.59(m, 3H), 1.45(s, 9H), 1.25(m, 19H), 0.89(t, J=6.3Hz, 3H).

### (step 2)

### Synthesis of N-dodecylpiperidine-4-carboxamide hydrochloride

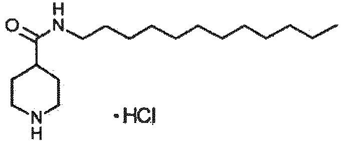

An operation similar to that in Example 13, step 2 was performed using tert-butyl 4-(dodecylcarbamoyl)piperidine-1-carboxylate (1.5 g, 3.8 mmol) obtained in step 1 instead of tert-butyl N-[(1S)-1-(dodecylcarbamoyl)pentyl]carbamate to give the title compound (1.2 g, yield 95%).

### (step 3)

### Synthesis of 1-docosanoyl-N-dodecyl-piperidine-4-carboxamide

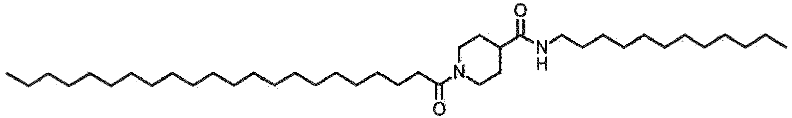

An operation similar to that in Example 13, step 3 was performed using N-dodecylpiperidine-4-carboxamide hydrochloride (1.3 g, 4.02 mmol) obtained in step 2 instead of (2S)-2-amino-N-dodecyl-hexanamide hydrochloride and diisopropylethylamine (hereinafter to be also referred to as "DIPEA") instead of triethylamine to give the title compound (0.28 g, yield 12%).
¹H-NMR (400 MHz, CDCl₃) δ5.44(brs, 1H), 4.62(d, J=14Hz, 1H), 3.95(d, J=12.4Hz, 1H), 3.24(q, J=6.8Hz, 2H), 3.07-3.01(m, 1H), 2.65-2.59(m, 1H), 2.33-2.26(m, 3H), 1.90-1.82(m, 2H), 1.65-1.58(m, 8H), 1.48(t, J=6.8Hz, 2H), 1.25(m, 50H), 0.89-0.86(m, 6H) .

### Example 15

### N-[(1S)-1-(diheptylcarbamoyl)-4-guanidino-butyl]docosanamide hydrochloride (SZ159)

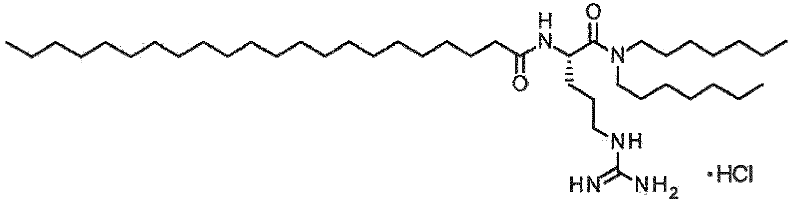

### (step 1)

### Synthesis of tert-butyl N-[(1S)-1-(diheptylcarbamoyl)-4-[(N-nitrocarbamimidoyl) amino]butyl]carbamate

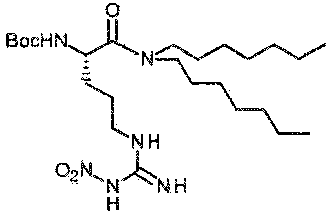

(2S)-2-(tert-Butoxycarbonylamino)-5-[(N-nitrocarbamimidoyl)amino]pentanoic acid (1.50 g, 4.69 mmol), diheptylamine (1.0 g, 4.69 mmol), EDCI (1.17 g, 6.07 mmol) and HOBt (820 mg, 6.07 mmol) were dissolved in DMF (30 mL), and the mixture was stirred at 30°C overnight. The reaction mixture was diluted with water and extracted with ethyl acetate. The organic layer was washed with 10% aqueous citric acid solution, saturated aqueous sodium hydrogen carbonate solution and saturated brine, dried over anhydrous sodium sulfate, and concentrated. The obtained residue was purified by silica gel chromatography (petroleum ether/ethyl acetate=1/1) to give the title compound (400 mg, yield 17%).
¹H-NMR (400 MHz, CDCl₃) δ8.73-8.65(m, 1H), 7.73-7.59(m, 2H), 5.68(d, J=8.0Hz, 1H), 4.49-4.44(m, 1H), 3.53-3.40(m, 2H), 3.19-2.94(m, 4H), 1.61-1.44(m, 8H), 1.38(s, 9H), 1.28-1.15(m, 16H), 0.83-0.80(m, 6H).

### (step 2)

### Synthesis of (2S)-2-amino-N,N-diheptyl-5-[(N-nitrocarbamimidoyl)amino]pentanamide hydrochloride

### ·HCl

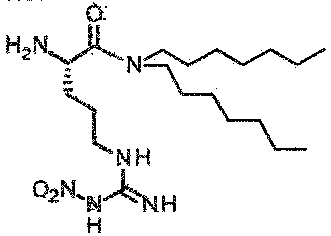

An operation similar to that in Synthetic Example 1, step 2 was performed using tert-butyl N-[(1S)-1-(diheptylcarbamoyl)-4-[(N-nitrocarbamimidoyl)amino]butyl]carbamate (400 mg, 0.78 mmol) obtained in step 1 instead of tert-butyl N-[(1S)-1-(dodecylcarbamoyl)-4-[(N-nitrocarbamimidoyl)amino]butyl]carbamate to give the title compound (350 mg, yield quantitative).
¹H-NMR (400 MHz, CDCl₃) δ8.62-8.48(m, 1H), 8.07-7.94(m, 2H), 3.64-3.60(m, 1H), 3.50-3.43(m, 1H), 3.32-2.98(m, 2H), 1.78-1.62(m, 4H), 1.55-1.42(m, 4H), 1.30-1.14(m, 16H), 0.84-0.79(m, 6H) .

### (step 3)

### Synthesis of N-[(1S)-1-(diheptylcarbamoyl)-4-[(N-nitrocarbamimidoyl)amino]butyl]docosanamide

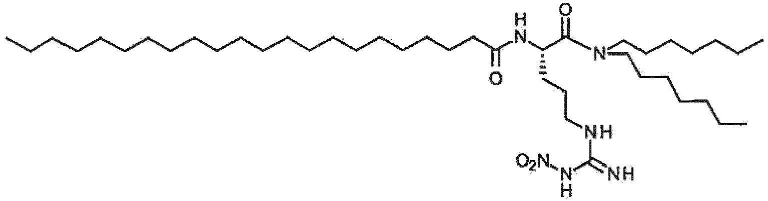

To a dichloromethane solution (10 mL) of behenic acid (241 mg, 0.74 mmol) were added under ice-cooling a catalytic amount of DMF and oxalyl dichloride (108 mg, 0.85 mmol) and the mixture was stirred at room temperature for 2 hr. The reaction mixture was concentrated and the obtained residue was dissolved in THF (2 mL). This was added to a THF solution (10 mL) of (2S)-2-amino-N,N-diheptyl-5-[(N-nitrocarbamimidoyl)amino]pentanamide hydrochloride (350 mg, 0.78 mmol) obtained in step 2 and triethylamine (285 mg, 2.82 mmol) and the mixture was stirred at room temperature overnight. The reaction mixture was concentrated and the obtained residue was purified by silica gel chromatography (dichloromethane/methanol=50/1) to give the title compound (400 mg, yield 77%).
¹H-NMR (400 MHz, CDCl₃) δ8.74-8.66(m, 1H), 7.86-7.70(m, 2H), 6.78(d, J=8.0Hz, 1H), 4.88-4.83 (m, 1H), 3.66-3.47(m, 2H), 3.25-2.99(m, 1H), 3.25(t, J=7.6Hz, 2H), 1.72-1.47(m, 10H), 1.28-1.17(m, 52H), 0.95-0.87 (m, 9H).

### (step 4)

### Synthesis of N-[(1S)-1-(diheptylcarbamoyl)-4-guanidino-butyl]docosanamide hydrochloride

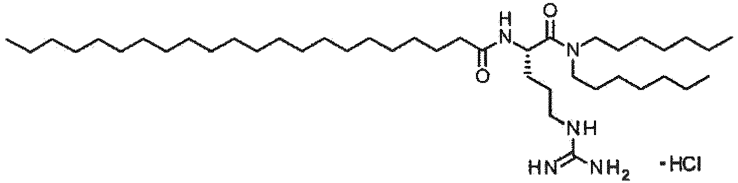

An operation similar to that in Example 2, step 2 was performed using N-[(1S)-1-(diheptylcarbamoyl)-4-[(N-nitrocarbamimidoyl)amino]butyl]docosanamide (400 mg, 0.54 mmol) obtained in step 3 instead of N-[(1S)-1-(dodecylcarbamoyl)-4-[(N-nitrocarbamimidoyl)amino]butyl]octadecanamide to give the title compound (230 mg, yield 58%).
¹H-NMR (400 MHz, CD₃OD) δ4.33-4.29(m, 1H), 3.46-3.36(m, 3H), 3.27-3.13(m, 3H), 2.22(t, J=7.6Hz, 2H), 1.75-1.48(m, 10H), 1.39-1.22(m, 52H), 0.93-0.88(m, 9H).
MS(ESI)m/z:692 [M+H]⁺

### Example 16

### N-[(1S)-5-amino-1-(dodecylcarbamoyl)pentyl]docosanamide hydrochloride (SZ160)

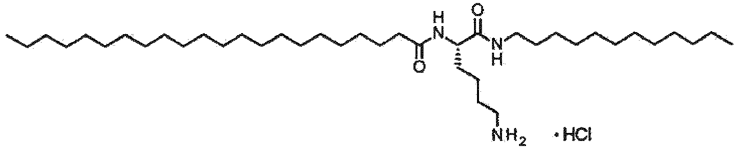

### (step 1)

### Synthesis of benzyl N-[(5S)-5-(docosanoylamino)-6-(dodecylamino)-6-oxo-hexyl]carbamate

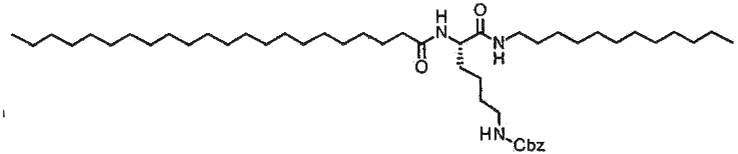

An operation similar to that in Example 12, step 4 was performed using behenic acid (2.8 g, 8.26 mmol) instead of palmitic acid to give the title compound (4.2 g, yield 66%).
¹H-NMR (300 MHz, CDCl₃) δ7.38-7.30(m, 5H), 6.47-6.43(m, 1H), 6.32(d, J=9.0Hz, 1H), 5.08(s, 2H), 4.98-4.94(m, 1H), 4.40-4.33(m, 1H), 3.23-3.14(m, 4H), 2.21-2.16(m, 2H), 1.88-1.79(m, 1H), 1.66-1.48(m, 7H), 1.45-1.39(m, 2H), 1.37-1.25(m, 54H), 0.90-0.86(m, 6H).

### (step 2)

### Synthesis of N-[(1S)-5-amino-1-(dodecylcarbamoyl)pentyl]docosanamide

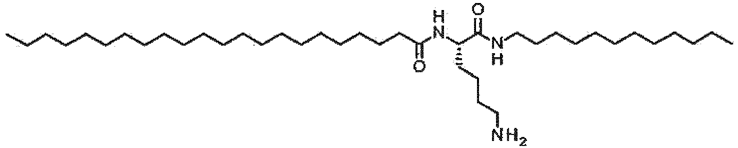

To a methanol solution (90 mL) of benzyl N-[(5S)-5-(docosanoylamino)-6-(dodecylamino)-6-oxo-hexyl]carbamate (4.2 g, 5.45 mmol) obtained in step 1 was added 10% palladium-carbon (420 mg) and the mixture was stirred under a hydrogen atmosphere at room temperature for 2 hr. Insoluble material was filtered off and the filtrate was concentrated to give the title compound (3.0 g, yield 88%).
¹H-NMR (300 MHz, CDCl₃) δ6.82-6.76(m, 1H), 6.45(d, J=7.5Hz, 1H), 4.46-4.38(m, 1H), 3.25-3.18(m, 2H), 2.79-2.75(m, 2H), 2.56-2.48(m, 4H), 2.22-2.17(m, 2H), 1.87-1.75(m, 1H), 1.66-1.41(m, 9H), 1.39-1.20(m, 52H), 0.90-0.86(m, 6H).

### (step 3)

### Synthesis of N-[(1S)-5-amino-1-(dodecylcarbamoyl)pentyl]docosanamide hydrochloride

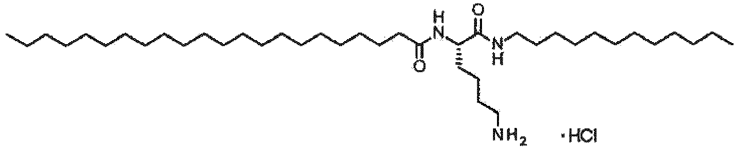

To N-[(1S)-5-amino-1-(dodecylcarbamoyl)pentyl]docosanamide (600 mg, 0.943 mmol) obtained in step 2 was added a hydrochloric acid/methanol solution (10 mL) and the mixture was stirred at room temperature for 1 hr. The reaction mixture was concentrated, methanol was added to the residue and the mixture was stirred at 70°C for 1 hr. The mixture was slowly cooled to room temperature and the solid was collected by filtration to give the title compound (380 mg, yield 60%).
¹H-NMR (400 MHz, CD₃OD) δ4.30-4.26(m, 1H), 3.20-3.10(m, 2H), 2.92-2.88(m, 2H), 2.26-2.22(m, 2H), 1.84-1.80(m, 1H), 1.75-1.65(m, 5H), 1.60-1.47(m, 4H), 1.42-1.28(m, 54H), 0.91-0.88(m, 6H).
MS(ESI)m/z:637[M+H]⁺

### Example 17

### (E)-N-[(1S)-1-(dodecylcarbamoyl)-4-guanidino-butyl]octadeca-9-enamide hydrochloride (SZ161)

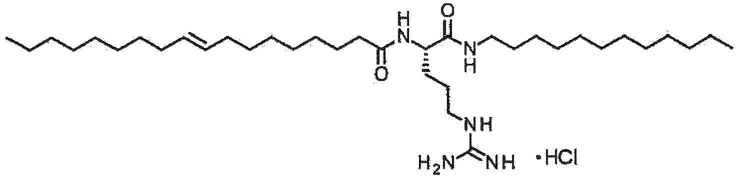

### (step 1)

### Synthesis of 9H-fluorene-9-ylmethyl N-[(1S)-4-[[(E)-N,N'-bis(tert-butoxycarbonyl)carbamimidoyl]amino]-1-(dodecylcarbamoyl)butyl]carbamate

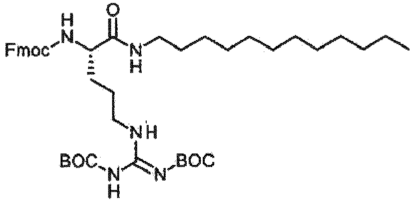

An operation similar to that in Synthetic Example 1, step 1 was performed using (2S)-5-[[(E)-N,N'-bis(tert-butoxycarbonyl)carbamimidoyl]amino]-2-(9H-fluorene-9-yl methoxycarbonylamino)pentanoic acid (3.5 g, 5.87 mmol) instead of (2S)-2-(tert-butoxycarbonylamino)-5-[(N-nitrocarbamimidoyl)amino]pentanoic acid to give the title compound (2.78 g, yield 62%).
¹H-NMR (400 MHz, CDCl₃) δ8.49(brs, 1H), 7.80(d, J=7.6Hz, 2H), 7.65(t, J=7.6Hz, 2H), 7.45-7.30(m, 4H), 4.51-4.41(m, 1H), 4.30-4.23(m, 2H), 4.17-4.13(m, 1H), 3.50-3.17(m, 4H), 1.86-1.65(m, 6H), 1.53(d, J=9.2Hz, 18H), 1.44-1.27(m, 18H), 0.92(t, J=7.2Hz, 3H) .

### (step 2)

### Synthesis of tert-butyl (NE)-N-[[[(4S)-4-amino-5-(dodecylamino)-5-oxo-pentyl]amino]-(tert-butoxycarbonylamino)methylene]carbamate

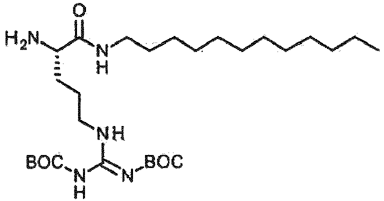

To 9H-fluorene-9-ylmethyl N-[(1S)-4-[[(E)-N,N'-bis(tert-butoxycarbonyl)carbamimidoyl]amino]-1-(dodecylcarbamoyl)butyl]carbamate (2.66 g, 3.49 mmol) obtained in step 1 was added 20% piperidine/dichloromethane solution (v/v=1/5, 30 mL) and the mixture was stirred at room temperature for 1 hr. The reaction mixture was concentrated and the obtained residue was purified by silica gel chromatography (dichloromethane/methanol=40/1) to give the title compound (2.0 g, yield 100%).
MS(ESI)m/z:542[M+H]⁺

### (step 3)

### Synthesis of tert-butyl (NE)-N-[(tert-butoxycarbonylamino)-[[(4S)-5-(dodecylamino)-4-[[(E)-octadeca-9-enoyl]amino]-5-oxo-pentyl]amino]methylene]carbamate

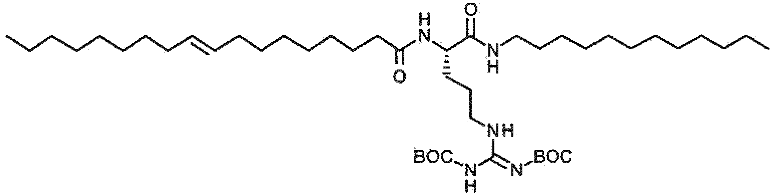

To a dichloromethane solution (15 mL) of 9-octadecenoic acid (260 mg, 0.92 mmol) were slowly added DMF (0.5 mL) and oxalyl dichloride (0.14 mg, 1.10 mmol) and the mixture was stirred at room temperature for 2 hr. The reaction mixture was concentrated and the obtained residue was dissolved in THF (3 mL). This was added to a THF solution (15 mL) of tert-butyl (NE)-N-[[[(4S)-4-amino-5-(dodecylamino)-5-oxo-pentyl]amino]-(tert-butoxycarbonylamino)methylene]carbamate (500 mg, 0.92 mmol) obtained in step 2 and triethylamine (371 mg, 3.68 mmol), and the mixture was stirred at room temperature overnight. The reaction mixture was diluted with water, extracted with ethyl acetate and the organic layer was dried over anhydrous sodium sulfate. The solvent was evaporated under reduced pressure to give the title compound (500 mg, yield 67%) without purification.
¹H-NMR (400 MHz, CDCl₃) δ5.35-5.33(m, 2H), 4.49-4.48(m, 1H), 3.55-3.42(m, 4H), 2.27-2.22(m, 2H), 2.01-2.00(m, 4H), 1.85-1.70(m, 8H), 1.49(d, J=4.0Hz, 18H), 1.37-1.21(m, 38H), 0.88(t, J=6.8Hz, 6H).

### (step 4)

### Synthesis of (E)-N-[(1S)-1-(dodecylcarbamoyl)-4-guanidino-butyl]octadeca-9-enamide hydrochloride

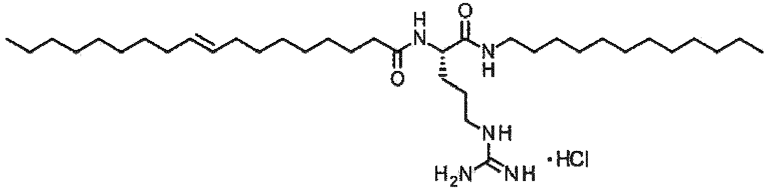

To tert-butyl (NE)-N-[(tert-butoxycarbonylamino)-[[(4S)-5-(dodecylamino)-4-[[(E)-octadeca-9-enoyl]amino]-5-oxo-pentyl]amino]methylene]carbamate (500 mg, 0.62 mmol) obtained in step 3 were added trifluoroacetic acid (hereinafter to be also referred to as "TFA") (5 mL) and dichloromethane (5 mL) and the mixture was stirred at room temperature for 2 hr. The reaction mixture was poured into water (30 mL), adjust same to pH8 - 9 with saturated aqueous sodium carbonate solution, and extracted with ethyl acetate. The organic layer was dried over anhydrous sodium sulfate, the solvent was evaporated, to the obtained residue was added a hydrochloric acid/1,4-dioxane solution (5 mL), and the mixture was stirred at room temperature for 2 hr. The solvent was evaporated under reduced pressure and the obtained residue was purified by silica gel chromatography (dichloromethane/methanol=10/1) to give the title compound (136 mg, yield 34%).
¹H-NMR (400 MHz, CD₃OD) δ5.38-5.30(m, 2H), 4.32(brs, 1H), 3.26-3.11(m, 4H), 2.29-2.20(m, 2H), 2.07-1.96(m, 4H), 1.85-1.77(m, 1H), 1.70-1.43(m, 7H), 1.32-1.10(m, 38H), 0.90(t, J=6.4Hz, 6H). MS(ESI)m/z:607[M+H]⁺

### Example 18

### N-[(1S)-4-guanidino-1-(heptylcarbamoyl)butyl]docosanamide hydrochloride (SZ163)

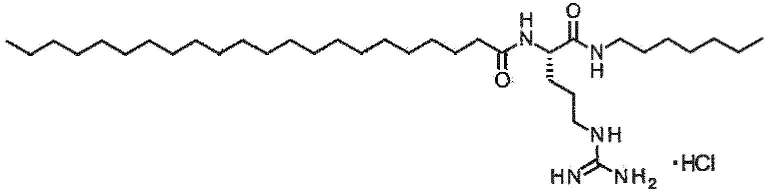

### (step 1)

### Synthesis of tert-butyl N-[(1S)-1-(heptylcarbamoyl)-4-[(N-nitrocarbamimidoyl)amino]butyl]carbamate

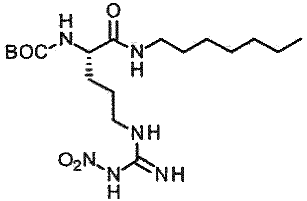

An operation similar to that in Synthetic Example 1, step 1 was performed using heptane-1-amine (649 mg, 5.64 mmol) instead of dodecylamine to give the title compound (1.45 g, yield 74%).
¹H-NMR (300 MHz, CDCl₃) δ7.69(brs, 1H), 6.68(brs, 1H), 5.53(brs, 1H), 4.22(brs, 1H), 3.55-3.20(m, 4H), 1.80-1.77(m, 2H), 1.56-1.39(m, 13H), 1.35-1.22(m, 8H), 0.88(t, J=8.0Hz, 3H).

### (step 2)

### Synthesis of (2S)-2-amino-N-heptyl-5-[(N-nitrocarbamimidoyl)amino]pentanamide hydrochloride

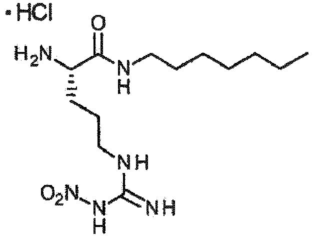

An operation similar to that in Example 7, step 2 was performed using tert-butyl N-[(1S)-1-(heptylcarbamoyl)-4-[(N-nitrocarbamimidoyl)amino]butyl]carbamate (1.45 g, 3.49 mmol) obtained in step 1 instead of tert-butyl N-[2-(dodecylamino)-1,1-dimethyl-2-oxo-ethyl]carbamate to give the title compound (1.30 g, yield quantitative).
¹H-NMR (400 MHz, DMSO-d₆) δ8.55(brs, 1H), 8.27(s, 3H), 8.20-7.92(m, 2H), 6.54(brs, 2H), 3.76-3.74(m, 1H), 3.19-3.01(m, 4H), 1.78-1.67(m, 2H), 153-1.41(m, 4H), 1.36-1.17(m, 8H), 0.86(t, J=7.2Hz, 3H).

### (step 3)

### Synthesis of N-[(1S)-1-(heptylcarbamoyl)-4-[(N-nitrocarbamimidoyl)amino]butyl]docosanamide

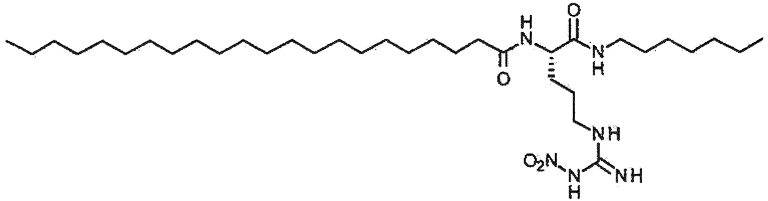

An operation similar to that in Example 13, step 3 was performed using (2S)-2-amino-N-heptyl-5-[(N-nitrocarbamimidoyl)amino]pentanamide hydrochloride (1.10 g, 3.47 mmol) obtained in step 2 instead of (2S)-2-amino-N-dodecyl-hexanamide hydrochloride to give the title compound (2.0 g, yield quantitative).
¹H-NMR (400 MHz, CDCl₃) δ4.69-4.66 (m, 1H), 3.61-3.49(m, 1H), 3.32-3.19(m, 3H), 2.24(t, J=7.2Hz, 2H), 1.75-1.41(m, 8H), 1.33-1.18(m, 44H), 0.93-0.84 (m, 6H).

### (step 4)

### Synthesis of N-[(1S)-4-guanidino-1-(heptylcarbamoyl)butyl]docosanamide hydrochloride

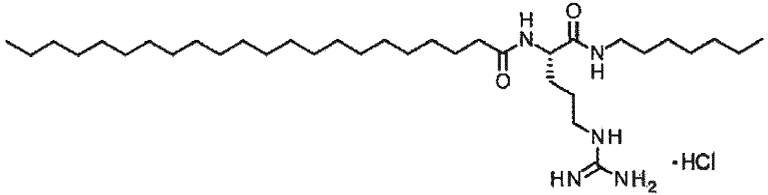

To a methanol solution (50 mL) of N-[(1S)-1-(heptylcarbamoyl)-4-[(N-nitrocarbamimidoyl)amino]butyl]docosanamide (2.0 g, 3.13 mmol) obtained in step 3 were added concentrated hydrochloric acid (1 mL) and 10% palladium-carbon (500 mg), and the mixture was stirred at 50°C overnight. Insoluble material was filtered off, the filtrate was recrystallized from methanol to give the title compound (500 mg, yield 25%).
¹H-NMR (400 MHz, DMSO-d₆) δ7.95-7.87(m, 2H), 7.57(brs, 1H), 7.31-6.83(m, 2H), 4.24-4.18(m, 1H), 3.10-3.00(m, 4H), 2.11(t, J=7.6Hz, 2H), 1.64-1.58(m, 1H), 1.48-1.32(m, 7H), 1.29-1.13(m, 44H), 0.85(t, J=6.8Hz, 6H).

### MS(ESI)m/z:594[M+H]⁺

### Example 19

### N-[(1S)-4-amino-1-(dodecylcarbamoyl)butyl]docosanamide hydrochloride (SZ165)

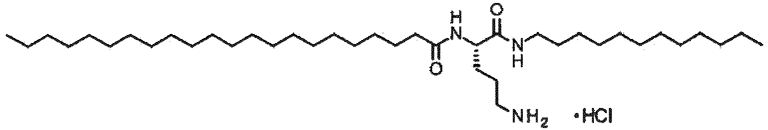

### (step 1)

### Synthesis of (2S)-5-(benzyloxycarbonylamino)-2-(tert-butoxycarbonylamino)pentanoic acid

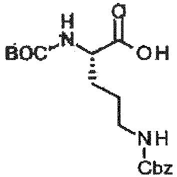

To a THF/water solution (250 mL/100 mL) of (2S)-2-amino-5-(benzyloxycarbonylamino)pentanoic acid (6.0 mg, 22.6 mmol) were added potassium carbonate (9.34 g, 67.7 mmol) and di-tert-butyl dicarbonate (5.9 g, 27.1 mmol), and the mixture was stirred at room temperature overnight. The mixture was extracted with ethyl acetate, and the organic layer was washed with saturated brine and concentrated. The obtained residue was purified by silica gel chromatography (dichloromethane/methanol=80/1) to give the title compound (8.0 g, yield 97%).
MS(ESI)m/z:367[M+H]⁺

### (step 2)

### Synthesis of tert-butyl N-[(1S)-4-(benzyloxycarbonylamino)-1-(dodecylcarbamoyl)butyl]carbamate

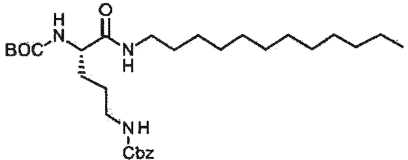

A DMF solution (40 mL) of (2S)-5-(benzyloxycarbonylamino)-2-(tert-butoxycarbonylamino)pentanoic acid (3.37 g, 9.21 mmol) obtained in step 1, HOBt (1.55 g, 11.5 mmol), EDCI (2.21 g, 11.5 mmol) and triethylamine (2.32 g, 23.0 mmol) was stirred at room temperature for 1 hr. Thereto was added dodecylamine (1.42 g, 7.67 mmol), and the mixture was stirred at room temperature overnight. The reaction mixture was concentrated, diluted with ethyl acetate, and the organic layer was washed with water and dried over anhydrous sodium sulfate. The solvent was evaporated and the obtained residue was purified by silica gel chromatography (petroleum ether/ethyl acetate=3/1) to give the title compound (3.4 g, yield 83%).
MS(ESI)m/z:534[M+H]⁺

### (step 3)

### Synthesis of benzyl N-[(4S)-4-amino-5-(dodecylamino)-5-oxo-pentyl]carbamate hydrochloride

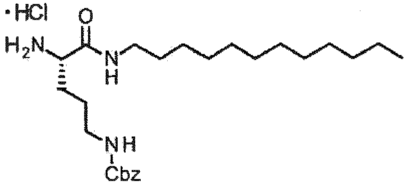

An operation similar to that in Synthetic Example 1, step 2 was performed using tert-butyl N-[(1S)-4-(benzyloxycarbonylamino)-1-(dodecylcarbamoyl)butyl]carbamate (3.4 g, 4.50 mmol) obtained in step 2 instead of tert-butyl N-[(1S)-1-(dodecylcarbamoyl)-4-[(N-nitrocarbamimidoyl)amino]butyl]carbamate to give the title compound (2.9 g, yield 97%).
MS(ESI)m/z:434[M+H]⁺

### (step 4)

### Synthesis of benzyl N-[(4S)-4-(docosanoylamino)-5-(dodecylamino)-5-oxo-pentyl]carbamate

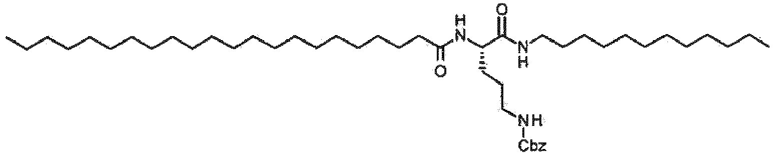

An operation similar to that in Example 13, step 3 was performed using benzyl N-[(4S)-4-amino-5-(dodecylamino)-5-oxo-pentyl]carbamate hydrochloride (1.5 g, 3.2 mmol) obtained in step 3 instead of (2S)-2-amino-N-dodecyl-hexanamide hydrochloride to give the title compound (1.2 g, yield 50%).
¹H-NMR (400 MHz, CDCl₃) δ7.35-7.31(m, 5H), 6.56(brs, 1H), 6.35(d, J=8.4Hz, 1H), 5.08-5.02(m, 3H), 4.56-4.53(m, 1H), 3.45-3.40(m, 1H), 3.21-3.15(m, 3H), 2.19(t, J=7.6Hz, 2H), 1.82-1.78(m, 1H), 1.61-1.51(m, 4H), 1.47-1.39(m, 2H), 1.25(s, 55H), 0.88(t, J=6.8Hz, 6H).

### (step 5)

### Synthesis of N-[(1S)-4-amino-1-(dodecylcarbamoyl)butyl]docosanamide hydrochloride

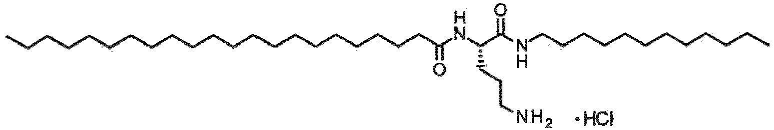

To a methanol solution (30 mL) of benzyl N-[(4S)-4-(docosanoylamino)-5-(dodecylamino)-5-oxo-pentyl]carbamate (1.2 g, 1.6 mmol) obtained in step 4 were added concentrated hydrochloric acid (0.5 mL) and 10% palladium-carbon (200 mg), and the mixture was stirred under 50 psi hydrogen atmosphere at 50°C for 4 hr. Insoluble material was filtered off, the filtrate was cooled to room temperature and the precipitated solid was collected by filtration to give the title compound (644 mg, yield 65%).
¹H-NMR (400 MHz, CD₃OD) δ4.32(t, J=6Hz, 1H), 3.23-3.11(m, 2H), 2.94(t, J=6.8Hz, 2H), 2.27-2.23(m, 2H), 1.85-1.82(m, 1H), 1.74-1.70(m, 2H), 1.69-1.60(m, 2H), 1.51-1.48(m, 2H), 1.28(s, 55H), 0.90(t, J=6.8Hz, 6H).
MS(ESI)m/z:622[M+H]⁺

### Example 20

### N-[(1S)-1-(dodecylcarbamoyl)-5-guanidino-pentyl]docosanamide hydrochloride (SZ166)

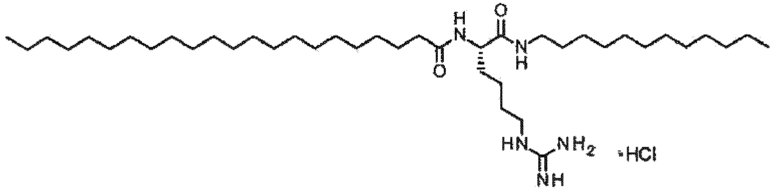

### (step 1)

### Synthesis of tert-butyl N-[(1S)-1-(dodecylcarbamoyl)-5-[(N-nitrocarbamimidoyl)amino]pentyl] carbamate

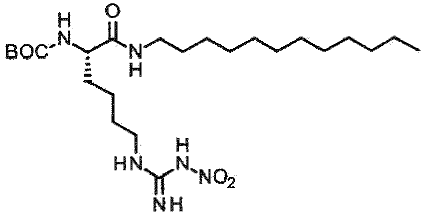

An operation similar to that in Synthetic Example 1, step 1 was performed using (2S)-2-(tert-butoxycarbonylamino)-6-[(N-nitrocarbamimidoyl)amino]hexanoic acid (1.0 g, 3.0 mmol) instead of (2S)-2-(tert-butoxycarbonylamino)-5-[(N-nitrocarbamimidoyl)amino]pentanoic acid to give the title compound (1.4 g, yield 93%).
¹H-NMR (400 MHz, CDCl₃) δ8.69(s, 1H), 7.42(brs, 2H), 6.80(s, 1H), 5.41(s, 1H), 4.27(s, 1H), 3.48(s, 2H), 3.32-3.14(m, 4H), 1.64(s, 2H), 1.56-1.47(m, 4H), 1.43(s, 9H), 1.24(s, 18H), 0.89-0.85(m, 3H).

### (step 2)

### Synthesis of (2S)-2-amino-N-dodecyl-6-[(N-nitrocarbamimidoyl)amino]hexanamide hydrochloride

### -HCl

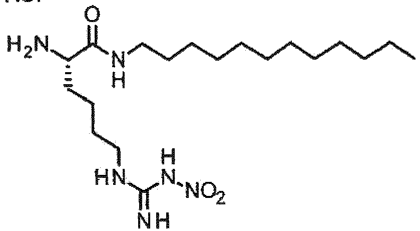

An operation similar to that in Example 7, step 2 was performed using tert-butyl N-[(1S)-1-(dodecylcarbamoyl)-5-[(N-nitrocarbamimidoyl)amino]pentyl]carbamate (1.4 g, 2.8 mmol) obtained in step 1 instead of tert-butyl N-[2-(dodecylamino)-1,1-dimethyl-2-oxo-ethyl]carbamate to give the title compound (1.1 g, yield 98%)
¹H-NMR (400 MHz, CD₃OD) δ3.85-3.82(m, 1H), 3.50-3.45(m, 1H), 3.34-3.30(m, 4H), 3.24-3.20 (m, 1H), 1.91-1.85(m, 1H), 1.67(s, 1H), 1.56-1.45(m, 4H), 1.32(s,18H), 0.91-0.87(t, J=6.8Hz, 3H).

### (step 3)

### Synthesis of N-[(1S)-1-(dodecylcarbamoyl)-5-[(N-nitrocarbamimidoyl)amino]pentyl]docosanamide

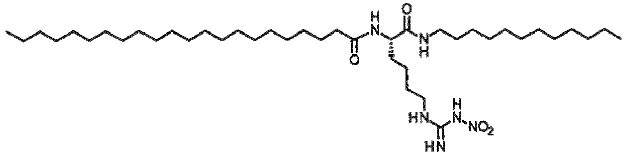

An operation similar to that in Example 3, step 1 was performed using (2S)-2-amino-N-dodecyl-6-[(N-nitrocarbamimidoyl)amino]hexanamide hydrochloride (1.3 g, 3.25 mmol) obtained in step 2 instead of (2S)-2-amino-N-dodecyl-5-[(N-nitrocarbamimidoyl)amino]pentanamide hydrochloride obtained in Synthetic Example 1 to give the title compound (980 mg, yield 41%).

### (step 4)

### Synthesis of N-[(1S)-1-(dodecylcarbamoyl)-5-guanidino-pentyl]docosanamide hydrochloride

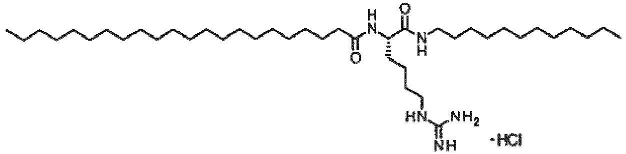

An operation similar to that in Example 2, step 2 was performed using N-[(1S)-1-(dodecylcarbamoyl)-5-[(N-nitrocarbamimidoyl)amino]pentyl]docosanamide (980 mg, 1.35 mmol) obtained in step 3 instead of N-[(1S)-1-(dodecylcarbamoyl)-4-[(N-nitrocarbamimidoyl)amino]butyl]octadecanamide to give the title compound (235 mg, yield 29%).
¹H-NMR (400 MHz, CD₃OD) δ4.28(t, J=6.0Hz, 1H), 3.19-3.14(m, 4H), 2.26-2.23(m, 2H), 1.83-1.74(m, 1H), 1.65-1.56(m, 5H), 1.49-1.42(m, 4H), 1.28(s, 54H), 0.91-0.88(m, 6H).
MS(ESI)m/z:678[M+H]⁺

### Example 21

### N-[(1S)-5-acetamide-1-(dodecylcarbamoyl)pentyl]docosanamide (SZ167)

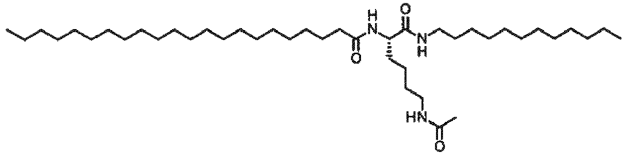

To a methanol solution (15 mL) of N-[(1S)-5-amino-1-(dodecylcarbamoyl)pentyl]docosanamide hydrochloride (500 mg, 0.786 mmol) obtained in Example 16 were added, under ice-cooling, triethylamine (239 mg, 3.36 mmol) and acetic anhydride (160 mg, 1.57 mmol), and the mixture was stirred at room temperature overnight. Water was added to the reaction mixture, the precipitated solid was washed with methanol, and the obtained crude crystals were purified by silica gel chromatography (dichloromethane/methanol=20/1) to give the title compound (525 mg, yield 98%).
¹H-NMR (400 MHz, CDCl₃) δ6.41-6.35(m, 2H), 5.85-5.80(m, 1H), 4.37-4.32(m, 1H), 3.28-3.18(m, 4H), 2.23-2.19(m, 2H), 1.97(s, 3H), 1.85-1.78(m, 1H), 1.69-1.63(m, 3H), 1.59-1.48(m, 4H), 1.46-1.38(m, 2H), 1.36-1.25(m, 54H), 0.89-0.86(m, 6H).

### Example 22

### (2S)-5-guanidino-2-(hexadecylcarbamoylamino)-N-hexyl-pentanamide hydrochloride (SZ168)

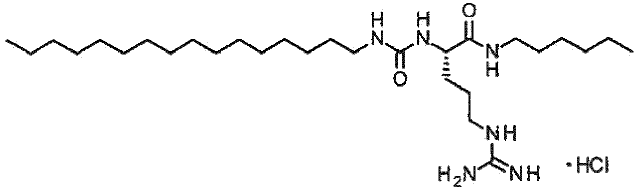

### (step 1)

### Synthesis of tert-butyl N-[(1S)-1-(hexylcarbamoyl)-4-[(N-nitrocarbamimidoyl)amino]butyl]carbamate

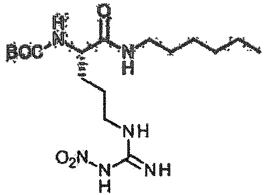

An operation similar to that in Synthetic Example 1, step 1 was performed using hexane-1-amine (2.28 g, 22.6 mmol) instead of dodecylamine to give the title compound (5.90 g, yield 78%).
¹H-NMR (400 MHz, CDCl₃) δ7.71(s, 1H), 6.70(s, 1H), 5.53(d, J=7.6Hz, 1H), 4.23(s, 1H), 3.48-3.18(m, 4H), 1.81(s, 2H), 1.78-1.66(m, 4H), 1.51-1.45(m, 2H), 1.43(s, 9H), 1.33-1.24(m, 6H), 0.89-0.85(m, 3H).

### (step 2)

### Synthesis of (2S)-2-amino-N-hexyl-5-[(N-nitrocarbamimidoyl)amino]pentanamide hydrochloride

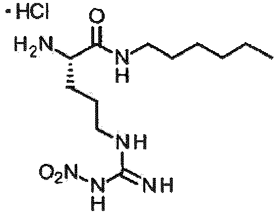

An operation similar to that in Synthetic Example 1, step 2 was performed using tert-butyl N-[(1S)-1-(hexylcarbamoyl)-4-[(N-nitrocarbamimidoyl)amino]butyl]carbamate (5.90 g, 14.7 mmol) obtained in step 1 instead of tert-butyl N-[(1S)-1-(dodecylcarbamoyl)-4-[(N-nitrocarbamimidoyl)amino]butyl]carbamate to give the title compound (4.93 g, yield 99%).
¹H-NMR (400 MHz, DMSO-d₆) δ8.74-8.60(m, 2H), 8.35(s, 3H), 8.06(s, 2H), 3.78(s, 1H), 3.17(s, 2H), 3.15-3.01(m, 2H), 1.80-1.65(m, 2H), 1.59-1.36(m, 4H), 1.25(s, 6H), 0.87-0.84(t, J=6.8Hz, 3H).

### (step 3)

### Synthesis of (2S)-5-guanidino-2-(hexadecylcarbamoylamino)-N-hexyl-pentanamide hydrochloride

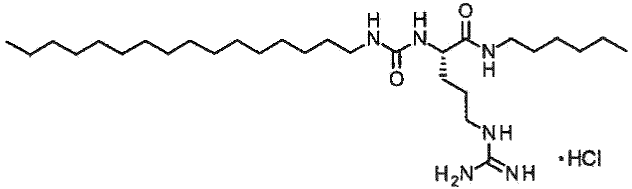

An operation similar to that in Example 10 was performed using (2S)-2-amino-N-hexyl-5-[(N-nitrocarbamimidoyl)amino]pentanamide hydrochloride (1.32 g, 3.90 mmol) obtained in step 2 instead of (2S)-2-amino-N-dodecyl-5-[(N-nitrocarbamimidoyl)amino]pentanamide hydrochloride obtained in Synthetic Example 1 to give the title compound (400 mg, yield 41%).
¹H-NMR (400 MHz, CD₃OD) δ4.20-4.17 (m, 1H), 3.31-3.07 (m, 6H), 1.78-1.46(m, 8H), 1.37-1.26(m, 32H), 0.94-0.88(m, 6H).
MS (ESI)m/z:525 [M+H]⁺

### Example 23

### Hexadecyl N-[(1S)-4-guanidino-1-(hexylcarbamoyl)butyl]carbamate hydrochloride (SZ169)

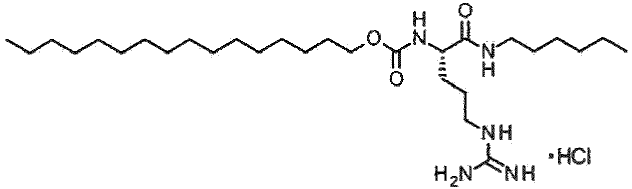

### (step 1)

### Synthesis of hexadecyl N-[(1S)-1-(hexylcarbamoyl)-4-[(N-nitrocarbamimidoyl)amino]butyl]carbamate

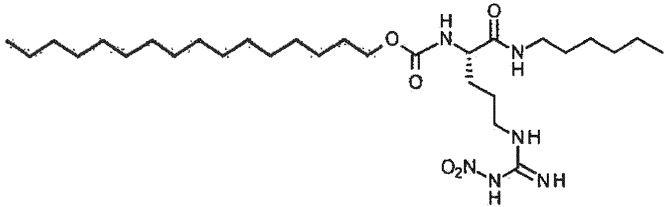

An operation similar to that in Example 11, step 1 was performed using (2S)-2-amino-N-hexyl-5-[(N-nitrocarbamimidoyl)amino]pentanamide hydrochloride obtained in Example 22, step 2 (1.58 g, 4.66 mmol) instead of (2S)-2-amino-N-dodecyl-5-[(N-nitrocarbamimidoyl)amino]pentanamide hydrochloride obtained in Synthetic Example 1 to give the title compound (2.5 g, yield 94%).
MS(ESI)m/z:571 [M+H]⁺

### (step 2)

### Synthesis of hexadecyl N-[(1S)-4-guanidino-1-(hexylcarbamoyl)butyl]carbamate hydrochloride

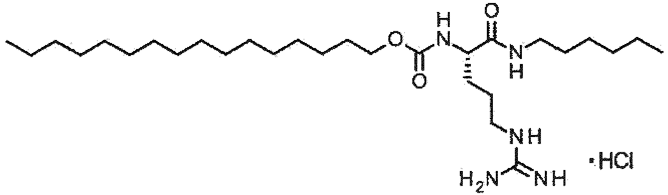

An operation similar to that in Example 11, step 2 was performed using hexadecyl N-[(1S)-1-(hexylcarbamoyl)-4-[(N-nitrocarbamimidoyl)amino]butyl]carbamate (2.5 g, 4.38 mmol) obtained in step 1 instead of hexadecyl N-[(1S)-1-(dodecylcarbamoyl)-4-[(N-nitrocarbamimidoyl)amino]butyl] carbamate to give the title compound (240 mg, yield 10%).
¹H-NMR (400 MHz, CD₃OD) δ4.07-4.00(m, 3H), 3.20-3.16(m, 4H), 1.83-1.76(m, 1H), 1.68-1.59(m, 5H), 1.51-1.48(m, 2H), 1.35-1.24(m, 32H), 0.92-0.88(m, 6H).
MS(ESI)m/z:526[M+H]⁺

### Example 24

### N-[(1S)-5-guanidino-1-(hexylcarbamoyl)pentyl]docosanamide hydrochloride (SZ170)

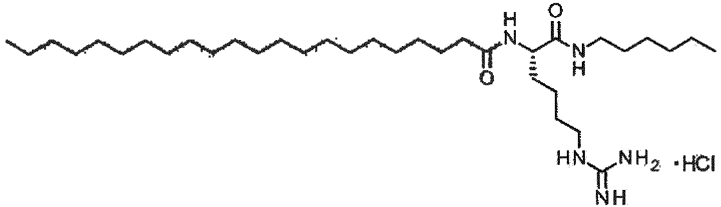

### (step 1)

### Synthesis of tert-butyl N-[(1S)-1-(hexylcarbamoyl)-5-[(N-nitrocarbamimidoyl)amino]pentyl]carbamate

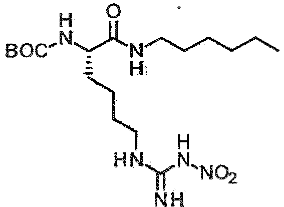

An operation similar to that in Example 1, step 1 was performed using (2S)-2-(tert-butoxycarbonylamino)-6-[(N-nitrocarbamimidoyl)amino]hexanoic acid (1.0 g, 3.0 mmol) instead of (2S)-2-(tert-butoxycarbonylamino)-5-[(N-nitrocarbamimidoyl)amino]pentanoic acid and hexane-1-amine (364 mg, 3.6 mmol) instead of dodecylamine to give the title compound (1.1 g, yield 88%).

### (step 2)

### Synthesis of (2S)-2-amino-N-hexyl-6-[(N-nitrocarbamimidoyl)amino]hexanamide hydrochloride

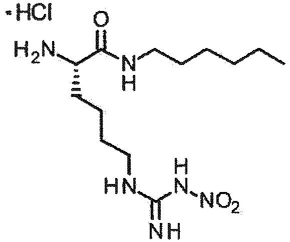

An operation similar to that in Example 7, step 2 was performed using tert-butyl N-[(1S)-1-(hexylcarbamoyl)-5-[(N-nitrocarbamimidoyl)amino]pentyl]carbamate (1.1 g, 2.64 mmol) obtained in step 1 instead of tert-butyl N-[2-(dodecylamino)-1,1-dimethyl-2-oxo-ethyl]carbamate to give the title compound (900 mg, yield quantitative).
¹H-NMR (400 MHz, CD₃OD) δ3.86-3.83(m, 1H), 3.63-3.62(m, 2H), 3.50-3.45(m, 2H), 3.34-3.30(m, 2H), 3.24-3.20(m, 1H), 1.91-1.83(m, 1H), 1.68(s, 1H), 1.58-1.47(m, 3H), 1.41-1.32(m, 4H), 0.94-0.89(m, 3H).

### (step 3)

### Synthesis of N-[(1S)-1-(hexylcarbamoyl)-5-[(N-nitrocarbamimidoyl)amino]pentyl]docosanamide

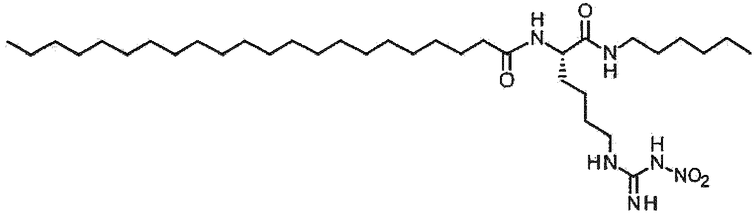

An operation similar to that in Example 3, step 1 was performed using (2S)-2-amino-N-hexyl-6-[(N-nitrocarbamimidoyl)amino]hexanamide hydrochloride (500 mg, 1.58 mmol) obtained in step 2 instead of (2S)-2-amino-N-dodecyl-5-[(N-nitrocarbamimidoyl)amino]pentanamide hydrochloride obtained in Synthetic Example 1 to give the title compound (580 mg, yield 72%).

### (step 4)

### Synthesis of N-[(1S)-5-guanidino-1-(hexylcarbamoyl)pentyl]docosanamide hydrochloride

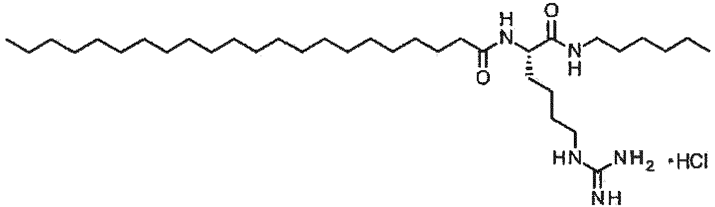

An operation similar to that in Example 18, step 4 was performed using N-[(1S)-1-(hexylcarbamoyl)-5-[(N-nitrocarbamimidoyl)amino]pentyl]docosanamide (580 mg, 0.90 mmol) obtained in step 3 instead of N-[(1S)-1-(heptylcarbamoyl)-4-[(N-nitrocarbamimidoyl)amino]butyl]docosanamide to give the title compound (509 mg, yield 38%).
¹H-NMR (400 MHz, CD₃OD) δ4.30-4.26(m, 1H), 3.19-3.12(m, 4H), 2.26-2.22(m, 2H), 1.82-1.74(m, 1H), 1.64-1.56(m, 5H), 1.50-1.41(m, 4H), 1.28(s, 42H), 0.897(t, J=6.8Hz, 6H). MS(ESI)m/z:594[M+H]⁺

### Example 25

### N-[(1R)-1-[[[(2R)-2-(docosanoylamino)-3-(dodecylamino)-3-oxo-propyl]disulfanyl]methyl]-2-(dodecylamino)-2-oxoethyl]docosanamide (SZ173)

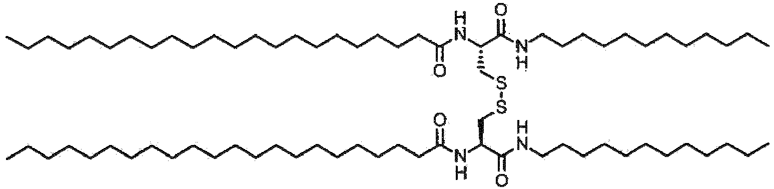

### (step 1)

### Synthesis of benzyl N-[(1R)-1-[[[(2R)-2-(benzyloxycarbonylamino)-3-(dodecylamino)-3-oxopropyl]disulfanyl]methyl]-2-(dodecylamino)-2-oxoethyl]carbamate

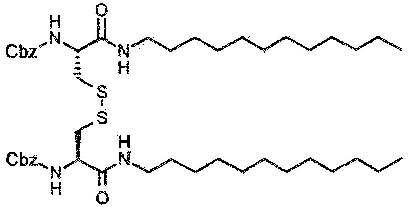

(2R)-2-(Benzyloxycarbonylamino)-3-[[(2R)-2-(benzyloxycarbonylamino)-3-hydroxy-3-oxopropyl]disulfanyl]propanoic acid (1.5 g, 2.9 mmol), dodecylamine (1.18 g, 6.4 mmol), HOBt (1.0 g, 7.4 mmol), EDCI (1.46 g, 7.4 mmol) and triethylamine (1.2 mL, 8.7 mmol) were stirred in DMF at room temperature. The reaction mixture was diluted with water (60 mL) and extracted with dichloromethane. The solvent was evaporated and the obtained residue was washed with dichloromethane (20 mL) to give the title compound (2.0 g, yield 82%).
¹H-NMR (400 MHz, DMSO-d₆) δ8.01(brs, 2H), 7.76(brs, 2H), 7.35-7.33(m, 10H), 5.02(s.4H), 4.24(brs, 2H), 3.08-3.00(m, 4H), 1.23(brs, 44H), 0.86-0.83(m, 6H).

### (step 2)

### Synthesis of (2R)-2-amino-3-[[(2R)-2-amino-3-(dodecylamino)-3-oxo-propyl]disulfanyl]-N-dodecyl-propanamide dihydrobromide

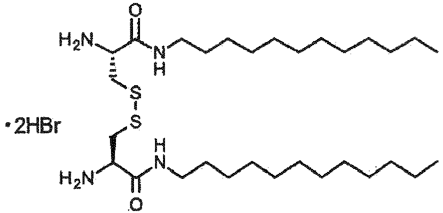

To a dichloromethane solution (2 mL) of benzyl N-[(1R)-1-[[[(2R)-2-(benzyloxycarbonylamino)-3-(dodecylamino)-3-oxopropyl]disulfanyl]methyl]-2-(dodecylamino)-2-oxoethyl]carbamate (300 mg, 0.35 mmol) obtained in step 1 was added a hydrobromic acid/acetic acid solution (4 mL) and the mixture was stirred at room temperature overnight. The reaction mixture was concentrated to give the title compound (200 mg, yield quantitative).
¹H-NMR (400 MHz, DMSO-d₆) δ8.34-8.22(m, 6H), 4.24 (brs, 2H), 3.10-3.08(m, 4H), 1.22(brs, 44H), 0.85-0.82(m, 6H).

### (step 3)

### Synthesis of N-[(1R)-1-[[[(2R)-2-(docosanoylamino)-3-(dodecylamino)-3-oxo-propyl]disulfanyl]methyl]-2-(dodecylamino)-2-oxo-ethyl]docosanamide

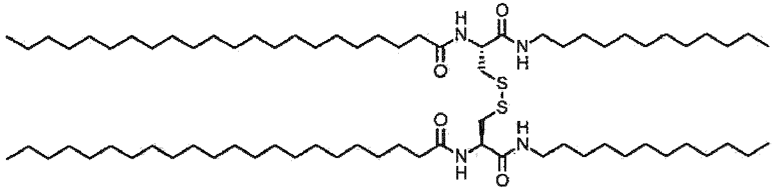

To a dichloromethane solution (4 mL) of behenic acid (238 mg, 0.7 mmol) were added a catalytic amount of DMF and oxalyl dichloride (196 mg, 1.54 mmol), and the mixture was stirred at room temperature for 1 hr. The reaction mixture was concentrated and the obtained residue was dissolved in THF (4 mL). This was added to a THF solution (4 mL) of (2R)-2-amino-3-[[(2R)-2-amino-3-(dodecylamino)-3-oxo-propyl]disulfanyl]-N-dodecyl-propanamide dihydrobromide (200 mg, 0.35 mmol) obtained in step 2, and the mixture was adjust same to pH10-11 by adding 5M aqueous sodium hydroxide solution and stirred at room temperature overnight. To the reaction mixture was added 1N hydrochloric acid to adjust same to pH6-7, and the precipitate was collected by filtration to give the title compound (140 mg, yield 33%).
¹H-NMR (400 MHz,THF-d₈) δ8.55(t, J=5.2Hz, 2H), 8.07(d, J=9.6Hz, 2H), 5.65-5.59(m, 2H), 3.62-3.43(m, 4H), 3.28-3.12(m, 3H), 2.61(t, J=6.4Hz, 3H), 2.07-1.83(m, 118H), 1.23(t, J=6.8Hz, 12H).

### Example 26

### (2S)-2-(dibenzylamino)-N-dodecyl-5-guanidino-pentanamide dihydrochloride (SZ174)

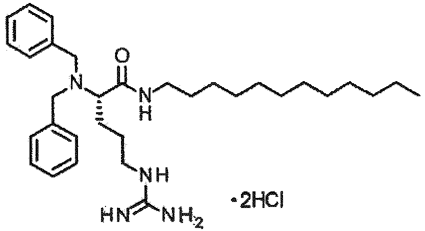

### (step 1)

### Synthesis of tert-butyl (NE)-N-[(tert-butoxycarbonylamino)-[[(4S)-4-(dibenzylamino)-5-(dodecylamino)-5-oxo-pentyl]amino]methylene]carbamate

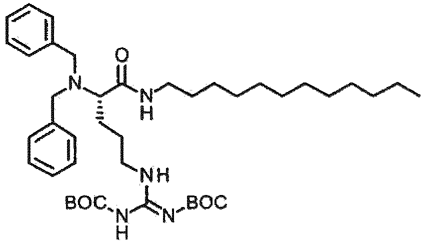

tert-Butyl (NE)-N-[[[(4S)-4-amino-5-(dodecylamino)-5-oxo-pentyl]amino]-(tert-butoxycarbonylamino)methylene]carbamate (500 mg, 0.92 mmol) obtained in Example 17, step 2 and benzyl bromide (473 mg, 2.77 mmol) were dissolved in DMF (10 mL), potassium carbonate (382 mg, 2.77 mmol) was added, and the mixture was stirred at room temperature overnight. The reaction mixture was diluted with water and extracted with ethyl acetate. The organic layer was dried over anhydrous sodium sulfate, the solvent was evaporated and the obtained residue was purified by silica gel chromatography (petroleum ether/ethyl acetate=10/1) to give the title compound (150 mg, 22%).
MS(ESI)m/z:722[M+H]⁺

### (step 2)

### Synthesis of (2S)-2-(dibenzylamino)-N-dodecyl-5-guanidino-pentanamide dihydrochloride

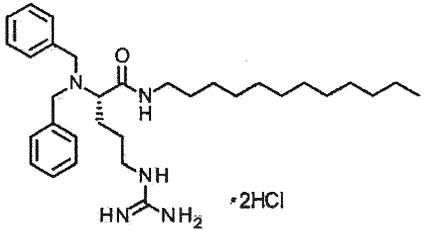

To tert-butyl (NE)-N-[(tert-butoxycarbonylamino)-[[(4S)-4-(dibenzylamino)-5-(dodecylamino)-5-oxo-pentyl]amino]methylene]carbamate (150 mg, 0.208 mmol) obtained in step 1 was added trifluoroacetic acid/dichloromethane solution (3 mL/3 mL) and the mixture was stirred at room temperature for 1.5 hr. The reaction mixture was concentrated, adjust same to pH8-9 with saturated aqueous sodium carbonate solution and extracted with ethyl acetate. The organic layer was dried over anhydrous sodium sulfate, the solvent was evaporated, hydrochloric acid/diethylether solution (10 mL) was added to the obtained residue, and the mixture was stirred at room temperature for 1 hr. The reaction mixture was concentrated, the obtained residue was purified by silica gel chromatography (dichloromethane/methanol=20/1) and the obtained crude product was treated with ethyl acetate to give the title compound (100 mg, yield 90%).
¹H-NMR (400 MHz, DMSO-d₆) δ7.73-7.70(m, 1H), 7.59-7.58(m, 1H), 7.42-7.20(m, 11H), 3.79-3.75(m, 2H), 3.59-3.56(m, 2H), 3.2-3.17(m, 1H), 3.06-2.94(m, 4H), 1.73-1.42(m, 6H), 1.41-1.17(m, 18H), 0.84(t, J=6.4Hz, 3H).
MS(ESI)m/z:522[M+H]⁺

### Example 27

### N-(5-guanidinopentyl)hexadecanamide hydrochloride (SZ175)

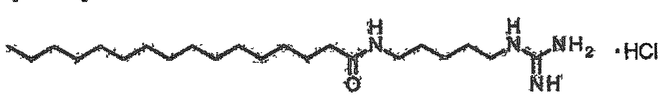

### (step 1)

### Synthesis of tert-butyl N-(5-guanidinopentyl)carbamate trifluoroacetate

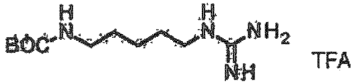

Under an argon atmosphere, to a solution of tert-butyl N-(5-aminopentyl)carbamate (300 mg, 1.46 mmol) in dichloromethane (15 mL) were added triethylamine (0.206 mL, 1.48 mmol) and 1-amidinopyrazole hydrochloride (217 mg, 1.46 mmol). After stirring at room temperature overnight, the mixture was purified by reversed-phase HPLC (0.05%(v/v)-containing TFA, water/acetonitrile) to give the title compound (408 mg, yield quantitative).
¹H-NMR (400 MHz, Deuterium Oxide) δ3.10(t, J=7.0Hz, 2H), 2.99(t, J=6.7Hz, 2H), 1.56-1.47(m, 2H), 1.46-1.37(m, 2H), 1.35(s, 9H), 1.32-1.23(m, 2H).
MS(ESI)m/z:245[M+H]⁺

### (step 2)

### Synthesis of N-(5-guanidinopentyl)amine dihydrochloride

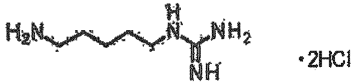

To tert-butyl N-(5-guanidinopentyl)carbamate trifluoroacetate (408 mg, 1.67 mmol) obtained in step 1 was added 4N hydrochloric acid/1,4-dioxane solution (17 mL), and the mixture was stirred for 30 min. The reaction mixture was concentrated and lyophilized to give the title compound (279 mg, yield 77%).
¹H-NMR (400 MHz, Deuterium Oxide) δ3.12(t, J=7.0Hz, 2H), 2.93(t, J=7.7Hz, 2H), 1.69-1.47(m, 4H), 1.43-1.29(m, 2H). MS(ESI)m/z:145[M+H]⁺

### (step 3)

### Synthesis of N-(5-guanidinopentyl)hexadecanamide trifluoroacetate

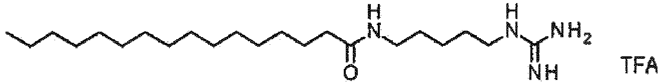

A 2-propanol/aqueous solution (37/63,v/v) (5 mL) of N-(5-guanidinopentyl)amine dihydrochloride (144 mg, 0.664 mmol) obtained in step 2 was cooled to 5°C, and adjust same to pH about 10 with 25% sodium hydroxide. To the reaction mixture was added palmitoylchloride (0.050 mL, 0.166 mmol), and the mixture was heated to room temperature. After stirring for 20 min, the mixture was cooled to 5°C again, 25% sodium hydroxide was added to adjust to pH8-11 and palmitoylchloride (0.050 mL, 0.166 mmol) was added. The mixture was heated to room temperature, and addition of palmitoylchloride, pH adjustment and temperature adjustment were repeated twice in the same manner (0.2 mL, 0.664 mmol added in total). After stirring at room temperature for 1 hr, the solid was collected by filtration and washed with water to give crude crystals (194 mg). This was subjected to reversed-phase HPLC in the same manner as in step 1 to give the title compound (93.5 mg, yield 29%).
¹H-NMR (400 MHz, DMSO-d₆) δ7.73(t, J=5.6Hz, 1H), 7.44(t, J=5.6Hz, 1H), 7.37-6.53(m, 4H), 3.17-2.90(m, 4H), 2.02(t, J=7.4Hz, 2H), 1.59-1.33(m, 6H), 1.33-0.99(m, 26H), 0.85(t,3H). MS(ESI)m/z:384[M+H]⁺

### (step 4)

### Synthesis of N-(5-guanidinopentyl)hexadecanamide hydrochloride

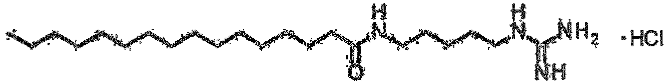

To N-(5-guanidinopentyl)hexadecanamide trifluoroacetate (93.5 mg, 0.195 mmol) obtained in step 3 was added ethyl acetate (2 mL), and a 4N hydrochloric acid/ethyl acetate solution (0.244 mL) was added under ice-cooling. After stirring at room temperature for 30 min, the reaction mixture was concentrated, and azeotropically distilled twice with ethyl acetate (2 mL). To the obtained residue was added ethyl acetate (2 mL) and the solid was collected by filtration to give the title compound (79.3 mg, yield 97%).
¹H-NMR (400 MHz, DMSO-d₆) δ7.81-7.72(m, 1H), 7.68-7.53(m, 1H), 7.50-6.62(m, 4H), 3.18-2.89(m, 4H), 2.03(t, J=7.4Hz, 2H), 1.51-1.32(m, 6H), 1.32-1.15(m, 26H), 0.90-0.82(m, 3H).

### Example 28

### N-(5-guanidinopentyl)docosanamide hydrochloride (SZ176)

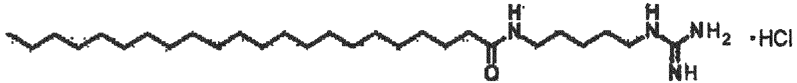

### (step 1)

### Synthesis of N-(5-guanidinopentyl)docosanamide trifluoroacetate

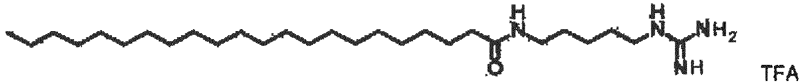

To a dichloromethane solution (4.6 mL) of behenic acid (232 mg, 0.680 mmol) were added oxalyl chloride (0.059 mL, 0.680 mmol), a catalytic amount of DMF and THF (1 mL). After stirring for 30 min, the mixture was concentrated under reduced pressure and the obtained residue was dissolved in THF (1 mL) to give a behenoyl chloride solution. In a separate flask, a 2-propanol/aqueous solution (v/v=37/63) (5 mL) of compound (134 mg, 0.618 mmol) obtained in Example 27, step 2 was prepared. A 25% aqueous sodium hydroxide solution was added at 5°C to adjust same to pH about 8-11 and a behenoyl chloride/THF solution was added by 0.25 mL. After stirring at room temperature for 1 hr, the solid was collected by filtration, and washed with water to give crude crystals (225 mg). The crude crystals (136 mg) were suspended in ethyl acetate (3.5 mL), 4N hydrochloric acid/ethyl acetate (0.29 mL) was added under ice-cooling, and the mixture was stirred at room temperature for 30 min and concentrated. To the obtained residue was added diethyl ether (5 mL) and the solid was collected by filtration to give a mixture (88.8 mg) of the object product and behenic acid. Thereto was added a saturated aqueous sodium hydrogen carbonate solution (5 mL) and the mixture was stirred for 1 hr, and the solid was collected by filtration. To the obtained residue was added methanol, and the mixture was acidified with trifluoroacetic acid and insoluble material was filtered off. The filtrate was subjected to reversed-phase HPLC in the same manner as in Example 27, step 1 to give the title compound (30.6 mg, 0.0543 mmol).
¹H-NMR (400 MHz, DMSO-d₆) δ7.73(t, J=5.6Hz, 1H), 7.44(t, J=5.6Hz, 1H), 7.37-6.55(m, 4H), 3.14-2.92(m, 4H), 2.02(t, J=7.4Hz, 2H), 1.55-1.32(m, 6H), 1.32-0.99(m, 38H), 0.85(t,3H). MS (ESI)m/z:468 [M+H]⁺

### (step 2)

### Synthesis of N-(5-guanidinopentyl)docosanamide hydrochloride

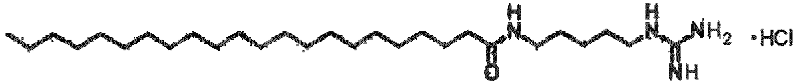

An operation similar to that in Example 27, step 4 was performed using N-(5-guanidinopentyl)docosanamide trifluoroacetate (30.6 mg, 0.0543 mmol) obtained in step 1 instead of N-(5-guanidinopentyl)hexadecanamide trifluoroacetate to give the title compound (20.6 mg, yield 75%).
¹H-NMR (400 MHz, DMSO-d₆) δ7.76(t, J=5.7Hz, 1H), 7.54(t, J=5.6Hz, 1H), 7.47-6.59(m, 4H), 3.15-2.93(m, 4H), 2.02(t, J=7.4Hz, 2H), 1.55-1.32(m, 6H), 1.33-0.98(m, 38H), 0.85(t,3H).

### Example 29

### N-[(1S)-1-(dodecylcarbamoyl)-4-guanidino-butyl]-2-oxo-octanamide hydrochloride (SZ177)

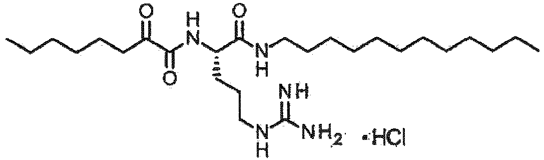

### (step 1)

### Synthesis of tert-butyl (NE)-N-[(tert-butoxycarbonylamino)-[[(4S)-5-(dodecylamino)-5-oxo-4-(2-oxooctanoylamino)pentyl]amino]methylene]carbamate

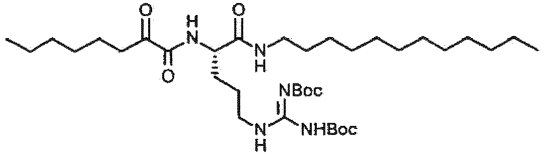

To a solution of tert-butyl (NE)-N-[[[(4S)-4-amino-5-(dodecylamino)-5-oxo-pentyl]amino]-(tert-butoxycarbonylamino)methylene]carbamate (73.8 mg, 0.136 mmol) obtained in Example 17, step 2 and O-(7-azabenzotriazol-1-yl)-N,N,N',N'-tetramethyluronium hexafluorophosphate (28.5 mg, 0.0749 mmol) were added a solution of 2-oxooctaneacid (36.9 mg, 0.0681 mmol) in acetonitrile (0.7 mL) and DIPEA (0.023 mL, 0.136 mmol), and the mixture was stirred at room temperature overnight. The reaction mixture was concentrated and the obtained residue was purified by silica gel chromatography (ethyl acetate/hexane=1/3) to give the title compound (26.2 mg, yield 56%).
¹H-NMR (400 MHz, CDCl₃) δ11.46(s, 1H), 8.42(t, J=5.9Hz, 1H), 7.75(d, J=8.5Hz, 1H), 6.37(t, J=5.9Hz, 1H), 4.44(q, J=7.1Hz, 1H), 3.69-3.50(m, 1H), 3.43-3.24(m, 2H), 3.24-3.06(m, 1H), 3.00-2.79(m, 2H), 1.97-1.80(m, 1H), 1.80-1.56(m, 6H), 1.53-1.39(m, 18H), 1.39-1.12(m, 23H), 0.87(t,6H).

### MS(ESI)m/z:683[M+H]⁺

### (step 2)

### Synthesis of N-[(1S)-1-(dodecylcarbamoyl)-4-guanidino-butyl]-2-oxo-octanamide hydrochloride

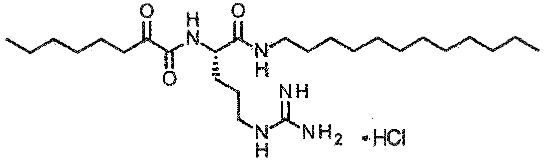

To a dichloromethane solution (0.4 mL) of tert-butyl (NE)-N-[(tert-butoxycarbonylamino)-[[(4S)-5-(dodecylamino)-5-oxo-4-(2-oxooctanoylamino)pentyl]amino]methylene]carbamate (26.2 mg, 0.0384 mmol) obtained in step 1 was added trifluoroacetic acid (0.18 mL) and the mixture was stirred at room temperature overnight. The reaction mixture was concentrated, azeotropically distilled three times with ethyl acetate (1 mL) and lyophilized to give trifluoroacetate (20.9 mg). To the obtained trifluoroacetate was added ethyl acetate (0.5 mL), 4N hydrochloric acid/ethyl acetate (1.5 mL) was added under ice-cooling and the mixture was stirred for 5 min and concentrated. The residue was azeotropically distilled twice with ethyl acetate (1 mL). To the obtained residue was added ethyl acetate and the solid was collected by filtration to give the title compound (13.4 mg, yield 67%).
¹H-NMR (400 MHz, DMSO-d₆) δ8.33(d, J=8.3Hz, 1H), 8.00(t, J=5.7Hz, 1H), 7.58(t, J=5.7Hz, 1H), 7.49-6.86(m, 4H), 4.27-4.12(m, 1H), 3.13-2.97(m, 4H), 2.79(dd,J=7.8,6.7Hz, 2H), 1.82-1.33(m, 8H), 1.32-0.98(m, 24H), 0.94-0.77(m, 6H).
MS(ESI)m/z:483[M+H]⁺

### Example 30

### Hexadecyl N-(4-guanidinobutyl)carbamate hydrochloride (SZ178)

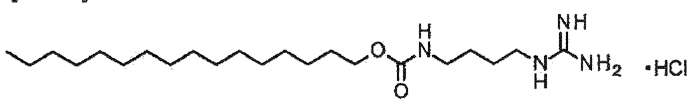

Agmatine sulfate (502 mg, 2.20 mmol) was dissolved in 2-propanol/water (8 mL/12 mL), and 25% aqueous sodium hydroxide solution was added at 10°C to adjust same to pH about 13. Hexadecyl chloroformate (0.65 mL, 1.98 mmol) was added at 12°C, and the mixture was adjust same to pH about 12-13 with 25% aqueous sodium hydroxide solution and heated to 17°C. After stirring at 17°C for 45 min, the mixture was stirred at room temperature for 3 hr. The precipitated solid was collected by filtration, washed with 2-propanol/water (1/1, v/v) to give crude crystals (747 mg). To the obtained crude crystals were added ethyl acetate (0.5 mL) and 4N hydrochloric acid/ethyl acetate (1.5 mL), and the mixture was stirred at room temperature for 30 min. The solvent was evaporated and the mixture was azeotropically distilled twice with ethyl acetate. To the obtained residue was added ethyl acetate and the mixture was collected by filtration to give the title compound (692 mg, yield 72%).
¹H-NMR (400 MHz, DMSO-d₆) δ9.42-9.29(m, 1H),9.02(s, 1H), 8.48(s, 1H), 7.80(t, J=5.7Hz, 1H), 3.16(q, J=6.6Hz, 2H), 3.05(q, J=6.4Hz, 2H), 2.12(s, 3H), 2.04(t, J=7.4Hz, 2H), 1.56-1.35(m, 5H), 1.34-1.14(m, 24H), 0.90-0.79 m,3H).
MS(ESI)m/z:399[M+H]⁺

### Example 31

### N-[4-(ethanimidoylamino)butyl]hexadecanamide hydrochloride (SZ179)

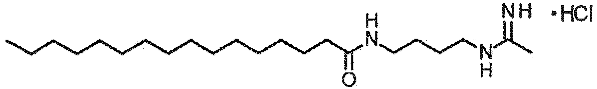

### (step 1)

### Synthesis of tert-butyl N-[4-(ethanimidoylamino)butyl]carbamate trifluoroacetate

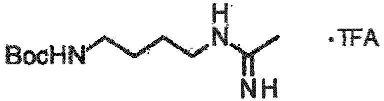

Under an argon atmosphere, to an ethanol solution (21 mL) of tert-butyl N-(4-aminobutyl)carbamate (400 mg, 2.13 mmol) were added triethylamine (0.196 mL, 2.12 mmol) and ethylacetimidate hydrochloride (262 mg, 2.12 mmol) under ice-cooling. After stirring at room temperature overnight, the reaction mixture was concentrated and the obtained residue was subjected to reversed-phase HPLC in the same manner as Example 27, step 1 to give the title compound (518 mg, yield quantitative).
¹H-NMR (400 MHz, CD₃OD) δ3.30-3.23(m, 2H), 3.10(t, J=6.7Hz, 2H), 2.23(s, 3H), 1.72-1.62(m, 2H), 1.62-1.51(m, 2H), 1.46(s, 9H).
MS(ESI)m/z:230[M+H]⁺

### (step 2)

### Synthesis of N-(4-aminobutyl)acetoamidine dihydrochloride

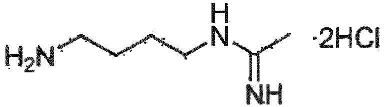

To tert-butyl N-[4-(ethanimidoylamino)butyl]carbamate trifluoroacetate (518 mg, 2.26 mmol) obtained in step 1 were added 1,4-dioxane/aqueous solution (10 mL/6 mL) and 4N hydrochloric acid/1,4-dioxane solution (2.83 mL) and the mixture was stirred at room temperature for 1 hr. After standing at 5°C overnight, a 4N hydrochloric acid/1,4-dioxane solution (2.83 mL) was added and the mixture was stirred at room temperature for 1.5 hr. A 4N hydrochloric acid/1,4-dioxane solution (2.83 mL) was further added and the mixture was stirred for 2 hr. The reaction mixture was concentrated and lyophilized to give the title compound (297 mg, yield 65%).
¹H-NMR (400 MHz, Deuterium Oxide) δ3.28-3.14(m, 2H), 3.01-2.87(m, 2H), 2.14(s, 3H), 1.73-1.56(m, 4H).
MS(ESI)m/z:130[M+H]⁺

### (step 3)

### Synthesis of N-[4-(ethanimidoylamino)butyl]hexadecanamide trifluoroacetate

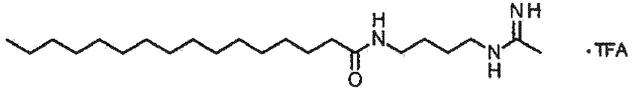

A 2-propanol/aqueous solution (37/63, v/v) (15 mL) of N-(4-aminobutyl)acetoamidine dihydrochloride (297 mg, 1.47 mmol) obtained in step 2 was cooled 15°C, a 25% aqueous sodium hydroxide solution was added to adjust to pH about 8-11 and palmitoylchloride (0.489 mL, 1.62 mmol) was added by 1/4 amount. After stirring at room temperature for 2 hr, the solid was filtered off, and the filtrate was concentrated and freeze-dried. This was subjected to reversed-phase HPLC in the same manner as in Example 27, step 1 to give the title compound (383 mg, yield 56%).
¹H-NMR (400 MHz, CD₃OD) δ3.38-3.18(m, 4H), 2.27-2.15(m, 5H), 1.72-1.54(m, 6H), 1.40-1.23(m, 24H), 0.92(t,3H).
MS (ESI)m/z:368[M+H]⁺

### (step 4)

### Synthesis of N-[4-(ethanimidoylamino)butyl]hexadecanamide hydrochloride

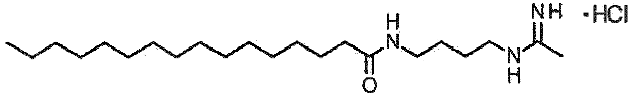

To an ethyl acetate solution (6 mL) of N-[4-(ethanimidoylamino)butyl]hexadecanamide trifluoroacetate (96.2 mg, 0.207 mmol) obtained in step 3 was added, under ice-cooling, 4N hydrochloric acid/ethyl acetate (0.33 mL) and the mixture was stirred for 5 min. The reaction mixture was concentrated, the obtained residue was suspended in ethyl acetate (2 mL), 4N hydrochloric acid/ethyl acetate (3 mL) was added under ice-cooling and the mixture was stirred for 5 min. The reaction mixture was concentrated and the obtained residue was suspended in ethyl acetate (2 mL). To the reaction mixture was added, under ice-cooling, 4N hydrochloric acid/ethyl acetate (3 mL) and the mixture was stirred for 10 min. The reaction mixture was concentrated, and azeotropically distilled three times with ethyl acetate (5 mL). To the obtained residue was added ethyl acetate, and the solid was collected by filtration to give the title compound (74.7 mg, yield 89%).
¹H-NMR (400 MHz, CD₃OD) δ9.30(s, 1H),9.01(s, 1H), 8.47(s, 1H), 3.32-3.20(m, 4H), 2.26-2.17(m, 5H), 1.73-1.54(m, 6H), 1.41-1.23(m, 24H), 0.92(t,3H).
MS(ESI)m/z:368[M+H]⁺

### Example 32

### N-[5-(dodecylamino)-5-oxo-pentyl]docosanamide (SZ164)

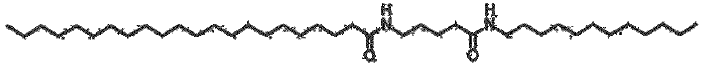

### (step 1)

### Synthesis of tert-butyl N-[5-(dodecylamino)-5-oxo-pentyl]carbamate

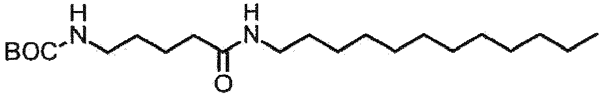

An operation similar to that in Synthetic Example 1, step 1 was performed using 5-(tert-butoxycarbonylamino)pentanoic acid (2.0 g, 9.20 mmol) instead of (2S)-2-(tert-butoxycarbonylamino)-5-[(N-nitrocarbamimidoyl)amino]pentanoic acid to give the title compound (3.4 g, yield 97%).
MS(ESI)m/z:385[M+H]⁺

### (step 2)

### Synthesis of 5-amino-N-dodecyl-pentanamide hydrochloride

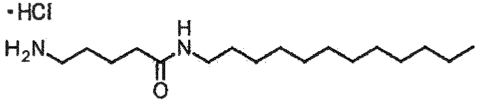

An operation similar to that in Synthetic Example 1, step 2 was performed using tert-butyl N-[5-(dodecylamino)-5-oxo-pentyl]carbamate (3.4 g, 8.84 mmol) obtained in step 1 instead of tert-butyl N-[(1S)-1-(dodecylcarbamoyl)-4-[(N-nitrocarbamimidoyl)amino]butyl]carbamate to give the title compound (2.8 g, yield quantitative).
MS(ESI)m/z:285[M+H]⁺

### (step 3)

### Synthesis of N-[5-(dodecylamino)-5-oxo-propyl]docosanamide

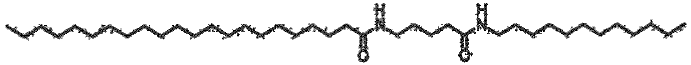

An operation similar to that in Example 7, step 3 was performed using 5-amino-N-dodecyl-pentanamide hydrochloride (500 mg, 1.56 mmol) obtained in step 2 instead of 2-amino-N-dodecyl-2-methyl-propanamide hydrochloride to give the title compound (480 mg, yield 63%).
¹H-NMR (400 MHz,CF₃COOD) δ3.63-3.55(m, 4H), 2.80-2.72(m, 4H), 1.86-1.70(m, 8H), 1.39-1.32(m, 54H), 0.89-0.87(m, 6H).

### Example 33

### N-[(1S)-1-(dodecylcarbamoyl)-4-guanidino-butyl]icosanamide hydrochloride (SZ180)

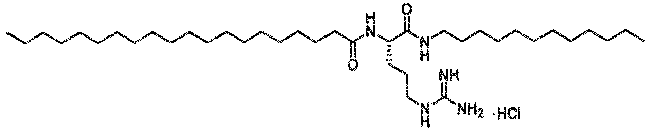

### (step 1)

### Synthesis of N-[(1S)-1-(dodecylcarbamoyl)-4-[(N-nitrocarbamimidoyl)amino]butyl]icosanamide

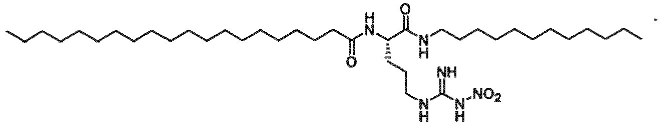

To a dichloromethane solution (10 mL) of icosanic acid (500 mg, 1.60 mmol) were added DMF (0.2 mL) and oxalyl dichloride (254 mg, 2.00 mmol), and the mixture was stirred at room temperature for 1 hr. The reaction mixture was concentrated and the obtained residue was dissolved in THF (10 mL), added to a THF solution (20 mL) of the compound (870 mg, 2.06 mmol) obtained in Synthetic Example 1, step 2 and triethylamine (606 mg, 6.00 mmol) and the mixture was stirred at room temperature overnight. The reaction mixture was concentrated under reduced pressure and the obtained residue was purified by silica gel column chromatography (dichloromethane/methanol=40/1) and concentrated under reduced pressure. The obtained solid was washed with water and dried to give the title compound (920 mg, 1.35 mmol, yield 65%).

### (step 2)

### Synthesis of N-[(1S)-1-(dodecylcarbamoyl)-4-guanidino-butyl]icosanamide hydrochloride

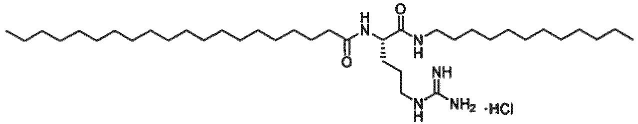

To a methanol solution (30 mL) of the compound (920 mg, 1.35 mmol) obtained in step 1 were added concentrated hydrochloric acid (0.5 mL) and 20%(w/w) palladium-carbon (10% wet) (184 mg), and the mixture was stirred under 50 psi hydrogen atmosphere at 50°C overnight. Insoluble material was filtered off, and the filtrate was concentrated. To the obtained residue was added methanol and the residue was collected by filtration and dried to give the title compound (500 mg, 0.743 mmol, yield 55%).
¹H-NMR (400 MHz, DMSO-d₆) δ7.95(brs, 1H), 7.87(brs, 1H), 7.60(brs, 1H), 7.33-6.90(m, 3H), 4.22-4.20(m, 1H), 3.08-2.99(m, 4H), 2.11-2.10(m, 2H), 1.63-1.60(m, 1H), 1.47-1.35(m, 7H), 1.23(m, 50H), 0.85(t, J=4.8Hz, 6H).
MS(ESI)m/z:637[M+H]⁺

### Example 34

### N-[(1S)-4-guanidino-1-(octylcarbamoyl)butyl]hexadecanamide hydrochloride (SZ181)

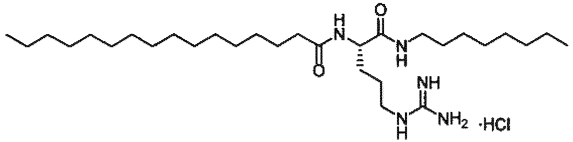

### (step 1)

### Synthesis of tert-butyl N-[(1S)-4-[(N-nitrocarbamimidoyl)amino]-1-(octylcarbamoyl)butyl]carbamate

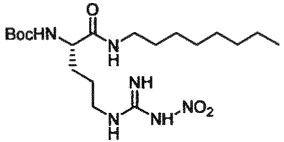

To a dichloromethane solution (200 mL) of (2S)-2-(tert-butoxycarbonylamino)-5-[(N-nitrocarbamimidoyl)amino]pentanoic acid (6.00 g, 18.8 mmol), HOBt (3.81 g, 28.2 mmol) and EDCI (5.41 g, 28.2 mmol) was added triethylamine (5.70 g, 56.4 mmol) and the mixture was stirred for 30 min. To the reaction mixture was added octylamine (2.43 g, 18.8 mmol), and the mixture was stirred at room temperature overnight. The reaction mixture was diluted with saturated aqueous ammonium chloride solution, and extracted with dichloromethane. The organic layer was washed with saturated brine, dried over anhydrous sodium sulfate and concentrated under reduced pressure. The obtained residue was purified by silica gel chromatography (dichloromethane/methanol=30/1) to give the title compound (4.50 g, 10.5 mmol, yield 56%).
MS(ESI)m/z:431[M+H]⁺

### (step 2)

### Synthesis of (2S)-2-amino-5-[(N-nitrocarbamimidoyl)amino]-N-octyl-pentanamide hydrochloride

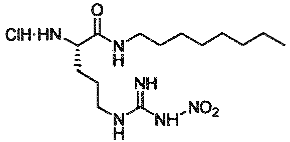

To the compound (4.50 g, 10.5 mmol) obtained in step 1 was added a 4N hydrochloric acid/ethyl acetate solution (30 mL) and the mixture was stirred at room temperature for 1 hr. The reaction mixture was concentrated under reduced pressure to give the title compound (4.00 g, 10.9 mmol, yield quantitative).
MS(ESI)m/z:331[M+H]⁺

### (step 3)

### Synthesis of N-[(1S)-4-[(N-nitrocarbamimidoyl)amino]-1-(octylcarbamoyl)butyl]hexadecanamide

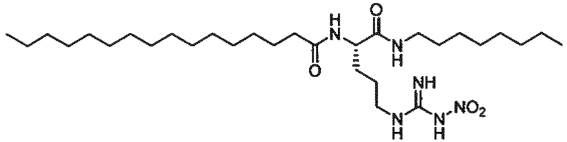

To a dichloromethane solution (10 mL) of palmitic acid (512 mg, 2.00 mmol) were added DMF (0.1 mL) and oxalyl dichloride (330 mg, 2.60 mmol), and the mixture was stirred at room temperature for 2 hr. The reaction mixture was concentrated and the obtained residue was dissolved in THF (6 mL). This was added to a THF solution (30 mL) of the compound (858 mg, 2.60 mmol) obtained in step 2 and triethylamine (788 mg, 7.80 mmol) and the mixture was stirred at room temperature overnight. The reaction mixture was concentrated and the obtained residue was suspended in a mixed solution of methanol/water (5 mL/10 mL) and collected by filtration to give the title compound (1.10 g, 1.94 mmol, yield 74%).
MS(ESI)m/z:569[M+H]⁺

### (step 4)

### Synthesis of N-[(1S)-4-guanidino-1-(octylcarbamoyl)butyl]hexadecanamide hydrochloride

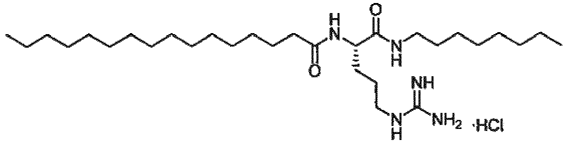

To a methanol solution (30 mL) of the compound (1.10 g, 1.94 mmol) obtained in step 3 were added concentrated hydrochloric acid (0.5 mL) and 20%(w/w) palladium-carbon (10% wet) (220 mg), and the mixture was stirred under 50 psi hydrogen atmosphere at 50°C overnight. Insoluble material was filtered off, the filtrate was concentrated and methanol/water (2 mL/5 mL) was added to the obtained residue. The residue was collected by filtration and dried to give the title compound (450 mg, 0.803 mmol, yield 41%).
¹H-NMR (400 MHz, DMSO-d₆) δ7.99(d, J=7.6Hz, 1H), 7.95(brs, 1H), 7.75(brs, 1H), 7.40-7.20(m, 3H), 4.23-4.17(m, 1H), 3.13-3.00(m, 4H), 2.14-2.06(m, 2H), 1.65-1.27(m, 8H), 1.24-1.22(m, 34H), 0.85(t, J=6.8Hz, 6H).
MS(ESI)m/z:524[M+H]⁺

### Example 35

### N-[(1S)-4-guanidino-1-(octylcarbamoyl)butyl]octadecanamide hydrochloride (SZ182)

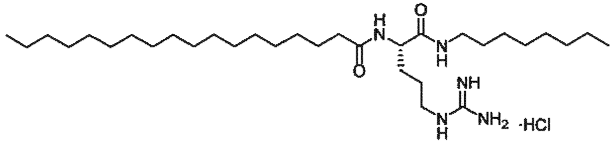

### (step 1)

### Synthesis of N-[(1S)-4-[(N-nitrocarbamimidoyl)amino]-1-(octylcarbamoyl)butyl]octadecanamide

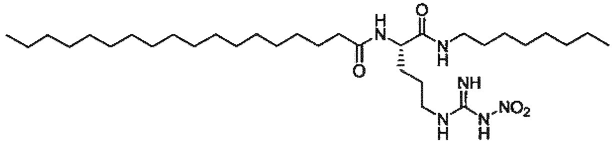

To a dichloromethane solution (10 mL) of stearic acid (568 mg, 2.00 mmol) were added DMF (0.2 mL) and oxalyl dichloride (330 mg, 2.60 mmol), and the mixture was stirred at room temperature for 2 hr. The reaction mixture was concentrated and the obtained residue was dissolved in THF (6 mL). This was added to a THF solution (30 mL) of the compound (858 mg, 2.34 mmol) obtained in Example 34, step 2 and triethylamine (788 mg, 7.80 mmol) and the mixture was stirred at room temperature overnight. The reaction mixture was concentrated and the obtained residue was suspended in methanol/water (5 mL/10 mL), collected by filtration, and purified by silica gel chromatography (dichloromethane/methanol=50/1) to give the title compound (650 mg, 1.09 mmol, yield 47%).
MS(ESI)m/z:597[M+H]⁺

### (step 2)

### Synthesis of N-[(1S)-4-guanidino-1-(octylcarbamoyl)butyl]octadecanamide hydrochloride

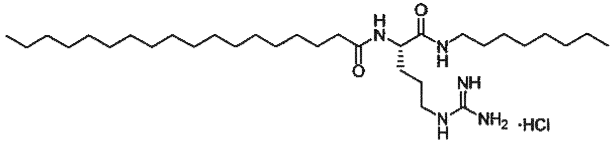

An operation similar to that in Example 33, step 2 was performed using the compound (650 mg, 1.09 mmol) obtained in step 1 to give the title compound (350 mg, 0.595 mmol, yield 55%).
¹H-NMR (400 MHz, DMSO-d₆) δ7.99(d, J=8.4Hz, 1H), 7.94-7.91(m, 1H), 7.78-7.75(m, 1H), 7.45-6.80(m, 3H), 4.23-4.17(m, 1H), 3.09-3.00(m, 4H), 2.11(t, J=7.2Hz, 2H), 1.65-1.30(m, 8H), 1.24-1.22(m, 40H), 0.85(t, J=6.8Hz, 6H).
MS(ESI)m/z:552[M+H]⁺

### Example 36

### N-[(1S)-4-guanidino-1-(octylcarbamoyl)butyl]icosanamide hydrochloride (SZ183)

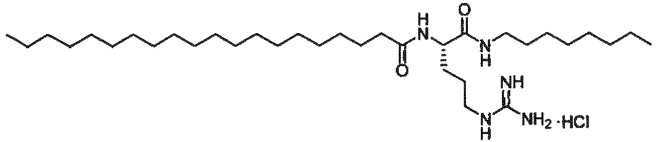

### (step 1)

### Synthesis of N-[(1S)-4-[(N-nitrocarbamimidoyl)amino]-1-(octylcarbamoyl)butyl]icosanamide

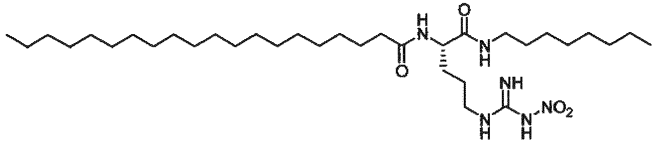

An operation similar to that in Example 34, step 3 was performed using icosanic acid (624 mg, 2.00 mmol) to give the title compound (1.10 g, 1.76 mm, yield 75%).
MS(ESI)m/z:625[M+H]⁺

### (step 2)

### Synthesis of N-[(1S)-4-guanidino-1-(octylcarbamoyl)butyl]icosanamide hydrochloride

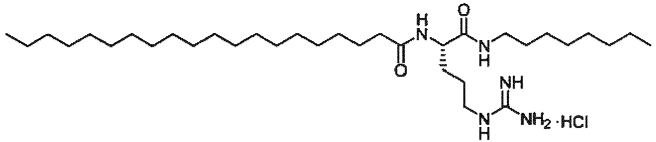

An operation similar to that in Example 33, step 2 was performed using the compound (1.10 g, 1.76 mmol) obtained in step 1 to give the title compound (270 mg, 0.438 mmol, yield 25%).
¹H-NMR (400 MHz, DMSO-d₆) δ8.00-7.92(m, 3H), 7.78-7.76(m, 1H), 7.50-6.80(m, 3H), 4.23-4.18(m, 1H), 3.09-3.02(m, 3H), 2.78-2.75(m, 1H), 2.11(t, J=6.8Hz, 2H), 1.70-1.33(m, 8H), 1.24-1.22(m, 42H), 0.85(t, J=6.8Hz, 6H).
MS(ESI)m/z:580[M+H]⁺

### Example 37

### N-[(1S)-5-amino-1-(octylcarbamoyl)pentyl]octadecanamide hydrochloride (SZ184)

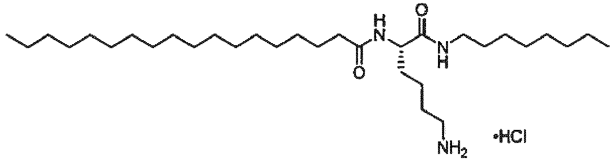

### (step 1)

### Synthesis of tert-butyl N-[(1S)-5-(benzyloxycarbonylamino)-1-(octylcarbamoyl)pentyl]carbamate

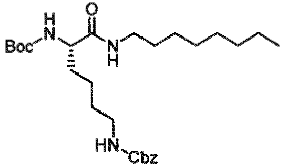

To a dichloromethane solution (20 mL) of the compound (1.00 g, 2.63 mmol) obtained in Example 12, step 1, HOBt (391 mg, 2.90 mmol) and EDCI (556 mg, 2.90 mmol) was added triethylamine (797 mg, 7.90 mmol) and the mixture was stirred for 15 min. To the reaction mixture was added octylamine (339 mg, 2.60 mmol) and the mixture was stirred at 30°C overnight. The reaction mixture was purified by silica gel chromatography (petroleum ether/ethyl acetate=3/1) to give the title compound (1.10 g, 2.24 mmol, yield 85%).
MS(ESI)m/z:492[M+H]⁺

### (step 2)

### Synthesis of benzyl N-[(5S)-5-amino-6-(octylamino)-6-oxohexyl]carbamate

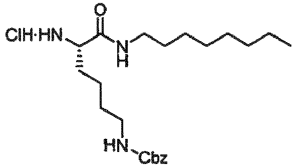

An operation similar to that in Example 34, step 2 was performed using the compound (1.10 g, 2.24 mmol) obtained in step 1 to give the title compound (940 mg, 2.20 mmol, yield 98%).
MS(ESI)m/z:392[M+H]⁺

### (step 3)

### Synthesis of benzyl N-[(5S)-5-(octadecanoylamino)-6-(octylamino)-6-oxo-hexyl]carbamate

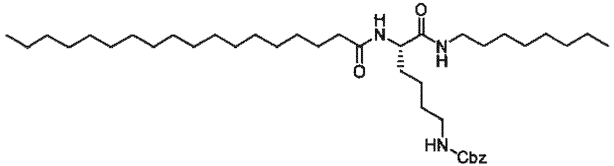

To a dichloromethane solution (10 mL) of stearic acid (509 mg, 1.80 mmol) were added DMF (0.2 mL) and oxalyl dichloride (285 mg, 2.20 mmol), and the mixture was stirred at room temperature for 1 hr. The reaction mixture was concentrated and the obtained residue was dissolved in THF (10 mL). This was added to a THF solution (30 mL) of the compound (941 mg, 2.41 mmol) obtained in step 2 and triethylamine (905 mg, 9.00 mmol) and the mixture was stirred at room temperature overnight. The reaction mixture was concentrated and the obtained residue was washed with water recrystallized from methanol to give the title compound (400 mg, 0.608 mmol, yield 25%).

### (step 4)

### Synthesis of N-[(1S)-5-amino-1-(octylcarbamoyl)pentyl]octadecanamide hydrochloride

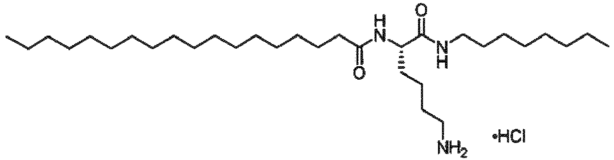

To a methanol solution (40 mL) of the compound (400 mg, 0.608 mmol) obtained in step 3 were added concentrated hydrochloric acid (0.5 mL) and 20%(w/w) palladium-carbon (10% wet) (80 mg), and the mixture was stirred under a hydrogen atmosphere at 70°C overnight. Insoluble material was filtered off, and the residue was collected by filtration and dried to give the title compound (150 mg, 0.268 mmol, yield 44%).
¹H-NMR (400 MHz, DMSO-d₆) δ7.95-7.83(m, 4H), 4.21-4.14(m, 1H), 3.04-2.99(m, 2H), 2.75-2.70(m, 2H), 2.12-2.09(m, 2H), 1.59-1.32(m, 48H), 0.85(t, J=6.4Hz, 6H).
MS(ESI)m/z:524[M+H]⁺

### Example 38

### N-[(1S)-5-amino-1-(dodecylcarbamoyl)pentyl]octadecanamide hydrochloride (SZ185)

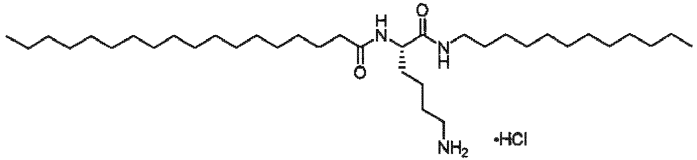

### (step 1)

### Synthesis of benzyl N-[(5S)-6-(dodecylamino)-5-(octadecanoylamino)-6-oxo-hexyl]carbamate

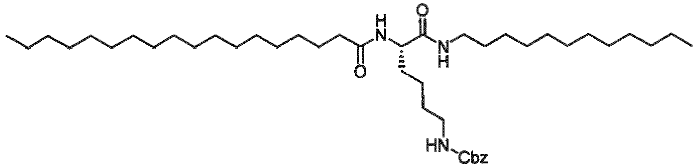

An operation similar to that in Example 37, step 3 was performed using the compound (191 mg, 0.395 mmol) obtained in Example 12, step 3 to give the title compound (320 mg, 0.448 mmol, quantitative).

### (step 2)

### Synthesis of N-[(1S)-5-amino-1-(dodecylcarbamoyl)pentyl]octadecanamide hydrochloride

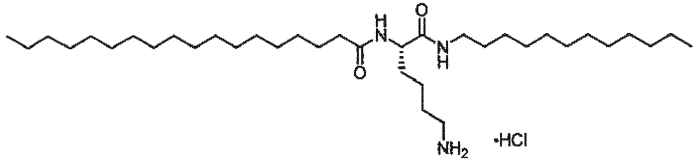

An operation similar to that in Example 37, step 4 was performed using the compound (320 mg, 0.448 mmol) obtained in step 1 to give the title compound (150 mg, 0.243 mmol, yield 54%).
¹H-NMR (400 MHz, DMSO-d₆) δ7.91-7.89(m, 1H), 7.82-7.70(m, 3H), 4.18-4.15(m, 1H), 3.05-2.98(m, 2H), 2.74-2.72(m, 2H), 2.12-2.08(m, 2H), 1.59-1.47(m, 8H), 1.42-1.23(m, 48H), 0.85(t, J=6.4Hz, 6H).
MS(ESI)m/z:580[M+H]⁺

### Example 39

### Octadecyl N-[(1S)-1-(dodecylcarbamoyl)-4-guanidino-butyl]carbamate hydrochloride (SZ186)

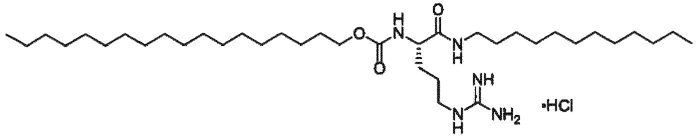

### (step 1)

### Synthesis of octadecyl N-[(1S)-1-(dodecylcarbamoyl)-4-[(N-nitrocarbamimidoyl)amino]butyl] carbamate

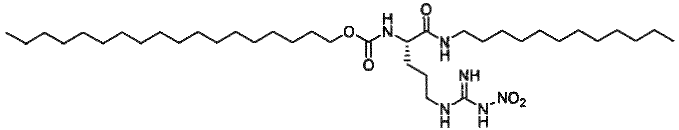

To a THF solution (15 mL) of octadecanol (473 mg, 1.75 mmol) was added, under ice-cooling, a THF solution (5 mL) of triethylamine (177 mg, 1.75 mmol) and triphosgene (208 mg, 0.700 mmol), and the mixture was stirred at room temperature for 2 hr. The reaction mixture was concentrated and the obtained residue was dissolved in THF (10 mL). This was added to a THF solution (20 mL) of the compound (900 mg, 2.13 mmol) obtained in Example 12, step 2 and triethylamine (530 mg, 5.25 mmol) and the mixture was stirred at room temperature overnight. The reaction mixture was concentrated and the obtained residue was purified by silica gel chromatography (dichloromethane/methanol=40/1) and concentrated. The obtained residue was suspended in water, collected by filtration and dried to give the title compound (830 mg, 1.22 mmol, yield 57%).

### (step 2)

### Synthesis of octadecyl N-[(1S)-1-(dodecylcarbamoyl)-4-guanidino-butyl]carbamate hydrochloride

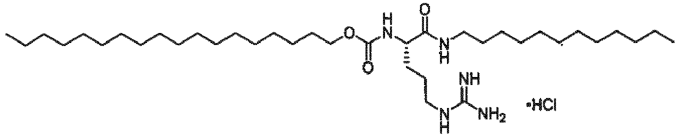

An operation similar to that in Example 33, step 2 was performed using the compound (830 mg, 1.22 mmol) obtained in step 1 to give the title compound (200 mg, 0.297 mmol, yield 24%).
¹H-NMR (400 MHz, CD₃OD) δ4.05-4.02(m, 3H), 3.20-3.16 (m, 4H), 1.65-1.60(m, 1H), 1.50-1.48(m, 7H), 1.28(m, 48H), 0.90(t, J=6.8Hz, 6H).
MS(ESI)m/z:638[M+H]⁺

### Example 40

### Dodecyl N-[(1S)-1-(dodecylcarbamoyl)-4-guanidino-butyl]carbamate hydrochloride (SZ187)

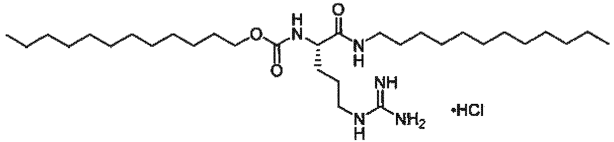

### (step 1)

### Synthesis of dodecyl N-[(1S)-1-(dodecylcarbamoyl)-4-[(N-nitrocarbamimidoyl)amino]butyl]carbamate

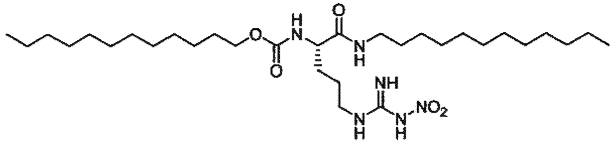

An operation similar to that in Example 39, step 1 was performed using dodecanol (326 mg, 1.75 mmol) instead of octadecanol to give the title compound (450 mg, 0.751 mmol, yield 97%).

### (step 2)

### Synthesis of dodecyl N-[(1S)-1-(dodecylcarbamoyl)-4-guanidino-butyl]carbamate hydrochloride

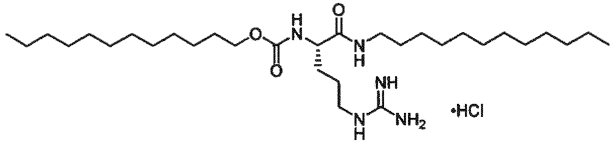

An operation similar to that in Example 34, step 4 was performed using the compound (450 mg, 0.751 mmol) obtained in step 1 to give the title compound (160 mg, 0.271 mmol, yield 36%).
¹H-NMR (400 MHz, DMSO-d₆) δ7.91-7.88(m, 1H), 7.72-7.70(m, 1H), 7.55-6.55(m, 4H), 3.96-3.87(m, 3H), 3.09-2.99(m, 2H), 1.61-1.36(m, 8H), 1.24(m, 38H), 0.85(t, J=6.8Hz, 6H).
MS(ESI)m/z:554[M+H]⁺

### Example 41

### Hexadecyl N-[(1S)-5-amino-1-(octylcarbamoyl)pentyl]carbamate hydrochloride (SZ188)

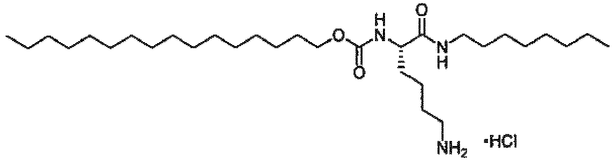

### (step 1)

### Synthesis of hexadecyl N-[(1S)-5-(benzyloxycarbonylamino)-1-(octylcarbamoyl)pentyl] carbamate

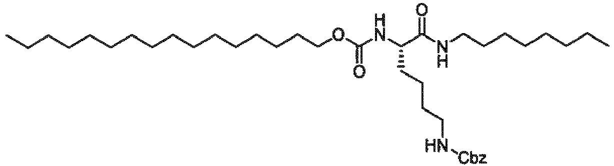

To a THF solution (20 mL) of hexadecanol (672 mg, 2.80 mmol) were added, under ice-cooling, triethylamine (283 mg, 2.80 mmol) and triphosgene (330 mg, 1.10 mmol), and the mixture was stirred at room temperature for 2 hr. The reaction mixture was concentrated and the obtained residue was dissolved in THF (10 mL). This was added to a THF solution (30 mL) of the compound (1.30 g, 3.04 mmol) obtained in Example 37, step 2 and triethylamine (838 mg, 8.30 mmol) and the mixture was stirred at room temperature overnight. The reaction mixture was concentrated and the obtained residue was washed with water, recrystallized from methanol, collected by filtration and dried to give the title compound (1.50 g, 2.27 mmol, yield 75%).

### (step 2)

### Synthesis of hexadecyl N-[(1S)-5-amino-1-(octylcarbamoyl)pentyl]carbamate hydrochloride

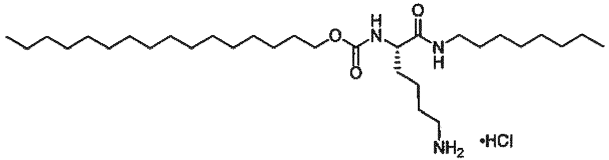

An operation similar to that in Example 37, step 4 was performed using the compound (1.50 g, 2.27 mmol) obtained in step 1 to give the title compound (930 mg, 1.65 mmol, yield 73%).
¹H-NMR (400 MHz, DMSO-d₆) δ7.85-7.82(m, 4H), 7.14(d, J=8.4Hz, 1H), 3.96-3.83(m, 3H), 3.04-3.00(m, 2H), 2.75-2.71(m, 2H), 1.53-1.47(m, 6H), 1.39-1.24(m, 40H), 0.85(t, J=6.0Hz, 6H).
MS(ESI)m/z:526[M+H]⁺

### Example 42

### Hexadecyl N-[(1S)-5-amino-1-(dodecylcarbamoyl)pentyl]carbamate hydrochloride (SZ189)

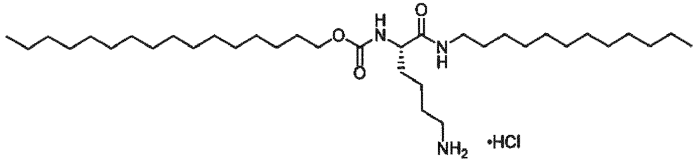

### (step 1)

### Synthesis of hexadecyl N-[(1S)-5-(benzyloxycarbonylamino)-1-(dodecylcarbamoyl)pentyl]carbamate

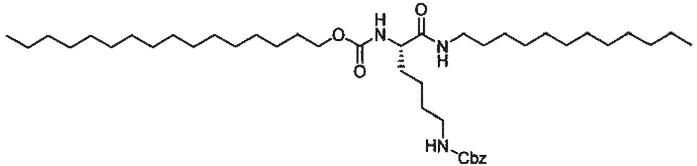

An operation similar to that in Example 41, step 1 was performed using the compound (1.80 g, 3.72 mmol) obtained in Example 12, step 3 to give the title compound (2.00 g, 2.79 mmol, yield 75%).

### (step 2)

### Synthesis of Hexadecyl N-[(1S)-5-amino-1-(dodecylcarbamoyl)pentyl]carbamate hydrochloride

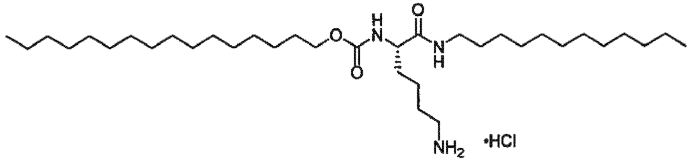

An operation similar to that in Example 37, step 4 was performed using the compound (2.00 g, 2.79 mmol) obtained in step 1 to give the title compound (1.10 g, 1.78 mmol, yield 64%).
¹H-NMR (400 MHz, DMSO-d₆) δ4.07-3.97(m, 3H), 3.17(t, J=6.8Hz, 2H), 2.91(t, J=7.6Hz, 2H), 1.80-1.78(m, 1H), 1.76-1.60(m, 5H), 1.50-1.38(m, 2H), 1.29-1.23(m, 46H), 0.90(t, J=6.8Hz, 6H).
MS(ESI)m/z:582[M+H]⁺

### Example 43

### 1-(4-guanidinobutyl)-3-hexadecyl-urea hydrochloride (SZ190)

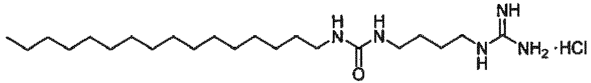

Agmatine sulfate (500 mg, 2.19 mmol) was dissolved in 2-propanol/water (12 mL/18 mL), and a 27% aqueous sodium hydroxide solution was added to adjust same to pH about 13. Hexadecyl isocyanate (646 mg, 2.42 mmol) was added and the mixture was stirred at 30°C overnight. The precipitated solid was collected by filtration, and washed with 2-propanol/water (1/1, v/v). To the obtained crude crystals was added 4N hydrochloric acid/ethyl acetate (5 mL), and the mixture was stirred at room temperature for 2 hr and concentrated. The obtained residue was dissolved in methanol (10 mL), filtered and the filtrate was concentrated to give the title compound (360 mg, 0.829 mmol, yield 38%).
¹H-NMR (400 MHz, CD₃OD) δ3.20-3.10(m, 6H), 1.57-1.48(m, 6H), 1.24-1.27(m, 26H), 0.88(t, J=6.8Hz, 3H).

### Example 44

### N-[(1R)-1-(dodecylcarbamoyl)-4-guanidino-butyl]octadecanamide hydrochloride (SZ191)

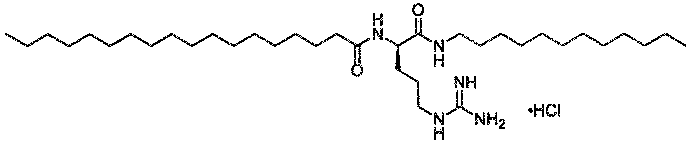

### (step 1)

### Synthesis of tert-butyl N-[(1R)-1-(dodecylcarbamoyl)-4-[(N-nitrocarbamimidoyl)amino]butyl]carbamate

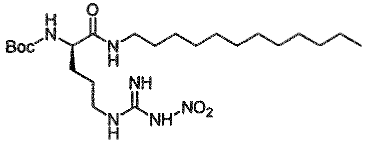

To a dichloromethane solution (50 mL) of (2R)-2-(tert-butoxycarbonylamino)-5-[(N-nitrocarbamimidoyl)amino]pentanoic acid (3.19 g, 11.6 mmol), HOBt (2.00 g, 15.0 mmol) and EDCI (2.90 g, 15.0 mmol) was added triethylamine (3.03 g, 30.0 mmol) and the mixture was stirred for 15 min. To the reaction mixture was added dodecylamine (2.22 g, 12.0 mmol) and the mixture was stirred at 30°C overnight. The reaction mixture was diluted with water and extracted with ethyl acetate. The organic layer was washed with saturated brine, dried over anhydrous sodium sulfate and concentrated under reduced pressure. The obtained residue was purified by silica gel chromatography (dichloromethane/methanol=20/1) to give the title compound (1.80 g, 3.70 mmol, yield 32%).
MS(ESI)m/z:487[M+H]⁺

### (step 2)

### Synthesis of (2R)-2-amino-N-dodecyl-5-[(N-nitrocarbamimidoyl)amino]pentanamide hydrochloride

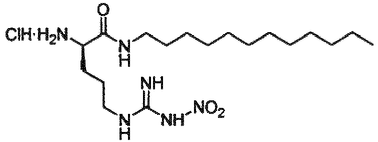

An operation similar to that in Example 34, step 2 was performed using the compound (1.80 g, 3.70 mmol) obtained in step 1 to give the title compound (1.60 g, 3.78 mmol, quantitative).
MS(ESI)m/z:387[M+H]⁺

### (step 3)

### Synthesis of N-[(1R)-1-(dodecylcarbamoyl)-4-[(N-nitrocarbamimidoyl)amino]butyl]octadecanamide

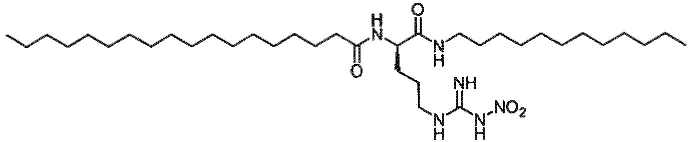

An operation similar to that in Example 34, step 3 was performed using stearic acid (500 mg, 1.76 mmol) instead of palmitic acid, and the compound (966 mg, 2.27 mol) obtained in step 2 instead of the compound obtained in Example 34, step 2 to give the title compound (1.20 g, 1.84 mol, yield 81%).
¹H-NMR (400 MHz, CDCl₃) δ7.42(brs, 1H), 6.73(d, J=8.0Hz, 1H), 6.63(brs, 1H), 4.66-4.63(m, 1H), 3.37-3.20(m, 4H), 2.26-2.22(m, 2H), 1.80-1.48(m, 8H), 1.26-1.24(m, 46H), 0.88(t, J=6.8Hz, 6H).

### (step 4)

### Synthesis of N-[(1R)-1-(dodecylcarbamoyl)-4-guanidino-butyl]octadecanamide hydrochloride

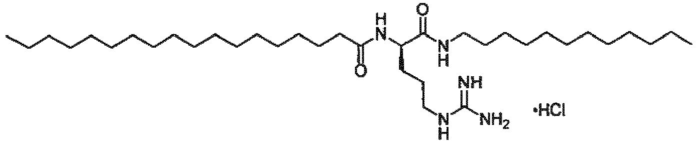

An operation similar to that in Example 2, step 2 was performed using the compound (1.20 g, 1.84 mmol) obtained in step 3 to give the title compound (300 mg, 0.465 mol, yield 25%).
¹H-NMR (400 MHz, DMSO-d₆) δ7.98(d, J=8.0Hz, 1H), 7.90(s, 1H), 7.78(s, 1H), 7.41(s, 1H), 7.28(s, 1H), 7.15(s, 1H), 4.21-4.18(m, 1H), 3.09-3.00(m, 4H), 2.12-2.10(m, 2H), 1.63-1.36(m, 8H), 1.24-1.22(m, 46H), 0.84(t, J=6.8Hz, 6H).
MS(ESI)m/z:608[M+H]⁺

### Example 45

### (2R)-N-dodecyl-5-guanidino-2-(hexadecylcarbamoylamino)pentanamide hydrochloride (SZ193)

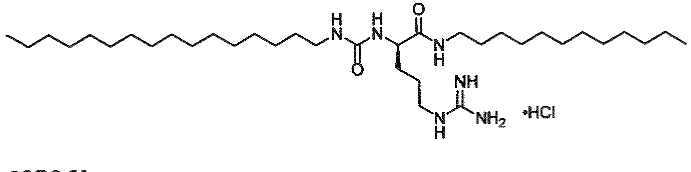

### (step 1)

### Synthesis of (2R)-N-dodecyl-2-(hexadecylcarbamoylamino)-5-[(N-nitrocarbamimidoyl)amino]pentanamide

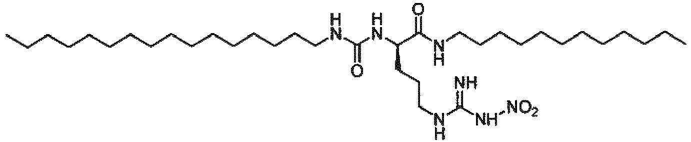

To a DMF solution (20 mL) of the compound (500 mg, 1.18 mmol) obtained in Example 44, step 2 were added triethylamine (364 mg, 3.60 mmol) and hexadecyl isocyanate (352 mg, 1.32 mmol), and the mixture was stirred at room temperature overnight. The precipitated solid was filtered and methanol was added to the obtained residue and the residue was collected by filtration and dried to give the title compound (700 mg, 1.07 mol, yield 91%).
MS(ESI)m/z:654[M+H]⁺

### (step 2)

### (2R)-N-dodecyl-5-guanidino-2-(hexadecylcarbamoylamino)pentanamide hydrochloride (SZ193)

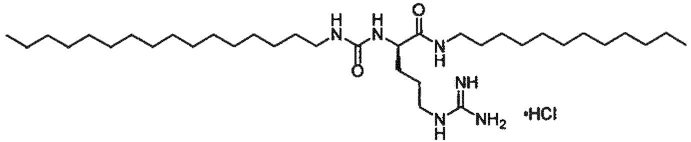

An operation similar to that in Example 33, step 2 was performed using the compound (700 mg, 1.07 mmol) obtained in step 1 to give the title compound (350 mg, 0.542 mol, yield 51%).
¹H-NMR (400 MHz, DMSO-d₆) δ7.96(brs, 1H), 7.63(brs, 1H), 7.31(brs, 1H), 6.69(brs, 1H), 6.12-6.07(m, 1H), 4.11-4.07(m, 1H), 3.11-2.91(m, 6H), 1.43-1.40(m, 1H), 1.39-1.27(m, 7H), 1.23(m, 45H), 0.85(t, J=6.8Hz, 6H).

### Example 46

### N-[(1R)-5-amino-1-(dodecylcarbamoyl)pentyl]hexadecanamide hydrochloride (SZ194)

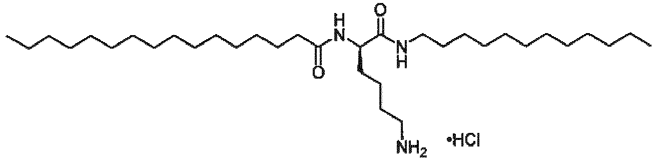

### (step 1)

### Synthesis of tert-butyl N-[(1R)-5-(benzyloxycarbonylamino)-1-(dodecylcarbamoyl)pentyl]carbamate

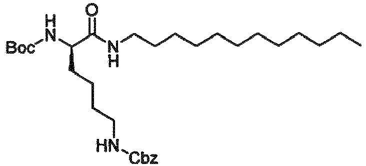

An operation similar to that in Example 37, step 1 was performed using (2R)-6-(benzyloxycarbonylamino)-2-(tert-butoxycarbonylamino)hexanoic acid (1.00 g, 2.63 mmol) to give the title compound (1.32 g, 2.41 mmol, yield 92%).
MS(ESI)m/z:548[M+H]⁺

### (step 2)

### Synthesis of N-[(5R)-5-amino-6-(dodecylamino)-6-oxo-hexyl]carbamate benzyl hydrochloride

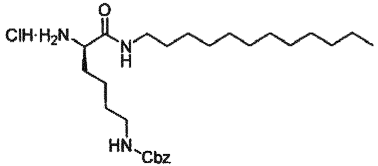

An operation similar to that in Example 34, step 2 was performed using the compound (1.32 g, 2.41 mmol) obtained in step 1 to give the title compound (1.16 g, 2.40 mmol, yield 99%).
MS(ESI)m/z:448 [M+H]⁺

### (step 3)

### Synthesis of benzyl N-[(5R)-6-(dodecylamino)-5-(hexadecanoylamino)-6-oxo-hexyl]carbamate

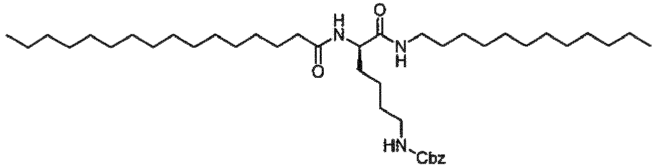

An operation similar to that in Example 37, step 3 was performed using palmitic acid (491 mg, 1.90 mmol) instead of stearic acid, and the compound (1.16 g, 2.40 mmol) obtained in step 2 instead of the compound obtained in Example 37, step 2 to give the title compound (1.10 g, 1.60 mmol, yield 67%).

### (step 4)

### N-[(1R)-5-amino-1-(dodecylcarbamoyl)pentyl]hexadecanamide hydrochloride

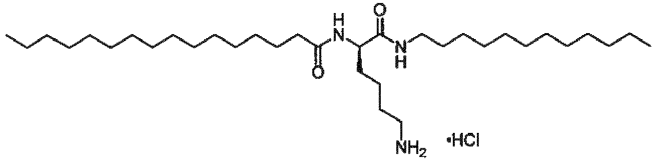

An operation similar to that in Example 37, step 4 was performed using the compound (1.10 g, 1.60 mmol) obtained in step 3 to give the title compound (556 mg, 0.945 mmol, yield 59%).
¹H-NMR (400 MHz, DMSO-d₆) δ7.94-7.92 (m, 2H), 7.83-7.78 (m, 3H), 4.17-4.16(m, 1H), 3.05-2.98(m, 2H), 2.73-2.69(m, 2H), 2.12-2.09(m, 2H), 1.60-1.05(m, 52H), 0.84(t, J=12.0Hz, 6H).
MS(ESI)m/z:552 [M+H]⁺

### Example 47

### N-[(1S)-1-(dodecylcarbamoyl)-3-methyl-butyl]octadecanamide (SZ195)

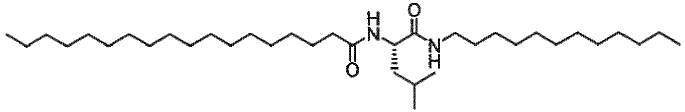

### (step 1)

### Synthesis of tert-butyl N-[(1S)-1-(dodecylcarbamoyl)-3-methyl-butyl]carbamate

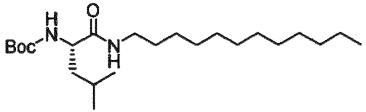

An operation similar to that in Example 6, step 1 was performed using (2S)-2-(tert-butoxycarbonylamino)-4-methyl-pentanoic acid (6 g, 26 mmol) instead of (2S)-2-(tert-butoxycarbonylamino)propanoic acid to give the title compound (6 g, 15.1 mmol, yield 58%).
MS(ESI)m/z:399[M+H]⁺

### (step 2)

### Synthesis of (2S)-2-amino-N-dodecyl-4-methyl-pentanamide hydrochloride

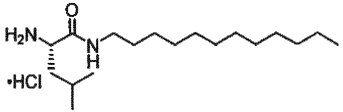

To an ethyl acetate solution (10 mL) of the compound (3.67 g, 9.2 mmol) obtained in step 1 was added 4N hydrochloric acid/ethyl acetate (10 mL) and the mixture was stirred at room temperature for 1 hr. The solvent was evaporated under reduced pressure to give the title compound (3.1 g, 9.2 mmol, yield quantitative).
MS(ESI)m/z:299[M+H]⁺

### (step 3)

### Synthesis of N-[(1S)-1-(dodecylcarbamoyl)-3-methyl-butyl]octadecanamide

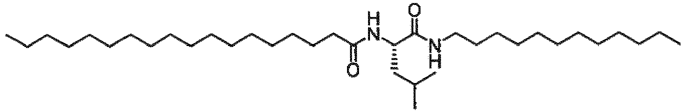

An operation similar to that in Example 13, step 3 was performed using the compound (1.17 g, 3.5 mmol) obtained in step 2 instead of (2S)-2-amino-N-dodecyl-hexanamide hydrochloride and stearic acid (797 mg, 2.8 mmol) instead of behenic acid to give the title compound (500 mg, 0.885 mmol, yield 32%).
¹H-NMR (400 MHz, CDCl₃) δ6.28-6.26(m, 1H), 6.01(d, J=8.4Hz, 1H), 4.43-4.01(m, 1H), 3.24-3.17(m, 2H), 2.20-2.16(m, 2H), 1.67-1.48(m, 7H), 1.28(m, 46H), 0.94-0.86(m, 12H).

### Example 48

### N-[(1S)-3-methyl-1-(octylcarbamoyl)butyl]octadecanamide (SZ196)

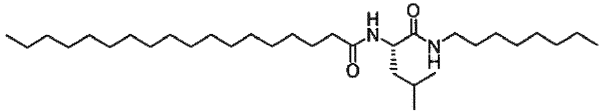

### (step 1)

### Synthesis of tert-butyl N-[(1S)-3-methyl-1-(octylcarbamoyl)butyl]carbamate

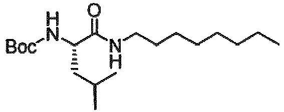

An operation similar to that in Example 47, step 1 was performed using octylamine (4 g, 31.2 mmol) instead of dodecylamine to give the title compound (6 g, 17.5 mmol, yield 67%).
MS(ESI)m/z:343[M+H]⁺

### (step 2)

### Synthesis of (2S)-2-amino-4-methyl-N-octyl-pentanamide hydrochloride

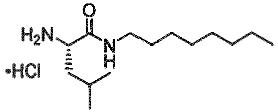

An operation similar to that in Example 47, step 2 was performed using the compound (6 g, 17.5 mmol) obtained in step 1 to give the title compound (4.9 g, 17.5 mmol, yield quantitative).
MS(ESI)m/z:243[M+H]⁺

### (step 3)

### Synthesis of N-[(1S)-3-methyl-1-(octylcarbamoyl)butyl]octadecanamide

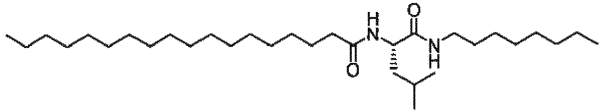

An operation similar to that in Example 47, step 2 was performed using the compound (1.3 g, 4.7 mmol) obtained in step 2 to give the title compound (500 mg, 0.983 mmol, yield 26%).
¹H-NMR (400 MHz, CDCl₃) δ6.30-6.27(m, 1H), 6.03-6.01(m, 1H), 4.43-4.39(m, 2H), 3.25-3.17(m, 2H), 2.20-2.16(m, 2H), 1.67-1.42(m, 7H), 1.25(m, 38H), 0.95-0.86(m, 12H).
MS(ESI)m/z:509[M+H]⁺

### Example 49

### N-[(1S)-1-(dodecylcarbamoyl)-3-methyl-butyl]docosanamide (SZ197)

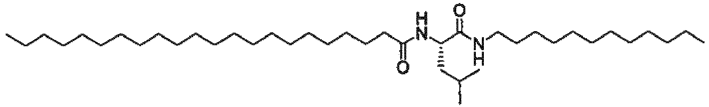

An operation similar to that in Example 47, step 3 was performed using behenic acid (681 mg, 2 mmol) instead of stearic acid to give the title compound (500 mg, 0.805 mmol, yield 40%).
¹H-NMR (400 MHz, CDCl₃) δ6.21-6.19(m, 1H), 5.95-5.93(m, 1H), 4.44-4.38(m, 1H), 3.24-3.18(m, 2H), 2.20-2.16(m, 2H), 1.65-1.44(m, 6H), 1.25(m, 55H), 0.95-0.86 m, 12H).

### Example 50 N-[(1S)-3-methyl-1-(octylcarbamoyl)butyl]docosanamide (SZ198)

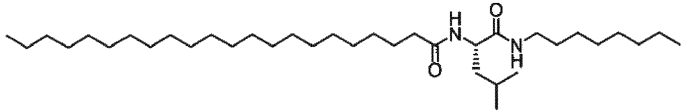

An operation similar to that in Example 48, step 3 was performed using behenic acid (790 mg, 2.3 mmol) instead of stearic acid to give the title compound (500 mg, 0.885 mmol, yield 38%).
¹H-NMR (400 MHz, CDCl₃) δ6.23-6.21(m, 1H), 5.97-5.95(m, 1H), 4.42-4.38(m, 1H), 3.25-3.18(m, 2H), 2.18(t, J=7.2Hz, 2H), 1.64-1.46(m, 6H), 1.25(m, 47H), 0.95-0.86(m, 12H).

### Example 51

### N-[(1S)-3-methyl-1-(octylcarbamoyl)butyl]hexadecanamide (SZ199)

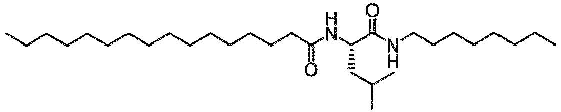

An operation similar to that in Example 48, step 3 was performed using palmitic acid (589 mg, 2.3 mmol) instead of stearic acid to give the title compound (500 mg, 11.04 mmol, yield 45%).
¹H-NMR (400 MHz, CDCl₃) δ6.21-6.18 (m, 1H), 5.95-5.93(m, 1H), 4.44-4.38(m, 1H), 3.25-3.18(m, 2H), 2.18(t, J=6.8Hz, 2H), 1.64-1.45(m, 7H), 1.25(m, 34H), 0.95-0.86(m, 12H).
MS(ESI)m/z:481[M+H]⁺

### Example 52

### (2S)-N-dodecyl-2-(hexadecylcarbamoylamino)-4-methyl-pentanamide (SZ200)

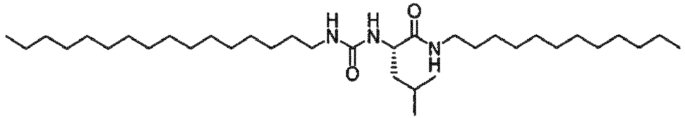

To a THF solution (15 mL) of hexadecylamine (482 mg, 2 mmol) were added triethylamine (202 mg, 2 mmol) and triphosgene (238 mg, 0.8 mmol) at 0°C, and the mixture was stirred at room temperature for 3 hr. The reaction mixture was concentrated and the obtained residue was dissolved in THF (10 mL). A THF solution (20 mL) of the compound (800 mg, 2.4 mmol) obtained in Example 47, step 2 and triethylamine (606 mg, 6 mmol) was added dropwise and the mixture was stirred at room temperature overnight. To the reaction mixture was added water (10 mL) and concentrated. Methanol (20 mL) to the residue and the solid was collected by filtration. To the obtained solid was added methanol (30 mL) and the mixture was stirred at 70°C for 1 hr, cooled to room temperature and the precipitated solid was collected by filtration to give the title compound (400 mg, 0.707 mmol, yield 36%).
¹H-NMR (400 MHz, CDCl₃) δ6.59-6.57(m, 1H), 5.59-5.57(m, 1H), 5.06(brs, 1H), 4.25-4.23(m, 1H), 3.26-3.07(m, 4H), 1.63-1.43(m, 6H), 1.25(m, 46H), 0.95-0.86(m, 12H).
MS(ESI)m/z:566[M+H]⁺

### Example 53

### (2S)-2-(hexadecylcarbamoylamino)-4-methyl-N-octyl-pentanamide (SZ201)

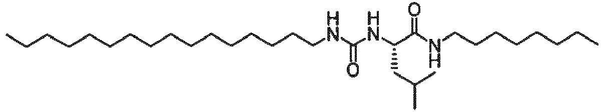

An operation similar to that in Example 52 was performed using the compound (900 mg, 3.2 mmol) obtained in Example 48, step 2 to give the title compound (500 mg, 0.981 mmol, yield 36%).
¹H-NMR (400 MHz, CDCl₃) δ5.62-5.59(m, 1H), 5.59(brs, 1H), 5.08(brs, 1H), 4.27-4.22(m, 1H), 3.27-3.05(m, 4H), 1.65-1.43(m, 6H), 1.25(m, 37H), 0.95-0.86(m, 12H).
MS(ESI)m/z:510[M+H]⁺

### Example 54

### Hexadecyl N-[(1S)-1-(dodecylcarbamoyl)-3-methyl-butyl]carbamate (SZ202)

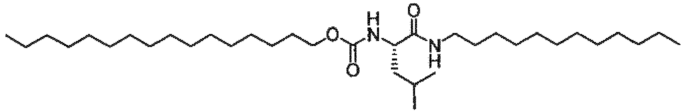

An operation similar to that in Example 52 was performed using 1-hexadecanol (484 mg, 2 mmol) instead of hexadecylamine to give the title compound (500 mg, 0.882 mmol, yield 44%). ¹H-NMR (400 MHz, CDCl₃) δ6.03-6.00(m, 1H), 5.03(brs, 1H), 4.10-4.03(m, 3H), 3.26-3.20(m, 2H), 1.68-1.56(m, 2H), 1.48-1.47(m, 3H), 1.25(m, 46H), 0.95-0.86(m, 12H).
MS (ESI)m/z:567 [M+H]⁺

### Example 55

### Hexadecyl N-[(1S)-3-methyl-1-(octylcarbamoyl)butyl]carbamate (SZ203)

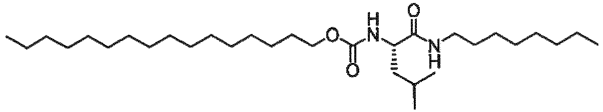

An operation similar to that in Example 53 was performed using 1-hexadecanol (653 mg, 2.7 mmol) instead of hexadecylamine to give the title compound (260 mg, 0.509 mmol, yield 19%).
¹H-NMR (400 MHz, CDCl₃) δ6.03-6.00(m, 1H), 5.03(brs, 1H), 4.10-4.03(m, 3H), 3.26-3.20(m, 2H), 1.67-1.64(m, 1H), 1.59-1.47(m, 5H), 1.25(m, 37H), 0.95-0.86(m, 12H).
MS(ESI)m/z:511[M+H]⁺

### Example 56

### N-[3-(dodecylamino)-2,2-dimethyl-3-oxo-propyl]octadecanamide (SZ204)

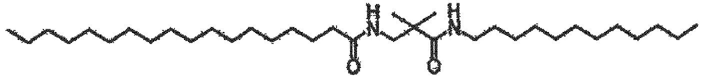

An operation similar to that in Example 9, step 3 was performed using stearic acid (592 mg, 2.08 mmol) instead of behenic acid to give the title compound (250 mg, 0.454 mmol, yield 22%).
¹H-NMR (400 MHz, CDCl₃) δ6.37(brs, 1H), 5.86(brs, 1H), 3.35-3.34(m, 2H), 3.23-3.19(m, 2H), 3.18-3.14(m, 2H), 1.64-1.61(m, 2H), 1.50-1.47 (m, 2H), 1.28(m, 46H), 1.18(s, 6H), 0.88(t, J=6.8Hz, 6H).

### Example 57

### N-[(1S)-1-(dodecylcarbamoyl)-5-guanidino-pentyl]octadecanamide hydrochloride (SZ205)

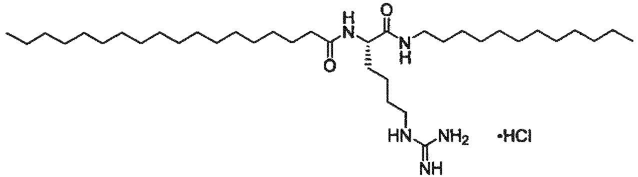

### (step 1)

### Synthesis of N-[(1S)-1-(dodecylcarbamoyl)-5-[(N-nitrocarbamimidoyl)amino]pentyl]octadecanamide

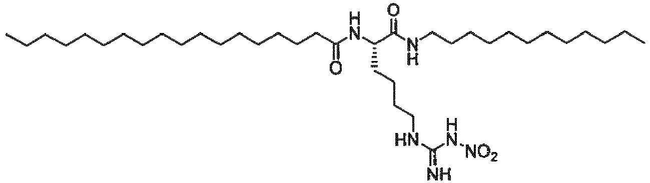

An operation similar to that in Example 20, step 3 was performed using stearic acid (568 mg, 2.0 mmol) instead of behenic acid to give the title compound (1.3 g, 1.95 mmol, yield 97%).

### (step 2)

### Synthesis of N-[(1S)-1-(dodecylcarbamoyl)-5-guanidino-pentyl]octadecanamide hydrochloride

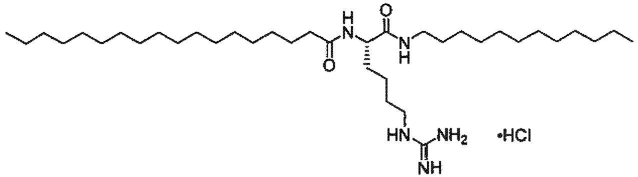

The compound (1 g, 1.5 mmol) obtained in step 1 was dissolved in methanol (40 mL), concentrated hydrochloric acid (0.5 mL) and 20%(w/w) palladium-carbon(10%wet) (200 mg) were added and the mixture was stirred under 50 psi hydrogen atmosphere at 50°C overnight. Insoluble material was filtered off, and the filtrate was concentrated to give the title compound (250 mg, 0.38 mmol, yield 25%).
¹H-NMR (400 MHz, DMSO-d₆) δ7.92-7.90(m, 1H), 7.85-7.84(m, 1H), 7.57-7.55(m, 1H), 7.39-6.73(m, 3H), 4.19-4.17(m, 1H), 3.09-2.98(m, 4H), 2.11-2.09(m, 2H), 1.62-1.11(m, 56H), 0.85(t, J=6.0Hz, 6H).
MS(ESI)m/z:622[M+H]⁺

### Example 58

### N-[(1S)-1-(hexylcarbamoyl)-5-guanidino-pentyl]octadecanamide hydrochloride (SZ206)

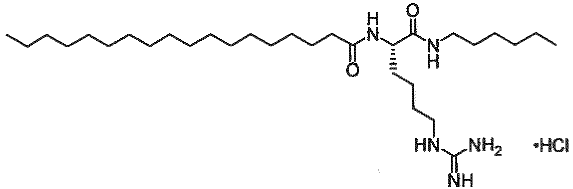

### (step 1)

### Synthesis of N-[(1S)-1-(hexylcarbamoyl)-5-[(N-nitrocarbamimidoyl)amino]pentyl]octadecanamide

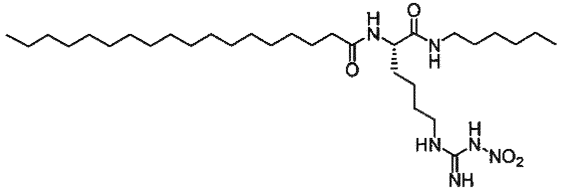

An operation similar to that in Example 24, step 3 was performed using stearic acid (568 mg, 2.0 mmol) instead of behenic acid to give the title compound (1.1 g, 1.9 mmol, yield 94%).

### (step 2)

### Synthesis of N-[(1S)-1-(hexylcarbamoyl)-5-guanidino-pentyl]octadecanamide hydrochloride

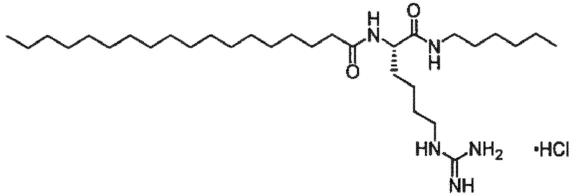

An operation similar to that in Example 57, step 2 was performed using the compound (1 g, 1.7 mmol) obtained in step 1 instead of N-[(1S)-1-(dodecylcarbamoyl)-5-[(N-nitrocarbamimidoyl)amino]pentyl]octadecanamide to give the title compound (230 mg, 0.40 mmol, yield 23%).
¹H-NMR (400 MHz, DMSO-d₆) δ7.91-7.85(m, 2H), 7.47-6.89(m, 4H), 4.22-4.16(m, 1H), 3.07-3.00(m, 4H), 2.10(t, J=6.8Hz, 2H), 1.63-1.23(m, 44H), 0.85(t, J=6.4Hz, 6H).
MS(ESI)m/z:538[M+H]⁺

### Example 59

### (2S)-2-(diheptylamino)-N-dodecyl-5-guanidino-pentanamide dihydrochloride (SZ207)

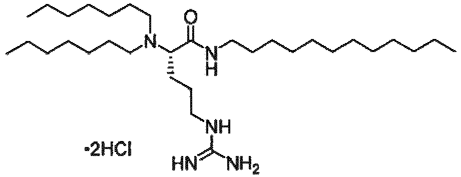

### (step 1)

### Synthesis of (2S)-2-(diheptylamino)-N-dodecyl-5-[(N-nitrocarbamimidoyl)amino]pentanamide

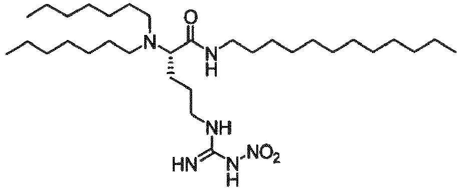

To a 1,2-dichloroethane solution (20 mL) of the compound (300 mg, 0.71 mmol) obtained in Synthetic Example 1, step 2, triethylamine (108 mg, 1.07 mmol) and heptanal (170 mg, 1.49 mmol) was added sodium triacetoxyborohydride (1.05 g, 4.95 mmol) and the mixture was stirred under a nitrogen atmosphere at 40°C for 20 hr. Insoluble material was filtered off from the reaction mixture and the residue obtained by concentration was purified by silica gel chromatography (petroleum ether/ethyl acetate) to give the title compound (149 mg, 0.256 mmol, yield 36%).
MS(ESI)m/z:583[M+H]⁺

### (step 2)

### Synthesis of (2S)-2-(diheptylamino)-N-dodecyl-5-guanidino-pentanamide dihydrochloride

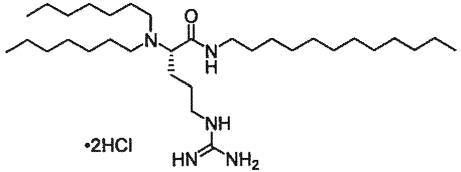

An operation similar to that in Example 57, step 2 was performed using the compound (400 mg, 0.69 mmol) obtained in step 1 instead of N-[(1S)-1-(dodecylcarbamoyl)-5-[(N-nitrocarbamimidoyl)amino]pentyl]octadecanamide to give the title compound (340 mg, 0.627 mmol, yield 91%).
¹H-NMR (400 MHz, DMSO-d₆) δ10.29(s, 1H), 10.14(s, 1H), 9.16(s, 1H), 7.95(s, 1H), 7.39(s, 1H), 7.27(s, 1H), 7.14(s, 1H), 4.01(t, J=4.0Hz, 1H), 3.25-3.18(m, 3H), 3.05-3.02(m, 5H), 1.98-1.63(m, 6H), 1.44-1.42(m, 4H), 1.26-1.23(s, 34H), 0.88-0.84(m, 9H).
MS(ESI)m/z:538[M+H]⁺

### Example 60

### N-[2-(4-pyridyl)ethyl]docosanamide hydrochloride (SZ209)

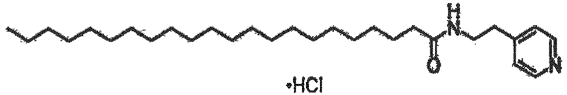

To a dichloromethane solution (10 mL) of behenic acid (1.3 g, 3.92 mmol) were added DMF (0.2 mL) and oxalyl dichloride (622 mg, 4.9 mmol), and the mixture was stirred at room temperature for 1 hr. The reaction mixture was concentrated and the obtained residue was dissolved in THF (5 mL), added dropwise to a THF solution (35 mL) of 2-(4-pyridyl)ethanamine (600 mg, 4.9 mmol) and triethylamine (1.48 g, 14.7 mmol) and the mixture was stirred at room temperature overnight. To the reaction mixture was added water (10 mL) and the mixture was concentrated. The residue was washed with methanol (20 mL), to the obtained solid was added methanol (30 mL) again and the mixture was stirred at 70°C for 30 min. The mixture was cooled to room temperature, and the solid collected by filtration was dissolved in dichloromethane (6 mL), 4N hydrochloric acid/ethyl acetate (6 mL) was added and the mixture was stirred at room temperature for 1 hr and concentrated to give the title compound (400 mg, 0.831 mmol, yield 21%).
¹H-NMR (400 MHz, DMSO-d₆) δ8.80(d, J=6.4Hz, 2H), 7.92-7.87(m, 3H), 3.44-3.90(m, 2H), 2.99(t, J=6.4Hz, 2H), 1.99(t, J=7.2Hz, 2H), 1.40-1.38(m, 2H), 1.23(m, 36H), 0.85(t, J=6.8Hz, 3H).
MS(ESI)m/z:445[M+H]⁺

### Example 61

### (2S)-5-guanidino-2-(hexadecylcarbamoylamino)-N-octyl-pentanamide hydrochloride (SZ210)

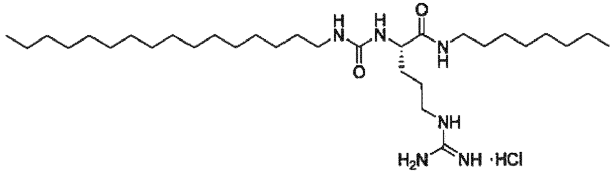

### (step 1)

### Synthesis of 9H-fluorene-9-ylmethyl N-[(1S)-4-[[N,N'-bis(tert-butoxycarbonyl)carbamimidoyl]amino]-1-(octylcarbamoyl)butyl]carbamate

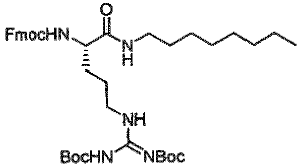

An operation similar to that in Example 17, step 1 was performed using octylamine (0.153 mL, 0.922 mmol) instead of dodecylamine to give the title compound (281 mg, yield 52%).
¹H-NMR (400 MHz, CDCl₃) δ11.47(s, 1H), 8.44(s, 1H), 7.76(d, J=7.5Hz, 2H), 7.65-7.56(m, 2H), 7.44-7.36(m, 2H), 7.30(tdd, J=7.4, 2.5, 1.2Hz, 2H), 6.38(s, 1H), 6.20(d, J=8.4Hz, 1H), 4.50-4.32(m, 2H), 4.29-4.18(m, 2H), 3.52-3.13(m, 4H), 1.89-1.79(m, 1H), 1.77-1.56(m, 3H), 1.51(s, 9H), 1.48(s, 9H), 1.32-1.19(m, 10H), 0.92-0.84(m, 3H).

### (step 2)

### Synthesis of tert-butyl N-[[[(4S)-4-amino-5-(octylamino)-5-oxo-pentyl]amino]-(tert-butoxycarbonylamino)methylene]carbamate

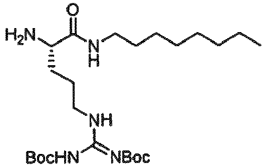

The compound (412 mg, 0.583 mmol) obtained in step 1 was dissolved in dichloromethane (8 mL), and piperidine (249 mg, 2.92 mmol) was added. After stirring for 1 hr, piperidine (249 mg, 2.92 mmol) was added, and the mixture was stirred for 2 hr. The reaction mixture was concentrated and the obtained residue was purified by silica gel chromatography (ethyl acetate/hexane=17/3) to give the title compound (207 mg, yield 73%).
¹H-NMR (400 MHz, CDCl₃) δ11.47(s, 1H), 8.40-8.32(m, 1H), 7.22-7.16(m, 1H), 3.47-3.37(m, 3H), 3.30-3.17(m, 2H), 1.93-1.82(m, 1H), 1.73-1.63(m, 2H), 1.61-1.56(m, 1H), 1.50(d, J=2.9Hz, 18H), 1.45-1.37(m, 2H), 1.33-1.23(m, 10H), 0.92-0.84(m, 3H).

### (step 3)

### Synthesis of tert-butyl N-[(tert-butoxycarbonylamino)-[[(4S)-4-(hexadecylcarbamoylamino)-5-(octylamino)-5-oxo-pentyl]amino]methylene]carbamate

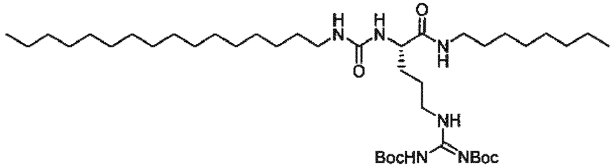

To a THF solution (3 mL) of the compound (110 mg, 0.227 mmol) obtained in step 2 were added triethylamine (68.9 mg, 0.681 mmol) and hexadecyl isocyanate (78.9 mg, 0.295 mmol) and the mixture was stirred at 30°C for 4 hr. The reaction mixture was diluted with water and extracted with dichloromethane. The organic layer was washed with saturated brine, dried over anhydrous sodium sulfate and concentrated. The obtained residue was purified by silica gel chromatography (ethyl acetate/hexane=13/7) to give the title compound (136 mg, yield 80%).
¹H-NMR (400 MHz, CDCl₃) δ11.43(s, 1H), 8.55(dd, J=8.4Hz, 4.8Hz, 1H), 6.96(d, J=8.0Hz, 1H), 6.69(t, J=5.8Hz, 1H), 5.25-5.19(m, 1H), 4.41-4.32(m, 1H), 3.80-3.69(m, 1H), 3.38-3.28(m, 1H), 3.27-3.04(m, 4H), 2.02-1.92(m, 1H), 1.76-1.60(m, 3H), 1.53-1.41(m, 24H), 1.37-1.17(m, 34H), 0.94-0.82(m, 6H).

### (step 4)

### Synthesis of (2S)-5-guanidino-2-(hexadecylcarbamoylamino)-N-octyl-pentanamide hydrochloride

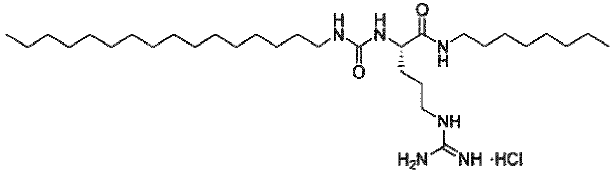

To the compound (136 mg, 0.181 mol) obtained in step 3 was added a TFA/dichloromethane solution (v/v=2/1, 6 mL), and the mixture was stirred at room temperature for 2 hr. The reaction mixture was concentrated, water (2 ml) was added and the mixture was freeze-dried. The obtained residue was suspended in ethyl acetate (2 mL), 4N hydrochloric acid/ethyl acetate (0.23 mL) was added and the mixture was stirred for 30 min. The reaction mixture was concentrated, water (2 mL) was added, and the mixture was lyophilized to give the title compound (104 mg, yield 97%).
¹H-NMR (400 MHz, DMSO-d₆) δ7.94(t, J=5.6Hz, 1H), 7.48(t, J=5.9Hz, 1H), 7.32-6.66(m, 3H), 6.09(t, J=5.7Hz, 1H), 6.03(d, J=8.5Hz, 1H), 4.15-4.06(m, 1H), 3.15-2.87(m, 6H), 1.58-1.17(m, 44H), 0.89-0.82(m, 6H).

### Example 62

(2S)-N-dodecyl-5-guanidino-2-(octadecylcarbamoylamino)pentanamide hydrochloride (SZ211)

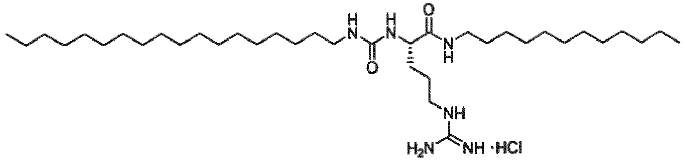

### (step 1)

### Synthesis of tert-butyl N-[(tert-butoxycarbonylamino)-[[(4S)-5-(dodecylamino)-4-(octadecylcarbamoylamino)-5-oxo-pentyl]amino]methylene]carbamate

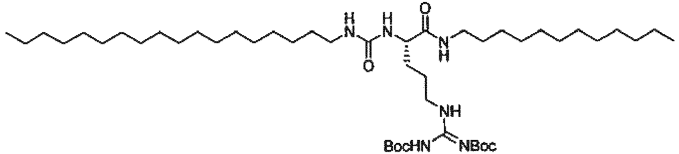

An operation similar to that in Example 61, step 3 was performed using the compound (111 mg, 0.204 mmol) obtained in Example 17, step 2 instead of the compound obtained in Example 61, step 2, and octadecyl isocyanate (78.3 mg, 0.265 mmol) instead of hexadecyl isocyanate to give the title compound (143 mg, yield 84%).
¹H-NMR (400 MHz, CDCl₃) δ11.43(s, 1H), 8.58-8.52(m, 1H), 6.98-6.94(m, 1H), 6.68(t, J=5.6Hz, 1H), 5.25-5.19(m, 1H), 4.40-4.32(m, 1H), 3.79-3.72(m, 1H), 3.33(td, J=13.7Hz, 6.9Hz, 1H), 3.25-3.05(m, 4H), 2.01-1.92(m, 1H), 1.74-1.62(m, 3H), 1.50(d, J=3.5Hz, 28H), 1.25(d, J=3.9Hz, 42H), 0.91-0.84(m, 6H).

### (step 2)

### Synthesis of (2S)-N-dodecyl-5-guanidino-2-(octadecylcarbamoylamino)pentanamide hydrochloride

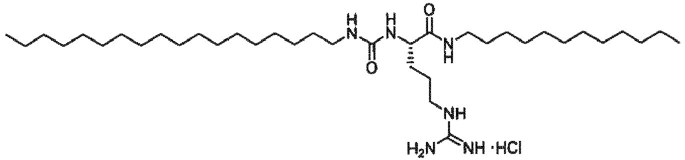

To the compound (143 mg, 0.171 mol) obtained in step 1 was added a TFA/dichloromethane solution (v/v=2/1, 6 mL) and the mixture was stirred at room temperature for 2 hr. The reaction mixture was concentrated, water (2 mL) was added and the mixture was freeze-dried. The obtained residue was suspended in ethyl acetate (3 mL), 4N hydrochloric acid/ethyl acetate (0.21 mL) was added and the mixture was stirred for 30 min. The reaction mixture was concentrated, water (2 mL) was added, and the mixture was lyophilized to give the title compound (117 mg, yield quantitative).
¹H-NMR (400 MHz, DMSO-d₆) δ7.93(t, J=5.6Hz, 1H), 7.53(t, J=5.8Hz, 1H), 7.34-6.68(m, 3H), 6.12-6.06(m, 1H), 6.04(d, J=8.5Hz, 1H), 4.13-4.05(m, 1H), 3.19-2.83(m, 6H), 1.62-1.05(m, 56H), 0.90-0.81(m, 6H).

### Example 63

### (2S)-N-dodecyl-2-(dodecylcarbamoylamino)-5-guanidino-pentanamide hydrochloride (SZ212)

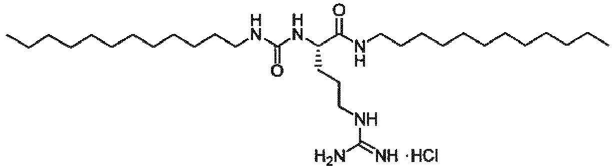

### (step 1)

### Synthesis of tert-butyl (NE)-N-[(tert-butoxycarbonylamino)-[[(4S)-5-(dodecylamino)-4-(dodecylcarbamoylamino)-5-oxo-pentyl]amino]methylene]carbamate

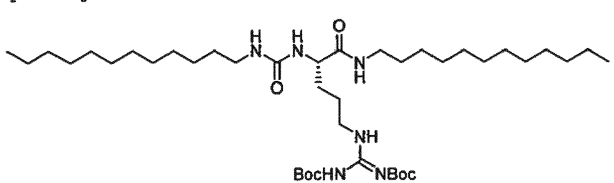

An operation similar to that in Example 61, step 3 was performed using the compound (131 mg, 0.226 mmol) obtained in Example 17, step 2 instead of the compound obtained in Example 61, step 2 and dodecyl isocyanate (0.0650 mg, 0.266 mmol) instead of hexadecyl isocyanate to give the title compound (160 mg, 0.213 mmol, yield 88%).
¹H-NMR (400 MHz, CDCl₃) δ11.43(s, 1H), 8.55(dd, J=8.2, 4.7Hz, 1H), 6.96(d, J=7.9Hz, 1H), 6.69(t, J=5.8Hz, 1H), 5.22(t, J=5.7Hz, 1H), 4.40-4.31(m, 1H), 3.81-3.69(m, 1H), 3.39-3.28(m, 1H), 3.26-3.02(m, 3H), 2.02-1.92(m, 1H), 1.77-1.61(m, 3H), 1.50(d, J=3.4Hz, 20H), 1.37-1.19(m, 38H), 0.92-0.83(m, 6H).

### (step 2)

### Synthesis of (2S)-N-dodecyl-2-(dodecylcarbamoylamino)-5-guanidino-pentanamide hydrochloride

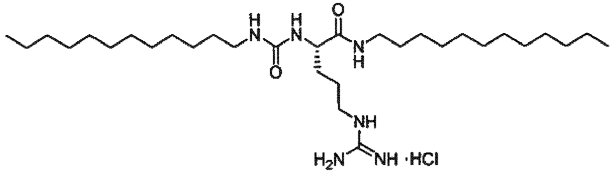

An operation similar to that in Example 61, step 4 was performed using the compound (160 mg, 0.213 mmol) obtained in step 3 to give the title compound (91.6 mg, 0.155 mmol, yield 73%).
¹H-NMR (400 MHz, DMSO-d₆) δ7.94(t, J=5.7Hz, 1H), 7.56(t, J=5.9Hz, 1H), 7.40-6.69(m, 2H), 6.10(t, J=5.6Hz, 1H), 6.05(d, J=8.5Hz, 1H), 4.14-4.07(m, 1H), 3.18-2.87(m, 6H), 1.24(s, 44H), 0.90-0.80(m, 6H).

### Example 64

### (2S)-6-amino-2-(hexadecylcarbamoylamino)-N-octyl-hexanamide hydrochloride (SZ213)

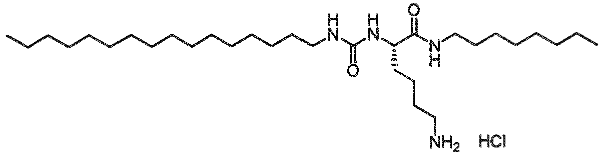

### (step 1)

### Synthesis of tert-butyl N-[(1S)-5-(benzyloxycarbonylamino)-1-(octylcarbamoyl)pentyl]carbamate

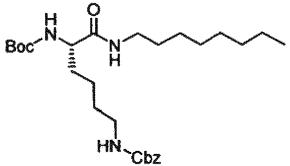

To the compound (495 mg, 1.30 mmol) obtained in Example 12, step 1, EDCI (299 mg, 1.56 mmol) and HOBt (211 mg, 1.56 mmol) was added dichloromethane (7 mL), octylamine (0.237 mL, 1.43 mmol) and triethylamine (0.217 mL, 1.56 mmol) were added, and the mixture was stirred at room temperature for 3 days. Water was added to the reaction mixture, and the mixture was extracted with dichloromethane, and the organic layer was washed with saturated aqueous sodium hydrogen carbonate solution and saturated aqueous ammonium chloride solution, and dried over anhydrous magnesium sulfate. The solvent was evaporated under reduced pressure and the obtained residue was purified by silica gel chromatography (hexane/ethyl acetate) to give the title compound (626 mg, 1.27 mmol, yield 98%).
¹H-NMR (400 MHz, CDCl₃) δ7.40-7.28(m, 5H), 6.14-5.97(m, 1H), 5.22-5.00(m, 3H), 4.89-4.73(m, 1H), 4.05-3.89(m, 1H), 3.30-3.10(m, 4H), 1.91-1.77(m, 1H), 1.68-1.33(m, 16H), 1.32-1.19(m, 10H), 0.93-0.82(m, 3H).

### (step 2)

### Synthesis of benzyl N-[(5S)-5-amino-6-(octylamino)-6-oxohexyl]carbamate hydrochloride

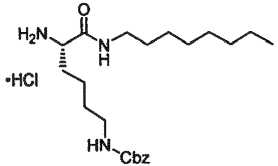

To a ethyl acetate solution (5 mL) of the compound (626 mg, 1.27 mmol) obtained in step 1 was added 4N hydrochloric acid/ethyl acetate (10 mL) and the mixture was stirred at room temperature for 3 hr. The solvent was evaporated under reduced pressure to give the title compound (513 mg, 1.20 mmol, yield 94%).

### (step 3)

### Synthesis of benzyl N-[(5S)-5-(hexadecylcarbamoylamino)-6-(octylamino)-6-oxo-hexyl]carbamate

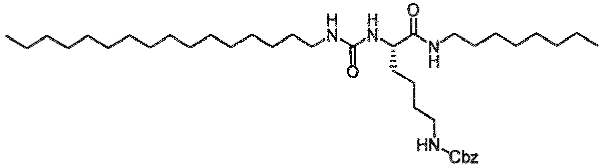

To the compound (253 mg, 0.591 mmol) obtained in step 2 were added tetrahydrofuran (6 mL), triethylamine (0.247 mL, 1.77 mmol), hexadecyl isocyanate (0.202 mL, 0.650 mmol) and tetrahydrofuran (14 mL), and the mixture was stirred at room temperature for 7 hr. To the reaction mixture was added water (40 mL), and the mixture was stirred at room temperature overnight. The solid was collected by filtration and washed with tetrahydrofuran/water (1/2) to give the title compound (406 mg, 0.616 mmol, yield quantitative).

### (step 4)

### Synthesis of (2S)-6-amino-2-(hexadecylcarbamoylamino)-N-octyl-hexanamide hydrochloride

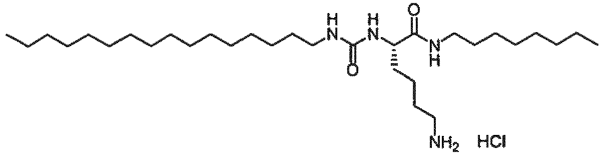

The compound (206 mg, 0.313 mmol) obtained in step 3 was dissolved in methanol (30 mL), a catalytic amount of 10% palladium/carbon was added and the mixture was stirred under a hydrogen atmosphere at 30°C overnight. To the reaction mixture was added a catalytic amount of concentrated hydrochloric acid, and the mixture was stirred under a hydrogen atmosphere at room temperature for 2 hr. Insoluble material was filtered off, and the solvent was evaporated under reduced pressure to give the title compound (148 mg, 0.264 mmol, yield 84%).
¹H-NMR (400 MHz, DMSO-d₆) δ7.88(t, J=5.5Hz, 1H), 7.79(s, 3H), 6.12-5.94(m, 2H), 4.05(d, J=6.6Hz, 1H), 3.10-2.64(m, 6H), 1.64-0.97(m, 46H), 0.92-0.78(m, 6H).

### Example 65

### (2S)-6-amino-N-dodecyl-2-(hexadecylcarbamoylamino)hexanamide hydrochloride (SZ214)

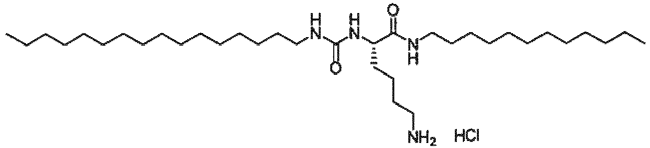

### (step 1)

### Synthesis of tert-butyl N-[(1S)-5-(benzyloxycarbonylamino)-1-(dodecylcarbamoyl)pentyl]carbamate

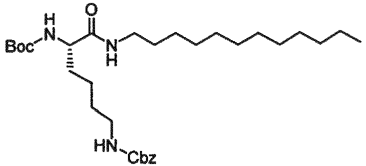

An operation similar to that in Example 64, step 1 was performed using dodecylamine (265 mg, 1.43 mmol) instead of octylamine to give the title compound (681 mg, 1.24 mmol, yield 96%).
¹H-NMR (400 MHz, CDCl₃) δ7.40-7.28(m, 5H), 6.15-5.97(m, 1H), 5.20-4.98(m, 3H), 4.90-4.74(m, 1H), 4.05-3.90(m, 1H), 3.29-3.10(m, 4H), 1.91-1.76(m, 1H), 1.68-1.34(m, 16H), 1.33-1.19(m, 18H), 0.93-0.83(m, 3H).

### (step 2)

### Synthesis of benzyl N-[(5S)-5-amino-6-(dodecylamino)-6-oxo-hexyl]carbamate hydrochloride

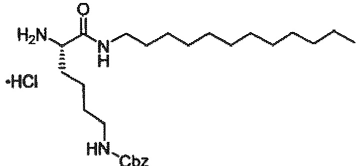

An operation similar to that in Example 64, step 2 was performed using the compound (681 mg, 1.24 mmol) obtained in step 1 to give the title compound (574 mg, 1.19 mmol, yield 96%).

### (step 3)

### Synthesis of benzyl N-[(5S)-6-(dodecylamino)-5-(hexadecylcarbamoylamino)-6-oxo-hexyl]carbamate

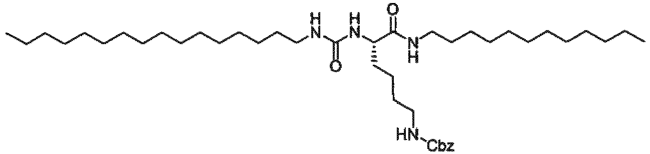

An operation similar to that in Example 64, step 3 was performed using the compound (296 mg, 0.611 mmol) obtained in step 2 to give the title compound (442 mg, 0.618 mmol, yield quantitative).

### (step 4)

### Synthesis of (2S)-6-amino-N-dodecyl-2-(hexadecylcarbamoylamino)hexanamide hydrochloride

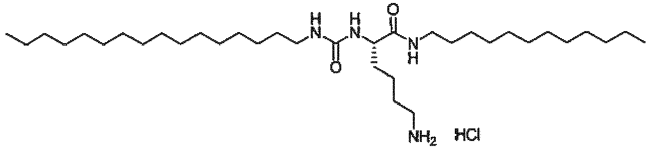

An operation similar to that in Example 64, step 4 was performed using the compound (225 mg, 0.315 mmol) obtained in step 3 to give the title compound (167 mg, 0.270 mmol, yield 86%).
¹H-NMR (400 MHz, DMSO-d₆) δ7.91-7.80(m, 1H), 7.69(s, 3H), 6.08-5.92(m, 2H), 4.11-3.99(m, 1H), 3.11-2.70(m, 6H), 1.61-0.93(m, 54H), 0.91-0.80(m, 6H).

### Example 66

### Hexadecyl N-[(1S)-4-guanidino-1-(octylcarbamoyl)butyl]carbamate hydrochloride (SZ215)

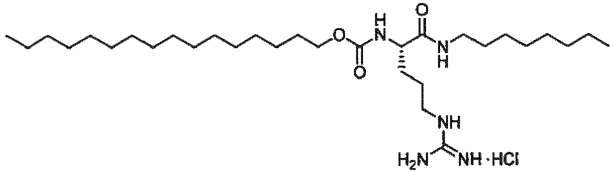

### (step 1)

### Synthesis of hexadecyl N-[(1S)-4-[[N,N'-bis(tert-butoxycarbonyl)carbamimidoyl]amino]-1-(octylcarbamoyl)butyl] carbamate

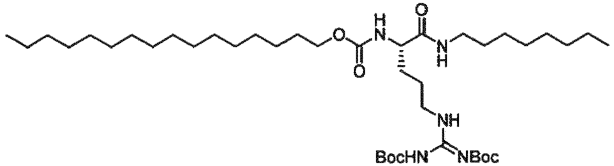

To a THF solution (2 mL) of the compound obtained in Example 61, step 2 were added triethylamine (60.4 mg, 0.597 mmol) and hexadecyl chloroformate (79.0 mg, 0.259 mmol) and the mixture was stirred at 30°C overnight. The reaction mixture was diluted with water, and extracted with dichloromethane.
The organic layer was washed with saturated brine, dried over anhydrous sodium sulfate, and concentrated. The obtained residue was purified by silica gel chromatography (ethyl acetate/hexane=1/4) to give the title compound (115 mg, yield 76%).
¹H-NMR (400 MHz, CDCl₃) δ11.45(s, 1H), 8.42(t, J=5.8Hz, 1H), 6.36(t, J=5.8Hz, 1H), 5.87(d, J=8.5Hz, 1H), 4.21(d, J=6.9Hz, 1H), 4.05(t, J=6.8Hz, 2H), 3.54-3.34(m, 2H), 3.24(ddt, J=31.6Hz, 13.4Hz, 6.2Hz, 2H), 1.88-1.79(m, 1H), 1.77-1.58(m, 5H), 1.53-1.43(m, 20H), 1.35-1.19(m, 36H), 0.91-0.83(m, 6H).

### (step 2)

### Synthesis of hexadecyl N-[(1S)-4-guanidino-1-(octylcarbamoyl)butyl]carbamate hydrochloride

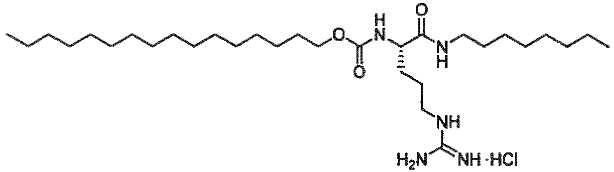

To the compound (115 mg, 0.152 mol) obtained in step 1 was added TFA/dichloromethane solution (v/v=5/1, 6 mL) and the mixture was stirred at room temperature for 1 hr. The reaction mixture was concentrated, water (2 ml) was added and the mixture was freeze-dried. To an ethyl acetate solution (2 mL) of the obtained residue was added 4N hydrochloric acid/ethyl acetate (0.19 mL) and the mixture was stirred for 30 min. The solid was collected by filtration to give the title compound (81.5 mg, yield 91%).
¹H-NMR (400 MHz, DMSO-d₆) δ7.80(t, J=5.7Hz, 1H), 7.43(t, J=5.8Hz, 1H), 7.30-6.69(m, 4H), 3.98-3.84(m, 3H), 3.10-2.98(m, 4H), 1.65-1.18(m, 44H), 0.89-0.82(m, 6H).

The correspondence between the respective compounds of Examples 1 - 32 and the formula (I) is shown in the following Table 1 and Table 2.

The correspondence between the respective compounds of Examples 1 - 13, 15 - 24 and 27 - 32 and the formula (II) (Y is a hydrogen atom and Z¹ is -CO-), and the formula (III) (X¹ is-NH- and Y¹ is a hydrogen atom) is shown in the following Table 3, Table 4 and Table 5. The structures of the respective compounds of Example 14, Example 25 and Example 26 are shown in Table 6.

The correspondence between the respective compounds of Examples 33 - 66 and the formula (I) is shown in the following Table 7 and Table 8.

**Table 1**

| | | | | | | | | | | | |
|---|---|---|---|---|---|---|---|---|---|---|---|
| 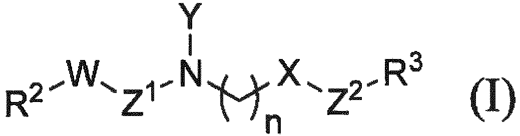 | | | | | | | | | | | |

| Ex. | SZ | R^{1a} | R^{1b} | R² | R³ | W | X | Y | Z¹ | Z² | n |
|---|---|---|---|---|---|---|---|---|---|---|---|
| 1 | 111 | 3-guanidylpropyl group | hydrogen atom | -C₁₅H₃₁ | -NH-C₁₂H₂₅ | bond | -C(R^{1a})(R^{1b})- | hydrogen atom | -CO- | -CO- | 0 |
| 2 | 137 | 3-guanidylpropyl group | hydrogen atom | -C₁₇H₃₅ | -NH-C₁₂H₂₅ | bond | -C(R^{1a})(R^{1b})- | hydrogen atom | -CO- | -CO- | 0 |
| 3 | 138 | 3-guanidylpropyl group | hydrogen atom | -C₂₁H₄₃ | -NH-C₁₂H₂₅ | bond | -C(R^{1a})(R^{1b})- | hydrogen atom | -CO- | -CO- | 0 |
| 4 | 142 | isobutyl group | hydrogen atom | -C₂₁H₄₃ | -NH-C₁₁H₂₃ | bond | -C(R^{1a})(R^{1b})- | hydrogen atom | -CO- | -CO- | 0 |
| 5 | 144 | isobutyl group | hydrogen atom | -C₁₅H₃₁ | -NH-C₁₂H₂₅ | bond | -C(R^{1a})(R^{1b})- | hydrogen atom | -CO- | -CO- | 0 |
| 6 | 145 | methyl group | hydrogen atom | -C₂₁H₄₃ | -NH-C₁₂H₂₅ | bond | -C(R^{1a})(R^{1b})- | hydrogen atom | -CO- | -CO- | 0 |
| 7 | 146 | methyl group | methyl group | -C₂₁H₄₃ | -NH-C₁₂H₂₅ | bond | -C(R^{1a})(R^{1b})- | hydrogen atom | -CO- | -CO- | 0 |
| 8 | 147 | hydrogen atom | hydrogen atom | -C₂₁H₄₃ | -NH-C₁₂H₂₅ | bond | -C(R^{1a})(R^{1b})- | hydrogen atom | -CO- | -CO- | 1 |
| 9 | 148 | methyl group | methyl group | -C₂₁H₄₃ | -NH-C₁₂H₂₅ | bond | -C(R^{1a})(R^{1b})- | hydrogen atom | -CO- | -CO- | 1 |
| 10 | 149 | 3-guanidylpropyl group | hydrogen atom | -C₁₆H₃₃ | -NH-C₁₂H₂₅ | -NH- | -C(R^{1a})(R^{1b})- | hydrogen atom | -CO- | -CO- | 0 |
| 11 | 150 | 3-guanidylpropyl group | hydrogen atom | -C₁₆H₃₃ | -NH-C₁₂H₂₅ | -O- | -C(R^{1a})(R^{1b})- | hydrogen atom | -CO- | -CO- | 0 |
| 12 | 153 | 4-aminobutyl group | hydrogen atom | -C₁₅H₃₁ | -NH-C₁₂H₂₅ | bond | -C(R^{1a})(R^{1b})- | hydrogen atom | -CO- | -CO- | 0 |
| 13 | 155 | n-butyl group | hydrogen atom | -C₂₁H₄₃ | -NH-C₁₂H₂₅ | bond | -C(R^{1a})(R^{1b})- | hydrogen atom | -CO- | -CO- | 0 |
| 14 | 156 | methyl group (ring formation) | hydrogen atom | -C₂₁H₄₃ | -NH-C₁₂H₂₅ | bond | -C(R^{1a}) (R^{1b})- | methyl group (ring formation) | -CO- | -CO- | 2 |
| 15 | 159 | 3-guanidylpropyl group | hydrogen atom | -C₂₁H₄₃ | -N(C₇H₁₅)(C₇H₁₅) | bond | -C(R^{1a})(R^{1b})- | hydrogen atom | -CO- | -CO- | 0 |
| 16 | 160 | 4-aminobutyl group | hydrogen atom | -C₂₁H₄₃ | -NH-C₁₂H₂₅ | bond | -C(R^{1a})(R^{1b})- | hydrogen atom | -CO- | -CO- | 0 |

**Table 2**

| | | | | | | | | | | | |
|---|---|---|---|---|---|---|---|---|---|---|---|
| 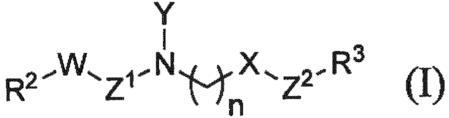 | | | | | | | | | | | |

| Ex. | SZ | R^{1a} | R^{1b} | R² | R³ | W | X | Y | Z¹ | Z² | n |
|---|---|---|---|---|---|---|---|---|---|---|---|
| 17 | 161 | 3-guanidylpropyl group | hydrogen atom | -C₁₇H₃₃ | -NH-C₁₂H₂₅ | bond | -C(R^{1a})(R^{1b})- | hydrogen atom | -CO- | -CO- | 0 |
| 18 | 163 | 3-guanidylpropyl group | hydrogen atom | -C₂₁H₄₃ | -NH-C₇H₁₅ | bond | -C(R^{1a})(R^{1b})- | hydrogen atom | -CO- | -CO- | 0 |
| 19 | 165 | 3-aminopropyl group | hydrogen atom | -C₂₁H₄₃ | -NH-C₁₂H₂₅ | bond | -C (R^{1a})(R^{1b})- | hydrogen atom | -CO- | -CO- | 0 |
| 20 | 166 | 4-guanidylbutyl group | hydrogen atom | -C₂₁H₄₃ | -NH-C₁₂H₂₅ | bond | -C(R^{1a})(R^{1b})- | hydrogen atom | -CO- | -CO- | 0 |
| 21 | 167 | 4-aminobutyl group substituted by acetyl group | hydrogen atom | -C₂₁H₄₃ | -NH-C₁₂H₂₅ | bond | -C(R^{1a})(R^{1b})- | hydrogen atom | -CO- | -CO- | 0 |
| 22 | 168 | 3-guanidylpropyl group | hydrogen atom | -C₁₆H₃₃ | -NH-C₆H₁₃ | -NH- | -C(R^{1a})(R^{1b})- | hydrogen atom | -CO- | -CO- | 0 |
| 23 | 169 | 3-guanidylpropyl group | hydrogen atom | -C₁₆H₃₃ | -NH-C₆H₁₃ | -O- | -C(R^{1a})- | hydrogen atom | -CO- | -CO- | 0 |
| 24 | 170 | 4-guanidylbutyl group | hydrogen atom | -C₂₁H₄₃ | -NH-C₆H₁₃ | bond | -C(R^{1a})(R^{1b})- | hydrogen atom | -CO- | -CO- | 0 |
| 25 | 173 | -CH₂-S-S-CH₂-(omitted) | hydrogen atom | -C₂₁H₄₃ | -NH-C₁₂H₂₅ | bond | -C(R^{1a})(R^{1b})- | hydrogen atom | -CO- | -CO- | 0 |
| 26 | 174 | 3-guanidylpropyl group | hydrogen atom | benzyl group | -NH-C₁₂H₂₅ | bond | -C(R^{1a})(R^{1b})- | benzyl group | bond | -CO- | 0 |
| 27 | 175 | | | -C₁₅H₃₁ | C (=NH) NH₂ | bond | -NH- | hydrogen atom | -CO- | bond | 5 |
| 28 | 176 | | | -C₂₁H₄₃ | -C(=NH)NH₂ | bond | -NH- | hydrogen atom | -CO- | bond | 5 |
| 29 | 177 | 3-guanidylpropyl group | hydrogen atom | -C₆H₁₃ | -NH-C₁₂H₂₅ | -CO- | -C (R^{1a})(R^{1b})- | hydrogen atom | -CO- | -CO- | 0 |
| 30 | 178 | | | -C₁₆H₃₃ | C(=NH) NH₂ | -O- | -NH- | hydrogen atom | -CO- | bond | 4 |
| 31 | 179 | | | -C₁₅H₃₁ | C(=NH)CH₃ | bond | -NH- | hydrogen atom | -CO- | bond | 4 |
| 32 | 164 | hydrogen atom | hydrogen atom | -C₂₁H₄₃ | -NH-C₁₂H₂₅ | bond | -C (R^{1a})(R^{1b})- | hydrogen atom | -CO- | -CO- | 3 |

**Table 3**

| | | | | | | | |
|---|---|---|---|---|---|---|---|
| 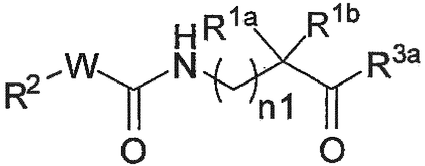 | | | | | | | |

| Ex. | SZ | W | R2 | R1a | R1b | R3a | n1 |
|---|---|---|---|---|---|---|---|
| 1 | 111 | bond | 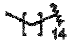 | 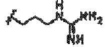 | H | 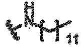 | 0 |
| 2 | 137 | bond | 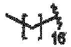 | 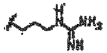 | H | 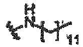 | 0 |
| 3 | 138 | bond | 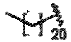 | 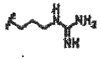 | H | 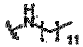 | 0 |
| 4 | 142 | bond | 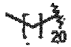 | 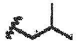 | H | 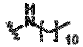 | 0 |
| 5 | 144 | bond | 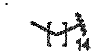 | 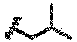 | H | 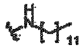 | 0 |
| 6 | 145 | bond | 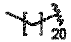 | Me | H | 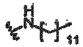 | 0 |
| 7 | 146 | bond | 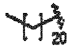 | Me | Me | 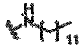 | 0 |
| 8 | 147 | bond | 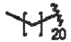 | H | H | 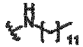 | 1 |
| 9 | 148 | bond | 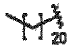 | Me | Me | 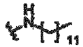 | 1 |
| 10 | 149 | -NH- | 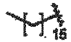 | 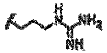 | H | 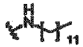 | 0 |
| 11 | 150 | -O- | 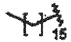 | 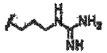 | H | 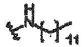 | 0 |
| 12 | 153 | bond | 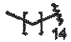 | 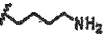 | H | 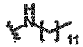 | 0 |
| 13 | 155 | bond | 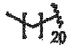 | 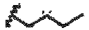 | H | 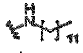 | 0 |
| 15 | 159 | bond | 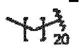 | 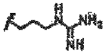 | H | 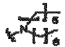 | 0 |

**[Table 4]**

| | | | | | | | |
|---|---|---|---|---|---|---|---|
| 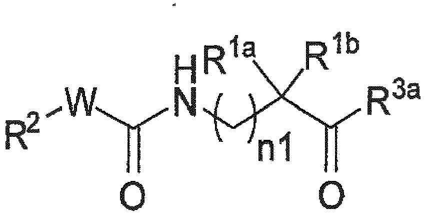 | | | | | | | |

| 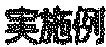 | SZ | W | R2 | R1a | R1b | R3a | n1 |
|---|---|---|---|---|---|---|---|
| 16 | 160 | bond | 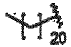 | 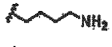 | H | 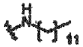 | 0 |
| 17 | 161 | bond | 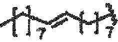 | 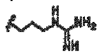 | H | 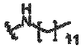 | 0 |
| 18 | 163 | bond | 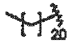 | 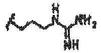 | H | 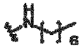 | 0 |
| 19 | 165 | bond | 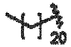 | 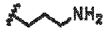 | H | 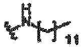 | 0 |
| 20 | 166 | bond | 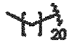 | 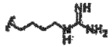 | H | 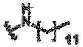 | 0 |
| 21 | 167 | bond | 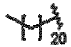 | 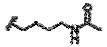 | H | 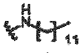 | 0 |
| 22 | 168 | bond | 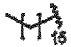 | 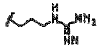 | H | 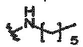 | 0 |
| 23 | 169 | bond | 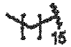 | 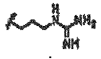 | H | 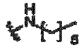 | 0 |
| 24 | 170 | bond | 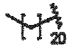 | 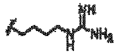 | H | 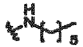 | 0 |
| 29 | 177 | -CO- | 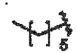 | 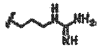 | H | 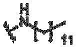 | 0 |
| 32 | 164 | bond | 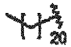 | H | H | 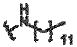 | 3 |

**[Table 5]**

| | | | | | |
|---|---|---|---|---|---|
| 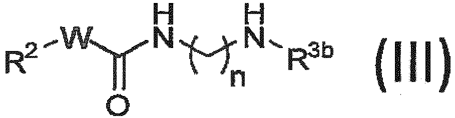 | | | | | |

| Ex. | SZ | W | R2 | R3b | n |
|---|---|---|---|---|---|
| 27 | 175 | bond | 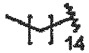 | 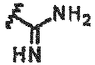 | 5 |
| 28 | 176 | bond | 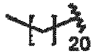 | 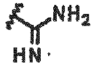 | 5 |
| 30 | 178 | -O- | 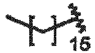 | 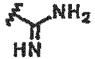 | 4 |
| 31 | 179 | bond | 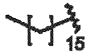 | 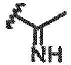 | 4 |

**[Table 6]**

| Ex. | SZ | structural formula. |
|---|---|---|
| 14 | 156 | 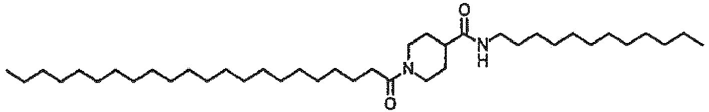 |
| 25 | 173 | 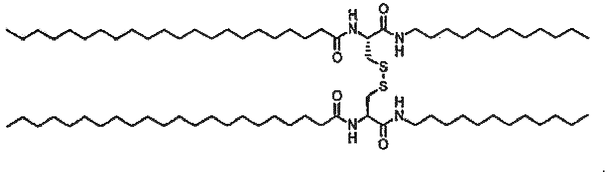 |
| 26 | 174 | 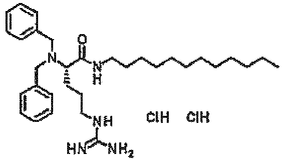 |

**[Table 7]**

| | | | | | | | | | | | |
|---|---|---|---|---|---|---|---|---|---|---|---|
| 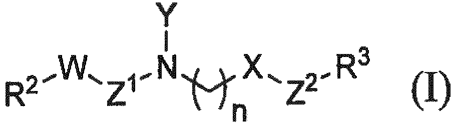 | | | | | | | | | | | |

| Ex. | SZ | R^{1a} | R^{1b} | R² | R³ | W | X | Y | Z¹ | Z² | n |
|---|---|---|---|---|---|---|---|---|---|---|---|
| 33 | 180 | 3-guanidylpropyl group | hydrogen atom | -C₁₉H₃₉ | -NH-C₁₂H₂₅ | bond | -C(R^{1a})(R^{1b})- | hydrogen atom | -CO- | -CO- | 0 |
| 34 | 181 | 3-guanidylpropyl group | hydrogen atom | -C₁₅H₃₁ | -NH-C₈H₁₇ | bond | -C(R^{1a}) (R^{1b})- | hydrogen atom | -CO- | -CO- | 0 |
| 35 | 182 | 3-guanidylpropyl group | hydrogen atom | -C₁₇H₃₅ | -NH-C₈H₁₇ | bond | -C(R^{1a}) (R^{1b})- | hydrogen atom | -CO- | -CO- | 0 |
| 36 | 183 | 3-guanidylpropyl group | hydrogen atom | -C₁₉H₃₉ | -NH-C₈H₁₇ | bond | -C (R^{1a})(R^{1b})- | hydrogen | -CO- | -CO- | 0 |
| 37 | 184 | 4-aminobutyl group | hydrogen atom | -C₁₇H₃₅ | -NH-C₈H₁₇ | bond | -C(R^{1a})(R^{1b})- | hydrogen atom | -CO- | -CO- | 0 |
| 38 | 185 | 4-aminobutyl group | hydrogen atom | -C₁₇H₃₅ | -NH-C₁₂H₂₅ | bond | -C(R^{1a}) (R^{1b})- | hydrogen atom | -CO- | -CO- | 0 |
| 39 | 186 | 3-guanidylpropyl group | hydrogen atom | -C₁₈H₃₇ | -NH-C₁₂H₂₅ | -O- | -C(R^{1a})(R^{1b})- | hydrogen atom | -CO- | -CO- | 0 |
| 40 | 187 | 3-guanidylpropyl group | hydrogen atom | -C₁₂H₂₅ | -NH-C₁₂H₂₅ | -O- | -C(R^{1a})(R^{1b})- | hydrogen atom | -CO- | -CO- | 0 |
| 41 | 188 | 4-aminobutyl group | hydrogen atom | -C₁₆H₃₃ | -NH-C₈H₁₇ | -O- | -C(R^{1a})(R^{1b})- | hydrogen atom | -CO- | -CO- | 0 |
| 42 | 189 | 4-aminobutyl group | hydrogen atom | -C₁₆H₃₃ | -NH-C₁₂H₂₅ | bond | -C(R^{1b})(R^{1b})- | hydrogen atom | -CO- | -CO- | 0 |
| 43 | 190 | | | -C₁₆H₃₃ | -C(=NH) NH₂ | -NH- | -NH- | hydrogen atom | -CO- | bond | 4 |
| 44 | 191 | 3-guanidylpropyl group | hydrogen atom | -C₁₇H₃₅ | -NH-C₁₂H₂₅ | bond | -C(R^{1a}) (R^{1b})- | hydrogen atom | -CO- | -CO- | 0 |
| 45 | 193 | 3-guanidylpropyl group | hydrogen atom | -C₁₆H₃₃ | -NH-C₁₂H₂₅ | -NH- | -C(R^{1a})(R^{1b})- | hydrogen atom | -CO- | -CO- | 0 |
| 46 | 194 | 4-aminobutyl group | hydrogen atom | -C₁₅H₃₁ | -NH-C₁₂H₂₅ | bond | -C(R^{1a})(R^{1b})- | hydrogen atom | -CO- | -CO- | 0 |
| 47 | 195 | isobutyl group | hydrogen atom | -C₁₇H₃₅ | -NH-C₁₂H₂₅ | bond | -C(R^{1a}) (R^{1b})- | hydrogen atom | -CO- | -CO- | 0 |
| 48 | 196 | isobutyl group | hydrogen atom | -C₁₇H₃₅ | -NH-C₈H₁₇ | bond | -C(R^{1a})(R^{1b})- | hydrogen atom | -CO- | -CO- | 0 |
| 49 | 197 | isobutyl group | hydrogen atom | -C₂₁H₄₃ | -NH-C₁₂H₂₅ | bond | -C(R^{1a})(R^{1b})- | hydrogen atom | -CO- | -CO- | 0 |

**[Table 8]**

| | | | | | | | | | | | |
|---|---|---|---|---|---|---|---|---|---|---|---|
| 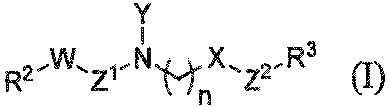 | | | | | | | | | | | |

| Ex. | SZ | R^{1a} | R^{1b} | R² | R³ | W | X | Y | Z¹ | Z² | n |
|---|---|---|---|---|---|---|---|---|---|---|---|
| 50 | 198 | isobutyl group | hydrogen atom | -C₂₁H₄₃ | -NH-C₈H₁₇ | bond | -C(R^{1a}) (R^{1b})- | hydrogen atom | -CO- | -CO- | 0 |
| 51 | 199 | isobutyl group | hydrogen atom | -C₁₅H₃₁ | -NH-C₈H₁₇ | bond | -C(R^{1a})(R^{1b})- | hydrogen atom | -CO- | -CO- | 0 |
| 52 | 200 | isobutyl group | hydrogen atom | -C₁₆H₃₃ | -NH-C₁₂H₂₅ | -NH- | -C(R^{1a})(R^{1b})- | hydrogen atom | -CO- | -CO- | 0 |
| 53 | 201 | isobutyl group | hydrogen atom | -C₁₆H₃₃ | -NH-C₈H₁₇ | -NH- | -C (R^{1a})(R^{1b})- | hydrogen atom | -CO- | -CO- | 0 |
| 54 | 202 | isobutyl group | hydrogen atom | -C₁₆H₃₃ | -NH-C₁₂H₂₅ | -O- | -C(R^{1a}) (R^{1b})- | hydrogen atom | -CO- | -CO- | 0 |
| 55 | 203 | isobutyl group | hydrogen atom | -C₁₆H₃₃ | -NH-C₈H₁₇ | -O- | -C(R^{1a})(R^{1b})- | hydrogen atom | -CO- | -CO- | 0 |
| 56 | 204 | methyl group | methyl group | -C₁₇H₃₅ | -NH-C₁₂H₂₅ | bond | -C(R^{1a})(R^{1b})- | hydrogen atom | -CO- | -CO- | 1 |
| 57 | 205 | 4-guanidylbutyl group | hydrogen atom | -C₁₇H₃₅ | -NH-C₁₂H₂₅ | bond | -C(R^{1a})(R^{1b})- | hydrogen atom | -CO- | -CO- | 0 |
| 58 | 206 | 4-guanidylbutyl group | hydrogen atom | -C₁₇H₃₅ | -NH-C6H13 | bond | -C(R^{1a})(R^{1b})- | hydrogen atom | -CO- | -CO- | 0 |
| 59 | 207 | 3-guanidylpropyl group | hydrogen atom | -C₇H₁₅ | -NH-C₁₂H₂₅ | bond | -C(R^{1a})(R^{1b})- | -C₇H₁₅ | bond | -CO- | 0 |
| 60 | 209 | | | -C₂₁H₄₃ | pyridyl group | bond | bond | hydrogen atom | -CO- | bond | 2 |
| 61 | 210 | 3-guanidylpropyl group | hydrogen atom | -C₁₆H₃₃ | -NH-C₈H₁₇ | -NH- | -C(R^{1a})(R^{1b})- | hydrogen atom | -CO- | -CO- | 0 |
| 62 | 211 | 3-guanidylpropyl group | hydrogen atom | -C₁₈H₃₇ | -NH-C₁₂H₂₅ | -NH- | -C(R^{1a})(R^{1b})- | hydrogen atom | -CO- | -CO- | 0 |
| 63 | 212 | 3-guanidylpropyl group | hydrogen atom | -C₁₂H₂₅ | -NH-C₁₂H₂₅ | -NH- | -C(R^{1a})(R^{1b})- | hydrogen atom | -CO- | -CO- | 0 |
| 64 | 213 | 4-aminobutyl group | hydrogen atom | -C₁₆H₃₃ | -NH-C₈H₁₇ | -NH- | -C (R^{1a})(R^{1b})- | hydrogen atom | -CO- | -CO- | 0 |
| 65 | 214 | 4-aminobutyl group | hydrogen atom | -C₁₆H₃₃ | -NH-C₁₂H₂₅ | -NH- | -C(R^{1a})(R^{1b})- | hydrogen atom | -CO- | -CO- | 0 |
| 66 | 215 | 3-guanidylpropyl group | hydrogen atom | -C₁₆H₃₃ | -NH-C₈H₁₇ | -O- | -C(R^{1a})(R^{1b})- | hydrogen atom | -CO- | -CO- | 0 |

### (2) Adjuvant activity test

### [Experimental Example 1]

### Evaluation test of adjuvant activity and allergy inducing activity after secondary immunization of N-[(1S)-1-(dodecylcarbamoyl)-4-guanidino-butyl]octadecanamide hydrochloride (SZ137), N-[(1S)-1-(dodecylcarbamoyl)-4-guanidino-butyl]docosanamide hydrochloride (SZ138), N-[(1S)-3-methyl-1-(undecylcarbamoyl)butyl]docosanamide (SZ142), N-[(1S)-1-(dodecylcarbamoyl)-3-methyl-butyl]hexadecanamide (SZ144), N-(1S)-2-(dodecylamino)-1-methyl-2-oxo-ethyl]docosanamide (SZ145), N-[2-(dodecylamino)-1,1-dimethyl-2-oxo-ethyl]docosanamide (SZ146), N-[3-(dodecylamino)-3-oxo-propyl]docosanamide (SZ147), N-[3-(dodecylamino)-2,2-dimethyl-3-oxo-propyl]docosanamide (SZ148)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 5 µg and Addavax™ (mixed with saline at 1:1) dissolved in saline (Addavax™ administration group) as positive control adjuvant, (3) OVA 5 µg and SZ137 0.256 µmol dissolved or dispersed in 5%α-cyclodextrin (hereinafter to be simply referred to as αCD)-added saline (SZ137 administration group), (4) OVA (5 µg) and SZ138 (0.256 µmol) dissolved or dispersed in 5% αCD-added saline (SZ138 administration group), (5) OVA (5 µg) and SZ142 (0.256 µmol) dissolved or dispersed in 5% αCD-added saline (SZ142 administration group), (6) OVA (5 µg) and SZ144 (0.256 µmol) dissolved or dispersed in 5% αCD-added saline (SZ144 administration group), (7) OVA (5 µg) and SZ145 (0.256 µmol) dissolved or dispersed in 5% αCD-added saline (SZ145 administration group), (8) OVA (5 µg) and SZ146 (0.256 µmol) dissolved or dispersed in 5% αCD-added saline (SZ146 administration group), (9) OVA (5 µg) and SZ147 (0.256 µmol) dissolved or dispersed in 5% αCD-added saline (SZ147 administration group), (10) OVA (5 µg) and SZ148 (0.256 µmol) dissolved or dispersed in 5% αCD-added saline (SZ148 administration group), each per mouse. Two week later, the secondary immunization was carried out by dorsal-subcutaneous administration of solutions or dispersions similar to those of the above-mentioned (1) - (10) again. Two weeks from the secondary immunization, the whole blood was extracted under anesthesia, and the obtained serum sample was measured for anti-OVA-specific IgG1 subclass antibody, anti-OVA-specific IgG2a subclass antibody, and anti-OVA-specific IgE antibody.

### [Significance of IgG1 subclass antibody, IgG2a subclass antibody measurements]

Helper T cell, which is one of the immunocompetent cells, includes several types and induces different immunofunction depending on the type thereof. In general, a cell called Th1 cell is considered to be involved in the induction of cellular immunity, finds, for example, virus infected cells and the like, and induces a function of killer T cells to directly attack them and the like. On the other hand, Th2 cell is considered to be involved in the induction of humoral immunity and induces, for example, IgE production from B cells. Th1 cell and Th2 cell are considered to be in a relationship where an increase in one of them suppresses the other. For example, it is considered that the onset of allergy can be suppressed by setting the Th1/Th2 balance to be Th1 dominant. IgG antibody includes some subclasses and, in the case of mouse, IgG1 subclass antibody is considered to be involved in the immunofunction of Th2 type, and IgG2a subclass antibody is considered to be involved in the immunofunction of Th1 type. It is said that whether the induced immunity is Th1 type or Th2 type can be known to a certain extent by measuring production of these subclass antibodies.

The measurement method of the anti-OVA-specific IgG1 subclass antibody and anti-OVA-specific IgG2a subclass antibody in Experimental Example 1 and the following Experimental Examples 2 - 10 is as follows.

### [Measurement method of anti-OVA-specific IgG1 subclass antibody and anti-OVA-specific IgG2a subclass antibody]

To a 96 well plate was added 5 µg/ml OVA at 100 µl/well, and the mixture was incubated at 4°C overnight. After incubation, the mixture was washed three times with 200 µl of PBS-T (PBS (phosphate buffered saline)+0.05% (v/v) Tween 20) per well, and blocked with 100 µl of 5% FCS (fetal calf serum)/PBS solution per well at room temperature for 1 - 2 hr. Thereafter, the mixture was washed three times with PBS-T (200 µl/well), a diluted serum sample (100 µl/well) or the same amount of 5% FCS/PBS solution as a control was added, and the mixture was incubated at 37°C for 1 hr. To calculate concentration of the antibody, the standard of an anti-OVA-specific IgG1 subclass antibody diluted with 2% FCS/PBS solution was added at 100 ng/ml, 50 ng/ml, 25 ng/ml, 12.5 ng/ml, 6.25 ng/ml, 3.125 ng/ml, 1.56 ng/ml, 0.781 ng/ml, 0.391 ng/ml, 0.195 ng/ml, 0.098 ng/ml, 0 ng/ml, per 1 well. When an anti-OVA-specific IgG2a subclass antibody was measured, the standard of an anti-OVA-specific IgG2a subclass antibody was added at 50 ng/ml, 25 ng/ml, 12.5 ng/ml, 6.25 ng/ml, 3.125 ng/ml, 1.56 ng/ml, 0.781 ng/ml, 0.391 ng/ml, 0.195 ng/ml, 0.098 ng/ml, 0.049 ng/ml, 0 ng/ml, per 1 well. Thereafter, similar to the samples, the mixture was washed five times with PBS-T (200 µl/well), a diluted biotinylated anti-mouse IgG1 antibody and IgG2a antibody were added (100 µl/well), and the mixture was incubated at 37°C for 45 min. Thereafter, the mixture was washed five times with PBS-T (200 µl/well), a diluted anti-biotin-HRP antibody was added (100 µl/well), and the mixture was incubated at 37°C for another 30 min. Thereafter, the mixture was washed five times with PBS-T (200 µl/well), TMB substrate solution was added (100 µl/well), and the mixture was incubated at room temperature for 10 - 15 min. Thereafter, a reaction quenching liquid (2N sulfuric acid solution) was added (50 µl/well) to discontinue a color developing reaction, and the absorbance (OD Value=450 nm) was measured by a microplate reader. A standard curve was drawn from the measured OD values, and the concentration was calculated.

The results are shown in Fig. 1.

As is clear from the results shown in Fig. 1, the anti-OVA-specific IgG1 and IgG2a subclass antibody production of Addavax™ administration group (Addavax in Figure), SZ137 administration group (SZ137 in Figure) significantly increased as compared to the OVA single administration group (saline in Figure). In addition, the anti-OVA-specific IgG1 and IgG2a subclass antibody production of SZ138 administration group (SZ138 in Figure), SZ142 administration group (SZ142 in Figure), SZ144 administration group (SZ144 in Figure), SZ145 administration group (SZ145 in Figure), SZ146 administration group (SZ146 in Figure), SZ147 administration group (SZ147 in Figure), SZ148 administration group (SZ148 in Figure) showed a tendency to increase. From these results, SZ138, SZ142, SZ144, SZ145, SZ146, SZ147 and SZ148 were confirmed to have an adjuvant activity.

The serum sample obtained in Experimental Example 1 was measured for an anti-OVA-specific IgE antibody.

The measurement method of the anti-OVA-specific IgE antibody in Experimental Example 1 and the following Experimental Examples 2 - 5, 8 - 10 is as follows.

### [Measurement method of anti-OVA-specific IgE antibody]

DS mouse IgE ELISA (OVA) kit manufactured by DS Pharma Biomedical Co., Ltd. was used. The protocol is briefly shown below.

The serum sample and a standard reagent for drawing an analytical curve are diluted with a buffer and, after stirring, left standing at room temperature for 10 min. The sample and the standard solution are added to a plate bound with an IgE capture antibody in advance and, after stirring, the mixture is left standing at room temperature for 60 min. The mixture is washed three times with wash, HRP-labeled OVA is added, and the mixture is left standing at room temperature for 30 min. The mixture is washed three times with wash, a substrate solution is added, and the mixture is left standing in dark at room temperature for 30 min. A reaction quenching liquid is added, the mixture is stirred, and the absorbance (OD Value=450 nm) is immediately measured.

The standard used is one attached to the kit, the standard curve is drawn from the measured OD Value, and the concentration is calculated.

The results are shown in Fig. 2.

As is clear from the results shown in Fig. 2, the anti-OVA-specific IgE antibody production of the Addavax™ administration group (Addavax in Figure) significantly increased as compared to the OVA single administration group (saline in Figure). In contrast, an increase in the anti-OVA-specific IgE antibody production was not observed in SZ137 administration group (SZ137 in Figure), SZ138 administration group (SZ138 in Figure), SZ142 administration group (SZ142 in Figure), SZ144 administration group (SZ144 in Figure), SZ148 administration group (SZ148 in Figure). From these results, it was clarified that SZ137, SZ138, SZ142, SZ144 and SZ148 may have a lower allergy inducing activity as compared to Addavax™ that may induce allergy by inoculation.

### [Evaluation of adjuvant enhancing antibody production and not inducing allergy]

The serum samples obtained in Experimental Example 1 were evaluated for utilizability as an adjuvant to cause enhancement of antibody production by each compound and not induce allergy based on the following index (Effective Index). Whether each compound can be used as an adjuvant to cause enhancement of antibody production and not induce allergy was also evaluated in the same manner in the following Experimental Examples 2 - 10.

### [Definition of index for evaluation of adjuvant not inducing allergy]

It is a problem that Addavax™ potentiates antigen-specific antibody production but induces allergy. Therefore, the index for adjuvant not inducing allergy (Effective Index) was defined as follows, and an index of each administration group in Experimental Examples 1 - 10 was calculated. That is, a value obtained by dividing mean of the concentrations of anti-OVA-specific IgG1 subclass antibody and anti-OVA-specific IgG2a subclass antibody of each group by mean of anti-OVA-specific IgE antibody concentration of each group was relatively compared with a value obtained by dividing mean of the concentrations of anti-OVA-specific IgG1 subclass antibody and anti-OVA-specific IgG2a subclass antibody of Addavax™ administration group by mean of anti-OVA-specific IgE antibody concentration of Addavax™ administration group as 1.0.

The results of Effective Index in Experimental Example 1 are shown in Fig. 3.

As is clear from the results shown in Fig. 3, it was clarified that the Effective Index of SZ137 administration group (SZ137 in Figure), SZ138 administration group (SZ138 in Figure), SZ142 administration group (SZ142 in Figure), SZ144 administration group (SZ144 in Figure) and SZ148 administration group (SZ148 in Figure) is higher than that of Addavax™ administration group (1.0). These results suggest that SZ137, SZ138, SZ142, SZ144 and SZ148 are utilizable as a particularly superior adjuvant that enhances antibody production and does not induce allergy as compared to Addavax™.

### [Experimental Example 2]

### Evaluation test of adjuvant activity and allergy inducing activity after secondary immunization of (2S)-N-dodecyl-5-guanidino-2-(hexadecylcarbamoylamino)pentanamide hydrochloride (SZ149), hexadecyl N-[(1S)-1-(dodecylcarbamoyl)-4-guanidino-butyl]carbamate hydrochloride (SZ150), N-[(1S)-5-amino-1-(dodecylcarbamoyl)pentyl]hexadecanamide hydrochloride (SZ153), N-[(1S)-1-(dodecylcarbamoyl)pentyl]docosanamide (SZ155), 1-docosanoyl-N-dodecyl-piperidine-4-carboxamide (SZ156), N-[(1S)-1-(diheptylcarbamoyl)-4-guanidino-butyl]docosanamide hydrochloride (SZ159), N-[(1S)-5-amino-1-(dodecylcarbamoyl)pentyl]docosanamide hydrochloride (SZ160)

8-Week-old female BALB/c mice were divided into groups (N=6), and each group was primarily immunized by dorsal-subcutaneous administration of (1) ovalbumin (OVA) (10 µg) dissolved in saline as an antigen (OVA single administration group), (2) OVA 5 µg and Addavax™ (mixed with saline at 1:1) dissolved in saline (Addavax™ administration group), (3) OVA (5 µg) and SZ149 (0.256 µmol) dissolved or dispersed in 5% αCD-added saline (SZ149 administration group), (4) OVA (5 µg) and SZ150 (0.256 µmol) dissolved or dispersed in 5% αCD-added saline (SZ150 administration group), (5) OVA (5 µg) and SZ153 (0.256 µmol) dissolved or dispersed in 5% αCD-added saline (SZ153 administration group), (6) OVA (5 µg) and SZ155 (0.256 µmol) dissolved or dispersed in 5% αCD-added saline (SZ155 administration group), (7) OVA (5 µg) and SZ156 (0.256 µmol) dissolved or dispersed in 5% αCD-added saline (SZ156 administration group), (8) OVA (5 µg) and SZ159 (0.256 µmol) dissolved or dispersed in 5% αCD-added saline (SZ159 administration group), (9) OVA (5 µg) and SZ160 (0.256 µmol) dissolved or dispersed in 5% aCD-added saline (SZ160 administration group), each per mouse. Two weeks later, the secondary immunization was carried out by dorsal-subcutaneous administration of solutions or dispersions similar to those of the above-mentioned (1) - (9) again. Two weeks from the secondary immunization, the blood was extracted under anesthesia, and the obtained serum samples were measured for anti-OVA-specific IgG1 subclass antibody, anti-OVA-specific IgG2a subclass antibody, and anti-OVA-specific IgE antibody. The results are shown in Fig. 4 and Fig. 5.

As is clear from the results shown in Fig. 4, the anti-OVA-specific IgG1 and IgG2a subclass antibody production of the Addavax™ administration group (Addavax in Figure), SZ149 administration group (SZ149 in Figure), SZ150 administration group (SZ150 in Figure) and SZ153 administration group (SZ153 in Figure) each significantly increased as compared to the OVA single administration group (saline in Figure). While a significant difference was not observed, it was found that the anti-OVA-specific IgG1 and IgG2a subclass antibody production in SZ155 administration group (SZ155 in Figure), SZ156 administration group (SZ156 in Figure), SZ159 administration group (SZ159 in Figure), SZ160 administration group (SZ160 in Figure) each showed a tendency to increase. These results suggest that SZ149, SZ150, SZ153, SZ155, SZ156, SZ159 and SZ160 have an adjuvant activity.

The allergy inducing activity test was performed in the same manner as in Experimental Example 1 except that N-[(1S)-5-amino-1-(dodecylcarbamoyl)pentyl]hexadecanamide hydrochloride (SZ153) was used. The results are shown in Fig. 5.

As is clear from the results shown in Fig. 5, the anti-OVA-specific IgE antibody production in the Addavax™ administration group (Addavax in Figure) increased as compared to the OVA single administration group (saline in Figure). In contrast, an increase in the anti-OVA-specific IgE antibody production was not observed in SZ153 administration group (SZ153 in Figure). From these results, it was clarified that SZ153 may have a lower allergy inducing activity as compared to Addavax™ that may induce allergy by inoculation.

An adjuvant that enhances antibody production and does not induce allergy was evaluated in the same manner as in Experimental Example 1 except that N-[(1S)-5-amino-1-(dodecylcarbamoyl)pentyl]hexadecanamide hydrochloride (SZ153) was used. The results are shown in Fig. 6.

As is clear from the results shown in Fig. 6, it was clarified that the Effective Index of SZ153 administration group (SZ153 in Figure) is larger than that of Addavax™ administration group (1.0). These results suggest that SZ153 is utilizable as a particularly superior adjuvant that enhances antibody production and does not induce allergy as compared to Addavax™.

### [Experimental Example 3]

### Evaluation test of adjuvant activity and allergy inducing activity after secondary immunization of (E)-N-[(1S)-1-(dodecylcarbamoyl)-4-guanidino-butyl]octadeca-9-enamide hydrochloride (SZ161), N-[(1S)-4-guanidino-1-(heptylcarbamoyl)butyl]docosanamide hydrochloride (SZ163), N-[3-(dodecylamino)-3-oxo-pentyl]docosanamide (SZ164), N-[(1S)-4-amino-1-(dodecylcarbamoyl)butyl]docosanamide hydrochloride (SZ165), N-[(1S)-1-(dodecylcarbamoyl)-5-guanidino-pentyl]docosanamide hydrochloride (SZ166), N-[(1S)-5-acetamide-1-(dodecylcarbamoyl)pentyl]docosanamide (SZ167), (2S)-5-guanidino-2-(hexadecylcarbamoylamino)-N-hexyl-pentanamide hydrochloride (SZ168), hexadecyl N-[(1S)-4-guanidino-1-(hexylcarbamoyl)butyl]carbamate hydrochloride (SZ169), N-[(1S)-5-guanidino-1-(hexylcarbamoyl)pentyl]docosanamide hydrochloride (SZ170)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 5 µg and Addavax™ (mixed with saline at 1:1) dissolved in saline (Addavax™ administration group), (3) OVA (5 µg) and SZ161 (0.256 µmol) dissolved or dispersed in 5% αCD-added saline (SZ161 administration group), (4) OVA (5 µg) and SZ163 (0.256 µmol) dissolved or dispersed in 5% aCD-added saline (SZ163 administration group), (5) OVA (5 µg) and SZ164 (0.256 µmol) dissolved or dispersed in 5% αCD-added saline (SZ164 administration group), (6) OVA (5 µg) and SZ165 (0.256 µmol) dissolved or dispersed in 5% aCD-added saline (SZ165 administration group), (7) OVA (5 µg) and SZ166 (0.256 µmol) dissolved or dispersed in 5% αCD-added saline (SZ166 administration group), (8) OVA (5 µg) and SZ167 (0.256 µmol) dissolved or dispersed in 5% aCD-added saline (SZ167 administration group), (9) OVA (5 µg) and SZ168 (0.256 µmol) dissolved or dispersed in 5% αCD-added saline (SZ168 administration group), (10) OVA (5 µg) and SZ169 (0.256 µmol) dissolved or dispersed in 5% aCD-added saline (SZ169 administration group), (11) OVA (5 µg) and SZ170 (0.256 µmol) dissolved or dispersed in 5% αCD-added saline (SZ170 administration group), each per mouse. Two weeks later, the secondary immunization was carried out by dorsal-subcutaneous administration of solutions or dispersions similar to those of the above-mentioned (1) - (11) again. Two weeks from the secondary immunization, the blood was extracted under anesthesia, and the obtained serum samples were measured for anti-OVA-specific IgG1 subclass antibody, anti-OVA-specific IgG2a subclass antibody, and anti-OVA-specific IgE antibody. The results thereof are shown in Fig. 7 and Fig. 8.

As is clear from the results shown in Fig. 7, the anti-OVA-specific IgG1 and IgG2a subclass antibody production of Addavax™ administration group (Addavax in Figure), SZ161 administration group (SZ161 in Figure) significantly increased as compared to OVA single administration group (saline in Figure). While a significant difference was not observed, it was found that the anti-OVA-specific IgG1 and IgG2a subclass antibody production also tended to increase in SZ163 administration group (SZ163 in Figure), SZ164 administration group (SZ164 in Figure), SZ165 administration group (SZ165 in Figure), SZ166 administration group (SZ166 in Figure), SZ167 administration group (SZ167 in Figure), SZ168 administration group (SZ168 in Figure), SZ169 administration group (SZ169 in Figure), SZ170 administration group (SZ170 in Figure). These results suggest that SZ161, SZ163, SZ164, SZ165, SZ166, SZ167, SZ168, SZ169 and SZ170 have an adjuvant activity.

The allergy inducing activity test was performed in the same manner as in Experimental Example 1 except that (E)-N-[(1S)-1-(dodecylcarbamoyl)-4-guanidino-butyl]octadeca-9-enamide hydrochloride (SZ161), N-[(1S)-4-guanidino-1-(heptylcarbamoyl)butyl]docosanamide hydrochloride (SZ163), N-[(1S)-4-amino-1-(dodecylcarbamoyl)butyl]docosanamide hydrochloride (SZ165), N-[(1S)-1-(dodecylcarbamoyl)-5-guanidino-pentyl]docosanamide hydrochloride (SZ166), N-[(1S)-5-acetamide-1-(dodecylcarbamoyl)pentyl]docosanamide (SZ167), (2S)-5-guanidino-2-(hexadecylcarbamoylamino)-N-hexyl-pentanamide hydrochloride (SZ168) were used. The results are shown in Fig. 8.

As is clear from the results shown in Fig. 8, anti-OVA-specific IgE antibody production increased in Addavax™ administration group (Addavax in Figure), as compared to OVA single administration group (saline in Figure). In addition, anti-OVA-specific IgE antibody production was lower in SZ161 administration group (SZ161 in Figure), SZ163 administration group (SZ163 in Figure), SZ165 administration group (SZ165 in Figure), SZ166 administration group (SZ166 in Figure), SZ167 administration group (SZ167 in Figure), SZ168 administration group (SZ168 in Figure) than Addavax™ administration group (Addavax in Figure). From these results, it was clarified that SZ161, SZ163, SZ165, SZ166, SZ167 and SZ168 may have an allergy inducing activity equivalent to or not more than that of Addavax™ known to induce allergy by inoculation.

An adjuvant that enhances antibody production and does not induce allergy was evaluated in the same manner as in Experimental Example 1 except that (E)-N-[(1S)-1-(dodecylcarbamoyl)-4-guanidino-butyl]octadeca-9-enamide hydrochloride (SZ161), (2S)-5-guanidino-2-(hexadecylcarbamoylamino)-N-hexyl-pentanamide hydrochloride (SZ168) were used. The results are shown in Fig. 9.

As is clear from the results shown in Fig. 9, it was clarified that the Effective Index of SZ161 administration group (SZ161 in Figure), SZ168 administration group (SZ168 in Figure) is larger than that of Addavax™ administration group (1.0). These results suggest that SZ161 and SZ168 are utilizable as a particularly superior adjuvant that enhances antibody production and does not induce allergy as compared to Addavax™.

### [Experimental Example 4]

### Adjuvant activity evaluation test of N-[(1S)-1-(dodecylcarbamoyl)-4-guanidino-butyl]hexadecanamide hydrochloride (SZ111) dissolved in saline after secondary immunization

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 5 µg and Addavax™ (mixed with saline at 1:1) dissolved in saline (Addavax™ administration group), (3) OVA (5 µg) and SZ111 (0.256 µmol) dissolved in saline (SZ111 (saline) administration group), each per mouse. Two weeks later, the secondary immunization was carried out by dorsal-subcutaneous administration of solutions similar to those of the above-mentioned (1) - (3) again. Two weeks from the secondary immunization, the blood was extracted under anesthesia, the blood was extracted under anesthesia, and the obtained serum samples were measured for anti-OVA-specific IgG1 subclass antibody, anti-OVA-specific IgG2a subclass antibody, and anti-OVA-specific IgE antibody. The results thereof are shown in Fig. 10 and Fig. 11.

As is clear from the results shown in Fig. 10, anti-OVA-specific IgG1 and IgG2a subclass antibody production significantly increased in Addavax™ administration group (Addavax in Figure), SZ111 administration group (SZ111(saline) in Figure) as compared to OVA single administration group (Saline in Figure). These results suggest that SZ111 dissolved in saline has an adjuvant activity.

An allergy inducing activity test was performed in the same manner as in Experimental Example 1 except that N-[(1S)-1-(dodecylcarbamoyl)-4-guanidino-butyl]hexadecanamide hydrochloride (SZ111) was used. The results are shown in Fig. 11.

As is clear from the results shown in Fig. 11, anti-OVA-specific IgE antibody production significantly increased in Addavax™ administration group (Addavax in Figure), SZ111 administration group (SZ111 (saline) in Figure) as compared to OVA single administration group (Saline in Figure). However, anti-OVA-specific IgE antibody production was lower in SZ111 administration group (SZ111 (Saline) in Figure) than Addavax™ administration group (Addavax in Figure). From these results, it was clarified that SZ111 dissolved in saline may have a lower allergy inducing activity as compared to Addavax™ known to induce allergy by inoculation.

An adjuvant that enhances antibody production and does not induce allergy was evaluated in the same manner as in Experimental Example 1 except that N-[(1S)-1-(dodecylcarbamoyl)-4-guanidino-butyl]hexadecanamide hydrochloride (SZ111) dissolved in saline was used. The results are shown in Fig. 12.

As is clear from the results shown in Fig. 12, it was clarified that the Effective Index of SZ111 administration group (SZ111 (saline) in Figure) is larger than that of Addavax™ administration group (1.0). These results suggest that SZ111 dissolved in saline is utilizable as a particularly superior adjuvant that enhances antibody production and does not induce allergy as compared to Addavax™.

### [Experimental Example 5]

### Adjuvant activity evaluation test of N-[(1S)-1-(dodecylcarbamoyl)-4-guanidino-butyl]hexadecanamide hydrochloride (SZ111) dissolved or dispersed in 5% αCD-added saline after secondary immunization

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 5 µg and Addavax™ (mixed with saline at 1:1) dissolved in saline (Addavax™ administration group), (3) OVA (5 µg) and SZ111 (0.256 µmol) dissolved or dispersed in 5% αCD-added saline (SZ111 (αCD) administration group), each per mouse. Two weeks later, the secondary immunization was carried out by dorsal-subcutaneous administration of solutions or dispersions similar to those of the above-mentioned (1) - (3) again. Two weeks from the secondary immunization, the blood was extracted under anesthesia, and the obtained serum samples were measured for anti-OVA-specific IgG1 subclass antibody, anti-OVA-specific IgG2a subclass antibody, and anti-OVA-specific IgE antibody. The results thereof are shown in Fig. 13 and Fig. 14.

As is clear from the results shown in Fig. 13, anti-OVA-specific IgG1 and IgG2a subclass antibody production significantly increased in Addavax™ administration group (Addavax in Figure), SZ111 administration group (SZ111 (αCD) in Figure) as compared to OVA single administration group (Saline in Figure). These results suggest that SZ111 dissolved or dispersed in 5% αCD-added saline has an adjuvant activity.

An allergy inducing activity test was performed in the same manner as in Experimental Example 1 except that N-[(1S)-1-(dodecylcarbamoyl)-4-guanidino-butyl]hexadecanamide hydrochloride (SZ111) was used. The results are shown in Fig. 14.

As is clear from the results shown in Fig. 14, anti-OVA-specific IgE antibody production significantly increased in Addavax™ administration group (Addavax in Figure) as compared to OVA single administration group (Saline in Figure). However, anti-OVA-specific IgE antibody production did not increase significantly in SZ111 (αCD) administration group (SZ111 (αCD) in Figure) as compared to OVA single administration group (saline in Figure) and was lower than in Addavax™ administration group (Addavax in Figure). From these results, it was clarified that SZ111 may have a lower allergy inducing activity as compared to Addavax™ known to induce allergy by inoculation.

An adjuvant that enhances antibody production and does not induce allergy was evaluated in the same manner as in Experimental Example 1 except that N-[(1S)-1-(dodecylcarbamoyl)-4-guanidino-butyl]hexadecanamide hydrochloride (SZ111) was used. The results are shown in Fig. 15.

As is clear from the results shown in Fig. 15, it was clarified that the Effective Index of SZ111 (αCD) administration group (SZ111 (αCD) in Figure) is larger than that of Addavax™ administration group (1.0). These results suggest that SZ111 dissolved or dispersed in 5% αCD-added saline is utilizable as a particularly superior adjuvant that enhances antibody production and does not induce allergy as compared to Addavax™.

### [Experimental Example 6]

### Adjuvant activity evaluation test of N-[(1R)-1-[[[(2R)-2-(docosanoylamino)-3-(dodecylamino)-3-oxopropyl]disulfanyl]methyl]-2-(dodecylamino)-2-oxoethyl]docosanamide (SZ173) after primary immunization

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 5 µg and Addavax™ (mixed with saline at 1:1) dissolved in saline (Addavax™ administration group), (3) OVA (5 µg) and SZ173 (0.256 µmol) dissolved or dispersed in 5% αCD-added saline (SZ173 administration group), each per mouse. Two weeks from the primary immunization, the blood was extracted under anesthesia, and the obtained serum samples were measured for anti-OVA-specific IgG1 subclass antibody and anti-OVA-specific IgG2a subclass antibody. The results thereof are Fig. 16.

As is clear from the results shown in Fig. 16, anti-OVA-specific IgG1 and IgG2a subclass antibody production was significantly enhanced in Addavax™ administration group (Addavax in Figure). The anti-OVA-specific IgG1 subclass antibody production in SZ173 administration group (SZ173 in Figure) showed a tendency to increase as compared to OVA single administration group (Saline in Figure), and the anti-OVA-specific IgG2a subclass antibody production in SZ173 administration group (SZ173 in Figure) significantly increased as compared to OVA single administration group (Saline in Figure). These results suggest that SZ173 has an adjuvant activity.

### [Experimental Example 7]

### Adjuvant activity evaluation test of (2S)-2-(dibenzylamino)-N-dodecyl-5-guanidino-pentanamide (SZ174), N-(5-guanidinopentyl)hexadecanamide (SZ175), N-(5-guanidinopentyl)docosanamide (SZ176), N-[(1S)-1-(dodecylcarbamoyl)-4-guanidino-butyl]-2-oxo-octanamide (SZ177) after primary immunization

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 5 µg and Addavax™ (mixed with saline at 1:1) dissolved in saline (Addavax™ administration group), (3) OVA (5 µg) and SZ174 (0.256 µmol) dissolved or dispersed in 5% αCD-added saline (SZ174 administration group), (4) OVA (5 µg) and SZ175 (0.256 µmol) dissolved or dispersed in 5% αCD-added saline (SZ175 administration group), (5) OVA (5 µg) and SZ176 (0.256 µmol) dissolved or dispersed in 5% αCD-added saline (SZ176 administration group), (6) OVA (5 µg) and SZ177 (0.256 µmol) dissolved or dispersed in 5% aCD-added saline (SZ177 administration group), each per mouse. Two weeks from the primary immunization, the blood was extracted under anesthesia, and the obtained serum samples were measured for anti-OVA-specific IgG1 subclass antibody and anti-OVA-specific IgG2a subclass antibody. The results thereof are Fig. 17.

As is clear from the results shown in Fig. 17, significant enhancement of the anti-OVA-specific IgG1 and IgG2a subclass antibody production was found in Addavax™ administration group (Addavax in Figure). The anti-OVA-specific IgG1 subclass antibody production in SZ174 administration group (SZ174 in Figure), SZ175 administration group (SZ175 in Figure), SZ176 administration group (SZ176 in Figure), SZ177 administration group (SZ177 in Figure) tended to increase as compared to OVA single administration group (saline in Figure). On the other hand, anti-OVA-specific IgG2a subclass antibody production in SZ174 administration group (SZ174 in Figure), SZ175 administration group (SZ175 in Figure), SZ176 administration group (SZ176 in Figure), SZ177 administration group (SZ177 in Figure) significantly increased as compared to OVA single administration group (Saline in Figure). These results suggest that SZ174, SZ175, SZ176 and SZ177 have an adjuvant activity.

### [Experimental Example 8]

### Adjuvant activity and allergy inducing activity evaluation test of N-[(1S)-1-(dodecylcarbamoyl)-4-guanidino-butyl]icosanamide hydrochloride (SZ180), N-[(1S)-4-guanidino-1-(octylcarbamoyl)butyl]hexadecanamide hydrochloride (SZ181), N-[(1S)-4-guanidino-1-(octylcarbamoyl)butyl]octadecanamide hydrochloride (SZ182), N-[(1S)-4-guanidino-1-(octylcarbamoyl)butyl]icosanamide hydrochloride (SZ183), N-[(1S)-5-amino-1-(octylcarbamoyl)pentyl]octadecanamide hydrochloride (SZ184), N-[(1S)-5-amino-1-(dodecylcarbamoyl)pentyl]octadecanamide hydrochloride (SZ185), octadecyl N-[(1S)-1-(dodecylcarbamoyl)-4-guanidino-butyl]carbamate hydrochloride (SZ186), dodecyl N-[(1S)-1-(dodecylcarbamoyl)-4-guanidino-butyl]carbamate hydrochloride (SZ187), hexadecyl N-[(1S)-5-amino-1-(octylcarbamoyl)pentyl]carbamate hydrochloride (SZ188), hexadecyl N-[(1S)-5-amino-1-(dodecylcarbamoyl)pentyl]carbamate hydrochloride (SZ189), 1-(4-guanidinobutyl)-3-hexadecyl-urea hydrochloride (SZ190), N-[(1R)-1-(dodecylcarbamoyl)-4-guanidino-butyl]octadecanamide hydrochloride (SZ191), (2R)-N-dodecyl-5-guanidino-2-(hexadecylcarbamoylamino)pentanamide hydrochloride (SZ193), N-[(1R)-5-amino-1-(dodecylcarbamoyl)pentyl]hexadecanamide hydrochloride (SZ194)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 5 µg and Addavax™ (mixed with saline at 1:1) dissolved in saline (Addavax™ administration group), (3) OVA (5 µg) and SZ180 (0.256 µmol) dissolved or dispersed in 5% αCD-added saline (SZ180 administration group), (4) OVA (5 µg) and SZ181 (0.256 µmol) dissolved or dispersed in 5% αCD-added saline (SZ181 administration group), (5) OVA (5 µg) and SZ182 (0.256 µmol) dissolved or dispersed in 5% αCD-added saline (SZ182 administration group), (6) OVA (5 µg) and SZ183 (0.256 µmol) dissolved or dispersed in 5% αCD-added saline (SZ183 administration group), (7) OVA (5 µg) and SZ184 (0.256 µmol) dissolved or dispersed in 5% aCD-added saline (SZ184 administration group), (8) OVA (5 µg) and SZ185 (0.256 µmol) dissolved or dispersed in 5% αCD-added saline (SZ185 administration group), (9) OVA (5 µg) and SZ186 (0.256 µmol) dissolved or dispersed in 5% αCD-added saline (SZ186 administration group), (10) OVA (5 µg) and SZ187 (0.256 µmol) dissolved or dispersed in 5% αCD-added saline (SZ187 administration group), (11) OVA (5 µg) and SZ188 (0.256 µmol) dissolved or dispersed in 5% αCD-added saline (SZ188 administration group), (12) OVA (5 µg) and SZ189 (0.256 µmol) dissolved or dispersed in 5% αCD-added saline (SZ189 administration group), (13) OVA (5 µg) and SZ190 (0.256 µmol) dissolved or dispersed in 5% aCD-added saline (SZ190 administration group), (14) OVA (5 µg) and SZ149 (0.256 pmol) dissolved or dispersed in 5% αCD-added saline SZ191 0.256 µmol (SZ191 administration group), (15) OVA (5 µg) and SZ193 (0.256 µmol) dissolved or dispersed in 5% αCD-added saline (SZ193 administration group), (16) OVA (5 µg) and SZ194 (0.256 µmol) dissolved or dispersed in 5% αCD-added saline (SZ194 administration group), each per mouse. Two weeks from the primary immunization, the blood was extracted under anesthesia, and the obtained serum samples were measured for anti-OVA-specific IgG1 subclass antibody and anti-OVA-specific IgG2a subclass antibody. One week from primary blood samples collection, each group underwent similar administration (secondary immunization) and two weeks from the secondary immunization, the blood was extracted under anesthesia, and the obtained serum samples were measured for anti-OVA-specific IgG1 subclass antibody, anti-OVA-specific IgG2a subclass antibody and anti-OVA-specific IgE antibody. The results are shown in Fig. 18 (upper: IgG1 subclass antibody after primary immunization, lower: IgG2a subclass antibody after primary immunization), Fig. 19 (upper: IgG1 subclass antibody after secondary immunization, lower: IgG2a subclass antibody after secondary immunization), Fig. 20 (IgE antibody after secondary immunization).

As is clear from the results shown in Fig. 18, significant enhancement of the anti-OVA-specific IgG1 and IgG2a subclass antibody production was found in Addavax™ administration group (Addavax in Figure), SZ180 administration group (SZ180 in Figure), SZ183 administration group (SZ183 in Figure), SZ185 administration group (SZ185 in Figure), SZ186 administration group (SZ186 in Figure), SZ187 administration group (SZ187 in Figure), SZ191 administration group (SZ191 in Figure), SZ193 administration group (SZ193 in Figure), as compared to OVA single administration group (saline in Figure). Significant enhancement of anti-OVA-specific IgG2a subclass antibody production was found in SZ194 administration group (SZ194 in Figure) as compared to OVA single administration group (saline in Figure). The anti-OVA-specific IgG1 and IgG2a subclass antibody production did not increase significantly in SZ181 administration group (SZ181 in Figure), SZ182 administration group (SZ182 in Figure), SZ184 administration group (SZ184 in Figure), SZ188 administration group (SZ188 in Figure), SZ189 administration group (SZ189 in Figure), SZ190 administration group (SZ190 in Figure), as compared to OVA single administration group (saline in Figure); however, a tendency toward increase was found. In addition, anti-OVA-specific IgG1 subclass antibody production tended to increase in SZ194 administration group (SZ194 in Figure), as compared to OVA single administration group (saline in Figure). These results suggest that SZ180, SZ181, SZ182, SZ183, SZ184, SZ185, SZ186, SZ187, SZ188, SZ189, SZ190, SZ191, SZ193 and SZ194 have an adjuvant activity.

As is clear from the results shown in Fig. 19, Addavax™ administration group (Addavax in Figure), SZ180 administration group (SZ180 in Figure), SZ183 administration group (SZ183 in Figure), SZ185 administration group (SZ185 in Figure), SZ189 administration group (SZ189 in Figure), SZ191 administration group (SZ191 in Figure) showed a significant enhancement of anti-OVA-specific IgG1 and IgG2a subclass antibody production as compared to OVA single administration group (saline in Figure). The SZ187 administration group (SZ187 in Figure) showed significant enhancement of anti-OVA-specific IgG2a subclass antibody production as compared to OVA single administration group (saline in Figure). The anti-OVA-specific IgG1 and IgG2a subclass antibody production in SZ181 administration group (SZ181 in Figure), SZ182 administration group (SZ182 in Figure), SZ184 administration group (SZ184 in Figure), SZ188 administration group (SZ188 in Figure), SZ190 administration group (SZ190 in Figure), SZ193 administration group (SZ193 in Figure), SZ194 administration group (SZ194 in Figure) did not show a significant increase as compared to OVA single administration group (saline in Figure) but showed a tendency to increase. In addition, anti-OVA-specific IgG1 subclass antibody production in SZ187 administration group (SZ187 in Figure) showed a tendency to increase as compared to OVA single administration group (saline in Figure). These results suggest that SZ180, SZ181, SZ182, SZ183, SZ184, SZ185, SZ186, SZ187, SZ188, SZ189, SZ190, SZ191, SZ193 and SZ194 have an adjuvant activity.

As is clear from the results shown in Fig. 20, significant increase in the production was not found in any group as compared to OVA single administration group (saline in Figure). The anti-OVA-specific IgE antibody production was high in Addavax™ administration group (Addavax in Figure), as compared to OVA single administration group (saline in Figure).

As is clear from the results shown in Fig. 21, it was clarified that the Effective Index of IgG1 of SZ180 administration group (SZ180 in Figure), SZ185 administration group (SZ185 in Figure), SZ189 administration group (SZ189 in Figure), SZ191 administration group (SZ191 in Figure) is larger than that of Addavax™ administration group (1.0). It was also clarified that the Effective Index of IgG2a of SZ182 administration group (SZ182 in Figure), SZ185 administration group (SZ185 in Figure), SZ189 administration group (SZ189 in Figure), SZ191 administration group (SZ191 in Figure) is larger than that of Addavax™ administration group (1.0). These results suggest that SZ180, SZ182, SZ185, SZ189 and SZ191 are utilizable as a particularly superior adjuvant that enhances antibody production and does not induce allergy as compared to Addavax™.

### [Experimental Example 9]

### Adjuvant activity and allergy inducing activity evaluation test of N-[(1S)-1-(dodecylcarbamoyl)-3-methyl-butyl]octadecanamide (SZ195), N-[(1S)-3-methyl-1-(octylcarbamoyl)butyl]octadecanamide (SZ196), N-[(1S)-1-(dodecylcarbamoyl)-3-methyl-butyl]docosanamide (SZ197), N-[(1S)-3-methyl-1-(octylcarbamoyl)butyl]docosanamide (SZ198), N-[(1S)-3-methyl-1-(octylcarbamoyl)butyl]hexadecanamide (SZ199), (2S)-N-dodecyl-2-(hexadecylcarbamoylamino)-4-methyl-pentanamide (SZ200), (2S)-2-(hexadecylcarbamoylamino)-4-methyl-N-octyl-pentanamide (SZ201), hexadecyl N-[(1S)-1-(dodecylcarbamoyl)-3-methyl-butyl]carbamate (SZ202), hexadecyl N-[(1S)-3-methyl-1-(octylcarbamoyl)butyl]carbamate (SZ203), N-[3-(dodecylamino)-2,2-dimethyl-3-oxo-propyl]octadecanamide (SZ204), N-[(1S)-1-(dodecylcarbamoyl)-5-guanidino-pentyl]octadecanamide hydrochloride (SZ205), N-[(1S)-1-(hexylcarbamoyl)-5-guanidino-pentyl]octadecanamide hydrochloride (SZ206), (2S)-2-(diheptylamino)-N-dodecyl-5-guanidino-pentanamide dihydrochloride (SZ207), N-[2-(4-pyridyl)ethyl]docosanamide hydrochloride (SZ209)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 5 µg and Addavax™ (mixed with saline at 1:1) dissolved in saline (Addavax™ administration group), (3) OVA (5 µg) and SZ195 (0.256 µmol) dissolved or dispersed in 5% αCD-added saline (SZ195 administration group), (4) OVA (5 µg) and SZ196 (0.256 µmol) dissolved or dispersed in 5% αCD-added saline (SZ196 administration group), (5) OVA (5 µg) and SZ197 (0.256 µmol) dissolved or dispersed in 5% αCD-added saline (SZ197 administration group), (6) OVA (5 µg) and SZ198 (0.256 µmol) dissolved or dispersed in 5% αCD-added saline (SZ198 administration group), (7) OVA (5 µg) and SZ199 (0.256 µmol) dissolved or dispersed in 5% αCD-added saline (SZ199 administration group), (8) OVA (5 µg) and SZ200 (0.256 µmol) dissolved or dispersed in 5% αCD-added saline (SZ200 administration group), (9) OVA (5 µg) and SZ201 (0.256 µmol) dissolved or dispersed in 5% aCD-added saline (SZ201 administration group), (10) OVA (5 µg) and SZ202 (0.256 µmol) dissolved or dispersed in 5% αCD-added saline (SZ202 administration group), (11) OVA (5 µg) and SZ203 (0.256 µmol) dissolved or dispersed in 5% αCD-added saline (SZ203 administration group), (12) OVA (5 µg) and SZ204 (0.256 µmol) dissolved or dispersed in 5% aCD-added saline (SZ204 administration group), (13) OVA (5 µg) and SZ205 (0.256 µmol) dissolved or dispersed in 5% αCD-added saline (SZ205 administration group), (14) OVA (5 µg) and SZ206 (0.256 µmol) dissolved or dispersed in 5% αCD-added saline (SZ206 administration group), (15) OVA (5 µg) and SZ207 (0.256 µmol) dissolved or dispersed in 5% αCD-added saline (SZ207 administration group), (16) OVA (5 µg) and SZ209 (0.256 µmol) dissolved or dispersed in 5% αCD-added saline (SZ209 administration group), each per mouse. Two weeks from the primary immunization, the blood was extracted under anesthesia, and the obtained serum samples were measured for anti-OVA-specific IgG1 subclass antibody and anti-OVA-specific IgG2a subclass antibody. One week from primary blood samples collection, each group underwent similar administration (secondary immunization) and two weeks from the secondary immunization, the blood was extracted under anesthesia, and the obtained serum samples were measured for anti-OVA-specific IgG1 subclass antibody, anti-OVA-specific IgG2a subclass antibody and anti-OVA-specific IgE antibody. The results are shown in Fig. 22 (upper: IgG1 subclass antibody after primary immunization, lower: IgG2a subclass antibody after primary immunization), Fig. 23 (upper: IgG1 subclass antibody after secondary immunization, lower: IgG2a subclass antibody after secondary immunization), Fig. 24 (IgE antibody after secondary immunization).

As is clear from the results shown in Fig. 22, significant enhancement of the anti-OVA-specific IgG1 and IgG2a subclass antibody production was found in Addavax™ administration group (Addavax in Figure), SZ199 administration group (SZ199 in Figure), SZ205 administration group (SZ205 in Figure), SZ206 administration group (SZ206 in Figure), as compared to OVA single administration group (saline in Figure). Significant enhancement of anti-OVA-specific IgG2a subclass antibody production was found in SZ202 administration group (SZ202 in Figure), SZ203 administration group (SZ203 in Figure), as compared to OVA single administration group (saline in Figure). Anti-OVA-specific IgG1 subclass antibody production and IgG2a subclass antibody production tended to increase in SZ195 administration group (SZ195 in Figure), SZ196 administration group (SZ196 in Figure), SZ197 administration group (SZ197 in Figure), SZ198 administration group (SZ198 in Figure), SZ200 administration group (SZ200 in Figure), SZ201 administration group (SZ201 in Figure), SZ204 administration group (SZ204 in Figure), SZ207 administration group (SZ207 in Figure), SZ209 administration group (SZ209 in Figure), as compared to OVA single administration group (saline in Figure). Anti-OVA-specific IgG1 subclass antibody production tended to increase in SZ202 administration group (SZ202 in Figure), SZ203 administration group (SZ203 in Figure), as compared to OVA single administration group (saline in Figure). These results suggest that SZ195, SZ196, SZ197, SZ198, SZ199, SZ200, SZ201, SZ202, SZ203, SZ204, SZ205, SZ206, SZ207 and SZ209 have an adjuvant activity.

As is clear from the results shown in Fig. 23, significant enhancement of anti-OVA-specific IgG1 and IgG2a subclass antibody production was found in Addavax™ administration group (Addavax in Figure), SZ205 administration group (SZ205 in Figure), SZ207 administration group (SZ207 in Figure), as compared to OVA single administration group (saline in Figure). In addition, significant enhancement of anti-OVA-specific IgG1 subclass antibody production was found in SZ196 administration group (SZ196 in Figure), SZ199 administration group (SZ199 in Figure), SZ202 administration group (SZ202 in Figure), SZ204 administration group (SZ204 in Figure), as compared to OVA single administration group (saline in Figure). In SZ206 administration group (SZ206 in Figure), significant enhancement of anti-OVA-specific IgG2a subclass antibody production was found as compared to OVA single administration group (saline in Figure). Furthermore, anti-OVA-specific IgG1 subclass antibody production and IgG2a subclass antibody production tended to increase in SZ195 administration group (SZ195 in Figure), SZ197 administration group (SZ197 in Figure), SZ198 administration group (SZ198 in Figure), SZ200 administration group (SZ200 in Figure), SZ201 administration group (SZ201 in Figure), SZ203 administration group (SZ203 in Figure), SZ209 administration group (SZ209 in Figure), as compared to OVA single administration group (saline in Figure). Moreover, anti-OVA-specific IgG2a subclass antibody production tended to increase in SZ196 administration group (SZ196 in Figure), SZ199 administration group (SZ199 in Figure), SZ202 administration group (SZ202 in Figure), SZ204 administration group (SZ204 in Figure), as compared to OVA single administration group (saline in Figure). These results suggest that SZ195, SZ196, SZ197, SZ198, SZ199, SZ200, SZ201, SZ202, SZ203, SZ204, SZ205, SZ206, SZ207 and SZ209 have an adjuvant activity.

As is clear from the results shown in Fig. 24, significant increase in the production was not found in any group as compared to OVA single administration group (saline in Figure). The anti-OVA-specific IgE antibody production was high in Addavax™ administration group (Addavax in Figure), as compared to OVA single administration group (saline in Figure).

As is clear from the results shown in Fig. 25, it was clarified that the Effective Index of IgG2a of SZ205 administration group (SZ205 in Figure), SZ206 administration group (SZ206 in Figure), SZ207 administration group (SZ207 in Figure) is larger than that of Addavax™ administration group (1.0). These results suggest that SZ205, SZ206 and SZ207 are utilizable as a particularly superior adjuvant that enhances antibody production and does not induce allergy as compared to Addavax™.

### [Experimental Example 10]

### Adjuvant activity and allergy inducing activity evaluation test of (2S)-5-guanidino-2-(hexadecylcarbamoylamino)-N-octyl-pentanamide hydrochloride (SZ210), (2S)-N-dodecyl-5-guanidino-2-(octadecylcarbamoylamino)pentanamide hydrochloride (SZ211), (2S)-N-dodecyl-2-(dodecylcarbamoylamino)-5-guanidino-pentanamide hydrochloride (SZ212), (2S)-6-amino-2-(hexadecylcarbamoylamino)-N-octyl-hexanamide hydrochloride (SZ213), (2S)-6-amino-N-dodecyl-2-(hexadecylcarbamoylamino)hexanamide hydrochloride (SZ214), hexadecyl N-[(1S)-4-guanidino-1-(octylcarbamoyl)butyl]carbamate hydrochloride (SZ215)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 5 µg and Addavax™ (mixed with saline at 1:1) dissolved in saline (Addavax™ administration group), (3) OVA (5 µg) and SZ210 (0.256 µmol) dissolved or dispersed in 5% αCD-added saline (SZ210 administration group), (4) OVA (5 µg) and SZ211 (0.256 µmol) dissolved or dispersed in 5% αCD-added saline (SZ211 administration group), (5) OVA (5 µg) and SZ212 (0.256 µmol) dissolved or dispersed in 5% αCD-added saline (SZ212 administration group), (6) OVA (5 µg) and SZ213 (0.256 µmol) dissolved or dispersed in 5% aCD-added saline (SZ213 administration group), (7) OVA (5 µg) and SZ214 (0.256 µmol) dissolved or dispersed in 5% aCD-added saline (SZ214 administration group), (8) OVA (5 µg) and SZ215 (0.256 µmol) dissolved or dispersed in 5% αCD-added saline (SZ215 administration group), each per mouse. Two weeks from the primary immunization, the blood was extracted under anesthesia, and the obtained serum samples were measured for anti-OVA-specific IgG1 subclass antibody and anti-OVA-specific IgG2a subclass antibody. One week from primary blood samples collection, each group underwent similar administration (secondary immunization) and two weeks from the secondary immunization, the blood was extracted under anesthesia, and the obtained serum samples were measured for anti-OVA-specific IgG1 subclass antibody, anti-OVA-specific IgG2a subclass antibody and anti-OVA-specific IgE antibody. The results are shown in Fig. 26 (upper: IgG1 subclass antibody after primary immunization, lower: IgG2a subclass antibody after primary immunization), Fig. 27 (upper: IgG1 subclass antibody after secondary immunization, lower: IgG2a subclass antibody after secondary immunization), Fig. 28 (IgE antibody after secondary immunization).

As is clear from the results shown in Fig. 26, significant enhancement of anti-OVA-specific IgG1 and IgG2a subclass antibody production was found in Addavax™ administration group (Addavax in Figure), SZ210 administration group (SZ210 in Figure), SZ211 administration group (SZ211 in Figure), SZ212 administration group (SZ212 in Figure), SZ214 administration group (SZ214 in Figure), as compared to OVA single administration group (saline in Figure). In SZ213 administration group (SZ213 in Figure), SZ215 administration group (SZ215 in Figure), significant enhancement of anti-OVA-specific IgG2a subclass antibody production was found as compared to OVA single administration group (saline in Figure). On the other hand, in SZ213 administration group (SZ213 in Figure), SZ215 administration group (SZ215 in Figure), a tendency toward increase in anti-OVA-specific IgG1 subclass antibody production was found as compared to OVA single administration group (saline in Figure). These results suggest that SZ210, SZ211, SZ212, SZ213, SZ214 and SZ215 have an adjuvant activity.

As is clear from the results shown in Fig. 27, significant enhancement of anti-OVA-specific IgG1 and IgG2a subclass antibody production was found in Addavax™ administration group (Addavax in Figure), SZ210 administration group (SZ210 in Figure), SZ211 administration group (SZ211 in Figure), SZ212 administration group (SZ212 in Figure), SZ213 administration group (SZ213 in Figure), SZ214 administration group (SZ214 in Figure), as compared to OVA single administration group (saline in Figure). In SZ215 administration group (SZ215 in Figure), significant enhancement of anti-OVA-specific IgG2a subclass antibody production was found as compared to OVA single administration group (saline in Figure). On the other hand, in SZ215 administration group (SZ215 in Figure), a tendency toward increase in anti-OVA-specific IgG1 subclass antibody production was found as compared to OVA single administration group (saline in Figure). These results suggest that SZ210, SZ211, SZ212, SZ213, SZ214 and SZ215 have an adjuvant activity.

As is clear from the results shown in Fig. 28, significant increase in the production was not found in any group as compared to OVA single administration group (saline in Figure). The anti-OVA-specific IgE antibody production was high in Addavax™ administration group (Addavax in Figure), as compared to OVA single administration group (saline in Figure).

As is clear from the results shown in Fig. 29, it was clarified that the Effective Index of IgG2a of SZ213 administration group (SZ213 in Figure), SZ215 administration group (SZ215 in Figure) is larger than that of Addavax™ administration group (1.0). These results suggest that SZ213 and SZ215 are utilizable as a particularly superior adjuvant that enhances antibody production and does not induce allergy as compared to Addavax™.

### [INDUSTRIAL APPLICABILITY]

Since the compound of the present invention has an antigen-specific IgG1 subclass antibody and IgG2a subclass antibody production-enhancing effect (immunostimulatory effect), it is useful as an immunostimulating agent. Particularly, since the compound of the present invention has an immunostimulatory effect equivalent to or not less than that of conventional aluminum gel adjuvants, suppresses induction of IgE antibody production, and sometimes suppresses problematic allergy inducing activity of conventional aluminum gel adjuvants, it can be an effective and safe adjuvant. Furthermore, since the compound of the present invention induces IgA antibody production on the mucosa, it can also be a mucosal vaccine adjuvant of which the research and development are ongoing at present. By combining with an existing adjuvant, the development as a combination adjuvant that enhances immune response is also expected.

## Claims

1. An immunostimulating agent comprising at least one kind of a compound represented by the formula (I): 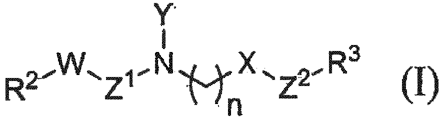 wherein
R² is an optionally substituted C₁₋₂₄ hydrocarbon group;
R³ is (a) a mono- or di-C₁₋₁₆ alkylamino group optionally substituted by a hydroxyl group or a C₁₋₄ alkoxy group, (b) a C₇₋₁₆ arylalkoxy group, (c) a hydrogen atom, (d) -C(=NH)R⁴ wherein R⁴ is an amino group, a C₁₋₁₆ alkyl-carbonylamino group or a C₁₋₄ alkyl group, or (e) a basic heterocyclic group;
W is a bond, -O-, -NH- or -CO-;
X is
(i) a group represented by the following formula 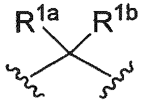 wherein
R^{1a} is
(1) a guanidyl-C₁₋₆ alkyl group,
(2) a C₁₋₁₂ alkyl group,
(3) an amino-C₁₋₆ alkyl group optionally substituted by an acetyl group or an aminocarbonyl group,
(4) a group represented by the following formula 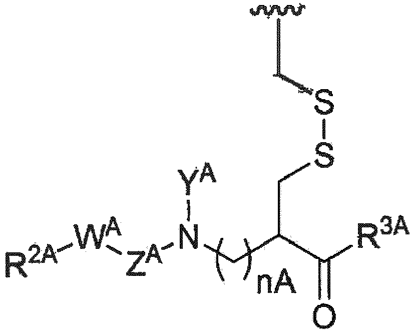 wherein
R^{2A} is an optionally substituted C₁₋₂₄ hydrocarbon group;
R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
W^{A} is a bond, -O-, -NH- or -CO-;
Y^{A} is a hydrogen atom, a C₁₋₁₀ alkyl group or a C₇₋₁₆ arylalkyl group;
Z^{A} is -CO- or a bond;
nA is an integer of 0 - 3 or
(5) a hydrogen atom;
R^{1b} is a C₁₋₄ alkyl group or a hydrogen atom;
when R^{1a} is a methyl group, Y is a methyl group and n is 2, then R^{1a} and Y are optionally bonded to form a ring,
(ii) -NR⁵- wherein R⁵ is a hydrogen atom or a methyl group, or
(iii) a bond;
Y is a hydrogen atom, a C₁₋₁₀ alkyl group or a C₇₋₁₆ arylalkyl group;
Z¹ and Z² are the same or different and each is -CO- or a bond;
n is an integer of 0 - 6;
when X is -N(CH₃)-, Y is a methyl group and n is 2, then the methyl group for X and Y are optionally bonded to form a ring,
provided that
a case in which R² is a C₁₋₂₄ alkyl group, R³ is a mono- or di-C₁₋₆ alkylamino group, W is a bond, X is the aforementioned (i), R^{1a} is a 3-guanidylpropyl group, a methyl group, an isopropyl group, an isobutyl group, a sec-butyl group, a 4-aminobutyl group or a hydrogen atom, R^{1b} is a hydrogen atom, Y is a hydrogen atom, Z¹ and Z² are both -CO-, and n is 0, and
a case in which R² is a C₁₋₂₄ alkyl group, R³ is -C(=NH)NH₂, W is a bond, X is -NH-, Y is a hydrogen atom, Z¹ is -CO-, Z² is a bond, and n is 4 are excluded,
or a salt thereof.

2. The immunostimulating agent according to claim 1, wherein R² is an optionally substituted C₆₋₂₁ hydrocarbon group.

3. The immunostimulating agent according to claim 1 or 2, wherein R³ is (a) a mono- or di-C₁₋₁₆ alkylamino group optionally substituted by a hydroxyl group or a C₁₋₄ alkoxy group, (c) a hydrogen atom, or (d) -C(=NH)R⁴ wherein R⁴ is an amino group, a C₁₋₁₆ alkyl-carbonylamino group or a C₁₋₄ alkyl group.

4. The immunostimulating agent according to any one of claims 1 to 3, wherein
X is
(i) a group represented by the following formula 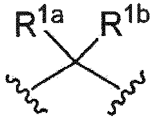 wherein
R^{1a} is
(1) a guanidyl-C₁₋₆ alkyl group,
(2) a C₁₋₁₂ alkyl group,
(3) an amino-C₁₋₆ alkyl group optionally substituted by an acetyl group or an aminocarbonyl group,
(4) a group represented by the following formula 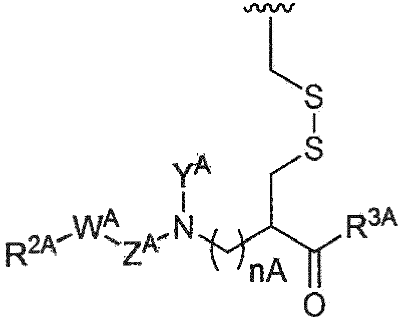 wherein
R^{2A} is an optionally substituted C₁₋₂₄ hydrocarbon group;
R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
W^{A} is a bond, -O-, -NH- or -CO-;
Y^{A} is a hydrogen atom, a C₁₋₁₀ alkyl group or a C₇₋₁₆ arylalkyl group;
Z^{A} is -CO- or a bond;
nA is an integer of 0 - 3, or
(5) a hydrogen atom;
R^{1b} is a C₁₋₄ alkyl group or a hydrogen atom;
when R^{1a} is a methyl group, Y is a methyl group and n is 2, then R^{1a} and Y are optionally bonded to form a ring, or
(ii) -NR⁵- wherein R⁵ is a hydrogen atom or a methyl group.

5. The immunostimulating agent according to any one of claims 1 to 4, wherein the compound represented by the formula (I) or a salt thereof is selected from the group consisting of
N-[(1S)-1-(dodecylcarbamoyl)-4-guanidino-butyl]hexadecanamide,
N-[(1S)-1-(dodecylcarbamoyl)-4-guanidino-butyl]octadecanamide,
N-[(1S)-1-(dodecylcarbamoyl)-4-guanidino-butyl]docosanamide,
N-[(1S)-3-methyl-1-(undecylcarbamoyl)butyl]docosanamide,
N-[(1S)-1-(dodecylcarbamoyl)-3-methylbutyl]hexadecanamide,
N-[(1S)-2-(dodecylamino)-1-methyl-2-oxoethyl]docosanamide,
N-[2-(dodecylamino)-1,1-dimethyl-2-oxoethyl]docosanamide,
N-[3-(dodecylamino)-3-oxo-propyl]docosanamide,
N-[3-(dodecylamino)-2,2-dimethyl-3-oxopropyl]docosanamide,
(2S)-N-dodecyl-5-guanidino-2-(hexadecylcarbamoylamino)pentanamide,
Hexadecyl N-[(1S)-1-(dodecylcarbamoyl)-4-guanidino-butyl]carbamate,
N-[(1S)-5-amino-1-(dodecylcarbamoyl)pentyl]hexadecanamide,
N-[(1S)-1-(dodecylcarbamoyl)pentyl]docosanamide,
1-docosanoyl-N-dodecyl-piperidine-4-carboxamide,
N-[(1S)-1-(diheptylcarbamoyl)-4-guanidino-butyl]docosanamide,
N-[(1S)-5-amino-1-(dodecylcarbamoyl)pentyl]docosanamide,
(E)-N-[(1S)-1-(dodecylcarbamoyl)-4-guanidino-butyl]octadeca-9-enamide,
N-[(1S)-4-guanidino-1-(heptylcarbamoyl)butyl]docosanamide,
N-[3-(dodecylamino)-3-oxo-pentyl]docosanamide,
N-[(1S)-4-amino-1-(dodecylcarbamoyl)butyl]docosanamide,
N-[(1S)-1-(dodecylcarbamoyl)-5-guanidino-pentyl]docosanamide,
N-[(1S)-5-acetamide-1-(dodecylcarbamoyl)pentyl]docosanamide,
(2S)-5-guanidino-2-(hexadecylcarbamoylamino)-N-hexyl-pentanamide,
Hexadecyl N-[(1S)-4-guanidino-1-(hexylcarbamoyl)butyl]carbamate,
N-[(1S)-5-guanidino-1-(hexylcarbamoyl)pentyl]docosanamide,
N-[(1R)-1-[[[(2R)-2-(docosanoylamino)-3-(dodecylamino)-3-oxo-propyl]disulfanyl]methyl]-2-(dodecylamino)-2-oxoethyl]docosanamide,
(2S)-2-(dibenzylamino)-N-dodecyl-5-guanidino-pentanamide,
N-(5-guanidinopentyl)hexadecanamide,
N-(5-guanidinopentyl)docosanamide,
N-[(1S)-1-(dodecylcarbamoyl)-4-guanidino-butyl]-2-oxo-octanamide,
Hexadecyl N-(4-guanidinobutyl)carbamate,
N-[4-(ethanimidoylamino)butyl]hexadecanamide,
N-[(1S)-1-(dodecylcarbamoyl)-4-guanidino-butyl]icosanamide,
N-[(1S)-4-guanidino-1-(octylcarbamoyl)butyl]hexadecanamide,
N-[(1S)-4-guanidino-1-(octylcarbamoyl)butyl]octadecanamide,
N-[(1S)-4-guanidino-1-(octylcarbamoyl)butyl]icosanamide,
N-[(1S)-5-amino-1-(octylcarbamoyl)pentyl]octadecanamide,
N-[(1S)-5-amino-1-(dodecylcarbamoyl)pentyl]octadecanamide,
Octadecyl N-[(1S)-1-(dodecylcarbamoyl)-4-guanidino-butyl] carbamate,
Dodecyl N-[(1S)-1-(dodecylcarbamoyl)-4-guanidino-butyl]carbamate,
Hexadecyl N-[(1S)-5-amino-1-(octylcarbamoyl)pentyl]carbamate,
Hexadecyl N-[(1S)-5-amino-1-(dodecylcarbamoyl)pentyl]carbamate,
1-(4-guanidinobutyl)-3-hexadecyl-urea,
N-[(1R)-1-(dodecylcarbamoyl)-4-guanidino-butyl]octadecanamide,
(2R)-N-dodecyl-5-guanidino-2-(hexadecylcarbamoylamino)pentanamide,
N-[(1R)-5-amino-1-(dodecylcarbamoyl)pentyl]hexadecanamide,
N-[(1S)-1-(dodecylcarbamoyl)-3-methylbutyl]octadecanamide,
N-[(1S)-3-methyl-1-(octylcarbamoyl)butyl]octadecanamide,
N-[(1S)-1-(dodecylcarbamoyl)-3-methyl-butyl]docosanamide,
N-[(1S)-3-methyl-1-(octylcarbamoyl)butyl]docosanamide,
N-[(1S)-3-methyl-1-(octylcarbamoyl)butyl]hexadecanamide,
(2S)-N-dodecyl-2-(hexadecylcarbamoylamino)-4-methylpentanamide,
(2S)-2-(hexadecylcarbamoylamino)-4-methyl-N-octyl-pentanamide,
Hexadecyl N-[(1S)-1-(dodecylcarbamoyl)-3-methylbutyl]carbamate,
Hexadecyl N-[(1S)-3-methyl-1-(octylcarbamoyl)butyl]carbamate,
N-[3-(dodecylamino)-2,2-dimethyl-3-oxopropyl]octadecanamide,
N-[(1S)-1-(dodecylcarbamoyl)-5-guanidino-pentyl]octadecanamide,
N-[(1S)-1-(hexylcarbamoyl)-5-guanidino-pentyl]octadecanamide,
(2S)-2-(diheptylamino)-N-dodecyl-5-guanidino-pentanamide,
N-[2-(4-pyridyl)ethyl]docosanamide,
(2S)-5-guanidino-2-(hexadecylcarbamoylamino)-N-octyl-pentanamide,
(2S)-N-dodeoyl-5-guanidino-2-(octadecylcarbamoylamino)pentanamide,
(2S)-N-dodecyl-2-(dodecylcarbamoylamino)-5-guanidino-pentanamide,
(2S)-6-amino-2-(hexadecylcarbamoylamino)-N-octyl-hexanamide,
(2S)-6-amino-N-dodecyl-2-(hexadecylcarbamoylamino)hexanamide,
Hexadecyl N-[(1S)-4-guanidino-1-(octylcarbamoyl)butyl]carbamate,
and salts thereof.

6. The immunostimulating agent according to any one of claims 1 to 5, wherein the aforementioned immunostimulating agent is a vaccine adjuvant.

7. A pharmaceutical composition comprising at least one kind of a compound represented by the formula (I): 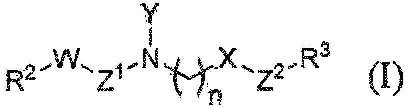 wherein
R² is an optionally substituted C₁₋₂₄ hydrocarbon group;
R³ is (a) a mono- or di-C₁₋₁₆ alkylamino group optionally substituted by a hydroxyl group or a C₁₋₄ alkoxy group, (b) a C₇₋₁₆ arylalkoxy group, (c) a hydrogen atom, (d) -C(=NH)R⁴ wherein R⁴ is an amino group, a C₁₋₁₆ alkyl-carbonylamino group or a C₁₋₄ alkyl group, or (e) a basic heterocyclic group;
W is a bond, -O-, -NH- or -CO-;
X is
(i) a group represented by the following formula 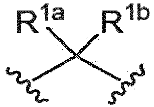 wherein
R^{1a} is
(1) a guanidyl-C₁₋₆ alkyl group,
(2) a C₁₋₁₂ alkyl group,
(3) an amino-C₁₋₆ alkyl group optionally substituted by an acetyl group or an aminocarbonyl group,
(4) a group represented by the following formula 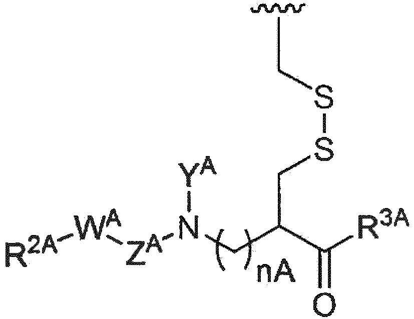 wherein
R^{2A} is an optionally substituted C₁₋₂₄ hydrocarbon group;
R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
W^{A} is a bond, -O-, -NH- or -CO-;
Y^{A} is a hydrogen atom, a C₁₋₁₀ alkyl group or a C₇₋₁₆ arylalkyl group;
Z^{A} is -CO- or a bond;
nA is an integer of 0 - 3, or
(5) a hydrogen atom;
R^{1b} is a C₁₋₄ alkyl group or a hydrogen atom;
when R^{1a} is a methyl group, Y is a methyl group and n is 2, then R^{1a} and Y are optionally bonded to form a ring,
(ii) -NR⁵- wherein R⁵ is a hydrogen atom or a methyl group, or
(iii) a bond;
Y is a hydrogen atom, a C₁₋₁₀ alkyl group or a C₇₋₁₆ arylalkyl group;
Z¹ and Z² are the same or different and each is -CO- or a bond;
n is an integer of 0 - 6;
when X is -N(CH₃)-, Y is a methyl group and n is 2, then the methyl group for X and Y are optionally bonded to form a ring,
provided that
a case in which R² is a C₁₋₂₄ alkyl group, R³ is a mono- or di-C₁₋₆ alkylamino group, W is a bond, X is the aforementioned (i), R^{1a} is a 3-guanidylpropyl group, a methyl group, an isopropyl group, an isobutyl group, a sec-butyl group, a 4-aminobutyl group or a hydrogen atom, R^{1b} is a hydrogen atom, Y is a hydrogen atom, Z¹ and Z² are both -CO-, and n is 0, and
a case in which R² is a C₁₋₂₄ alkyl group, R³ is -C(=NH)NH₂, W is a bond, X is -NH-, Y is a hydrogen atom, Z¹ is -CO-, Z² is a bond and n is 4 are excluded,
or a salt thereof, and α-cyclodextrin.

8. The pharmaceutical composition according to claim 7, wherein
R² is an optionally substituted C₆₋₂₁ hydrocarbon group.

9. The pharmaceutical composition according to claim 7 or 8, wherein
R³ is (a) a mono- or di-C₁₋₁₆ alkylamino group optionally substituted by a hydroxyl group or a C₁₋₄ alkoxy group, (c) a hydrogen atom, or (d) -C(=NH)R⁴ wherein R⁴ is an amino group, a C₁₋₁₆ alkyl-carbonylamino group or a C₁₋₄ alkyl group.

10. The pharmaceutical composition according to any one of claims 7 to 9, wherein
X is
(i) a group represented by the following formula 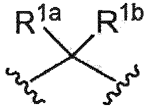 wherein
R^{1a} is
(1) a guanidyl-C₁₋₆ alkyl group,
(2) a C₁₋₁₂ alkyl group,
(3) an amino-C₁₋₆ alkyl group optionally substituted by an acetyl group or an aminocarbonyl group,
(4) a group represented by the following formula 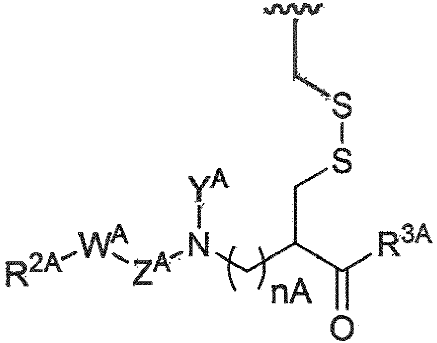 wherein
R^{2A} is an optionally substituted C₁₋₂₄ hydrocarbon group;
R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
W^{a} is a bond, -O-, -NH- or -CO-;
Y^{a} is a hydrogen atom, a C₁₋₁₀ alkyl group or a C₇₋₁₆ arylalkyl group;
Z^{A} is -CO- or a bond;
nA is an integer of 0 - 3, or
(5) a hydrogen atom;
R^{1b} is a C₁₋₄ alkyl group or a hydrogen atom;
when R^{1a} is a methyl group, Y is a methyl group and n is 2, then R^{1a} and Y are optionally bonded to form a ring, or
(ii) -NR⁵- wherein R⁵ is a hydrogen atom or a methyl group.

11. The pharmaceutical composition according to any one of claims 7 to 10, wherein the compound represented by the formula (I) or a salt thereof is selected from the group consisting of
N-[(1S)-1-(dodecylcarbamoyl)-4-guanidino-butyl]hexadecanamide,
N-[(1S)-1-(dodecylcarbamoyl)-4-guanidino-butyl]octadecanamide,
N-[(1S)-1-(dodecylcarbamoyl)-4-guanidino-butyl]docosanamide,
N-[(1S)-3-methyl-1-(undecylcarbamoyl)butyl]docosanamide,
N-[(1S)-1-(dodecylcarbamoyl)-3-methylbutyl]hexadecanamide,
N-[(1S)-2-(dodecylamino)-1-methyl-2-oxoethyl]docosanamide,
N-[2-(dodecylamino)-1,1-dimethyl-2-oxoethyl]docosanamide,
N-[3-(dodecylamino)-3-oxo-propyl]docosanamide,
N-[3-(dodecylamino)-2,2-dimethyl-3-oxopropyl]docosanamide,
(2S)-N-dodecyl-5-guanidino-2-(hexadecylcarbamoylamino)pentanamide,
Hexadecyl N-[(1S)-1-(dodecylcarbamoyl)-4-guanidino-butyl]carbamate,
N-[(1S)-5-amino-1-(dodecylcarbamoyl)pentyl]hexadecanamide,
N-[(1S)-1-(dodecylcarbamoyl)pentyl]docosanamide,
1-docosanoyl-N-dodecyl-piperidine-4-carboxamide,
N-[(1S)-1-(diheptylcarbamoyl)-4-guanidino-butyl]docosanamide,
N-[(1S)-5-amino-1-(dodecylcarbamoyl)pentyl]docosanamide,
(E)-N-[(1S)-1-(dodecylcarbamoyl)-4-guanidino-butyl]octadeca-9-enamide,
N-[(1S)-4-guanidino-1-(heptylcarbamoyl)butyl]docosanamide,
N-[3-(dodecylamino)-3-oxo-pentyl]docosanamide,
N-[(1S)-4-amino-1-(dodecylcarbamoyl)butyl]docosanamide,
N-[(1S)-1-(dodecylcarbamoyl)-5-guanidino-pentyl]docosanamide,
N-[(1S)-5-acetamide-1-(dodecylcarbamoyl)pentyl]docosanamide,
(2S)-5-guanidino-2-(hexadecylcarbamoylamino)-N-hexyl-pentanamide,
Hexadecyl N-[(1S)-4-guanidino-1-(hexylcarbamoyl)butyl]carbamate,
N-[(1S)-5-guanidino-1-(hexylcarbamoyl)pentyl]docosanamide,
N-[(1R)-1-[[[(2R)-2-(docosanoylamino)-3-(dodecylamino)-3-oxo-propyl]disulfanyl]methyl]-2-(dodecylamino)-2-oxoethyl]docosanamide,
(2S)-2-(dibenzylamino)-N-dodecyl-5-guanidino-pentanamide,
N-(5-guanidinopentyl)hexadecanamide,
N-(5-guanidinopentyl)docosanamide,
N-[(1S)-1-(dodecylcarbamoyl)-4-guanidino-butyl]-2-oxo-octanamide,
Hexadecyl N-(4-guanidinobutyl)carbamate,
N-[4-(ethanimidoylamino)butyl]hexadecanamide, N-[(1S)-1-(dodecylcarbamoyl)-4-guanidino-butyl]icosanamide,
N-[(1S)-4-guanidino-1-(octylcarbamoyl)butyl]hexadecanamide,
N-[(1S)-4-guanidino-1-(octylcarbamoyl)butyl]octadecanamide,
N-[(1S)-4-guanidino-1-(octylcarbamoyl)butyl]icosanamide,
N-[(1S)-5-amino-1-(octylcarbamoyl)pentyl]octadecanamide,
N-[(1S)-5-amino-1-(dodecylcarbamoyl)pentyl]octadecanamide,
Octadecyl N-[(1S)-1-(dodecylcarbamoyl)-4-guanidino-butyl]carbamate,
Dodecyl N-[(1S)-1-(dodecylcarbamoyl)-4-guanidino-butyl]carbamate,
Hexadecyl N-[(1S)-5-amino-1-(octylcarbamoyl)pentyl] carbamate,
Hexadecyl N-[(1S)-5-amino-1-(dodecylcarbamoyl)pentyl] carbamate,
1-(4-guanidinobutyl)-3-hexadecyl-urea,
N-[(1R)-1-(dodecylcarbamoyl)-4-guanidino-butyl]octadecanamide,
(2R)-N-dodecyl-5-guanidino-2-(hexadecylcarbamoylamino)pentanamide,
N-[(1R)-5-amino-1-(dodecylcarbamoyl)pentyl]hexadecanamide,
N-[(1S)-1-(dodecylcarbamoyl)-3-methylbutyl]octadecanamide,
N-[(1S)-3-methyl-1-(octylcarbamoyl)butyl]octadecanamide,
N-[(1S)-1-(dodecylcarbamoyl)-3-methyl-butyl]docosanamide,
N-[(1S)-3-methyl-1-(octylcarbamoyl)butyl]docosanamide,
N-[(1S)-3-methyl-1-(octylcarbamoyl)butyl]hexadecanamide,
(2S)-N-dodecyl-2-(hexadecylcarbamoylamino)-4-methylpentanamide,
(2S)-2-(hexadecylcarbamoylamino)-4-methyl-N-octyl-pentanamide,
Hexadecyl N-[(1S)-1-(dodecylcarbamoyl)-3-methylbutyl]carbamate,
Hexadecyl N-[(1S)-3-methyl-1-(octylcarbamoyl)butyl]carbamate,
N-[3-(dodecylamino)-2,2-dimethyl-3-oxopropyl]octadecanamide,
N-[(1S)-1-(dodecylcarbamoyl)-5-guanidino-pentyl]octadecanamide,
N-[(1S)-1-(hexylcarbamoyl)-5-guanidino-pentyl]octadecanamide,
(2S)-2-(diheptylamino)-N-dodecyl-5-guanidino-pentanamide,
N-[2-(4-pyridyl)ethyl]docosanamide,
(2S)-5-guanidino-2-(hexadecylcarbamoylamino)-N-octyl-pentanamide,
(2S)-N-dodecyl-5-guanidino-2-(octadecylcarbamoylamino)pentanamide,
(2S)-N-dodecyl-2-(dodecylcarbamoylamino)-5-guanidino-pentanamide,
(2S)-6-amino-2-(hexadecylcarbamoylamino)-N-octyl-hexanamide,
(2S)-6-amino-N-dodecyl-2-(hexadecylcarbamoylamino)hexanamide,
Hexadecyl N-[(1S)-4-guanidino-1-(octylcarbamoyl)butyl]carbamate,
and salts thereof.

12. A vaccine comprising at least one kind of a compound represented by the formula (I): 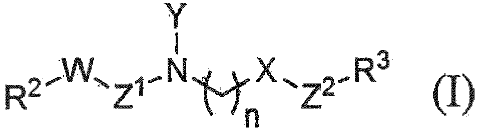 wherein
R² is an optionally substituted C₁₋₂₄ hydrocarbon group;
R³ is (a) a mono- or di-C₁₋₁₆ alkylamino group optionally substituted by a hydroxyl group or a C₁₋₄ alkoxy group, (b) a C₇₋₁₆ arylalkoxy group, (c) a hydrogen atom, (d) -C(=NH)R⁴ wherein R⁴ is an amino group, a C₁₋₁₆ alkyl-carbonylamino group or a C₁₋₄ alkyl group, or (e) a basic heterocyclic group;
W is a bond, -O-, -NH- or -CO-;
X is
(i) a group represented by the following formula 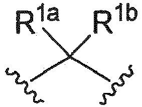 wherein
R^{1a} is
(1) a guanidyl-C₁₋₆ alkyl group,
(2) a C₁₋₁₂ alkyl group,
(3) an amino-C₁₋₆ alkyl group optionally substituted by an acetyl group or an aminocarbonyl group,
(4) a group represented by the following formula 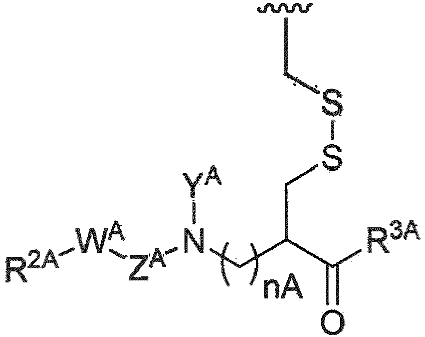 wherein
R^{2A} is an optionally substituted C₁₋₂₄ hydrocarbon group;
R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
W^{A} is a bond, -O-, -NH- or -CO-;
Y^{A} is a hydrogen atom, a C₁₋₁₀ alkyl group or a C₇₋₁₆ arylalkyl group;
Z^{A} is -CO- or a bond;
nA is an integer of 0 - 3, or
(5) a hydrogen atom;
R^{1b} is a C₁₋₄ alkyl group or a hydrogen atom;
when R^{1a} is a methyl group, Y is a methyl group and n is 2, R^{1a} and Y are optionally bonded to form a ring,
(ii) -NR⁵- wherein R⁵ is a hydrogen atom or a methyl group, or
(iii) a bond;
Y is a hydrogen atom, a C₁₋₁₀ alkyl group or a C₇₋₁₆ arylalkyl group;
Z¹ and Z² are the same or different and each is -CO- or a bond;
n is an integer of 0 - 6;
when X is -N(CH₃)-, Y is a methyl group and n is 2, then the methyl group for X and Y are optionally bonded to form a ring,
provided that
a case in which R² is a C₁₋₂₄ alkyl group, R³ is a mono- or di-C₁₋₆ alkylamino group, W is a bond, X is the aforementioned (i), R^{1a} is a 3-guanidylpropyl group, a methyl group, an isopropyl group, an isobutyl group, a sec-butyl group, a 4-aminobutyl group or a hydrogen atom, R^{1b} is a hydrogen atom, Y is a hydrogen atom, Z¹ and Z² are both -CO-, and n is 0, and
a case in which R² is a C₁₋₂₄ alkyl group, R³ is -C(=NH)NH₂, W is a bond, X is -NH-, Y is a hydrogen atom, Z¹ is -CO-, Z² is a bond, and n is 4 are excluded,
or a salt thereof, and an antigen.

13. The vaccine according to claim 12, wherein R² is an optionally substituted C₆₋₂₁ hydrocarbon group.

14. The vaccine according to claim 12 or 13, wherein R³ is (a) a mono- or di-C₁₋₁₆ alkylamino group optionally substituted by a hydroxyl group or a C₁₋₄ alkoxy group, (c) a hydrogen atom, or (d) -C(=NH)R⁴ wherein R⁴ is an amino group, a C₁₋₁₆ alkyl-carbonylamino group or a C₁₋₄ alkyl group.

15. The vaccine according to any one of claims 12 to 14, wherein
X is
(i) a group represented by the following formula 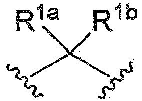 wherein
R^{1a} is
(1) a guanidyl-C₁₋₆ alkyl group,
(2) a C₁₋₁₂ alkyl group,
(3) an amino-C₁₋₆ alkyl group optionally substituted by an acetyl group or an aminocarbonyl group,
(4) a group represented by the following formula 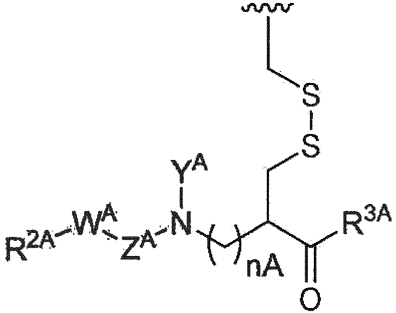 wherein
R^{2A} is an optionally substituted C₁₋₂₄ hydrocarbon group;
R^{3A} is (a) a mono- or di-C₁₋₁₆ alkylamino group optionally substituted by a hydroxyl group or a C₁₋₄ alkoxy group, or (b) a C₇₋₁₆ arylalkoxy group;
W^{A} is a bond, -O-, -NH- or -CO-;
Y^{A} is a hydrogen atom, a C₁₋₁₀ alkyl group or a C₇₋₁₆ arylalkyl group;
Z^{A} is -CO- or a bond;
nA is an integer of 0 - 3, or
(5) a hydrogen atom;
R^{1b} is a C₁₋₄ alkyl group or a hydrogen atom;
when R^{1a} is a methyl group, Y is a methyl group and n is 2, then R^{1a} and Y are optionally bonded to form a ring, or
(ii) -NR⁵- wherein R⁵ is a hydrogen atom or a methyl group.

16. The vaccine according to any one of claims 12 to 15, wherein the compound represented by the formula (I) or a salt thereof is selected from the group consisting of
N-[(1S)-1-(dodecylcarbamoyl)-4-guanidino-butyl]hexadecanamide,
N-[(1S)-1-(dodecylcarbamoyl)-4-guanidino-butyl]octadecanamide,
N-[(1S)-1-(dodecylcarbamoyl)-4-guanidino-butyl]docosanamide,
N-[(1S)-3-methyl-1-(undecylcarbamoyl)butyl]docosanamide,
N-[(1S)-1-(dodecylcarbamoyl)-3-methylbutyl]hexadecanamide,
N-[(1S)-2-(dodecylamino)-1-methyl-2-oxoethyl]docosanamide,
N-[2-(dodecylamino)-1,1-dimethyl-2-oxoethyl]docosanamide,
N-[3-(dodecylamino)-3-oxo-propyl]docosanamide,
N-[3-(dodecylamino)-2,2-dimethyl-3-oxopropyl]docosanamide,
(2S)-N-dodecyl-5-guanidino-2-(hexadecylcarbamoylamino)pentanamide,
Hexadecyl N-[(1S)-1-(dodecylcarbamoyl)-4-guanidino-butyl]carbamate,
N-[(1S)-5-amino-1-(dodecylcarbamoyl)pentyl]hexadecanamide,
N-[(1S)-1-(dodecylcarbamoyl)pentyl]docosanamide,
1-docosanoyl-N-dodecyl-piperidine-4-carboxamide,
N-[(1S)-1-(diheptylcarbamoyl)-4-guanidino-butyl]docosanamide,
N-[(1S)-5-amino-1-(dodecylcarbamoyl)pentyl]docosanamide,
(E)-N-[(1S)-1-(dodecylcarbamoyl)-4-guanidino-butyl]octadeca-9-enamide,
N-[(1S)-4-guanidino-1-(heptylcarbamoyl)butyl]docosanamide,
N-[3-(dodecylamino)-3-oxo-pentyl]docosanamide,
N-[(1S)-4-amino-1-(dodecylcarbamoyl)butyl]docosanamide,
N-[(1S)-1-(dodecylcarbamoyl)-5-guanidino-pentyl]docosanamide,
N-[(1S)-5-acetamide-1-(dodecylcarbamoyl)pentyl]docosanamide,
(2S)-5-guanidino-2-(hexadecylcarbamoylamino)-N-hexyl-pentanamide,
Hexadecyl N-[(1S)-4-guanidino-1-(hexylcarbamoyl)butyl]carbamate,
N-[(1S)-5-guanidino-1-(hexylcarbamoyl)pentyl]docosanamide,
N-[(1R)-1-[[[(2R)-2-(docosanoylamino)-3-(dodecylamino)-3-oxo-propyl]disulfanyl]methyl]-2-(dodecylamino)-2-oxoethyl]docosanamide,
(2S)-2-(dibenzylamino)-N-dodecyl-5-guanidino-pentanamide,
N-(5-guanidinopentyl)hexadecanamide,
N-(5-guanidinopentyl)docosanamide,
N-[(1S)-1-(dodecylcarbamoyl)-4-guanidino-butyl]-2-oxo-octanamide,
Hexadecyl N-(4-guanidinobutyl)carbamate,
N-[4-(ethanimidoylamino)butyl]hexadecanamide,
N-[(1S)-1-(dodecylcarbamoyl)-4-guanidino-butyl]icosanamide,
N-[(1S)-4-guanidino-1-(octylcarbamoyl)butyl]hexadecanamide,
N-[(1S)-4-guanidino-1-(octylcarbamoyl)butyl]octadecanamide,
N-[(1S)-4-guanidino-1-(octylcarbamoyl)butyl]icosanamide,
N-[(1S)-5-amino-1-(octylcarbamoyl)pentyl]octadecanamide,
N-[(1S)-5-amino-1-(dodecylcarbamoyl)pentyl]octadecanamide,
Octadecyl N-[(1S)-1-(dodecylcarbamoyl)-4-guanidino-butyl]carbamate,
Dodecyl N-[(1S)-1-(dodecylcarbamoyl)-4-guanidino-butyl]carbamate,
Hexadecyl N-[(1S)-5-amino-1-(octylcarbamoyl)pentyl]carbamate,
Hexadecyl N-[(1S)-5-amino-1-(dodecylcarbamoyl)pentyl]carbamate,
1-(4-guanidinobutyl)-3-hexadecyl-urea,
N-[(1R)-1-(dodecylcarbamoyl)-4-guanidino-butyl]octadecanamide,
(2R)-N-dodecyl-5-guanidino-2-(hexadecylcarbamoylamino)pentanamide,
N-[(1R)-5-amino-1-(dodecylcarbamoyl)pentyl]hexadecanamide,
N-[(1S)-1-(dodecylcarbamoyl)-3-methyl-butyl]octadecanamide,
N-[(1S)-3-methyl-1-(octylcarbamoyl)butyl]octadecanamide,
N-[(1S)-1-(dodecylcarbamoyl)-3-methyl-butyl]docosanamide,
N-[(1S)-3-methyl-1-(octylcarbamoyl)butyl]docosanamide,
N-[(1S)-3-methyl-1-(octylcarbamoyl)butyl]hexadecanamide,
(2S)-N-dodecyl-2-(hexadecylcarbamoylamino)-4-methylpentanamide,
(2S)-2-(hexadecylcarbamoylamino)-4-methyl-N-octyl-pentanamide,
Hexadecyl N-[(1S)-1-(dodecylcarbamoyl)-3-methylbutyl]carbamate,
Hexadecyl N-[(1S)-3-methyl-1-(octylcarbamoyl)butyl]carbamate,
N-[3-(dodecylamino)-2,2-dimethyl-3-oxopropyl]octadecanamide,
N-[(1S)-1-(dodecylcarbamoyl)-5-guanidino-pentyl]octadecanamide,
N-[(1S)-1-(hexylcarbamoyl)-5-guanidino-pentyl]octadecanamide,
(2S)-2-(diheptylamino)-N-dodecyl-5-guanidino-pentanamide,
N-[2-(4-pyridyl)ethyl]docosanamide,
(2S)-5-guanidino-2-(hexadecylcarbamoylamino)-N-octyl-pentanamide,
(2S)-N-dodecyl-5-guanidino-2-(octadecylcarbamoylamino)pentanamide,
(2S)-N-dodecyl-2-(dodecylcarbamoylamino)-5-guanidino-pentanamide,
(2S)-6-amino-2-(hexadecylcarbamoylamino)-N-octyl-hexanamide,
(2S)-6-amino-N-dodecyl-2-(hexadecylcarbamoylamino)hexanamide,
Hexadecyl N-[(1S)-4-guanidino-1-(octylcarbamoyl)butyl]carbamate,
and salts thereof.

17. A compound represented by the formula (I'): 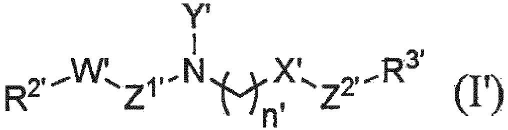 wherein
X' is a group represented by the following formula 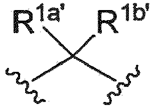 wherein
R^{1a}' is
(1) a guanidyl-C₃₋₄ alkyl group,
(2) a C₄ alkyl group,
(3) an amino-C₃₋₄ alkyl group optionally substituted by an acetyl group or an aminocarbonyl group, or
(4) a group represented by the following formula 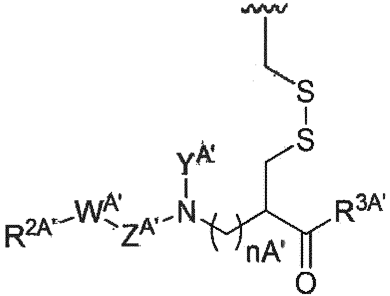 wherein
R^{2A}' is a C₁₈₋₂₄ hydrocarbon group;
R^{3A}' is a mono- or di-C₆₋₁₆ alkylamino group;
W^{A}' is a bond;
Y^{A}' is a hydrogen atom;
Z^{A}' is -CO-;
nA' is 0;
R^{1b}' is a hydrogen atom;
Y' is a hydrogen atom;
in R²', R³' and W',
(I') when R^{1a}' is the aforementioned (1),
(I'-1) R²' is a C₁₈₋₂₄ hydrocarbon group, R³' is a mono- or di-C₇₋₁₆ alkylamino group and W' is a bond,
(I'-2) R²' is a C₁₂₋₂₄ hydrocarbon group, R³' is a mono- or di-C₆₋₁₆ alkylamino group and W' is -O- or -NH-, or
(I'-3) R²' is a C₅₋₂₄ hydrocarbon group, R³' is a mono- or di-C₆₋₁₆ alkylamino group and W' is -CO-,
(II') when R^{1a}' is the aforementioned (2),
(II'-1) R²' is a C₁₂₋₂₄ hydrocarbon group, R³' is a mono- or di-C₇₋₁₆ alkylamino group and W' is a bond (provided that a case in which R²' is a C₂₁ hydrocarbon group and R³' is a mono-C₈ alkylamino group is excluded),
(II'-2) R²' is a C₁₂₋₂₄ hydrocarbon group, R³' is a mono- or di-C₆₋₁₆ alkylamino group and W' is -O- or -NH-, or
(II'-3) R²' is a C₅₋₂₄ hydrocarbon group, R³' is a mono- or di-C₆₋₁₆ alkylamino group and W' is -CO-,
(III') when R^{1a}' is the aforementioned (3),
(III'-1) R²' is a C₁₂₋₂₄ alkyl group, R³' is a mono- or di-C₇₋₁₆ alkylamino group and W' is a bond,
(III'-2) R²' is a C₁₂₋₂₄ hydrocarbon group, R³' is a mono- or di-C₆₋₁₆ alkylamino group and W' is -O- or -NH-, or
(III'-3) R²' is a C₅₋₂₄ hydrocarbon group, R³' is a mono- or di-C₆₋₁₆ alkylamino group and W' is -CO-,
(IV') when R^{1a}' is the aforementioned (4),
(IV'-1) R²' is a C₁₈₋₂₄ hydrocarbon group, R³' is a mono- or di-C₇₋₁₆ alkylamino group and W' is a bond,
(IV-2) R²' is a C₁₂₋₂₄ hydrocarbon group, R³' is a mono- or di-C₆₋₁₆ alkylamino group and W' is -O- or -NH-, or
(IV'-3) R²' is a C₅₋₂₄ hydrocarbon group, R³' is a mono- or di-C₆₋₁₆ alkylamino group and W' is -CO-;
Z¹' and Z²' are each -CO-;
n' is 0,
or a salt thereof.

18. A compound selected from the group consisting of
N-[(1S)-1-(dodecylcarbamoyl)-4-guanidino-butyl]hexadecanamide,
N-[(1S)-1-(dodecylcarbamoyl)-4-guanidino-butyl]octadecanamide,
N-[(1S)-1-(dodecylcarbamoyl)-4-guanidino-butyl]docosanamide,
N-[(1S)-3-methyl-1-(undecylcarbamoyl)butyl]docosanamide,
N-[(1S)-1-(dodecylcarbamoyl)-3-methylbutyl]hexadecanamide,
(2S)-N-dodecyl-5-guanidino-2-(hexadecylcarbamoylamino)pentanamide,
Hexadecyl N-[(1S)-1-(dodecylcarbamoyl)-4-guanidino-butyl]carbamate,
N-[(1S)-5-amino-1-(dodecylcarbamoyl)pentyl]hexadecanamide,
N-[(1S)-1-(dodecylcarbamoyl)pentyl]docosanamide,
N-[(1S)-1-(diheptylcarbamoyl)-4-guanidino-butyl]docosanamide,
N-[(1S)-5-amino-1-(dodecylcarbamoyl)pentyl]docosanamide,
(E)-N-[(1S)-1-(dodecylcarbamoyl)-4-guanidino-butyl]octadeca-9-enamide,
N-[(1S)-4-guanidino-1-(heptylcarbamoyl)butyl]docosanamide,
N-[(1S)-4-amino-1-(dodecylcarbamoyl)butyl]docosanamide,
N-[(1S)-1-(dodecylcarbamoyl)-5-guanidino-pentyl]docosanamide,
N-[(1S)-5-acetamide-1-(dodecylcarbamoyl)pentyl]docosanamide,
(2S)-5-guanidino-2-(hexadecylcarbamoylamino)-N-hexyl-pentanamide,
Hexadecyl N-[(1S)-4-guanidino-1-(hexylcarbamoyl)butyl]carbamate,
N-[(1S)-5-guanidino-1-(hexylcarbamoyl)pentyl]docosanamide,
N-[(1R)-1-[[[(2R)-2-(docosanoylamino)-3-(dodecylamino)-3-oxo-propyl]disulfanyl]methyl]-2-(dodecylamino)-2-oxoethyl]docosanamide,
N-[(1S)-1-(dodecylcarbamoyl)-4-guanidino-butyl]-2-oxo-octanamide,
N-[(1S)-1-(dodecylcarbamoyl)-4-guanidino-butyl]icosanamide,
N-[(1S)-4-guanidino-1-(octylcarbamoyl)butyl]icosanamide,
N-[(1S)-5-amino-1-(octylcarbamoyl)pentyl]octadecanamide,
N-[(1S)-5-amino-1-(dodecylcarbamoyl)pentyl]octadecanamide,
Octadecyl N-[(1S)-1-(dodecylcarbamoyl)-4-guanidino-butyl]carbamate,
Dodecyl N-[(1S)-1-(dodecylcarbamoyl)-4-guanidino-butyl]carbamate,
Hexadecyl N-[(1S)-5-amino-1-(octylcarbamoyl)pentyl] carbamate,
Hexadecyl N-[(1S)-5-amino-1-(dodecylcarbamoyl)pentyl]carbamate,
(2R)-N-dodecyl-5-guanidino-2-(hexadecylcarbamoylamino)pentanamide,
N-[(1R)-5-amino-1-(dodecylcarbamoyl)pentyl]hexadecanamide,
N-[(1S)-1-(dodecylcarbamoyl)-3-methylbutyl]octadecanamide,
N-[(1S)-3-methyl-1-(octylcarbamoyl)butyl]octadecanamide,
N-[(1S)-1-(dodecylcarbamoyl)-3-methyl-butyl]docosanamide,
N-[(1S)-3-methyl-1-(octylcarbamoyl)butyl]hexadecanamide,
(2S)-N-dodecyl-2-(hexadecylcarbamoylamino)-4-methylpentanamide,
(2S)-2-(hexadecylcarbamoylamino)-4-methyl-N-octyl-pentanamide,
Hexadecyl N-[(1S)-1-(dodecylcarbamoyl)-3-methylbutyl] carbamate,
Hexadecyl N-[(1S)-3-methyl-1-(octylcarbamoyl)butyl]carbamate,
(2S)-5-guanidino-2-(hexadecylcarbamoylamino)-N-octyl-pentanamide,
(2S)-N-dodecyl-5-guanidino-2-(octadecylcarbamoylamino)pentanamide,
(2S)-N-dodecyl-2-(dodecylcarbamoylamino)-5-guanidino-pentanamide,
(2S)-6-amino-2-(hexadecylcarbamoylamino)-N-octyl-hexanamide,
(2S)-6-amino-N-dodecyl-2-(hexadecylcarbamoylamino)hexanamide, and
Hexadecyl N-[(1S)-4-guanidino-1-(octylcarbamoyl)butyl]carbamate
or a salt thereof.

19. A compound selected from the group consisting of
N-[(1S)-1-(dodecylcarbamoyl)-4-guanidino-butyl]hexadecanamide,
N-[(1S)-1-(dodecylcarbamoyl)-4-guanidino-butyl]octadecanamide,
(E)-N-[(1S)-1-(dodecylcarbamoyl)-4-guanidino-butyl]octadeca-9-enamide,
N-[(1S)-4-guanidino-1-(octylcarbamoyl)butyl]hexadecanamide,
N-[(1S)-4-guanidino-1-(octylcarbamoyl)butyl]octadecanamide,
N-[(1R)-1-(dodecylcarbamoyl)-4-guanidino-butyl]octadecanamide,
N-[(1S)-3-methyl-1-(octylcarbamoyl)butyl]docosanamide,
N-[(1S)-1-(dodecylcarbamoyl)-5-guanidino-pentyl]octadecanamide,
N-[(1S)-1-(hexylcarbamoyl)-5-guanidino-pentyl]octadecanamide,
(2S)-2-(diheptylamino)-N-dodecyl-5-guanidino-pentanamide, and
N-[2-(4-pyridyl)ethyl]docosanamide
or a salt thereof.

20. A compound represented by the formula (I"): 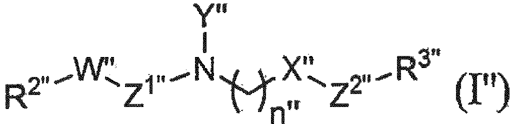 wherein
in R²", R³" and W",
(I"-1) R²" is a C₁₂₋₂₄ hydrocarbon group, R³" is a mono- or di-C₇₋₁₆ alkylamino group and W" is a bond,
(I"-2) R²" is a C₁₂₋₂₄ hydrocarbon group, R³" is a mono- or di-C₆₋₁₆ alkylamino group and W" is -O- or -NH-, or
(I"-3) R²" is a C₅₋₂₄ hydrocarbon group, R³" is a mono- or di-C₆₋₁₆ alkylamino group and W" is -CO-;
X" is a group represented by the following formula 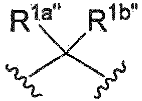 wherein
R^{1a}" is a hydrogen atom or a methyl group;
R^{1b}" is a hydrogen atom or a methyl group;
Y" is a hydrogen atom or a methyl group;
Z¹" and Z²" are each -CO-;
n" is an integer of 0 - 3;
when R^{1a}" is a methyl group, R^{1b}" is a hydrogen atom, Y" is a hydrogen atom and n" is 2, then R^{1a}" and Y" are optionally bonded to form a ring,
or a salt thereof.

21. A compound selected from the group consisting of
N-[(1S)-2-(dodecylamino)-1-methyl-2-oxoethyl]docosanamide,
N-[2-(dodecylamino)-1,1-dimethyl-2-oxo-ethyl]docosanamide,
N-[3-(dodecylamino)-3-oxo-propyl]docosanamide,
N-[3-(dodecylamino)-2,2-dimethyl-3-oxopropyl]docosanamide,
1-docosanoyl-N-dodecyl-piperidine-4-carboxamide,
N-[3-(dodecylamino)-3-oxo-pentyl]docosanamide, and
N-[3-(dodecylamino)-2,2-dimethyl-3-oxopropyl]octadecanamide
or a salt thereof.

22. A compound represented by the formula (I"'): 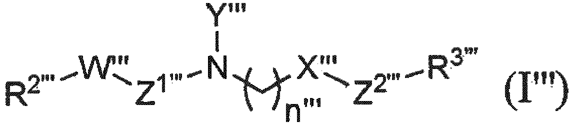 wherein
R³"' is -C(=NH)R⁴"' wherein R⁴"' is an amino group or a C₁₋₄ alkyl group;
in R²''' and W''',
(I'''-1) R²"' is a C₁₂₋₂₄ hydrocarbon group and W''' is a bond, -O-, or -NH-, or
(I'''-2) R²''' is a C₅₋₂₄ hydrocarbon group and W''' is -CO-;
in X''', Y''' and n''',
(II'''-1) X''' is -NH-, Y''' is a hydrogen atom, n''' is 4 or 5 (provided that a case in which W''' is a bond, n"' is 4 and R⁴''' is an amino group is excluded), or
(II'''-2) X''' is -NR⁵'''- wherein R⁵''' is a methyl group, Y''' is a methyl group, n''' is 2 and R⁵''' and Y''' are bonded to form a ring;
Z¹''' is -CO-; and
Z²''' is a bond,
or a salt thereof.

23. The compound or a salt thereof according to claim 22, wherein in X''', Y''' and n''',
(II''' -1') X''' is -NH-, Y''' is a hydrogen atom, n''' is 4 or 5 (provided that a case in which n''' is 4 and R⁴''' is an amino group is excluded), or
(II'''-2) X''' is -NR⁵'''- wherein R⁵''' is a methyl group, Y''' is a methyl group, n''' is 2 and R⁵''' and Y''' are bonded to form a ring.

24. A compound selected from the group consisting of
N-(5-guanidinopentyl)hexadecanamide,
N-(5-guanidinopentyl)docosanamide,
Hexadecyl N-(4-guanidinobutyl)carbamate,
N-[4-(ethanimidoylamino)butyl]hexadecanamide, and
1-(4-guanidinobutyl)-3-hexadecyl-urea
or a salt thereof.

25. (2S)-2-(dibenzylamino)-N-dodecyl-5-guanidino-pentanamide or a salt thereof.
